# EUROPEAN PATENT SPECIFICATION

(11) **EP 4 366 627 B1**
(45) Date of publication and mention of the grant of the patent: **04.11.2026**
(21) Application number: 22777403.1
(22) Date of filing: 26.08.2022
(51) Int. Cl.: A61B 17/04

(54) **HEART WALL REMODELING DEVICES AND METHODS**
VORRICHTUNGEN UND VERFAHREN ZUR REMODELLIERUNG DER HERZWAND
DISPOSITIFS ET PROCÉDÉS DE REMODELAGE DE PAROI CARDIAQUE

(30) Priority: 01.09.2021 US 202163239873 P; 03.09.2021 US 202163240712 P
(43) Date of publication of application: 15.05.2024
(73) Proprietor: Edwards Lifesciences Corporation, Irvine, CA 92614 (US)
(72) Inventor: AMEFIA, Kokou Anani, Aliso Viejo, CA 92656 (US); CATANIA, Pablo Hernan, Irvine, CA 92614 (US); TUASON, Arnold Cruz, Irvine, CA 92614 (US); KWON, Yoon Hee, Mission Viejo, CA 92691 (US); TRAN, Tiana, Irvine, CA 92614 (US); VALDEZ, Michael G., Irvine, CA 92614 (US); ALCASID, Anne Bernadette Aragon, Irvine, CA 92614 (US); CHANG, Arvin T., Irvine, CA 92614 (US); PARK, Steven, Irvine, CA 92614 (US); VALENCIA, Juan, Fullerton, CA 92833 (US)
(74) Representative: Venner, Julia Ann
(86) International application number: PCT/US2022/041717
(87) International publication number: WO 2023/034144

(56) References cited:
- WO-A2-2016/189391
- US-A1- 2014 236 127

## Description

### RELATED APPLICATIONS

The present application claims the benefit of U.S. Provisional Application No. 63/240,712, filed on September 3, 2021, titled "Heart Wall Remodeling Devices and Methods, and the benefit of U.S. Provisional Application No. 63/239,873 filed on September 1, 2021, titled "Heart Wall Remodeling Devices and Methods.

### BACKGROUND

Ischemic heart failure and systolic heart failure are conditions whereby the left ventricle becomes enlarged and dilated. With ischemic heart failure, a cardiac infarction occurs and the left ventricle remodels over a period of time, such as over days or months. With systolic heart failure, the left ventricle undergoes dilation for some other reason. For example, initial causes of heart systolic heart failure include chronic hypertension, mitral valve incompetency, and other dilated cardiomyopathies. A dilated heart, and particularly a dilated left ventricle, may significantly increase the tension and/or stress in the heart wall both during diastolic filling and systolic contraction, which contributes to ongoing dilatation of the left ventricular chamber.

Mitral valve incompetency or mitral valve regurgitation often accompanies ischemic and systolic heart failure. As the dilation of the ventricle proceeds, valve function may worsen. For example, as the dilation of the left ventricle progresses, the papillary muscles (to which the leaflets are connected via the chordae tendinea) may move radially outward and downward relative to the mitral valve, and relative to their normal positions. During this movement of the papillary muscles, however, the various chordae lengths remain substantially constant. This compromises the full closure ability of the leaflets by exerting tension prematurely on the leaflets. In addition, the enlargement of the left ventricle may cause the size of the mitral valve annulus to increase, while the area of the leaflets of the valve remains constant. This may lead to an area of less coaptation of the valve leaflets. Moreover, in normal hearts, the size of the mitral valve contracts during systole, aiding in valve coaptation. Right ventricular enlargement reduces annular contraction and distorts annular size, often exacerbating mitral valve regurgitation. The combination of the changes to the mitral valve annulus and the movement of the papillary muscles may result in a regurgitant mitral valve. This increase in regurgitation may, in turn, increase ventricular wall stress thereby advancing the dilation process, which may even further worsen mitral valve dysfunction.

WO 2016/189391 describes a tissue anchor including an anchor shaft; a tissue-coupling element, which (a) extends from a distal end of the anchor shaft, (b) comprises a wire, and (c) is shaped as an open shape when the tissue anchor is unconstrained by a deployment tool; and a flexible elongate tension member, which (a) extends from a distal site on the open shape, the distal site located within 7 mm of a distal end of the open shape, and (b) includes a proximal portion, which has a longitudinal segment that runs alongside at least a portion of the anchor shaft when the tissue anchor is unconstrained by the deployment tool. The tissue anchor is configured to allow relative axial motion between the at least a portion of the anchor shaft and the longitudinal segment of the proximal portion of the flexible elongate tension member when the tissue anchor is unconstrained by the deployment tool.

### SUMMARY

Aspects of the presently claimed invention are set out in the independent claims. Particular embodiments of these aspects are set out in the dependent claims. Any subject matter contained herein that does not fall within the scope of the appended claims is considered as being useful for understanding the invention.

This summary is meant to provide some examples and is not intended to be limiting of the scope of the invention in any way. For example, any feature included in an example of this summary is not required by the claims, unless the claims explicitly recite the feature. Also, the features, components, steps, concepts, etc. described in examples in this summary and elsewhere in this disclosure can be combined in a variety of ways. Various features and steps as described elsewhere in this disclosure can be included in the examples summarized here.

Several heart wall remodeling devices and methods are disclosed. In one example, a device for remodeling the shape of one or more walls of a human heart includes an anchoring portion having an elongated anchor body with a distal anchor end portion and a proximal anchor end portion opposite the distal anchor end portion, a joint portion having a distal joint end portion fixedly attached to the proximal anchor end portion and a proximal joint end portion opposite the distal joint end portion, and a hemostasis element having a distal hemostasis end portion detachably connected to the proximal joint end portion. In some examples, the device is configured to receive an elongating device through an inner passage in the device, wherein when the elongating device is received in the inner passage through the anchoring portion, the anchoring portion is held in an elongated delivered state, and when the elongating device is removed from the inner passage of the anchoring portion, the anchoring portion forms a curved deployed state.

In some implementations useful for understanding the presently claimed invention, a device for remodeling the shape of one or more walls of a human heart is provided. The device comprises a first anchor configured to engage an endocardium of a first heart wall at least partially defining an internal chamber of the heart; a first tether connected to the first anchor; a second anchor configured to engage an endocardium of a second heart wall at least partially defining the internal chamber of the heart; a second tether connected to the second anchor; and, a tether lock connected to the first line and the second line, wherein the tether lock is configured to create a variable tension between the first tether and the second tether.

In some implementations useful for understanding the presently claimed invention, a device for remodeling the shape of one or more walls of a human heart is provided. The device comprises a first anchor configured to engage an endocardium of a first heart wall at least partially defining an internal chamber of the heart; a first tether connected to the first anchor; a second anchor configured to engage an endocardium of a second heart wall at least partially defining the internal chamber of the heart; a second tether connected to the second anchor; and, a tether lock connected to the first line and the second line, wherein the tether lock is configured to create a variable tension between the first tether and the second tether, wherein the tension increases over time based on degradation of a bio-absorbable material.

In some implementations useful for understanding the presently claimed invention, a device for remodeling the shape of one or more walls of a human heart is provided. The device comprises a first anchor configured to engage an endocardium of a first heart wall at least partially defining an internal chamber of the heart; a first tether connected to the first anchor; a second anchor configured to engage an endocardium of a second heart wall at least partially defining the internal chamber of the heart; a second tether connected to the second anchor; and, a tether lock connected to the first line and the second line, wherein the tether lock is configured to create a variable tension between the first tether and the second tether, wherein the tension increases over time based on degradation of a bio-absorbable material.

In some implementations useful for understanding the presently claimed invention, a device for remodeling the shape of one or more walls of a human heart is provided. The device comprises a first anchor configured to engage an endocardium of a first heart wall at least partially defining an internal chamber of the heart; a first tether connected to the first anchor; and, a variable tension module comprising a spring connected to the first anchor and the first tether, wherein the spring is held in tension by a bio-absorbable material configured to degrade over time and increase tension at the first tether.

A further understanding of the nature and advantages of the present invention are set forth in the following description and claims, particularly when considered in conjunction with the accompanying drawings in which like parts bear like reference numerals.

### BRIEF DESCRIPTION OF THE DRAWINGS

To further clarify various aspects of implementations of the present disclosure, a more particular description of the certain examples and implementations will be made by reference to various aspects of the appended drawings. These drawings depict only example implementations of the present disclosure and are therefore not to be considered limiting of the scope of the disclosure. Moreover, while the FIGS. can be drawn to scale for some examples, the FIGS. are not necessarily drawn to scale for all examples. Examples and other features and advantages of the present disclosure will be described and explained with additional specificity and detail through the use of the accompanying drawings in which:
Figure 1 illustrates a cutaway view of the human heart in a diastolic phase;
Figure 2 illustrates a cutaway view of the human heart in a systolic phase;
Figure 3 illustrates a cutaway view of the human heart in a diastolic phase, in which the chordae tendineae are shown attaching the leaflets of the mitral and tricuspid valves to ventricle walls;
Figure 4 illustrates a healthy mitral valve with the leaflets closed as viewed from an atrial side of the mitral valve;
Figure 5 illustrates a dysfunctional mitral valve with a visible gap between the leaflets as viewed from an atrial side of the mitral valve;
Figure 6 illustrates a cutaway view of the human heart showing the papillary muscles;
Figure 7 illustrates a cutaway view of the human heart showing the multilayer heart wall;
Figure 8 is an enlarged cutaway view of the human heart wall;
Figure 9 is an enlarged cutaway view of the human heart wall of Figure 8 showing a needle about to pierce the heart wall;
Figure 10 is an enlarged cutaway view of the human heart wall of Figure 8 showing a needle inserted into the myocardium of the heart wall;
Figure 11 is an enlarged cutaway view of the human heart wall of Figure 8 showing a needle inserted into the parietal tissue of the pericardium of the heart wall;
Figure 12 is an enlarged cutaway view of the human heart wall of Figure 8 showing a needle inserted into the pericardial cavity of the heart wall;
Figure 13 is an enlarged cutaway view of the human heart wall of Figure 8 showing a needle injecting a dye into the pericardial cavity of the heart wall;
Figure 14 is an enlarged cutaway view of the human heart wall of Figure 8 showing a needle inserted into the pericardial cavity of the heart wall and a catheter adjacent the endocardium of the heart wall;
Figure 15 is an enlarged cutaway view of the human heart wall of Figure 8 showing a needle and a catheter inserted into the pericardial cavity of the heart wall;
Figure 16 is an enlarged cutaway view of the human heart wall of Figure 8 showing an anchor deployed into the pericardial cavity of the heart wall;
Figure 16A is a view similar to the view of Figure 16 where the anchor is deployed through an introducer or needle;
Figures 16B and 16C are views similar to Figure 16 where the anchor is deployed over an introducer or needle;
Figure 17 is an enlarged cutaway view of the human heart wall of Figure 8 showing the anchor of Figure 16 seated in the pericardial cavity of the heart wall;
Figure 18 is an enlarged cutaway view of the human heart wall of Figure 8 showing a screw catheter adjacent the endocardium of the heart wall;
Figure 19 is an enlarged cutaway view of the human heart wall of Figure 8 showing a screw catheter anchored into the myocardium of the heart wall;
Figure 20 is an enlarged cutaway view of the human heart wall of Figure 19 showing a needle delivered through the screw catheter;
Figure 21 is an enlarged cutaway view of the human heart wall of Figure 19 showing a needle inserted into the myocardium of the heart wall;
Figure 22 is an enlarged cutaway view of the human heart wall of Figure 19 showing a needle inserted into the parietal tissue of the heart wall;
Figure 23 is an enlarged cutaway view of the human heart wall of Figure 19 showing a needle inserted into the pericardial cavity of the heart wall;
Figure 24 is an enlarged cutaway view of the human heart wall of Figure 19 showing a needle injecting a dye into the pericardial cavity of the heart wall;
Figure 25 is an enlarged cutaway view of the human heart wall of Figure 19 showing a needle inserted into the pericardial cavity of the heart wall and a secondary catheter adjacent the endocardium of the heart wall;
Figure 26 is an enlarged cutaway view of the human heart wall of Figure 19 a needle and a secondary catheter inserted into the pericardial cavity of the heart wall;
Figure 26A is an enlarged cutaway view of the human heart wall showing a needle and a secondary catheter inserted into the pericardial cavity of the heart wall;
Figure 27 is an enlarged cutaway view of the human heart wall of Figure 19 showing an anchor deployed into the pericardial cavity of the heart wall;
Figure 27A is an enlarged cutaway view of the human heart wall showing an anchor deployed into the pericardial cavity of the heart wall;
Figure 28 is an enlarged cutaway view of the human heart wall of Figure 19 showing the anchor of Figure 26 seated in the pericardial cavity of the heart wall;
Figure 28A is an enlarged cutaway view of the human heart wall showing an anchor seated in the pericardial cavity of the heart wall
Figure 29 is an enlarged cutaway view of a human heart wall showing a needle about to pierce the heart wall;
Figure 30 is an enlarged cutaway view of the human heart wall of Figure 29 showing the needle extending through the heart wall;
Figure 31 is an enlarged cutaway view of the human heart wall of Figure 29 showing the needle extending through the heart wall and a catheter adjacent the endocardium of the heart wall;
Figure 32 is an enlarged cutaway view of the human heart wall of Figure 29 showing the needle and a catheter extending through the heart wall;
Figure 33 is an enlarged cutaway view of the human heart wall of Figure 32 showing an anchor deployed through the catheter;
Figure 34 is an enlarged cutaway view of the human heart wall of Figure 29 showing the anchor seated against the parietal tissue of the heart wall;
Figure 35 is an enlarged cutaway view of the human heart wall showing a screw catheter anchored into the myocardium of the heart wall;
Figure 36 is an enlarged cutaway view of the human heart wall of Figure 35 showing a needle delivered through the screw catheter;
Figure 37 is an enlarged cutaway view of the human heart wall of Figure 35 showing a needle inserted through the heart wall;
Figure 38 is an enlarged cutaway view of the human heart wall of Figure 35 showing a needle inserted through the heart wall and a secondary catheter adjacent the endocardium of the heart wall;
Figure 39 is an enlarged cutaway view of the human heart wall of Figure 35 showing a needle and a secondary catheter inserted through the heart wall;
Figure 40 is an enlarged cutaway view of the human heart wall of Figure 35 showing an anchor deployed through the secondary catheter;
Figure 41 is an enlarged cutaway view of the human heart wall of Figure 35 showing the anchor of Figure 40 seated against the parietal tissue of the heart wall;
Figure 42 illustrates a cutaway view of the human heart showing a needle inserted through a papillary muscle of the heart and through the heart wall;
Figure 43 illustrates a cutaway view of the human heart of Figure 42 showing the needle inserted through the papillary muscle and a delivery catheter positioned adjacent the papillary muscle;
Figure 44 is an enlarged cutaway view of the human heart wall showing a needle and a catheter inserted through a papillary muscle of the heart and into the pericardial cavity of the heart wall;
Figure 45 is an enlarged cutaway view of the human heart wall of Figure 44 showing an anchor deployed into the pericardial cavity of the heart wall;
Figure 46 is an enlarged cutaway view of the human heart wall of Figure 44 showing an anchor seated in the pericardial cavity of the heart wall;
Figure 47 illustrates a cutaway view of the human heart showing an anchor seated against the exterior of the heart wall with a line extending through a papillary muscle;
Figure 48 illustrates a cutaway view of the human heart showing a first anchor seated against the exterior of the heart wall with a line extending through a first papillary muscle and a second anchor seated against the exterior of the heart wall with a second line extending through a second papillary muscle;
Figure 49A illustrates the cutaway view of the human heart of Figure 48 showing the first line and a second line being pulled through a connector to pull the papillary muscles toward one another;
Figure 49B shows the first and second lines secured in the connector and trimmed;
Figure 50 illustrates the cutaway view of the human heart of Figure 49B showing a third anchor seated against the intraventricular septum and a third line coupled to the first and second lines;
Figure 51 illustrates a cutaway view of the human heart showing a first anchor seated against the exterior of the heart wall with a first line extending through a papillary muscle and a second anchor seated against the intraventricular septum and a second line connected to the first line;
Figure 52 illustrates a cutaway view of the human heart showing a first anchor seated against the exterior of the heart wall with a first line extending through the heart wall and a second anchor seated against the intraventricular septum and a second line connected to the first line;
Figures 53 and 54 are exploded views of a line clamp according to an example;
Figures 53A and 54A are exploded views of a line clamp according to an example;
Figures 55 and 56 are cross-section views of a line clamp according to an example;
Figures 55A and 56A are cross-section views of a line clamp according to an example;
Figures 57A-57E are illustrations of anchors, lines, and a line clamp located between tissue walls according to an example;
Figures 57F-57J are illustrations of anchors, lines, and a line clamp located between tissue walls according to an example;
Figure 58 is a perspective view of the operating end of a line clamp installation tool according to an example;
Figure 58A is a perspective view of the operating end of a line clamp installation tool according to an example;
Figure 59 is a cross-section view of the operating end of a line clamp installation tool according to an example;
Figure 59A is a cross-section view of the operating end of a line clamp installation tool according to an example;
Figure 60 is a perspective view of a line clamp according to an example;
Figure 60A is a perspective view of a line clamp according to an example;
Figure 61 is a perspective view of an engagement device according to an example;
Figure 61A is a perspective view of an engagement device according to an example;
Figure 62 is a cross-section view of an engagement device secured to a component of a line clamp according to an example;
Figure 62A illustrates a cross-section view of an engagement device secured to a component of a line clamp according to an example;
Figure 63 is an exploded view of Figure 62;
Figure 63A is an exploded view of the components illustrated by Figure 62A;
Figures 63B-63G are schematic illustrations of an engagement device being used to place an insert in a body of a locking device according to an example;
Figure 64 illustrates a socket positioned adjacent to the operating end of a line clamp installation tool according to an example;
Figure 64A illustrates a socket positioned adjacent to the operating end of a line clamp installation tool according to an example;
Figure 65 is a perspective cross-section view of a line clamp installation tool according to an example;
Figure 66 is a perspective cross-section view of the operating handle portion of a line clamp installation tool according to an example;
Figure 67 is a cross-section view of a portion of the operating handle portion of a line clamp installation tool according to an example;
Figure 68 is a cross-section illustration of the operating end and operating handle portion of a line clamp installation tool according to an example;
Figure 69 is an enlarged perspective cross-section view of the operating handle portion of a line clamp installation tool according to an example;
Figures 70A-70G are illustrations of the line clamp being installed by a line clamp installation tool according to an example;
Figures 71A-71E are illustrations of a line trimming tool in use according to an example;
Figures 72A-72B illustrate an alternative example of a line trimming tool according to an example;
Figures 73A-73B are views of the components of the line trimming tool of Figures 72A-72B;
Figures 74A-74C are illustrations of a line clamp according to an example;
Figures 75A-75B are illustrations of a line clamp according to an example;
Figures 76A-76B are illustrations of a line clamp according to an example;
Figures 77A-77B are illustrations of a line clamp according to an example;
Figures 78A-78B are illustrations of a line clamp according to an example;
Figures 79A-79B are illustrations of a line clamp according to an example;
Figures 80A-80B are illustrations of a line clamp according to an example;
Figures 81A-81B are illustrations of a line clamp according to an example;
Figure 82 is an illustration of a line clamp according to an example;
Figure 83 is an illustration of a line clamp according to an example;
Figure 84 is an illustration of a line clamp according to an example;
Figures 85A-85B are illustrations of a line clamp according to an example;
Figure 86 is an illustration of a line clamp according to an example;
Figures 87A-87B are illustrations of a line clamp according to an example;
Figures 88A-88B are illustrations of a line clamp according to an example;
Figures 89A-89B are illustrations of a line clamp according to an example;
Figures 90A-90B are illustrations of a line clamp according to an example;
Figures 91A-91B are illustrations of a line clamp according to an example;
Figure 92 is an illustration of a line clamp according to an example;
Figures 93A-93B are illustrations of a line clamp according to an example.
Figure 94 illustrates an example of an anchor for a papillary muscle approximation system, the anchor illustrated in an extended configuration;
Figure 95 illustrates the anchor of Figure 94 in a non-deployed, extended configuration;
Figure 95A illustrates another example of an anchor in a substantially non-deployed configuration;
Figure 95B is a plan view of a piece of material that has been cut in a pattern for use in a connector of the anchor illustrated by Figure 95A;
Figure 95C is a side view of the material illustrated by Figure 95B;
Figure 95D is a plan view of a connector made by folding the material illustrated by Figure 95B;
Figure 95E is a side view of the connector illustrated by Figure 95D;
Figure 95F is a plan view of a portion of the connector illustrated by Figure 95D attached to a portion of an anchor;
Figure 95G is a side view of the connector and anchor illustrated by Figure 95F;
Figure 95H is a plan view of another example of a piece of material that has been cut in a pattern for use in a connector of the anchor illustrated by Figure 95A;
Figure 95I is illustrates an example of an anchor that is similar to the example illustrated by Figure 95A where high strength fibers are oriented to increase pull strength;
Figure 96 illustrates the anchor of Figure 94 in a deployed configuration;
Figure 96A illustrates the anchor of Figure 95A in a deployed configuration;
Figures 96B and 96C illustrates examples of anchors that are similar to the anchor illustrated by Figure 96A where high strength fibers are oriented to increase strength of the anchor;
Figure 96D illustrates an example of a hybrid cloth;
Figure 96E illustrates an example of a hybrid cloth;
Figure 97 illustrates the anchor of Figure 94 in a non-deployed, extended configuration along with a hemostatic plug;
Figure 98 illustrates the anchor of Figure 94 in a deployed configuration along with a hemostatic plug;
Figure 99 illustrates a delivery sheath and a steerable catheter for delivering the anchor of Figure 94;
Figure 100 illustrates an anchoring delivery catheter extending from the delivery sheath and the steerable catheter of Figure 99;
Figure 101 illustrates a needle extending from the anchoring delivery catheter of Figure 100;
Figure 102 illustrates the anchor extending along the needle of Figure 101;
Figure 103 illustrates the anchor and hemostatic plug along with the needle and a pusher of the delivery system of Figure 98;
Figure 104 illustrates the anchor and hemostatic plug of Figure 103;
Figure 105 illustrates the anchor and hemostatic plug of Figure 103 with lines attached;
Figure 106 illustrates the anchor with lines attached without a hemostatic plug;
Figure 107 illustrates the delivery system deploying the anchor and hemostatic plug without lines;
Figure 108 illustrates the delivery system, anchor, and hemostatic plug of Figure 107 with lines;
Figure 109 illustrates the delivery system, anchor, and hemostatic plug of Figure 108 with the anchor in a deployed state;
Figure 110 illustrates an enlarged view of the anchor and hemostatic plug in a deployed state;
Figure 110A illustrates an enlarged view of the anchor of Figure 95A and a hemostatic plug in a deployed state;
Figure 111 illustrates the anchor and hemostatic plug in a deployed state with the needle and delivery catheter of the delivery system withdrawn;
Figure 112 is an enlarged cutaway view of the human heart wall showing the delivery sheath and steerable catheter positioned adjacent the heart wall;
Figure 112A illustrates a delivery catheter, a piston, and an anchoring device for delivering an anchor;
Figure 112B is a cross sectional view of the delivery catheter, piston, and anchoring device of Figure 112A;
Figure 112C is a cross sectional view of the delivery catheter of Figure 112B;
Figure 112D is a cross sectional view of the delivery catheter of Figure 112C taken along the plane indicted by lines 133-133 in Figure 112C;
Figure 112E is a cross sectional view of the piston of Figure 112B;
Figure 112F is a cross sectional view of the piston of Figure 112E taken along the plane indicted by lines 135-135 in Figure 112E;
Figure 112G is a cross sectional view of the delivery catheter and piston of Figure 112B;
Figure 113 is an enlarged cutaway view of the human heart wall showing the delivery catheter anchored to the myocardium of the heart wall;
Figure 113A is a cross sectional view of the piston of Figure 112B, illustrating a clutch mechanism;
Figure 113B is a cross sectional view of the piston of Figure 113A taken along the plane indicated by lines 138-138 in Figure 113A;
Figures 113C-113E are views similar to Figure 113B, illustrating increasing torque applied between the piston and anchor;
Figure 113F and 113G are cross sectional views of an alternate clutch arrangement between the piston and the clutch;
Figure 113H is an enlarged cutaway view of a human heart wall showing the delivery catheter, piston, and anchoring device positioned adjacent the heart wall;
Figure 113I is an enlarged cutaway view of the human heart wall showing the delivery catheter, piston, and anchoring device positioned adjacent the heart wall with the piston compressed;
Figure 113J is an enlarged cutaway view of the human heart wall showing the anchoring device partially anchored to the myocardium of the heart wall;
Figure 113K is a cross sectional view showing the position of the clutch of the piston of Figure 113J;
Figure 113L is an enlarged cutaway view of the human heart wall showing the anchoring device anchored to the myocardium of the heart wall;
Figures 113M and 113N are cross sectional views showing the movement of the clutch of the piston when the anchor reaches the position illustrated by Figure 113L;
Figure 114 is an enlarged cutaway view of the human heart wall showing the needle inserted into the pericardial cavity of the heart wall;
Figure 114A is an enlarged cutaway view of the human heart wall showing a needle inserted into the pericardial cavity of the heart wall;
Figure 115 is an enlarged cutaway view of the human heart wall showing a needle injecting a dye into the pericardial cavity of the heart wall;
Figure 115A is an enlarged cutaway view of the human heart wall showing the needle injecting a dye into the pericardial cavity of the heart wall;
Figure 115B is an enlarged cutaway view of the human heart wall showing a needle injecting a wire into the pericardial cavity of the heart wall;
Figure 116 is an enlarged cutaway view of the human heart wall showing the anchor being deployed along the needle;
Figure 116A is an enlarged cutaway view of the human heart wall showing the anchor being deployed along the needle;
Figure 117 is an enlarged cutaway view of the human heart wall showing the anchor extending inside the pericardial cavity;
Figure 117A is an enlarged cutaway view of the human heart wall showing the anchor extending inside the pericardial cavity;
Figure 118 is an enlarged cutaway view of the human heart wall showing the anchor deployed inside the pericardial cavity;
Figure 118A is an enlarged cutaway view of the human heart wall showing the anchor deployed inside the pericardial cavity;
Figure 119 is an enlarged cutaway view of the human heart wall showing the anchor being seated in the pericardial cavity;
Figure 119A is an enlarged cutaway view of the human heart wall showing the anchor seated in the pericardial cavity;
Figure 119B is an enlarged cutaway view of the human heart wall showing the anchor seated in the pericardial cavity;
Figure 119C is an enlarged cutaway view of the human heart wall showing the anchoring device being detached from the myocardium of the heart wall;
Figure 119D is a cross sectional view of the piston of Figure 119C, illustrating the position of the clutch as the anchor is removed from the heart wall;
Figure 120 is an enlarged cutaway view of the human heart wall showing the anchor seated in the pericardial cavity and the delivery system removed;
Figure 120A is an enlarged cutaway view of the human heart wall showing the anchor seated in the pericardial cavity and the delivery system removed;
Figure 121 is an enlarged cutaway view of the human heart wall showing the delivery sheath and steerable catheter positioned adjacent a papillary muscle of the heart;
Figure 121A illustrates a cutaway view of the human heart in a diastolic phase, in which a steerable catheter and delivery catheter are positioned adjacent a papillary muscle of the heart;
Figure 122 is an enlarged cutaway view of the human heart wall showing the delivery catheter anchored to the papillary muscle of the heart;
Figure 122A illustrates a cutaway view of the human heart in a diastolic phase, in which a steerable catheter and delivery catheter are positioned adjacent a papillary muscle of the heart
Figure 123 is an enlarged cutaway view of the human heart wall showing the needle inserted through the papillary muscle and into the pericardial cavity of the heart wall;
Figure 124 is an enlarged cutaway view of the human heart wall showing a needle injecting a dye into the pericardial cavity of the heart wall;
Figure 125 is an enlarged cutaway view of the human heart wall showing the anchor being deployed along the needle;
Figure 126 is an enlarged cutaway view of the human heart wall showing the anchor extending inside the pericardial cavity;
Figure 127 is an enlarged cutaway view of the human heart wall showing the anchor deployed inside the pericardial cavity;
Figure 128 is an enlarged cutaway view of the human heart wall showing the anchor being seated in the pericardial cavity;
Figure 129 is an enlarged cutaway view of the human heart wall showing the anchor seated in the pericardial cavity and the delivery system removed.
Figure 130 illustrates an example of a device for a papillary muscle approximation system, the anchor illustrated in an elongated delivered state;
Figure 131 illustrates the device of Figure 130 with a piercing device received through an inner passage of the device;
Figure 132 illustrates a top perspective view of an attachment portion of the device;
Figure 133 illustrates a side perspective view of the attachment portion of Figure 132;
Figure 134 illustrates a partial view of the device with the piercing device extending through a section of an anchoring portion of the device;
Figure 135 illustrates a perspective view of the device of Figure 134;
Figure 136 illustrates a partial view of the device with the piercing device extending through a section of the joint portion of the device;
Figure 137 illustrates a perspective view of the device of Figure 136;
Figure 138 is an enlarged cutaway view of the human heart wall showing the device positioned within the pericardial cavity;
Figure 139 illustrates a partial view of the device with the attachment portion connected;
Figure 140 illustrates a partial view of the device with the attachment portion disconnected;
Figure 141 illustrates a perspective view of the device with a hemostasis portion of the device disconnected;
Figure 142 is an enlarged cutaway view of the human heart wall showing the device of Figure 141 positioned within the pericardial cavity;
Figures 143A-143B illustrate deployment of an example anchor and an attached tether;
Figures 144A-144D illustrate use of an example of a tissue remodeling system;
Figure 145A illustrates a cutaway view of the human heart showing a first anchor attached to the intraventricular septum and a second anchor attached to a ventricular wall with lines connecting the first and second anchors;
Figure 145B illustrates a cutaway view of the human heart showing a first anchor attached to the intraventricular septum, a second anchor attached to a ventricular wall, and a third anchor attached to a ventricular wall with lines connecting the first, second, and third anchors;
Figure 145C illustrates a cutaway view of the human heart showing a first anchor attached to the intraventricular septum, a second anchor attached to a ventricular wall, a third anchor attached to a ventricular wall, and a fourth anchor attached to a ventricular wall with lines connecting the first, second, third and fourth anchors;
Figure 145D illustrates a cutaway view of the human heart showing a first anchor attached to a papillary muscle and a second anchor attached to a papillary muscle with lines connecting the first and second anchors;
Figure 145E illustrates a cutaway view of the human heart showing a first anchor attached to an intraventricular septum, a second anchor attached to a papillary muscle, and a third anchor attached to a papillary muscle with lines connecting the first, second, and third anchors;
Figures 146A-146B illustrate an example tether tensioner connecting tethers from first and second anchors;
Figure 147 illustrates an example tether tensioner being modified outside the body;
Figures 148A-148C illustrate another example tether tensioner;
Figures 149A-149C illustrate yet another example tether tensioner;
Figure 150 illustrates an example bio-absorbable material used in connection with a tether tensioner;
Figure 151 illustrates an example tether tensioner after a bio-absorbable material has completely degraded;
Figure 152 illustrates and example tether tensioner as deployed into tissue;
Figures 153A-153B illustrate yet another example tether tensioner with bio-absorbable material;
Figure 154 illustrates yet another example tether tensioner;
Figure 155 illustrates an example release member used in connection with tether tensioners as disclosed herein;
Figure 156 illustrates another example tether tensioner;
Figure 157 illustrates yet another example tether tensioner;
Figure 158 illustrates yet another example tether tensioner;
Figure 159 illustrates a side view of a plug nut used in the example illustrated by Figure 158;
Figure 160 illustrates an end view of the plug nut of Figure 159;
Figures 161A-161C illustrate an example magnetic tether tensioner;
Figures 162A-162C illustrate stepper motor theory;
Figures 163A-163B illustrate an example magnetic cinching tether tensioner connecting a first and second anchor;
Figures 164A-164D illustrate an example ratcheting anchor device;
Figures 165A-165B illustrates an example ratcheting anchor device deployed in heart tissue;
Figures 166A-166C illustrate an example bellows based tether tensioner; and
Figure 167 illustrates an example bellows based tissue remodeling system.

### DETAILED DESCRIPTION

The following description refers to the accompanying drawings, which illustrate example implementations of the present disclosure. Other implementations having different structures and operation do not depart from the scope of the present disclosure.

Example implementations of the present disclosure are directed to systems, devices, methods, etc. for repairing a defective heart valve. For example, various implementations of valve repair devices, implantable devices, implants, and systems (including systems for delivery thereof) are disclosed herein, and any combination of these options can be made unless specifically excluded. In other words, individual components of the disclosed devices and systems can be combined unless mutually exclusive or otherwise physically impossible. Further, the techniques and methods disclosed herein can be performed on a living animal. The methods of the presently claimed invention are performed on a simulation, such as on a cadaver, cadaver heart, simulator (e.g., with the body parts, heart, tissue, etc. being simulated), etc.

As described herein, when one or more components are described as being connected, joined, affixed, coupled, attached, or otherwise interconnected, such interconnection can be direct as between the components or can be indirect such as through the use of one or more intermediary components. Also as described herein, reference to a "member," "component," or "portion" shall not be limited to a single structural member, component, or element but can include an assembly of components, members, or elements. Also as described herein, the terms "substantially" and "about" are defined as at least close to (and includes) a given value or state (preferably within 10% of, more preferably within 1% of, and most preferably within 0.1% of).

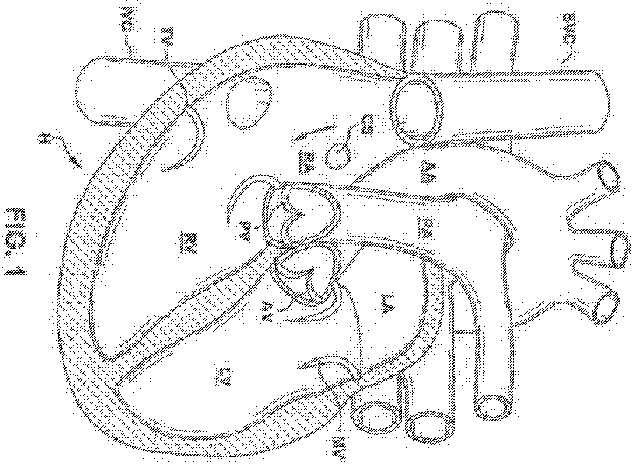

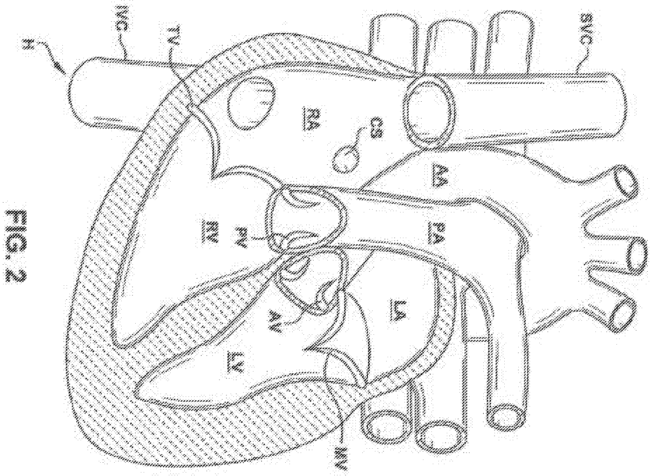

Figures 1 and 2 are cutaway views of the human heart H in diastolic and systolic phases, respectively. The right ventricle RV and left ventricle LV are separated from the right atrium RA and left atrium LA, respectively, by the tricuspid valve TV and mitral valve MV; i.e., the atrioventricular valves. Additionally, the aortic valve AV separates the left ventricle LV from the ascending aorta AA, and the pulmonary valve PV separates the right ventricle from the pulmonary artery PA. Each of these valves has flexible leaflets extending inward across the respective orifices that come together or "coapt" in the flow stream to form the one-way, fluid-occluding surfaces. The remodeling devices, systems, and methods of the present application are described primarily with respect to the left ventricle LV. Therefore, anatomical structures of the left side of the heart will be explained in greater detail. It should be understood that the devices, systems, and methods described herein can also be used in remodeling the right ventricle.

The left atrium LA receives oxygenated blood from the lungs. During the diastolic phase, or diastole, seen in Figure 1, the blood that was previously collected in the left atrium LA (during the systolic phase) moves through the mitral valve MV and into the left ventricle LV by expansion of the left ventricle LV. In the systolic phase, or systole, seen in Figure 2, the left ventricle LV contracts to force the blood through the aortic valve AV and ascending aorta AA into the body. During systole, the leaflets of the mitral valve MV close to prevent the blood from regurgitating from the left ventricle LV and back into the left atrium LA, and blood is collected in the left atrium from the pulmonary vein.

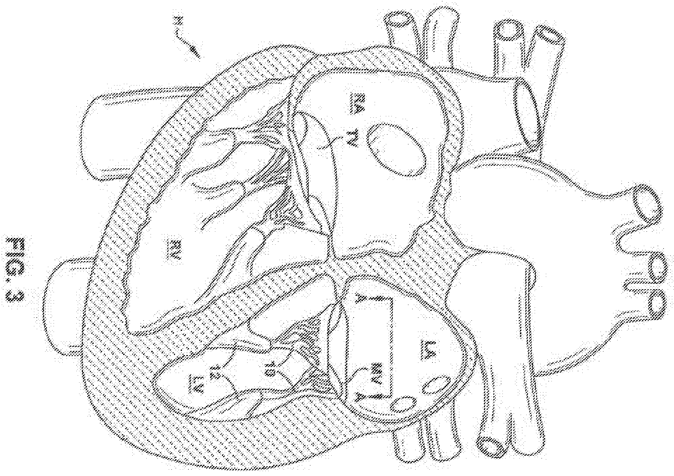

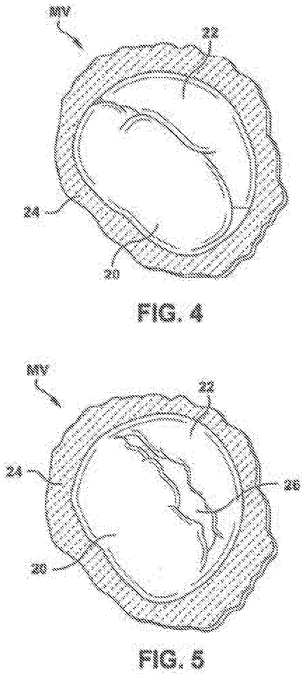

Referring now to Figures 1-5, the mitral valve MV includes two leaflets, the anterior leaflet 20 and the posterior leaflet 22. The mitral valve MV also includes an annulus 24, which is a variably dense fibrous ring of tissues that encircles the leaflets 20, 22. Referring to Figure 3, the mitral valve MV is anchored to the wall of the left ventricle LV by chordae tendineae 10. The chordae tendineae 10 are cord-like tendons that connect the papillary muscles 12 (i.e., the muscles located at the base of the chordae tendineae and within the walls of the left ventricle) to the leaflets 20, 22 of the mitral valve MV. The papillary muscles 12 serve to limit the movements of the mitral valve MV and prevent the mitral valve from being inverted or prolapsed. The mitral valve MV opens and closes in response to pressure changes in the left atrium LA and the left ventricle LV. The papillary muscles do not open or close the mitral valve MV. Rather, the papillary muscles brace the mitral valve MV against the high pressure needed to circulate blood throughout the body. Together the papillary muscles and the chordae tendineae are known as the subvalvular apparatus, which functions to keep the mitral valve MV from prolapsing into the left atrium LA when the mitral valve closes.

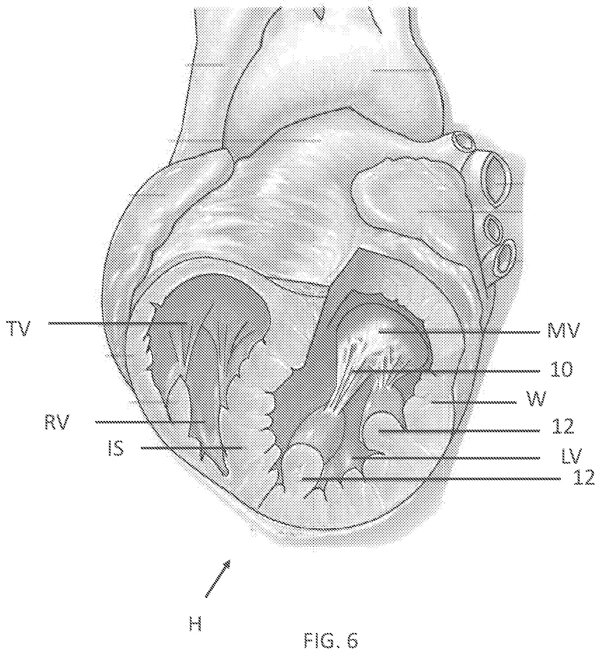

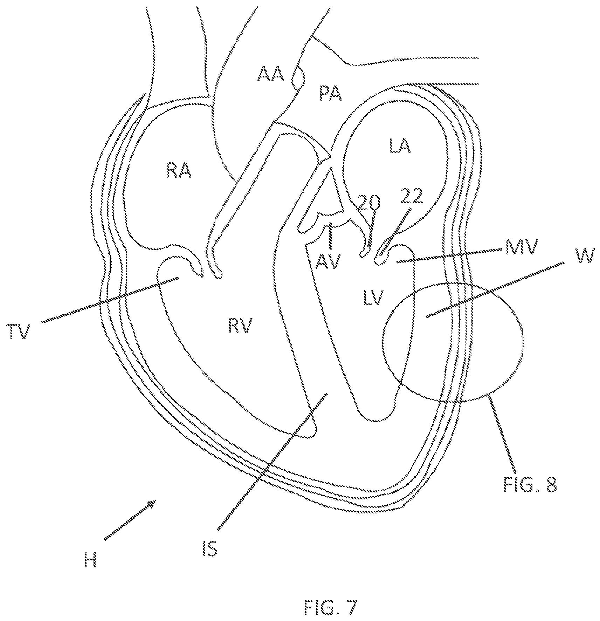

Figures 6 is a cutaway view of the human heart with the section through the papillary muscles of the left ventricle. The right ventricle RV is separated from the left ventricle LV by the interventricular septum IS. The mitral valve leaflets 20, 22 shown Figure 7) extending inward across the respective orifices that come together or "coapt" in the flowstream to form the one-way, fluid-occluding surfaces. The devices and methods for remodeling the shape of the heart walls W are described primarily with respect to the left ventricle LV. The devices and methods can be used to approximate the papillary muscles in some examples, which are also described primarily with respect to the left ventricle LV. In addition to reducing the size of the ventricle to increase the ventricular function, bringing the papillary muscles closer together can cause the valve leaflets to coapt and inhibit or prevent mitral valve regurgitation. It should be understood that the devices described herein can also be used in remodeling the right ventricle RV and approximate the papillary muscles of the tricuspid valve TV.

**In** one example, the devices described by the present application are used to remodel the shape of a ventricle to improve heart function. Heart function can be improved by reducing the size of the ventricle, approximating the papillary muscles, and/or correcting the function of the mitral valve MV. **In** one example, the devices are configured to reshape the wall of a human heart H in a way that causes the mitral valve MV to inhibit or prevent blood from regurgitating from the left ventricle LV and back into the left atrium LA.

When a healthy mitral valve MV is in a closed position, the leaflets 20, 22 coapt, which prevents blood from leaking from the left ventricle LV to the left atrium LA. Regurgitation can occur when one or both of the leaflets 20, 22 of the mitral valve MV prolapse into the left atrium LA during systole or the leaflets fail to coapt or close together against one another. This prolapse and/or a failure to coapt causes a gap between the leaflets 20, 22, which allows blood to flow back into the left atrium LA from the left ventricle LV during systole.

The devices and procedures disclosed herein make reference to remodeling the left ventricle, with the possible consequence of better coaption of the leaflets of the mitral valve. However, it should be understood that the devices and concepts provided herein can be used to remodel the right ventricle, with the possible consequence of better coaption of the tricuspid valve TV leaflets.

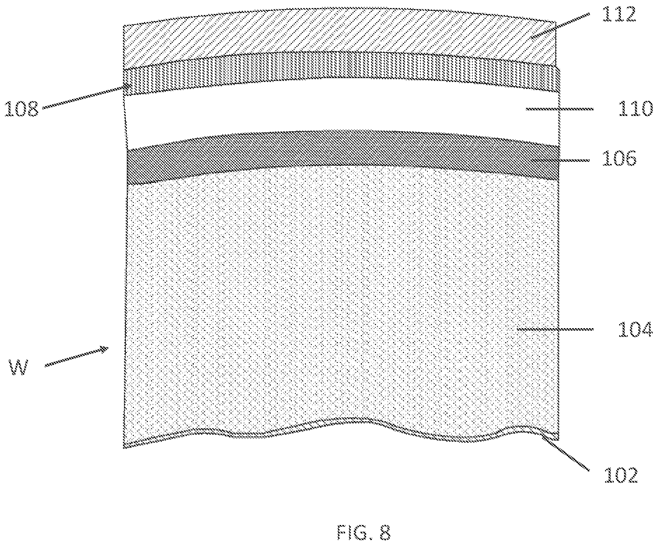

Referring now to Figure 8, an enlarged cutaway view of the human heart wall W is illustrated. The heart wall W has multiple layers, which include the endocardium 102, the myocardium 104, and the epicardium 106. The endocardium 102 is the most inner layer of the heart H. It forms the inner layer of all four heart chambers and is directly connected to all the inner cardiac appendages, such as the bicuspid valve BV, the tricuspid valve TV, the pulmonary valve (not shown), the aortic valve AV, and the chordae tendineae CT by way of the papillary muscles 12.

The myocardium 104 sits between the inner endocardium 102 and the outer epicardium 106. The myocardium 104 is the basic muscle that makes up the heart H and it functions by providing a scaffolding for the heart chambers. The myocardium 104 contracts and relaxes the cardiac walls so that blood can pass between the chambers.

The epicardium 106 is a visceral layer of serous pericardium. The epicardium is the innermost of the two layers of the pericardium. The epicardium covers the external surfaces of the heart. It is directly fused with the myocardium internally. It is comprised mainly of connective tissue and protectively encompasses the heart.

The pericardium 108 is the double-walled sac that contains the heart and roots of the great vessels that leave from or enter the heart. A space is formed between epicardium 106 and the serous layer of the pericardium 108, which is known as the pericardial cavity 110, which contains pericardial fluid. A layer of parietal pericardium 112 is disposed around the heart. The outer parietal layer 112 and the inner serous pericardium layer are on the outside of the pericardial cavity 110.

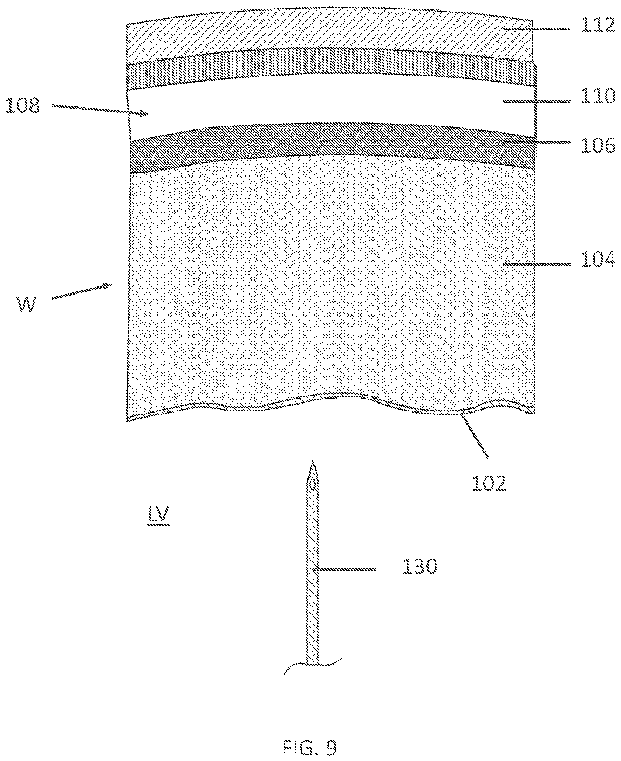

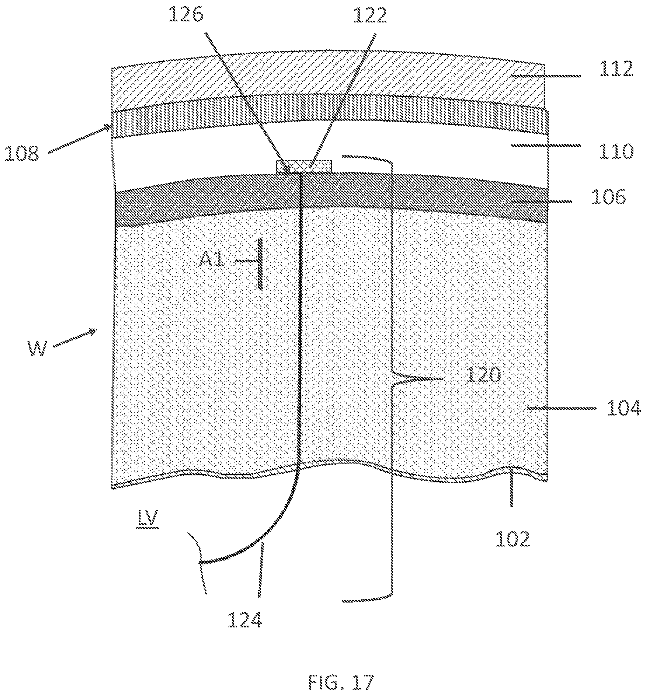

Referring to Figures 9-17, an example of a device 120 for remodeling the shape of a heart wall W, and an example of a system and method for delivering and deploying the device 120 into the pericardial cavity 110 is illustrated. Referring to Figure 17, the device 120 includes an anchor 122 and a line 124 engaging or connected to the anchor 122 and extending therefrom. The line 124 can take a wide variety of different forms. Examples of lines 124 include, but are not limited to, sutures, wires, cables, chords, bendable rods, any combination thereof, etc. The line 124 can be any element or combination of elements that is configured to extend from the anchor 122, through the heart wall W, and into the internal chamber.

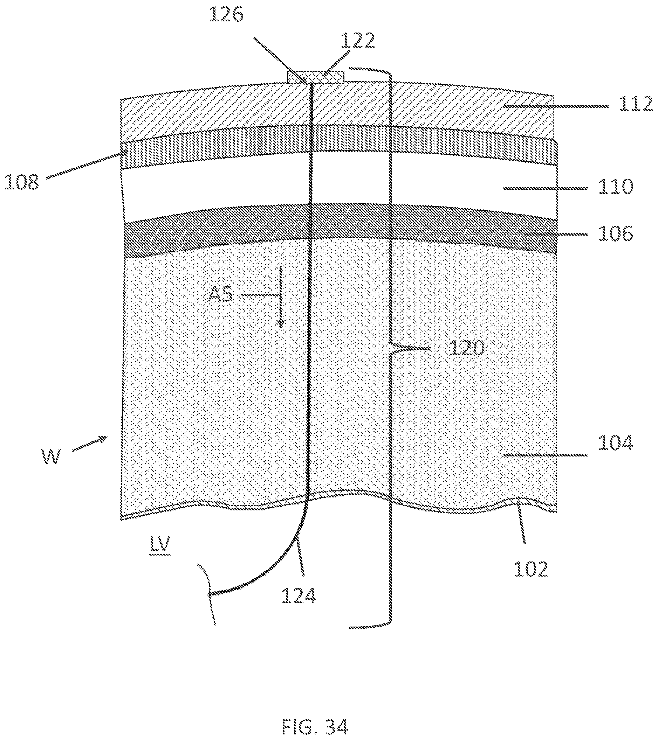

The anchor 122 is configured to be positioned against a surface 126 facing outward relative to an internal chamber of the heart H, such as for example, the left ventricle LV or right ventricle of the heart H. In the illustrated example of Figure 17, the outward facing surface 126 is a portion of the epicardium 106 and the anchor 122 is disposed in the pericardial cavity 110. In the example illustrated by Figure 34, the outward facing surface 126 is the pericardium 108.

The anchor 122 can be configured in a variety of ways. Any configuration that can be positioned to engage an outward facing surface 126 of the heart wall W to support pulling a portion of the heart wall W inward (i.e., toward the internal chamber) can be used. For example, the anchor 122 can be a pledget, a sufficiently sized knot formed in the line 124, a stop, or some other line anchoring device. The anchor 122 can be collapsible/expandable or reconfigurable, such that the anchor can be delivered through a catheter or sheath in a delivered state (e.g., collapsed or elongated) that fits within a lumen of the catheter, and can be reshaped or expanded to a deployed state once it has been delivered to the appropriate location. In one example, the anchor 122 includes a shape-memory alloy-such as Nitinol-to provide shape-setting capability.

Referring to Figure 9, in one example, deployment of the device 120 includes delivering a piercing device 130 into an internal chamber (e.g., left ventricle LV) of the heart H and adjacent the heart wall W. The piercing device 130 can be any suitable device for piercing or creating a passage into the human heart wall W, such as for example, a needle, wire, or other similar device. In the illustrated example, the piercing device 130 is a needle or hollow wire having an inner passage (not shown) and an opening 131 proximate the distal end 133 of the piercing device the fluidly connects the inner passage (not shown) to the exterior of the piercing device 130. However, in other examples, the piercing device is not hollow. In the illustrated example, the piercing device 130 is pointed or has a sharp tip. However, in other examples, the piercing device 130 has a blunt tip.

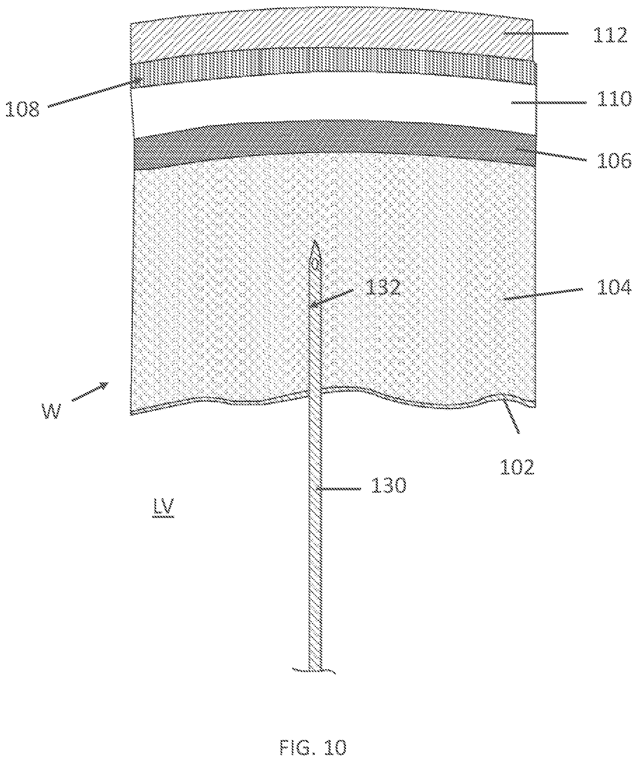

In Figure 10, the piercing device 130 is extended into the heart wall W through the endocardium 102 and into the myocardium 104 to create a passage 132 through the heart wall W. The piercing device 130, however, is not yet sufficiently inserted to deploy the anchor 122 into the pericardial cavity 110. Thus, the piercing device 130 in Figure 10 is shown in an under-inserted position.

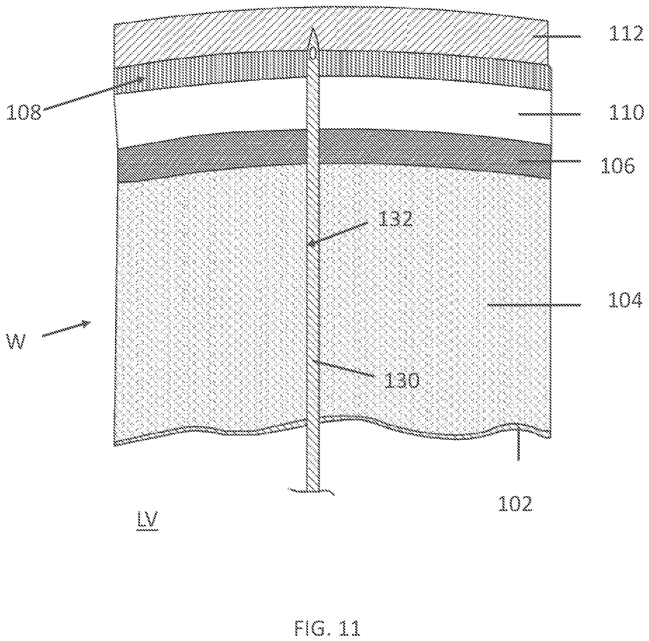

In Figure 11, the piercing device 130 is extended through the endocardium 102, the myocardium 104, the epicardium 106, and the pericardium 108, and into the external parietal pericardium tissue 112 to extend the passage 132 in the heart wall W. The piercing device 130, however, has extended past the pericardial cavity 110 where the anchor 122 is to be positioned in this example. Thus, the piercing device 130 in Figure 11 is shown in an over-inserted position for this example (however, this can be the correct position for other examples).

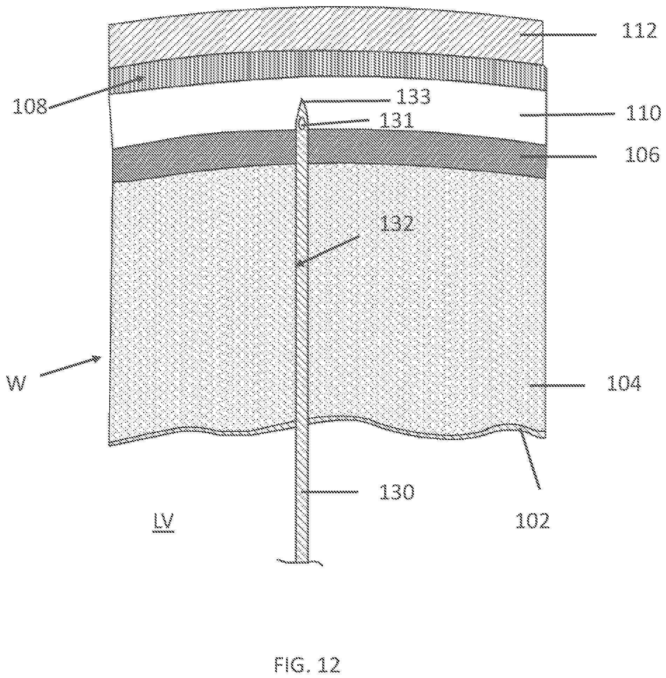

In Figure 12, the piercing device 130 is extended through the endocardium 102, the myocardium 104, the epicardium 106, and into the pericardial cavity 110, such that the opening 131 and the distal end 133 are within the within the pericardial cavity 110. Thus, the piercing device 130 in Figure 12 is properly positioned for deploying the anchor 122 into the pericardial cavity 110.

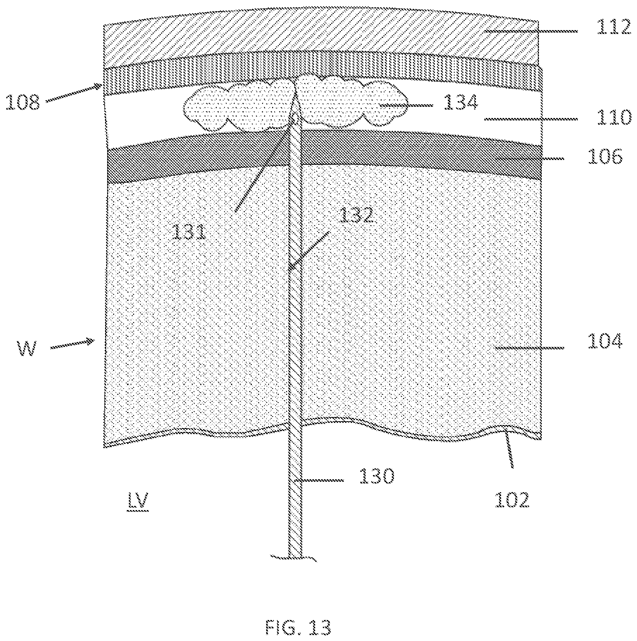

In one example, proper positioning of the piercing device 130 is optionally verified. The proper positioning of the piercing device 130 can be verified in a wide variety of different ways. For example, the positioning of the piercing device can be visually determined by providing the piercing device with a marker, such as a radio-opaque marker, by discharging a material into cavity, such as the pericardial cavity, by sensing a pressure required to discharge fluid from the piercing device, by sensing a force required to advance the piercing device, by positioning a small guide wire, or other guide element or device, in the pericardial cavity, and/or with electrical signals, such as by electrical signals provided by and/or sensed by the piercing device, etc. In one example, to verify that the piercing device 130 is properly positioned for deploying the anchor 122 into the pericardial cavity 110, a dye 134, or other detectable fluid, is delivered through the piercing device 130 and injected into the pericardial cavity 110, as shown in Figure 13. The dye 134 can be detected by any suitable technique such as X-ray or other imaging techniques, to verify that the piercing device 130 is properly positioned.

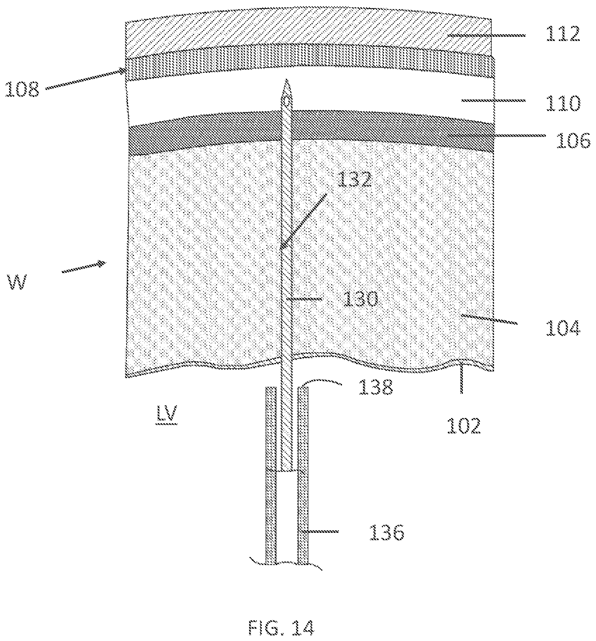

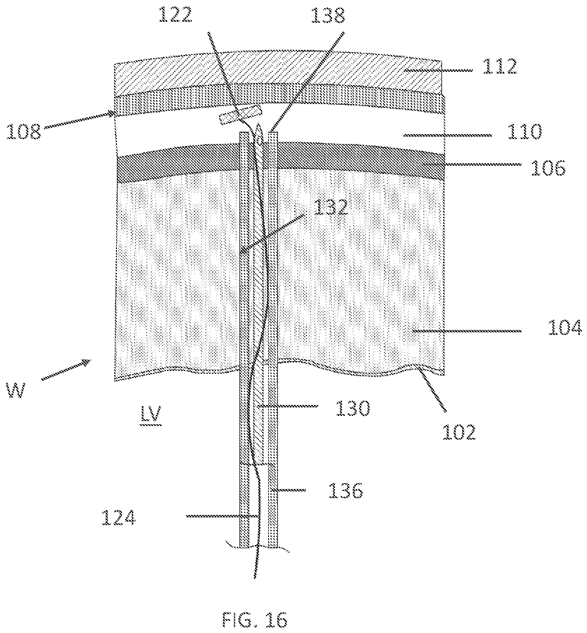

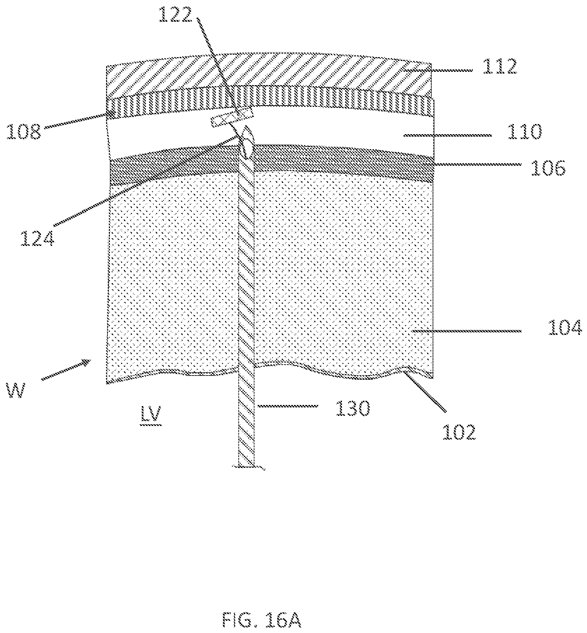

In Figure 14, the piercing device 130 is extended into the pericardial cavity 110 and a delivery catheter 136 is positioned within the heart chamber (e.g., the left ventricle LV) such that a distal end 138 of the delivery catheter 136 is adjacent the endocardium 102. In the illustrated example, the delivery catheter 136 is concentric with the piercing device 130. In other examples, however, the delivery catheter 136 is not concentric with the piercing device 130. In yet other examples, the delivery catheter 136 can be omitted. For example, the device 120 can be delivered directly through or over the piercing device 130, rather than through a separate catheter (See FIGS 16A-16C).

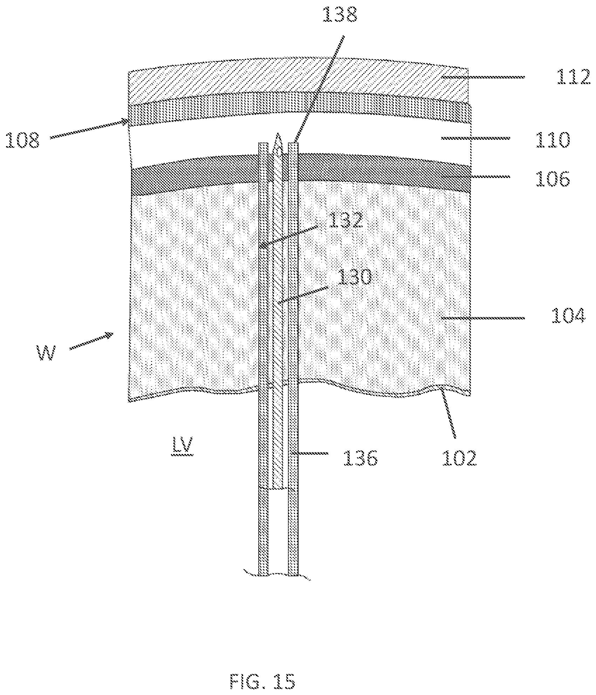

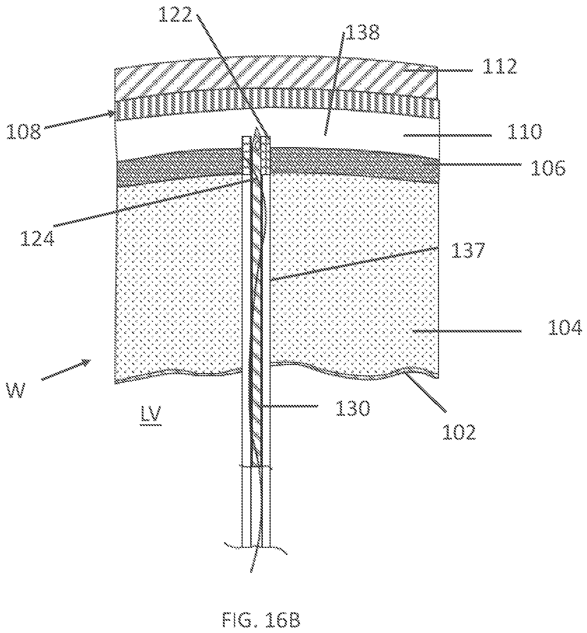

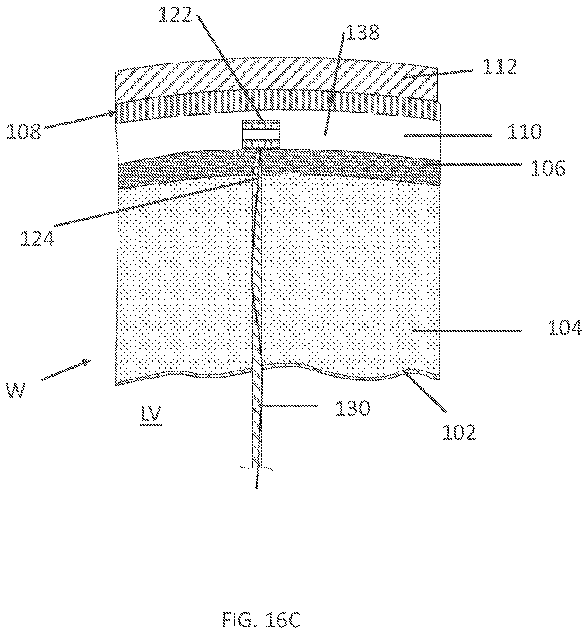

In Figure 15, the delivery catheter 136 is extended through the passage 132 such that the distal end 138 of the delivery catheter 136 is positioned within the pericardial cavity 110. Referring to Figure 16, with the distal end 138 of the delivery catheter 136 is positioned within the pericardial cavity 110, the device 120 (See Figure 17) for remodeling the shape of a heart wall W can be delivered through the delivery catheter 136. In particular, the anchor 122 (See Figures 16 and 17) can be extended or pushed out of the distal end of the 138 of the delivery catheter 136 and into the pericardial cavity 110 while the attached line 124 (See Figures 16 and 17) extends through the delivery catheter 136 in the passage 132.

As is mentioned above, the anchor 122 can take a wide variety of different forms and can be delivered in a wide variety of different ways. In the examples illustrated by Figures 16A-16C, the delivery catheter 136 is omitted. In Figure 16A, the anchor 122 is delivered through the piercing device 130 and the catheter 136 can be omitted. The anchor 122 can be delivered through the piercing device 130 in any of the examples disclosed herein. In Figure 16B, the anchor 122 is delivered over the piercing device 130. In this example, a pusher 137 pushes the anchor 122 along the outside surface of the piercing device 130 to deploy the anchor 122 as illustrated by Figure 16C. The anchor 122 can be delivered over the piercing device 130 in any of the examples disclosed herein.

As shown by comparing Figures 16 and 17, the delivery catheter 136 and the piercing device 130 can be removed by withdrawing them from the passage 132 and from the heart chamber. The device 120 is left deployed in the heart wall W with the anchor 122 within the pericardial cavity 110 and the line 124 extending through the epicardium 106, the myocardium 104, and the endocardium 102 and into the heart chamber (e.g., the left ventricle LV or the right ventricle RV).

To seat the anchor 122 and to remodel the heart wall W, the line 124 can be placed in tension by pulling the line 124 in an inward direction toward the heart chamber as shown by the arrow A1 in Figure 17. As will be described in more detail below, two or more devices can be deployed, coupled, and pull against one another to pull the heart wall inward and remodel the shape of the heart wall(s). The anchor 122 will engage and press in on the outward facing surface 126, which in the illustrated example is the epicardium 106. The anchor 122 (See Figure 17) in its deployed state is too large to fit through the passage 132 (See Figure 14) formed by the piercing device 130 (See Figure 14). Thus, further tensioning of the line 124 can pull the heart wall W inward toward the heart chamber (e.g., the left ventricle LV).

In one example, the device 120 is configured to prevent or inhibit blood leakage through the passage 132 (see Figure 14) into the pericardial space 110. The blood leakage can be blocked in a wide variety of different ways. For example, the anchor 122 can cover the hole through the epicardium, a seal separate from the anchor 122 can be placed over the hole in the epicardium, a seal can be placed over the hole in the endocardium, a portion of the anchor 122 can plug the passage 132, the line 124 can be configured to plug the passage, and/or a component disposed over the line can plug the passage. In one example, the piercing device 130 and/or the delivery catheter 136 are configured such that the passage 132 closes immediately or substantially immediately upon withdrawal of the piercing device 130 and/or the delivery catheter 136.

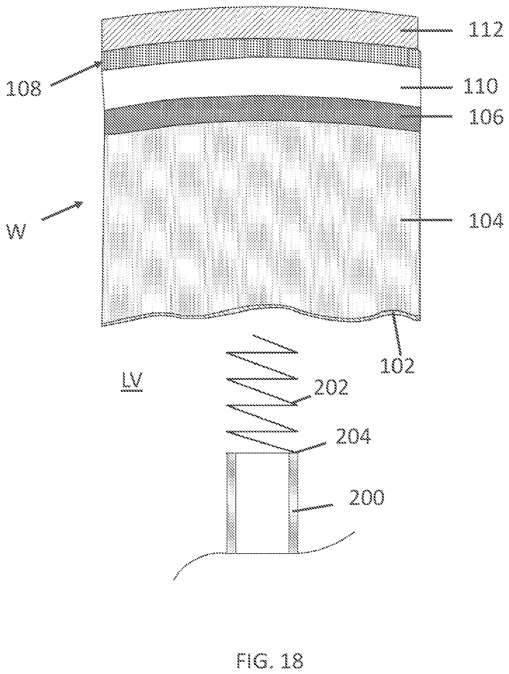

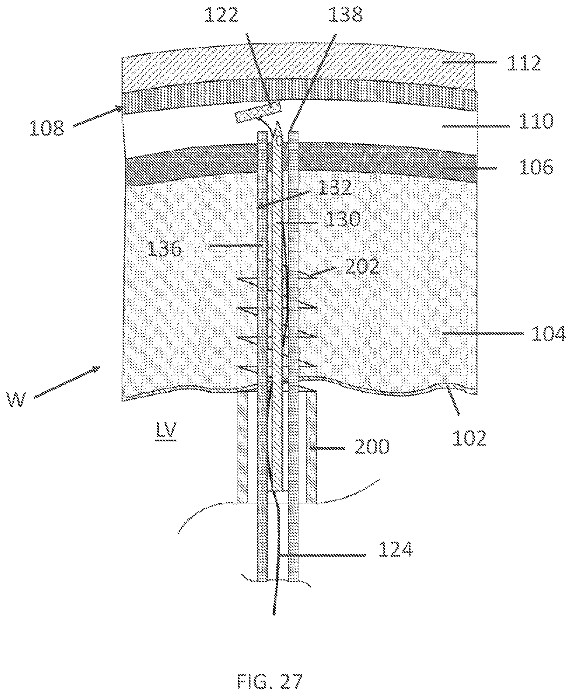

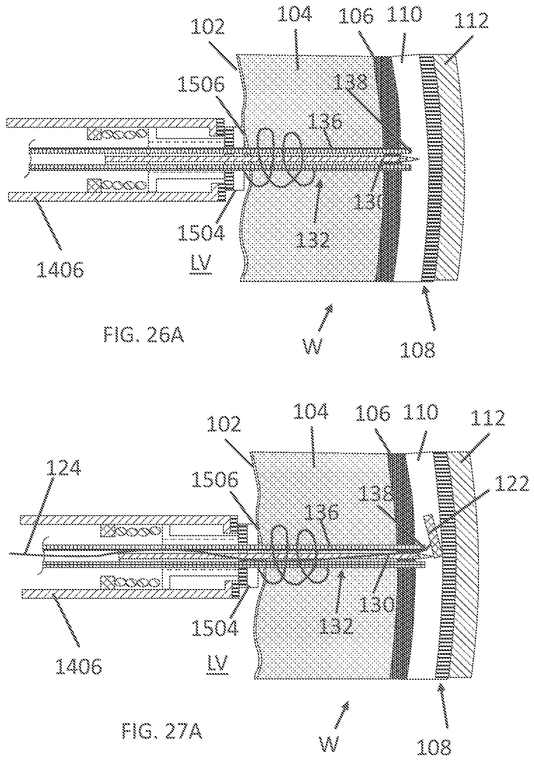

Referring to Figures 18-28, another example of deployment of the device 120 (See Figure 27) into the pericardial cavity 110 for remodeling the shape of a heart wall W and system and method for delivering the device 120 is illustrated. Referring to Figure 18, deployment of the device 120 includes delivering an anchoring catheter 200 into an internal chamber (e.g., left ventricle LV) of the heart H and adjacent the heart wall W. The anchoring catheter 200 can be any suitable catheter or catheter-like device that is capable of attaching to, or anchoring to, the heart wall W.

In the example, the anchoring catheter 200 includes an anchoring device 202 attached to a distal end 204 of the anchoring catheter 200. Examples of suitable anchoring devices include, but are not limited to expandable barbs, a suction tip, such as a suction cone, and/or a corkscrew shaped tip as illustrated. The anchoring device 202 can be any device capable of temporarily attaching the anchoring catheter 200 to the heart wall W. In the illustrated example, the anchoring device 202 is a wire formed into a helical shape.

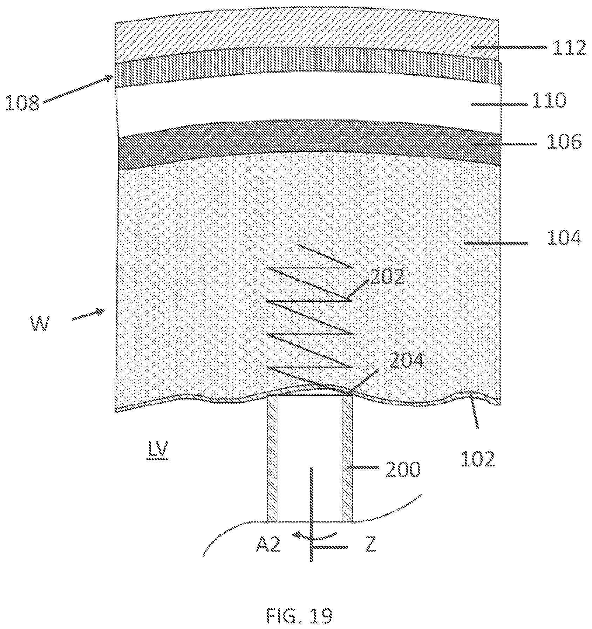

Referring to Figure 19, the anchoring catheter 200 is attached to the heart wall W. In the illustrated example, the anchoring catheter 200 can be attached to the heart wall W by rotating the anchoring catheter 200 about illustrated axis Z in the direction of arrow A2, as shown in Figure 19. As a result, the helical anchoring device 202 can screw into the heart wall W through the endocardium 102 and into the myocardium 104 to secure the anchoring catheter 200 to the heart wall W. The anchoring device 202 thereby fixes the position of the catheter 200 relative to the heart wall W. Since the heart H is beating during the procedures described herein, the anchoring of the catheter 200 relative to the heart wall greatly simplifies and increases the accuracy of the procedure of piercing the wall W with the piercing device 130, positioning the distal end 133 of the piecing device in the pericardial space 110, positioning the delivery catheter 136 in the pericardial space 110, and/or deploying the anchor 122 into the pericardial space 110 (See Figure 27).

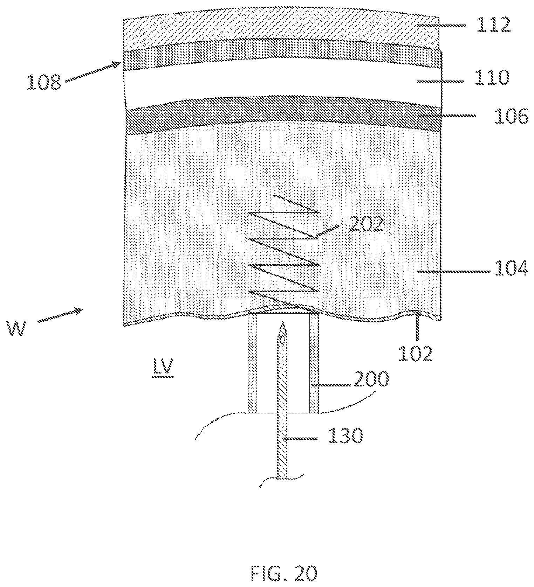

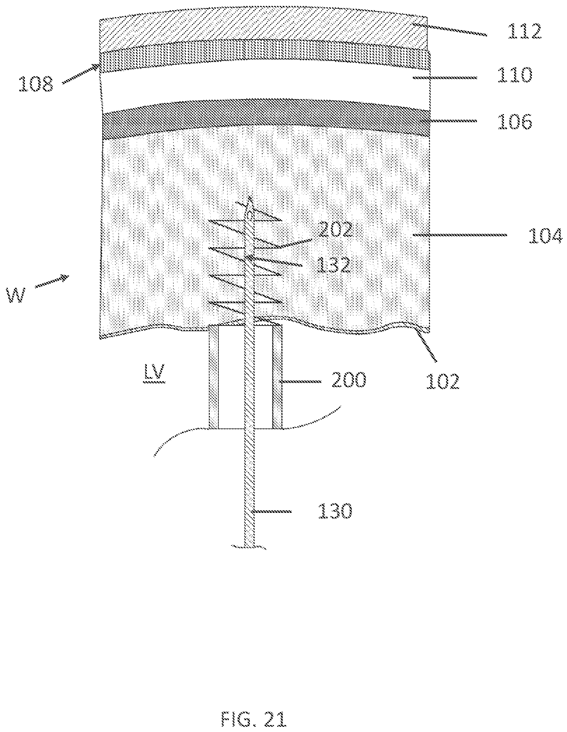

Referring to Figure 20, the piercing device 130 is delivered through the anchoring catheter 200 into an internal chamber (e.g., left ventricle LV) of the heart H such that the distal end of the piercing device is adjacent the heart wall W. In Figure 21, the piercing device 130 is extended into the heart wall W through the endocardium 102 and into the myocardium 104 to create the passage 132 into the heart wall W. The piercing device 130, however, is not yet sufficiently inserted to deploy the anchor 122 (See Figure 27) into the pericardial cavity 110. Thus, the piercing device 130 in Figure 21 is shown in an under-inserted position.

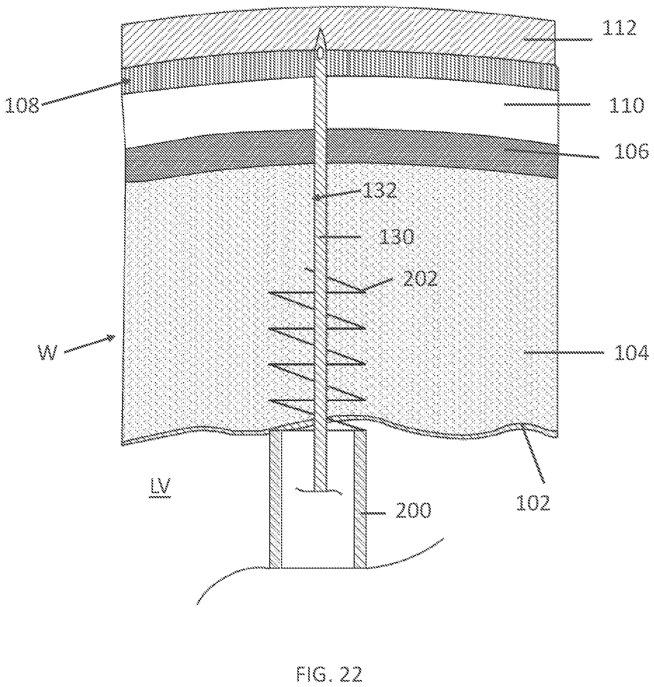

In Figure 22, the piercing device 130 is extended through the endocardium 102, the myocardium 104, the epicardium 106, and the pericardium 108, and into the external parietal pericardium tissue 112. The piercing device 130, however, has extended past the pericardial cavity 110 where the anchor 122 (See Figure 27) is to be positioned in this example. Thus, the piercing device 130 in Figure 22 is shown in an over-inserted position in this example.

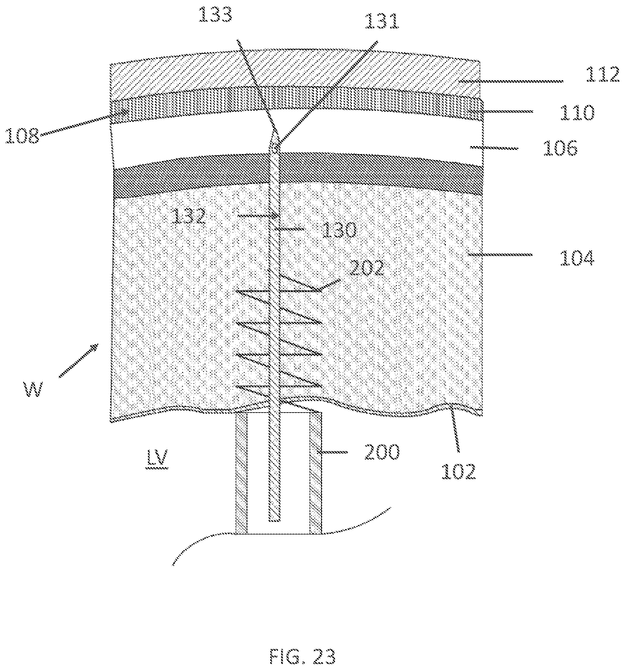

In Figure 23, the piercing device 130 is extended through the endocardium 102, the myocardium 104, the epicardium 106, and into the pericardial cavity 110, such that the opening 131 and the distal end 133 are within the pericardial cavity 110. Thus, the piercing device 130 in Figure 23 is properly positioned for deploying the anchor 122 (See Figure 27) into the pericardial cavity 110.

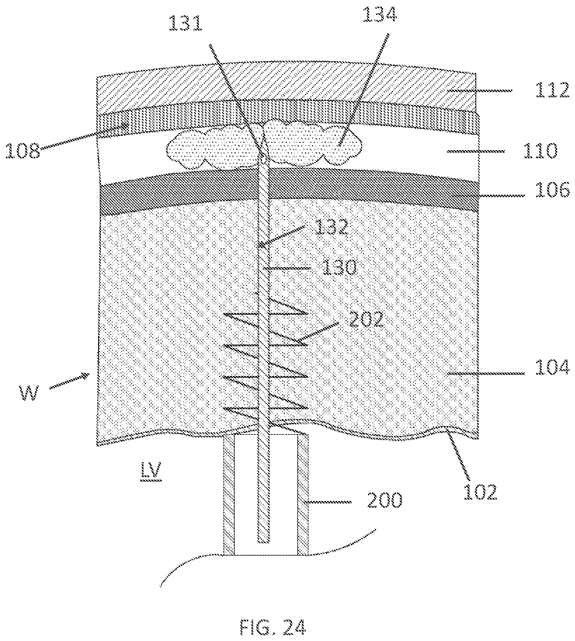

In one example, proper positioning of the piercing device 130 is optionally verified. The proper positioning of the piercing device 130 can be verified in a wide variety of different ways. For example, the positioning of the piercing device can be visually determined by providing the piercing device with a marker, such as a radio-opaque marker, by discharging a material into cavity, such as the pericardial cavity, by sensing a pressure required to discharge fluid from the piercing device, by sensing a force required to advance the piercing device, by providing and/or sensing an electrical signal with the piercing device (e.g., the piercing device can be used to sense an ECG signal generated by the heart). In one example, to verify that the piercing device 130 is properly positioned for deploying the anchor 122 into the pericardial cavity 110, a dye 134, or other detectable fluid, is delivered through the piercing device 130 and injected into the pericardial cavity 110, as shown in Figure 24. The dye 134 can be detected by any suitable technique such as X-ray or other imaging techniques, to verify that the piercing device 130 is properly positioned.

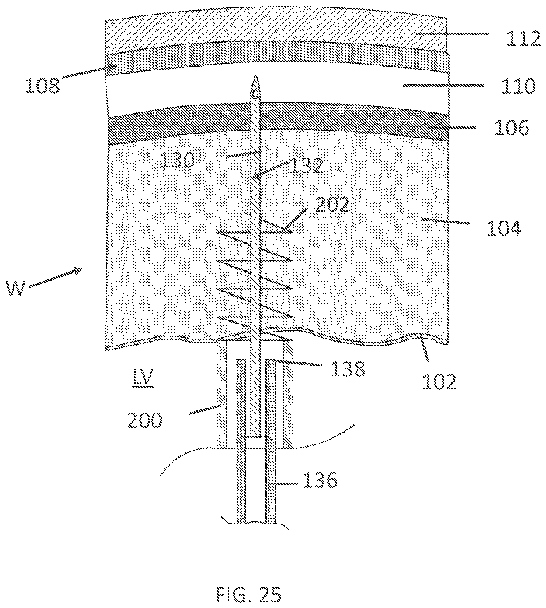

In Figure 25, the piercing device 130 is extended into the pericardial cavity 110 and the delivery catheter 136 is extended through the anchoring catheter 200 such that the distal end 138 of the delivery catheter 136 is adjacent the endocardium 102.

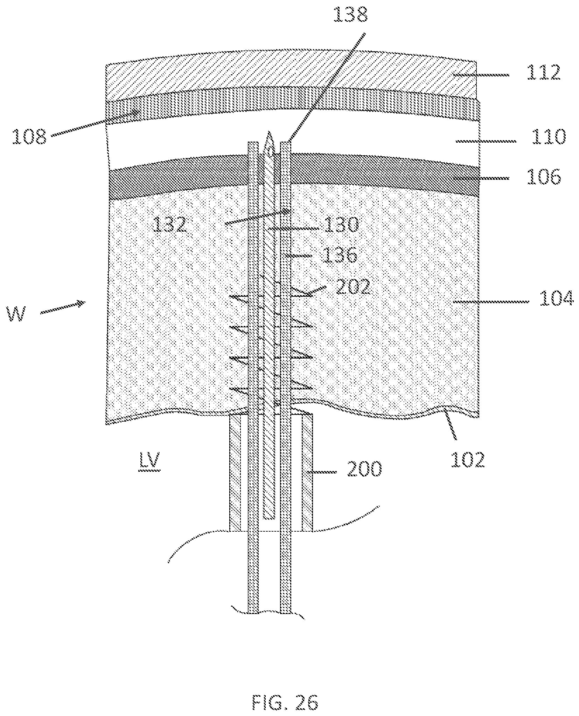

In Figure 26, the delivery catheter 136 is extended through the passage 132 such that the distal end 138 of the delivery catheter 136 is positioned within the pericardial cavity 110. As shown in Figure 27, with the distal end 138 of the delivery catheter 136 is positioned within the pericardial cavity 110, the device 120 for remodeling the shape of a heart wall W can be delivered through the delivery catheter 136. In particular, the anchor 122 can be extended out of the distal end of the 138 of the delivery catheter 136 and into the pericardial cavity 110 while the attached line 124 extends through the delivery catheter 136 in the passage 132.

As shown by comparing Figures 26-28, the delivery catheter 136 and the piercing device 130 can be removed by withdrawing them from the passage 132 and from the heart chamber. The device 120 is left deployed in the heart wall W with the anchor 122 within the pericardial cavity 110 and the line 124 extending through the epicardium 106, the myocardium 104, and the endocardium 102 and into the heart chamber (e.g., the left ventricle LV).

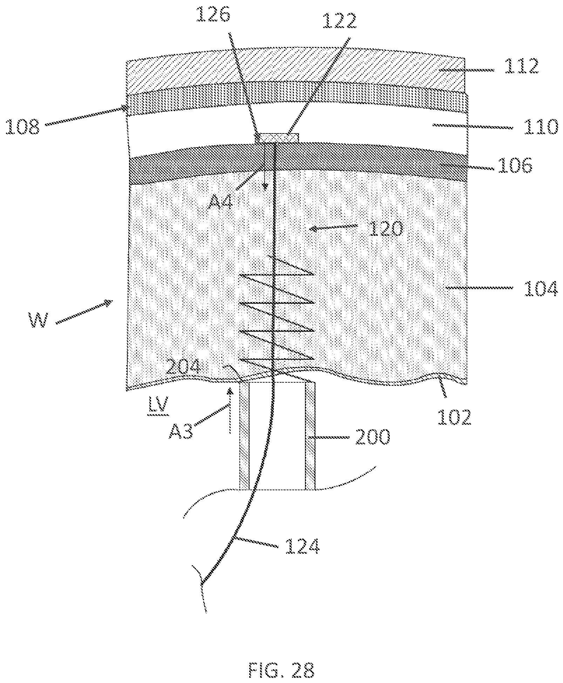

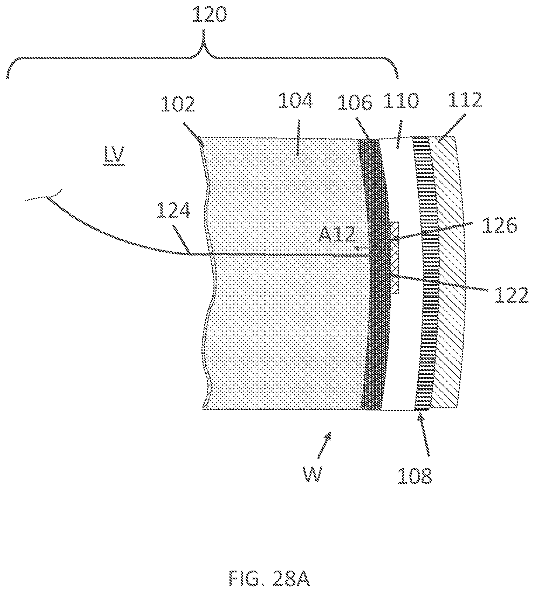

To seat the anchor 122 against the heart wall W, the anchoring catheter 200 can optionally remain attached to the heart wall W and the distal end 204 can be pushed against the heart wall W, as shown by arrow A3. At the same time, the line 124 can be placed in tension by pulling the line 124 in an inward direction toward the heart chamber as shown by the arrow A4 in Figure 28.

To remodel the heart wall W, the anchoring catheter 200 is removed (if it had not previously been removed) The line 124 can be placed in tension by pulling the line 124 in an inward direction toward the heart chamber as shown by the arrow A4 in Figure 28. As will be described in more detail below, two or more devices can be deployed, coupled, and pull toward one another to pull the heart wall inward and remodel the shape of the heart wall(s). The anchor 122 will engage and press in on the outward facing surface 126, which in the illustrated example is the epicardium 106. The anchor 122 in its deployed state is too large to fit through the passage 132 formed by the piercing device 130. Thus, once the anchoring catheter 200 is removed, further tensioning of the line 124 can pull the heart wall W inward toward the heart chamber (e.g., the left ventricle LV).

In one example, the device 120 is configured to prevent or inhibit blood leakage through the passage 132 into the pericardial space 110. The blood leakage can be blocked in a wide variety of different ways. For example, the anchor 122 can cover the hole through the epicardium, a seal separate from the anchor 122 can be placed over the hole in the epicardium, a seal can be placed over the hole in the endocardium, a portion of the anchor 122 can plug the passage 132, the line 124 can be configured to plug the passage, and/or a component disposed over the line can plug the passage. In one example, the piercing device 130 and/or the delivery catheter 136 are configured such that the passage 132 closes immediately or substantially immediately upon withdrawal of the piercing device 130 and/or the delivery catheter 136.

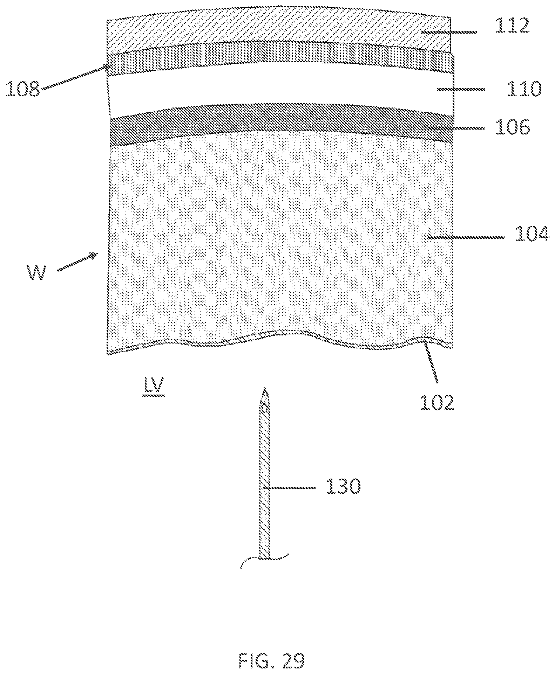

Referring to Figures 29-34, an example of a device 120 (Figure 34) for remodeling the shape of a heart wall W, and an example of a system and method for delivering and deploying the device 120 against an exterior surface of the heart H is illustrated. Referring to Figure 29, in one example, deployment of the device 120 includes delivering the piercing device 130 into an internal chamber (e.g., left ventricle LV) of the heart H and adjacent the heart wall W.

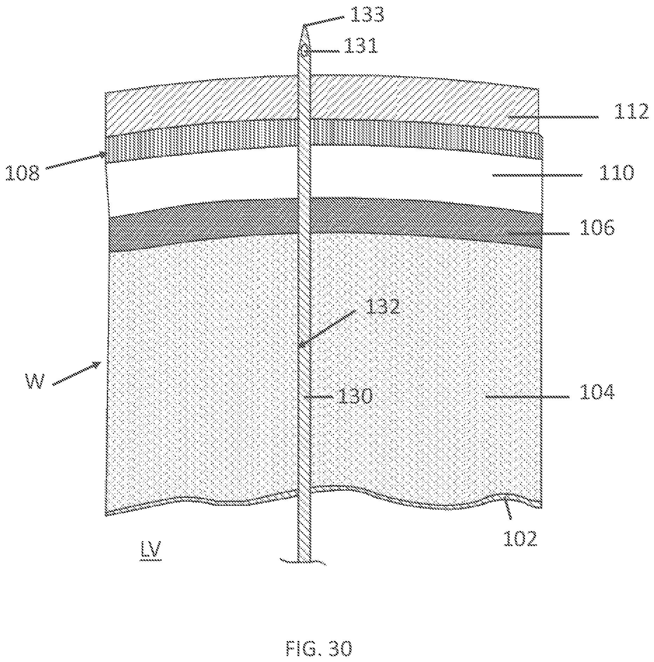

In Figure 30, the piercing device 130 is extended into the heart wall W through the endocardium 102, the myocardium 104, the epicardium 106, the pericardium 108, and the external parietal pericardium tissue 112 to create a passage 132 through the heart wall W, such that the opening 131 and the distal end 133 of the piercing device 130 are external to the heart H. Thus, the piercing device 130 in Figure 30 is properly positioned for deploying the anchor 122 in this example.

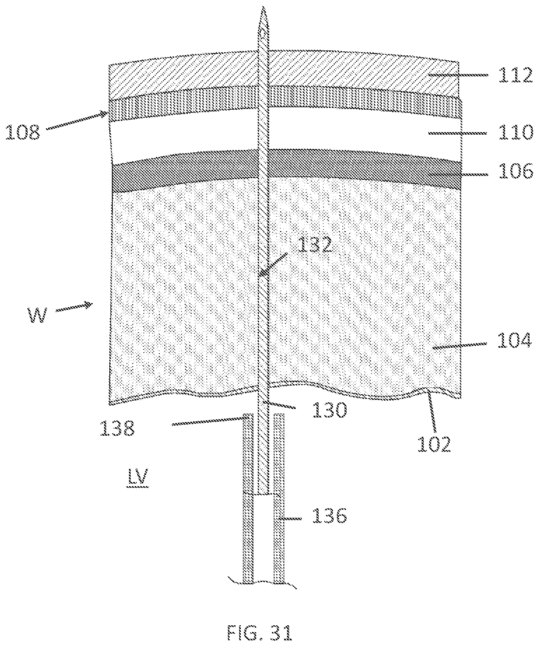

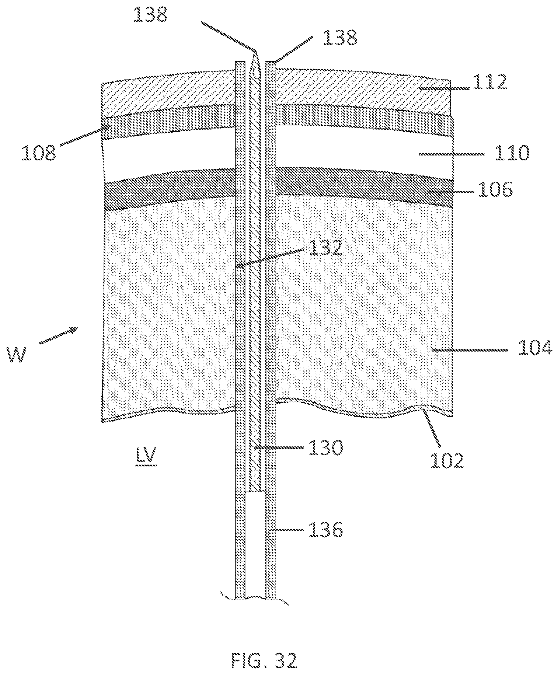

In Figure 31, the piercing device 130 is extended though the heart wall W and the delivery catheter 136 is positioned within the heart chamber (e.g., the left ventricle LV) such that the distal end 138 of the delivery catheter 136 is adjacent the endocardium 102. In Figure 32, the delivery catheter 136 is extended through the passage 132 such that the distal end 138 of the delivery catheter 136 is external the heart H, such as adjacent the distal end 133 of the piercing device 130. In the illustrated example, the delivery catheter 136 is concentric with the piercing device 130. In other examples, however, the delivery catheter 136 is not concentric with the piercing device 130. In yet other examples, the delivery catheter 136 can be omitted. For example, the device 120 can be delivered directly through the piercing device 130, rather than through a separate catheter.

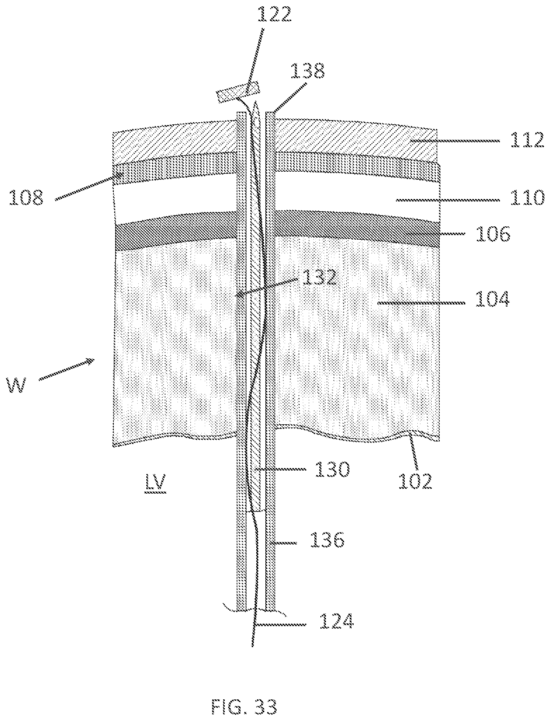

In Figure 33, the device 120 (Figure 34) for remodeling the shape of a heart wall W can be delivered through the delivery catheter 136. In particular, the anchor 122 can be extended out of the distal end of the 138 of the delivery catheter 136 external to the heart wall H while the attached line 124 extends through the delivery catheter 136 in the passage 132.

As shown in Figure 34, the delivery catheter 136 and the piercing device 130 can be removed by withdrawing them from the passage 132 and from the heart chamber. The device 120 is left deployed through heart wall W with the anchor 122 external to the heart wall W and the line 124 extending through the parietal pericardium tissue 112, the pericardium 108, the pericardial space 110, the epicardium 106, the myocardium 104, and the endocardium 102 and into the heart chamber (e.g., the left ventricle LV).

To seat the anchor 122 and to remodel the heart wall W, the line 124 can be placed in tension by pulling the line 124 in an inward direction toward the heart chamber as shown by the arrow A5 in Figure 34. As will be described in more detail below, two or more devices can be deployed, coupled, and pulled toward one another to pull the heart wall inward and remodel the shape of the heart wall(s). The anchor 122 will engage the outward facing surface 126, which in the illustrated example is the exterior parietal pericardium tissue layer 112. In one example, the anchor 122 presses a localized area of the pericardium 108 against the epicardium and thus pushes the myocardium 104 inward to remodel the heart wall W. The anchor 122 in its deployed state is too large to fit through the passage 132 formed by the piercing device 130. Thus, further tensioning of the line 124 can pull the heart wall W inward toward the heart chamber (e.g., the left ventricle LV).

In one example, the device 120 is configured to prevent or inhibit blood leakage through the passage 132 into the pericardial space 110. The blood leakage can be blocked in a wide variety of different ways. For example, the anchor 122 can locally pull the pericardium into contact with the epicardium to block the hole through the epicardium, a seal separate from the anchor 122 can be placed over the hole in the epicardium, a seal can be placed over the hole in the endocardium, a portion of the anchor 122 can plug the passage 132, the line 124 can be configured to plug the passage, and/or a component disposed over the line can plug the passage. **In** one example, the piercing device 130 and/or the delivery catheter 136 are configured such that the passage 132 and/or the hole in the pericardium closes immediately or substantially immediately upon withdrawal of the piercing device 130 and/or the delivery catheter 136.

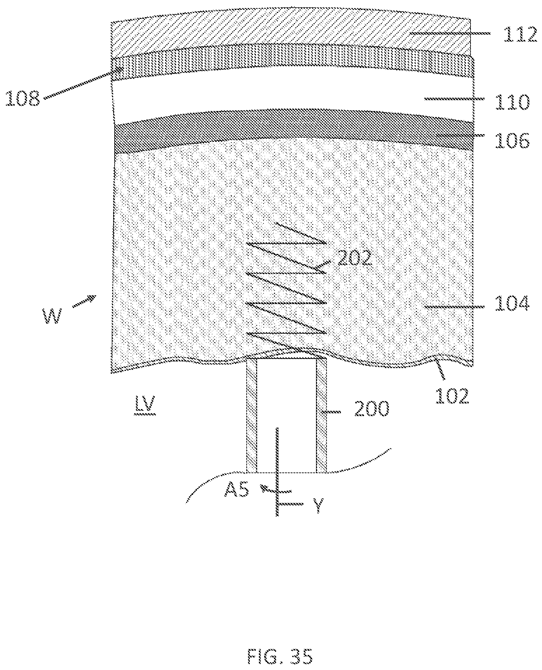

Referring to Figures 35-41, another example of deployment of the device 120 for remodeling the shape of a heart wall W and system and method for delivering the device 120 against an exterior surface of the heart H is illustrated. Referring to Figure 35 deployment of the device 120 includes delivering the anchoring catheter 200 into an internal chamber (e.g., left ventricle LV) of the heart H and attaching the anchoring catheter to the heart wall W.

In the example, the anchoring catheter 200 includes an anchoring device 202 attached to a distal end 204 of the anchoring catheter 200. Examples of suitable anchoring devices include, but are not limited to expandable barbs, a suction tip, such as a suction cone, and/or a corkscrew shaped tip as illustrated. The anchoring device 202 can be any device capable of temporarily attaching the anchoring catheter 200 to the heart wall W. In the illustrated example, the anchoring device 202 is a wire formed into a helical shape.

In the illustrated example, the anchoring catheter 200 can be attached to the heart wall W by rotating the anchoring catheter 200 about axis Y in the direction of arrow A5, as shown in Figure 35. As a result, the helical anchoring device 202 can screw into the heart wall W through the endocardium 102 and into the myocardium 104 to secure the anchoring catheter 200 to the heart wall W.

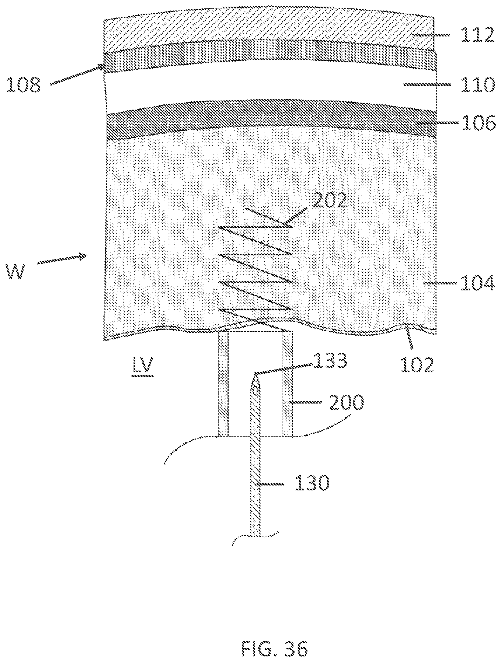

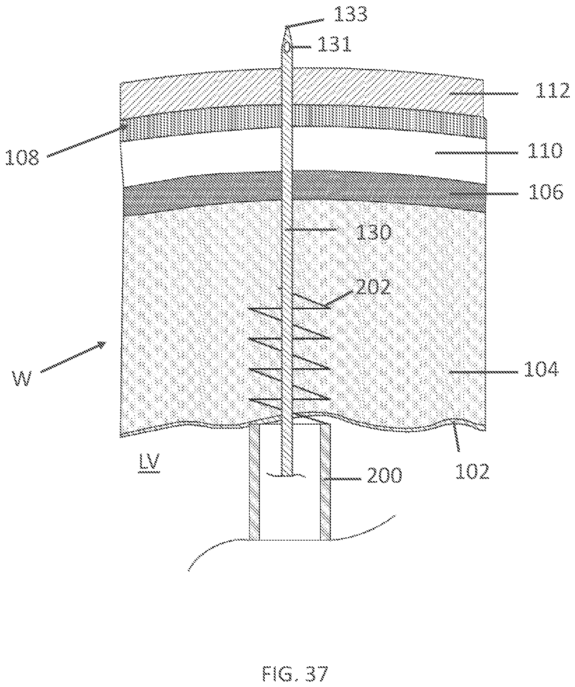

Referring to Figure 36, the piercing device 130 is delivered into an internal chamber (e.g., left ventricle LV) of the heart H via the anchoring catheter 200. The distal end 133 of the piercing device is adjacent the heart wall W. In Figure 37, the piercing device 130 is extended into the heart wall W through the endocardium 102, the myocardium 104, the epicardium 106, and the pericardium 108 to create the passage 132 through the heart wall W. The opening 131 and the distal end 133 of the piercing device 130 are external to the heart H. The piercing device 130 in Figure 37 is properly positioned for deploying the anchor 122 in this example.

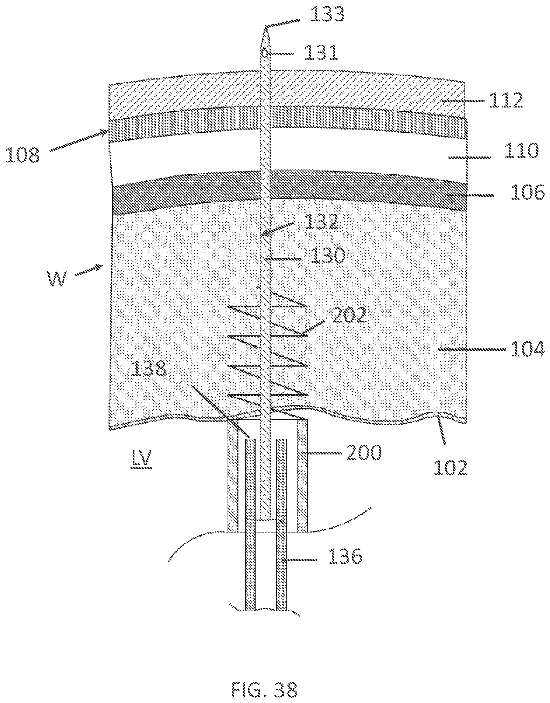

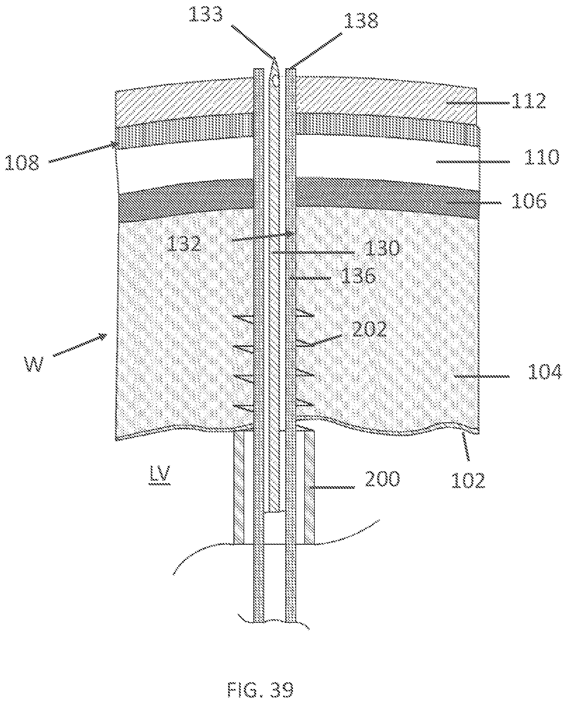

In Figure 38, the piercing device 130 is extended though the heart wall W and the delivery catheter 136 is positioned within the temporary anchoring catheter 200 such that the distal end 138 of the delivery catheter 136 is adjacent the endocardium 102. In Figure 39, the delivery catheter 136 is extended through the passage 132 such that the distal end 138 of the delivery catheter 136 is external the heart H, such as adjacent the distal end 133 of the piercing device 130.

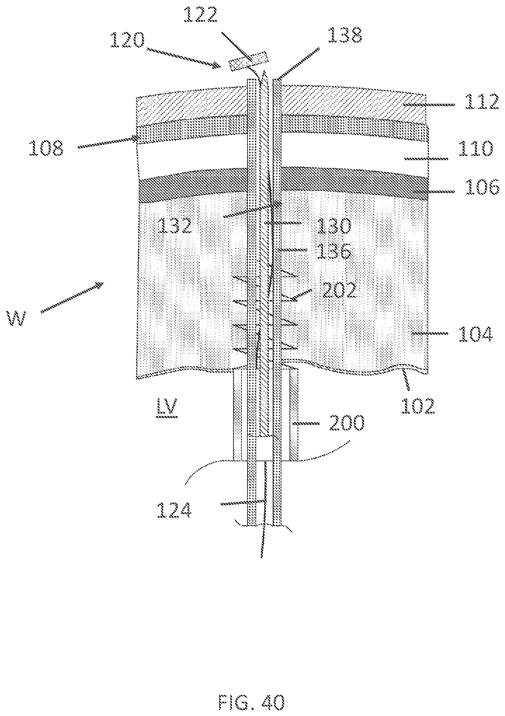

In Figure 40, the device 120 for remodeling the shape of a heart wall W can be delivered through the delivery catheter 136. In particular, the anchor 122 can be extended out of the distal end of the 138 of the delivery catheter 136 external to the heart wall H while the attached line 124 extends through the delivery catheter 136 in the passage 132.

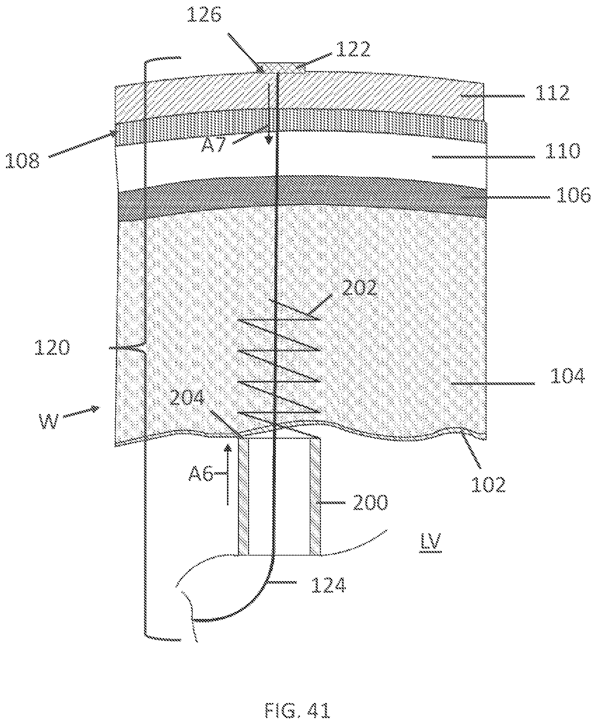

As shown in Figure 41, the delivery catheter 136 and the piercing device 130 can be removed by withdrawing them from the passage 132 and from the heart chamber. The device 120 is left deployed through heart wall W with the anchor 122 external to the heart wall W and the line 124 extending through the epicardium 106, the myocardium 104, and the endocardium 102 and into the heart chamber (e.g., the left ventricle LV).

To seat the anchor 122 firmly against the heart wall W, the anchoring catheter 200 can optionally remain attached to the heart wall W and the distal end 204 can be maintained against the heart wall W, as shown by arrow A6. At the same time, the line 124 can be placed in tension by pulling the line 124 in an inward direction toward the heart chamber as shown by the arrow A7 in Figure 41. This seating step is optional and can be omitted.

Once the anchor 122 is firmly seated, the anchoring catheter 200 is removed and the line 124 is placed in tension to remodel the heart wall. As will be described in more detail below, two or more devices can be deployed, coupled, and pulled toward one another to pull the heart wall inward and remodel the shape of the heart wall(s). The anchor 122 will engage the outward facing surface 126, which in the illustrated example is the exterior parietal tissue layer 112. In one example, the anchor 122 presses a localized area of the pericardium 108 against the epicardium and thus pushes the myocardium 104 inward to remodel the heart wall W. The anchor 122 in its deployed state is too large to fit through the passage 132 formed by the piercing device 130. Thus, once the anchoring catheter 200 is removed, further tensioning of the line 124 can pull the heart wall W inward toward the heart chamber (e.g., the left ventricle LV).

In one example, the device 120 is configured to prevent or inhibit blood leakage through the passage 132 into the pericardial space 110. The blood leakage can be blocked in a wide variety of different ways. For example, the anchor 122 can locally pull the pericardium into contact with the epicardium to block the hole through the epicardium, a seal separate from the anchor 122 can be placed over the hole in the epicardium, a seal can be placed over the hole in the endocardium, a portion of the anchor 122 can plug the passage 132, the line 124 can be configured to plug the passage, and/or a component disposed over the line can plug the passage. In one example, the piercing device 130 and/or the delivery catheter 136 are configured such that the passage 132 and/or the hole in the pericardium closes immediately or substantially immediately upon withdrawal of the piercing device 130 and/or the delivery catheter 136.

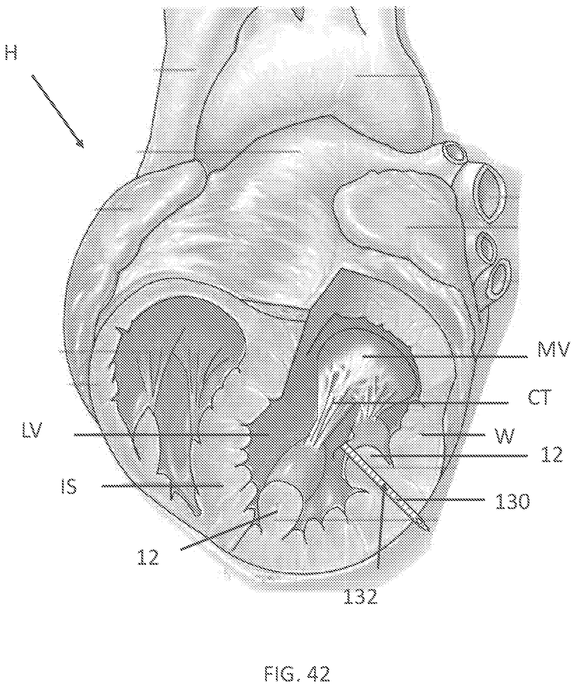

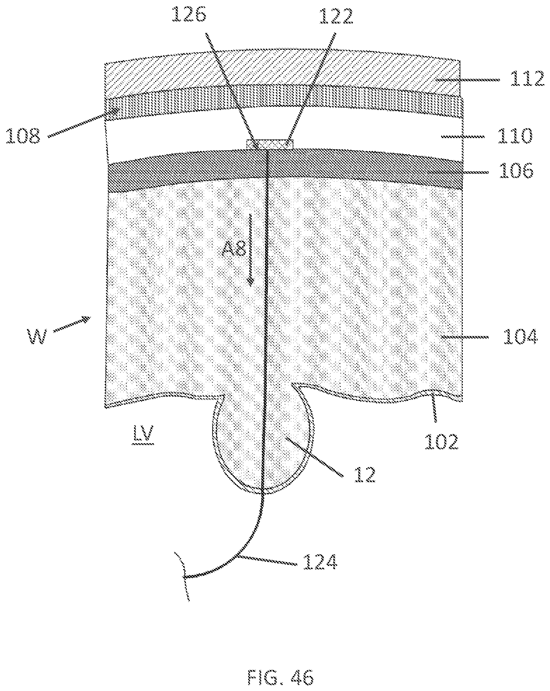

Referring to Figures 42-47, another example of deployment of the device 120 for remodeling the shape of a heart wall W and system and method for delivering the device 120. The example illustrated by Figures 42-47 can deploy the anchor 122 within the pericardial cavity 110 as illustrated by Figure 46 or outside the pericardium (see, for example Figure 34 - location can also be selected to pass the line 124 through the papillary muscle). Referring to Figure 42, in one example, deployment of the device 120 includes delivering the piercing device 130 into an internal chamber (e.g., left ventricle LV or right ventricle RV) of the heart H. The piercing device 130 is then extended through one of the papillary muscles 12 and through the heart wall W to create a passage 132.

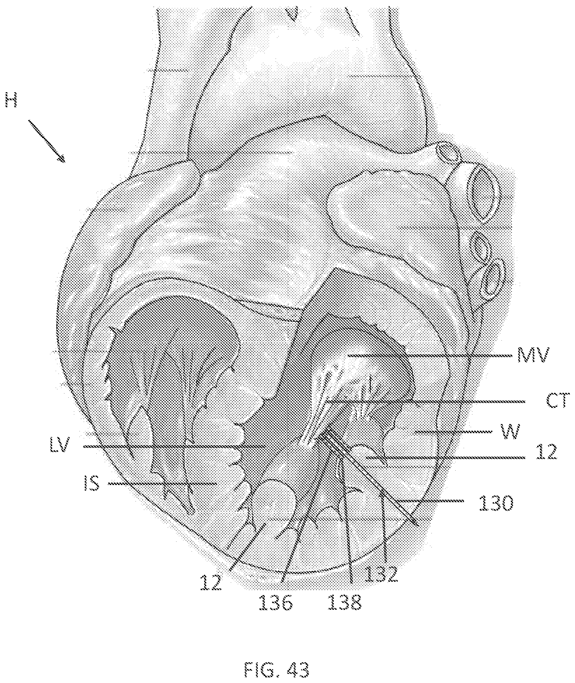

In Figure 43, the delivery catheter 136 is disposed around the piercing device 130 and positioned within the heart chamber (e.g., the left ventricle LV). The distal end 138 of the delivery catheter 136 is positioned adjacent the papillary muscle 12. In Figures 42 and 43, the pericardium 108 is not illustrated. As is noted above, in this example the anchor 122 can be deployed into the pericardial space 110 or onto the outside of the pericardium.

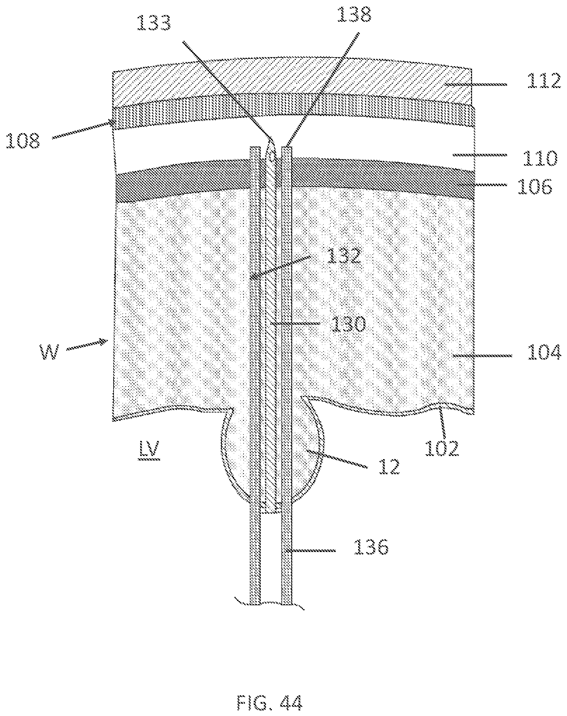

In Figure 44, the piercing device 130 is illustrated in the proper position for deploying the anchor 122 in the pericardial cavity 110. The delivery catheter 136 is extended through the passage 132 such that the distal end 138 of the delivery catheter 136 is within the pericardial cavity 110 adjacent the distal end 133 of the piercing device 130.

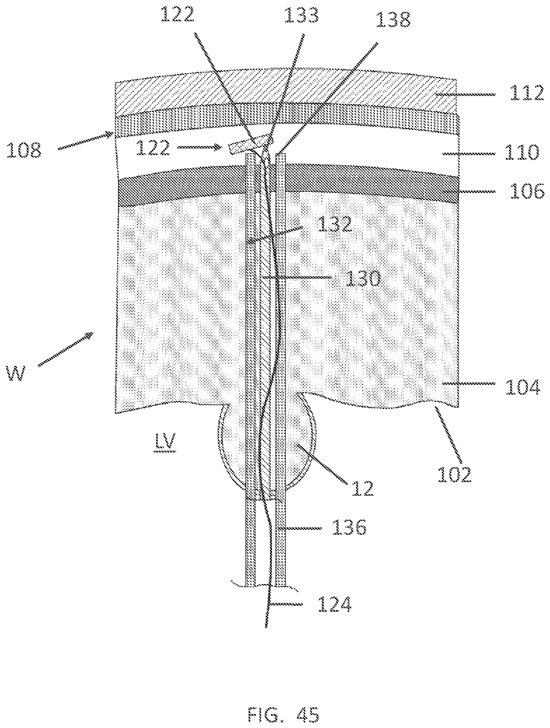

In Figure 45, the device 120 for remodeling the shape of a heart wall W can be delivered through the delivery catheter 136. In particular, the anchor 122 can be extended out of the distal end of the 138 of the delivery catheter 136 into the pericardial cavity 110 while the attached line 124 extends through the delivery catheter 136 in the passage 132 through the heart wall and the papillary muscle 12.

As shown in Figure 46, the delivery catheter 136 and the piercing device 130 can be removed by withdrawing them from the passage 132 and from the heart chamber. The device 120 is left deployed through heart wall W with the anchor 122 within the pericardial cavity 110 and the line 124 extending through the epicardium 106, the myocardium 104, and the endocardium 102 and into the heart chamber (e.g., the left ventricle LV).

To seat the anchor 122 and to remodel the heart wall W, the line 124 can be placed in tension by pulling the line 124 in an inward direction toward the heart chamber as shown by the arrow A8 in Figure 46. As will be described in more detail below, two or more devices can be deployed, coupled, and pulled toward one another to pull the heart wall inward and remodel the shape of the heart wall(s). The anchor 122 will engage and press in on the outward facing surface 126, which in the illustrated example is the epicardium 106. The anchor 122 in its deployed state is too large to fit through the passage 132 formed by the piercing device 130. Thus, further tensioning of the line 124 can pull the heart wall W inward toward the heart chamber (e.g., the left ventricle LV).

In one example, the device 120 is configured to prevent or inhibit blood leakage through the passage 132 into the pericardial space 110. The blood leakage can be blocked in a wide variety of different ways. For example, the anchor 122 can locally pull the pericardium into contact with the epicardium to block the hole through the epicardium, a seal separate from the anchor 122 can be placed over the hole in the epicardium, a seal can be placed over the hole in the endocardium, a portion of the anchor 122 can plug the passage 132, the line 124 can be configured to plug the passage, and/or a component disposed over the line can plug the passage. In one example, the piercing device 130 and/or the delivery catheter 136 are configured such that the passage 132 and/or the hole in the pericardium closes immediately or substantially immediately upon withdrawal of the piercing device 130 and/or the delivery catheter 136.

In one example, the device 120 is configured to prevent or inhibit blood leakage through the passage 132 into the pericardial space 110. The blood leakage can be blocked in a wide variety of different ways. For example, the anchor 122 can cover the hole through the epicardium, a seal separate from the anchor 122 can be placed over the hole in the epicardium, a seal can be placed over the hole in the endocardium, a portion of the anchor 122 can plug the passage 132, the line 124 can be configured to plug the passage, and/or a component disposed over the line can plug the passage. In one example, the piercing device 130 and/or the delivery catheter 136 are configured such that the passage 132 closes immediately or substantially immediately upon withdrawal of the piercing device 130 and/or the delivery catheter 136.

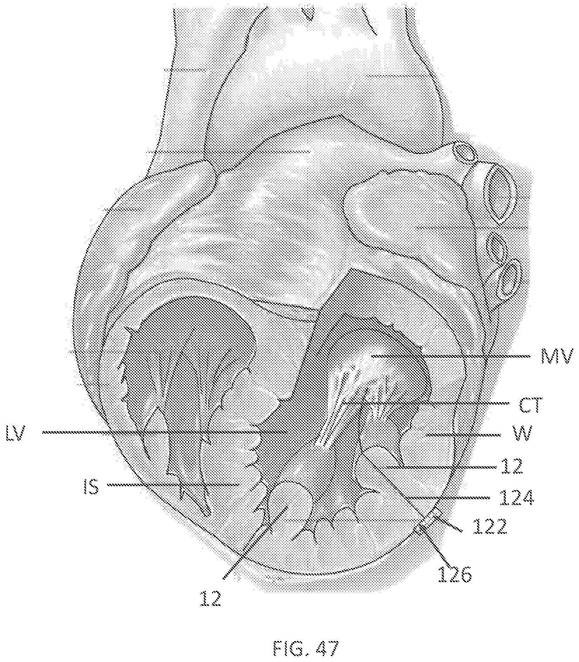

In Figure 47, the device 120 is illustrated in an installed position with the anchor 122 engaging the outward facing surface 126 and the line extending through the heart wall W and one of the papillary muscles 12 and into the left ventricle LV and through the mitral valve MV. The device illustrated by Figure 47 can be installed in any manner described herein. In the example illustrated by Figures 42-47, the device 120 is installed without and anchoring catheter 200. However, in other examples, the device 120 can be installed using and anchoring catheter 200 in any of the manners described herein.

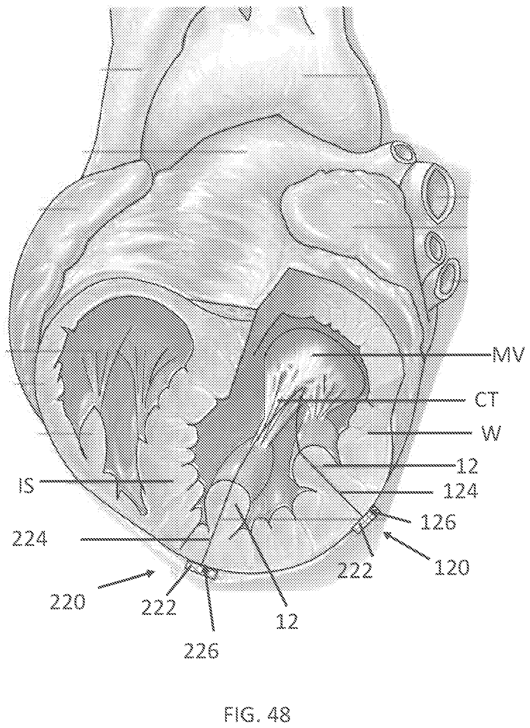

In Figure 48, along with the installed first device 120, a second device 220 is illustrated in an installed position. The second device 220 includes a second anchor 222 engaging a second outward facing surface 126 and a second line extending through the heart wall W and through another of the papillary muscles 12 and into the left ventricle LV and through the mitral valve MV. The second device 120 can be installed in any of the manners described herein. In one example, the second device 220 is installed in the same manner as the first device 120.

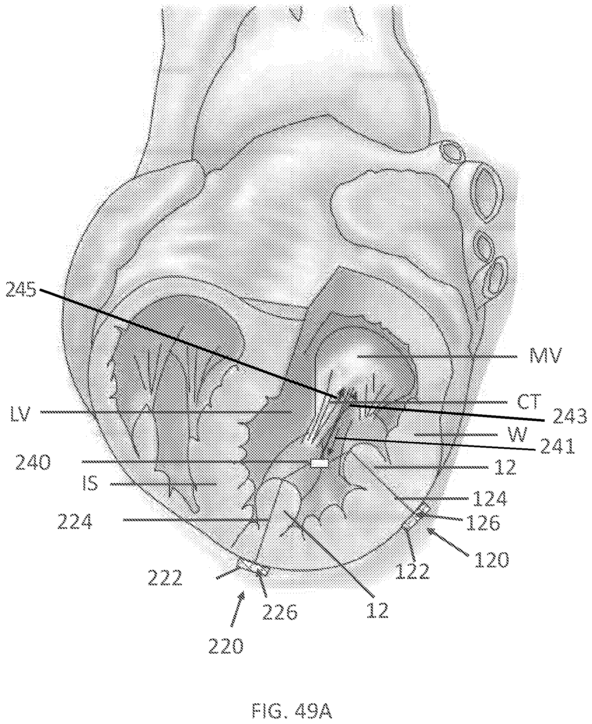

Referring to Figure 49A, in one example the first and second lines are routed through a connector 240. In the example illustrated by Figure 49A, the papillary muscles 12 are pulled together or approximated by pushing or holding the position of the connector 240 as indicated by arrow 241 and pulling on the lines 124, 224 as indicated by arrows 243, 245 respectively. The distance the connector 240 is pushed as indicated by arrow 241 and the distances the lines 124, 224 are pulled controls how far the papillary muscles 12 are pulled toward one another, which in turn determines the remodeling of the heart walls.

Still referring to Figure 49A, in one example, the papillary muscles 12 can be pulled toward one another in a manner that improves coaption between the leaflets of the mitral valve MV. That is, the chordae tendinea are attached to the mitral valve MV leaflets and the papillary muscles. Approximating the papillary muscles toward one another causes the chordae tendinea CT to pull the mitral valve leaflets toward one another and enhance coaption of the mitral valve leaflets. The enhanced or corrected leaflet coaption can reduce or eliminate mitral valve regurgitation.

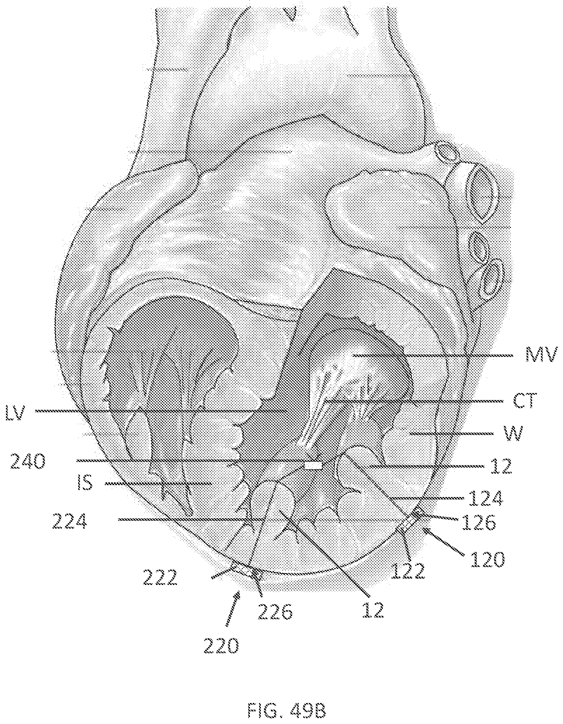

Referring to Figure 49B, after the papillary muscles 12 and the heart walls W are pulled to the desired remodeled position by the lines 124, 224, the connector 240 secures the positions of the lines in the connector. Once secured, the lines can be trimmed as shown. As such, both the device 120 and the second device 220 are shown in installed positions with the anchors 122, 222 engaging the outward facing surfaces 126, 226 and the lines 124, 224 extending through the heart wall W, through papillary muscles 12 and into the left ventricle LV. The lines 124, 224 remain in tension to pull inward to pull the heart wall W inward to remodel the shape of the heart wall W.

The lines 124, 224 can be connected together in a variety of ways. For example, the lines can be tied together or held together by a line locking device 240. The line locking device 240 can be any suitable device that can hold the lines 124, 224 together in tension such that the heart wall W is held in the remodeled position. A variety of different line locking devices 240 are shown and described below.

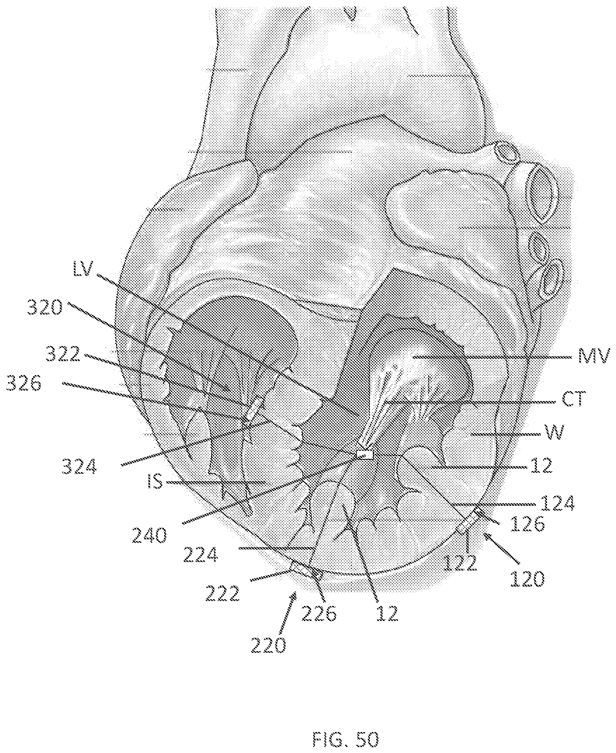

Any number of devices 120 and any number of line locking devices 240 can be used to tailor the heart wall remodeling to each individual patient. In one example, two or more lines 124 are locked together in each of the locking devices. Figure 50 illustrates one example where three lines are connected together by one locking device. In some examples, more than one locking device is used, with at least two lines being connected together by each locking device. In the example illustrated by Figure 50, along with the installed first device 120 and a second device 220, a third device 320 is illustrated in an installed position with a third anchor 322 engaging a third outward facing surface 326 and a third line 324 extending through a heart wall and into the left ventricle LV. In the illustrated example of Figure 48, the heart wall associated with the third device 320 is the interventricular septum IS. Thus, the interventricular septum IS defines the third outward facing surface 326 and the third line 324 extends through the interventricular septum IS.

As with the devices of Figure 49B, the lines 124, 224, 324 can be pulled inward to pull the heart wall W inward to remodel the shape of the heart wall W. To hold the heart wall W in the remodeled position, the lines 124, 224, 324, while in tension, can be connected within the left ventricle LV, such as for example, by the line locking device 240 or other suitable means for connecting the lines 124, 224, 324.

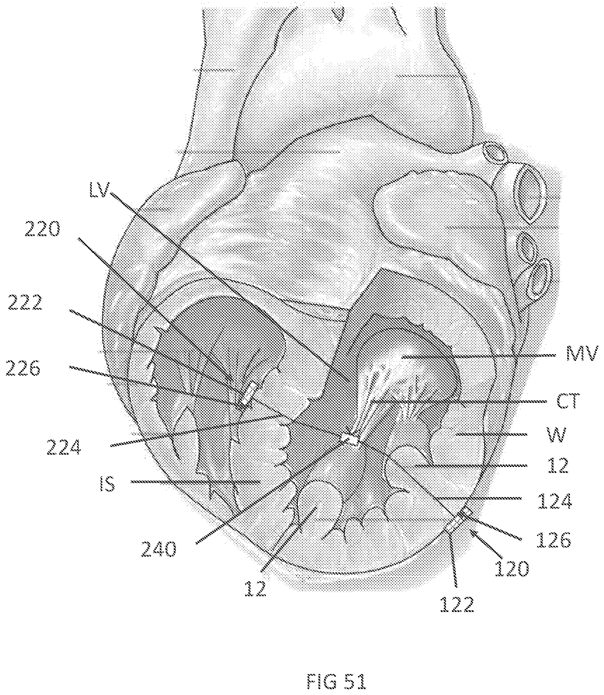

In Figure 51, both the device 120 and the second device 220 are shown in installed positions with the anchors 122, 222 engaging the outward facing surfaces 126, 226. The line 124 extends through the heart wall W, through a papillary muscle 12 and into the left ventricle LV. The second device 220 is installed such that the second anchor 222 engages the outward facing surface 226 on the interventricular septum IS and the second line 224 extends through the interventricular septum IS and into the left ventricle LV.

As with the devices of Figure 49B, the lines 124, 224, can be pulled inward to pull the heart wall W inward and to pull the intraventricular septum IS inward to remodel the shape of the heart wall W and the intraventricular septum IS. To hold the heart wall W and the intraventricular septum IS in the remodeled positions, the lines 124, 224, while in tension, can be connected within the left ventricle LV, such as for example, by the line locking device 240 or other suitable means for connecting the lines 124, 224.

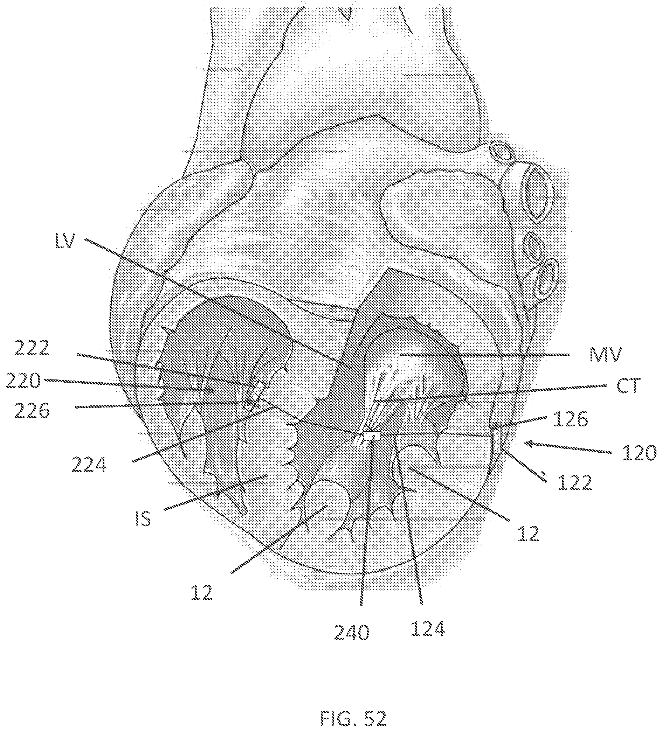

In Figure 52, both the device 120 and the second device 220 are shown in installed positions with the anchors 122, 222 engaging the outward facing surfaces 126, 226. The line 124 extends through the heart wall W and into the left ventricle LV, but not through a papillary muscle 12. The second device 220 is installed such that the second anchor 222 engages the outward facing surface 226 on the interventricular septum IS and the second line 224 extends through the interventricular septum IS and into the left ventricle LV.

As with the devices of Figure 49B, the lines 124, 224, can be pulled inward to pull the heart wall W inward to remodel the shape of the heart wall W and the intraventricular septum IS. To hold the heart wall W and the intraventricular septum IS in the remodeled position, the lines 124, 224, while in tension, can be connected within the left ventricle LV, such as for example, by the line locking device 240 or other suitable means for connecting the lines 124, 224.

The devices 120 can be used in a wide variety of different ways to remodel the heart and/or approximate the papillary muscles. A single locking device 240 and two or more devices 120 can be deployed or more than one line locking device 240 with two or more devices 120 per locking device 240 can be deployed to remodel the heart of a single patient. For example, any of the configurations illustrated by Figures 49B, 50, 51, and 52 can be used in combination on the heart of a single patient. For example, one pair of devices 120, 220 can be used to approximate the papillary muscles 12, while one or more additional pairs (or three, or four, etc.) of devices can be used to remodel the shape of the right ventricle, in the same patient. Or, in one example, one pair of devices 120, 220 pulls one papillary muscle and a portion of the heart wall or intraventricular septum IS relatively toward one another and another on pair of devices 120, 220 pulls another papillary muscle and a portion of the heart wall or intraventricular septum IS relatively toward one another. In another example, the lines are not deployed through the papillary muscles and one pair of devices 120, 220 pulls two portions of the heart wall or intraventricular septum IS relatively toward one another and another pair of devices 120, 220 pulls two other portions of the heart wall or intraventricular septum IS relatively toward one another.

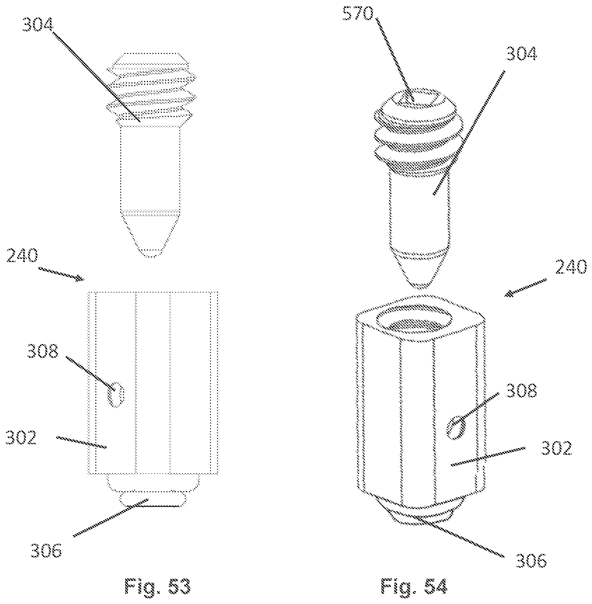

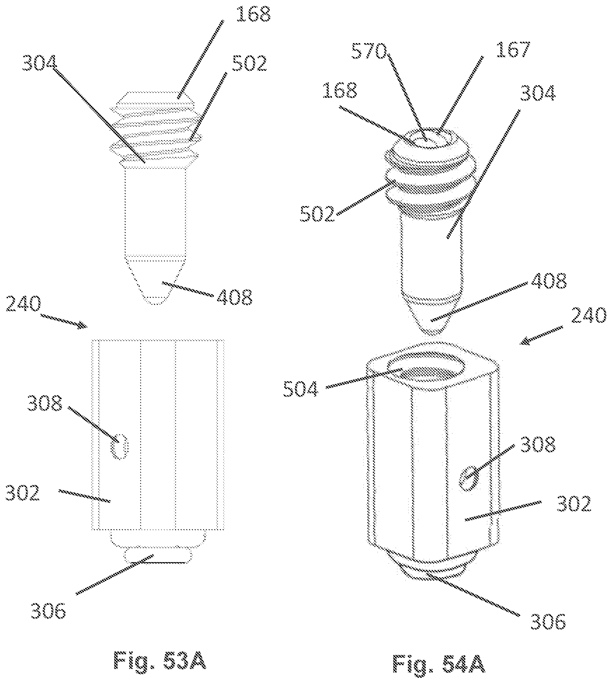

The line locking device 240 can take a wide variety of different forms. An example of a line locking device 240 is illustrated in Figures 53 and 54. Figure 53 illustrates an exploded side view of the line locking device 240 which is comprised of a body 302 and a threaded insert 304. The body 302 has an input opening 306 and two outlets 308 (the second outlet is not visible in each of the figures). Figure 54 illustrates an orthogonal view of Figure 53.

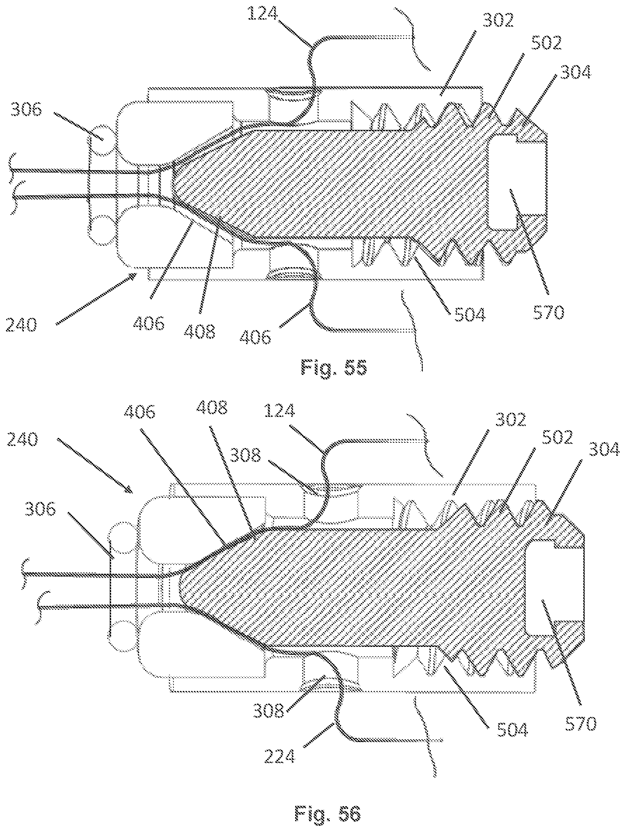

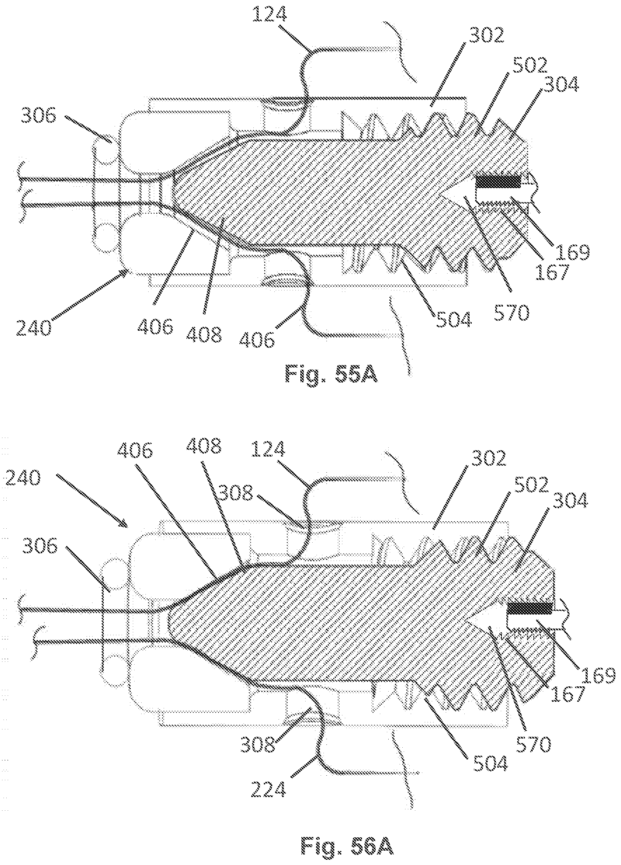

Figure 55 illustrates cross section view of the line locking device 240 of Figure 53 and 54. As illustrated, the threaded insert 304 is partially inserted into the body 302. A first line 124 and a second line 224 are shown entering the body 302 via the input opening 306 where they pass between a conical-shaped clamping surface 406 of the body 302 and a conical surface 408 of the insert 304. After passing between the two clamping surfaces, the first line 124 exits the line locking device 240 through one outlet 308 and the second line 224 extends through the other outlet 308. The lines 124, 224 can take a wide variety of different forms. For example, the lines can be sutures, wires, cables, chords, bendable rods, any combination thereof, etc.

Figure 56 illustrates the threaded insert 304 inserted into the body 302 such that the first and second lines (124 and 224) are captured or clamped between the conical-shaped clamping surface 406 of the body 302 and the conical surface 408 of the insert 304. This insertion is performed by causing the threaded insert 304 to be rotated such that the threaded insert is drawn into the body 302 by the action of threads 502 formed on the insert and mating threads 504 formed in a body chamber.

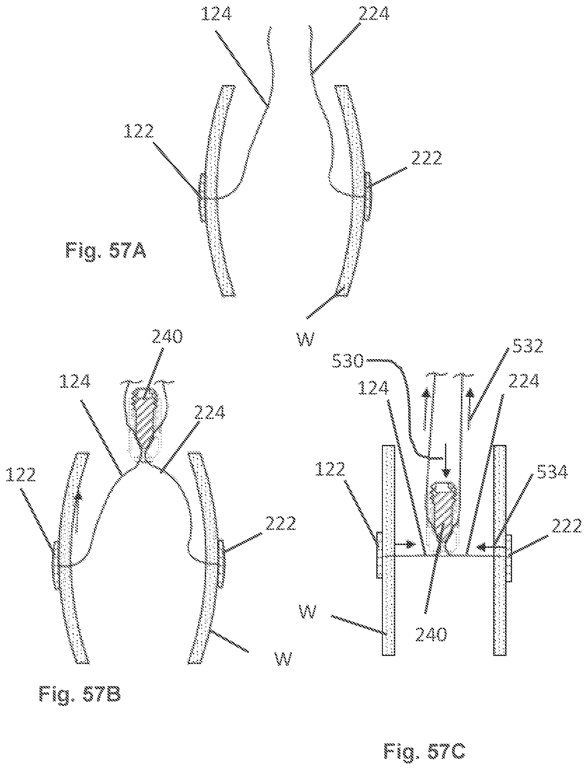

Figures 57A-57E illustrate the clamp in use. As shown, a first anchor 122 and second anchor 222 are positioned on an outside surface 126 of a heart wall W. The first anchor 122 is attached to the first line 124 and the second anchor 222 is attached to the second line 224. The first and second anchors 122, 222 and lines 124, 224 can be deployed in the manner described above.

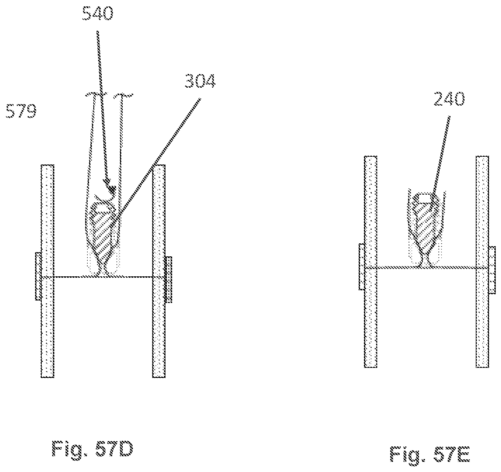

As illustrated in Figure 57B, the first and second lines 124 and 224 are positioned in a line locking device 240, such as the device illustrated by Figure 55. In Figure 57C, the line locking device 240 is pushed along the lines 124, 224 as indicated by arrow 530 and is positioned and held proximately to the first and second anchors 122 and 222. The first and second lines 124 and 224 are pulled as indicated by arrows 532 through the clamp, causing the first and second anchors to be drawn inward as shown as indicated by arrows 534. This has the effect of drawing the heart walls W together. Once the heart walls W are in the position desired, the threaded insert 304 is rotated as shown in Figure 57D at 540. This serves to secure the first and second lines 124 and 224 as illustrated in Figure 56. This securing maintains the tension on the walls W and thereby keeps the heart walls in the remodeled shape. When the first and second lines 124 and 224 are secured they can be cut as illustrated in Figure 57E.

Still referring to Figures 57A-57E, in one example the line locking device 240 is configured to delivered through a catheter (i.e. transcatheter) to a ventricle LV, RV. Tension is applied to the lines 124, 224 while the line locking device is inside the ventricle by pulling on the lines from outside the patient's body (i.e. from the patient's groin, collar bone area, or chest). The line locking device 240 is locked while the line locking device is inside the ventricle by rotating (or otherwise actuating) a line locking tool or driver 550 from outside the patient's body (i.e. also from the patient's groin, collar bone area, or chest). As such, in one example the operation of rotating the threaded insert 304 includes a tool or driver 550 that can position the line locking device 240 and rotate the threaded insert 304 inside a heart.

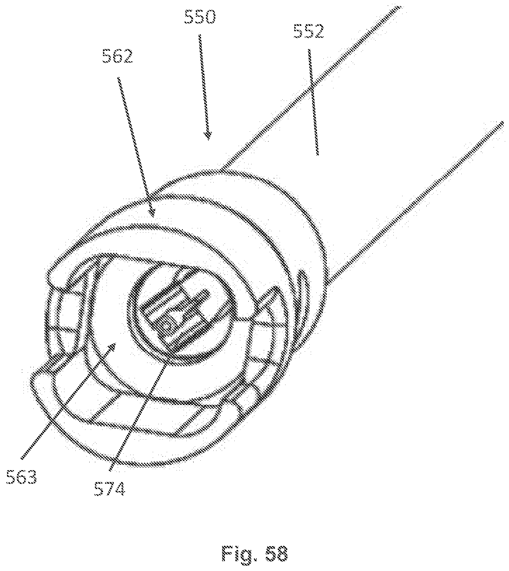

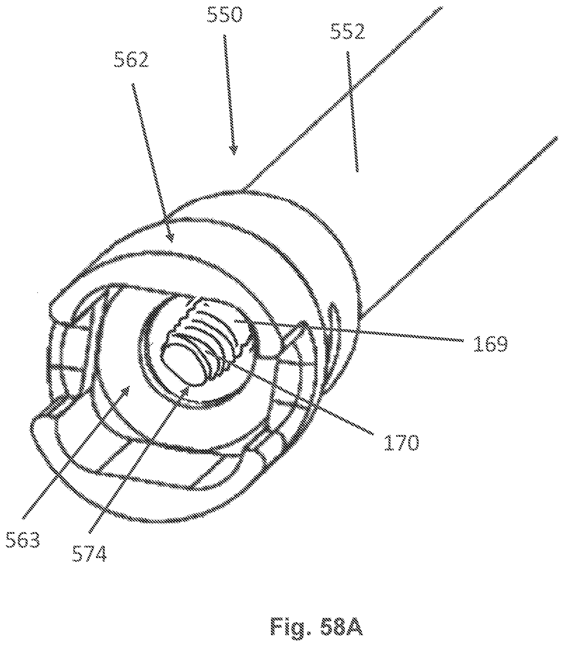

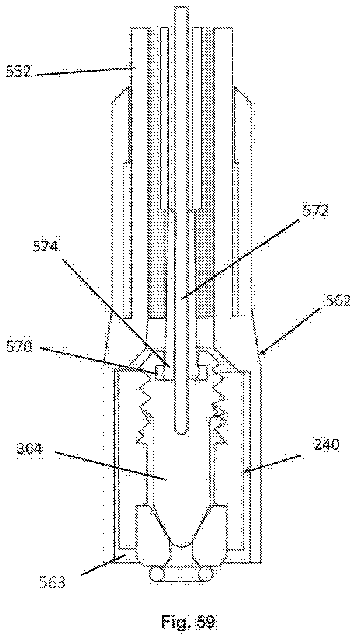

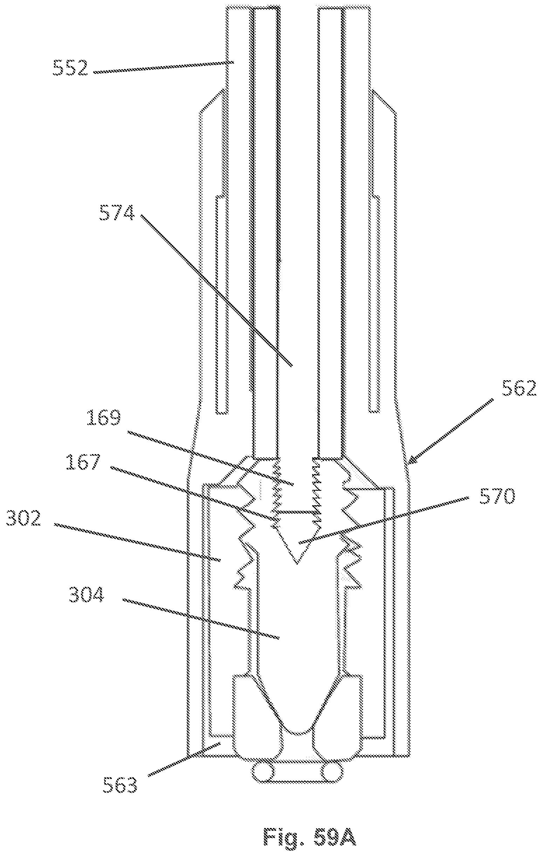

The tool or driver 550 can take a wide variety of different forms. In the example illustrated by Figures 58 and 59, the tool 550 includes a catheter 552, a socket 562, and a drive member 574. The socket 562 and drive member 574 are configured to retain the line locking device 240, while the catheter 552 moves the line locking device 240 along the lines 124, 224 through the patient's vasculature (typically through one or more guide catheters) to the patient's ventricle. The socket 562 includes a recess 562 for receiving the body 302 and is shaped to releasably engage and prevent or inhibit rotation of the body 302 when the threaded insert 304 is rotated. Referring again to Figure 55, a recess 570 for engagement with the drive member 574 is located in the threaded insert 304, opposite the conical surface 408 of the insert 304. The drive member 574 is positionable in the recess 570 and can latch to and disengage from the threaded insert 304.

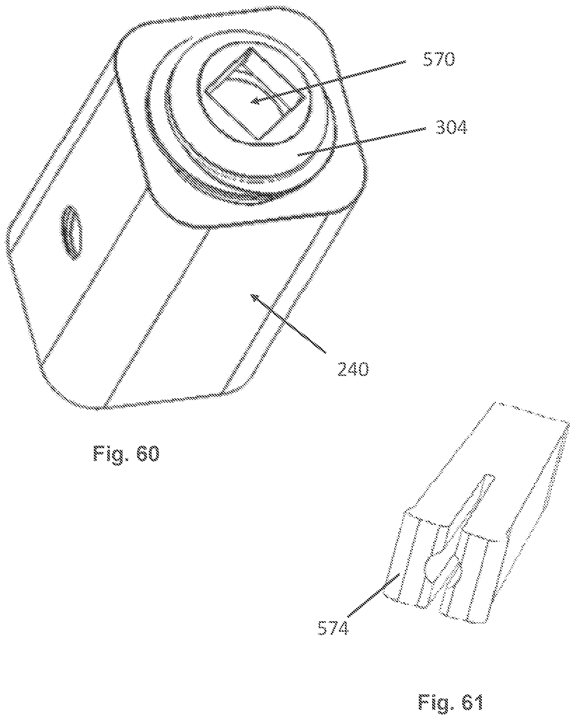

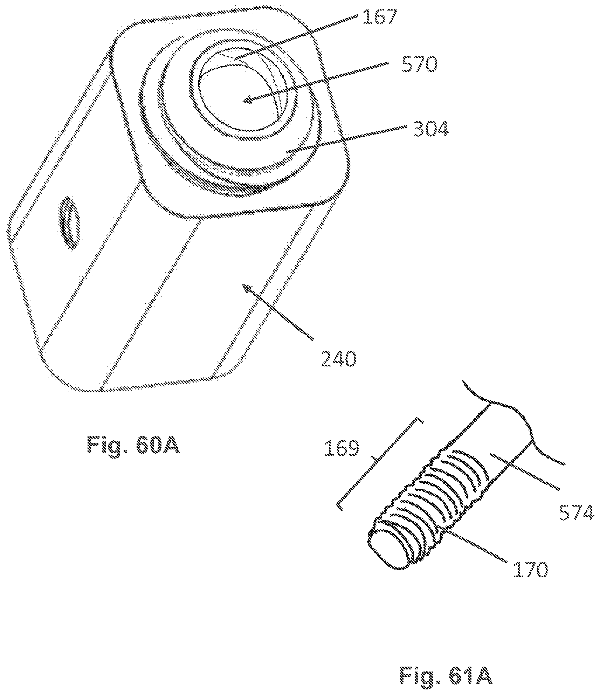

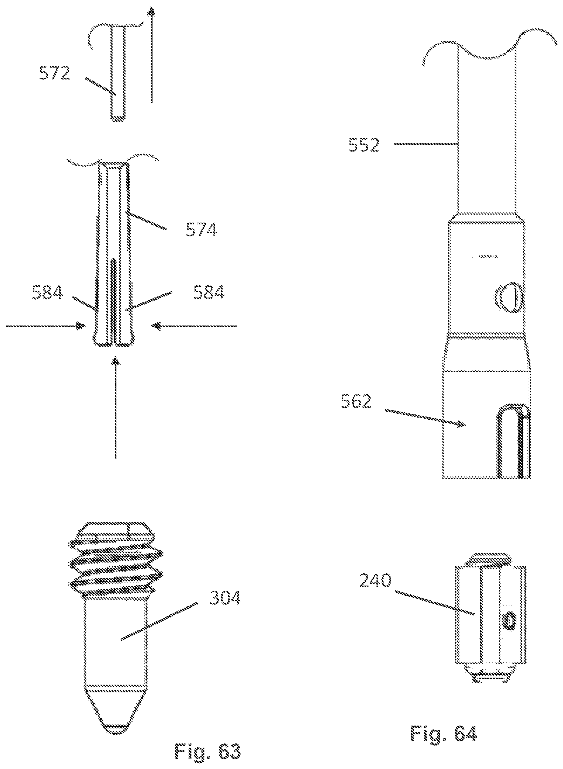

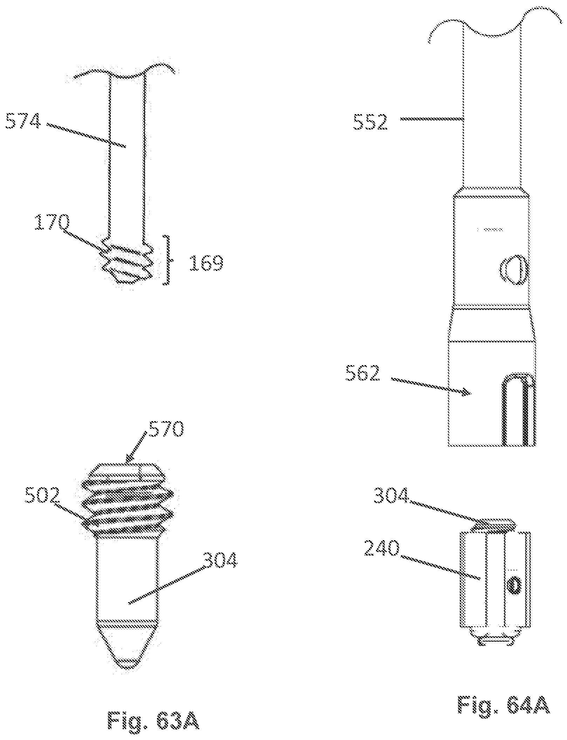

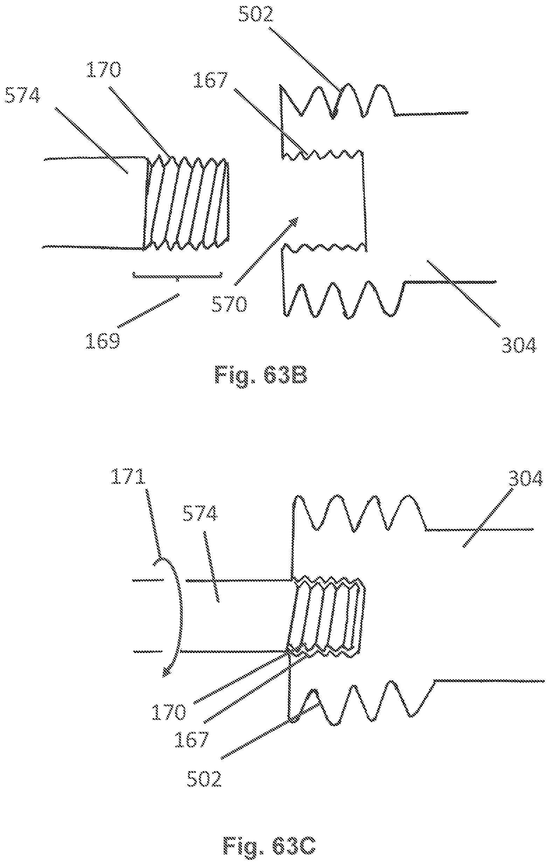

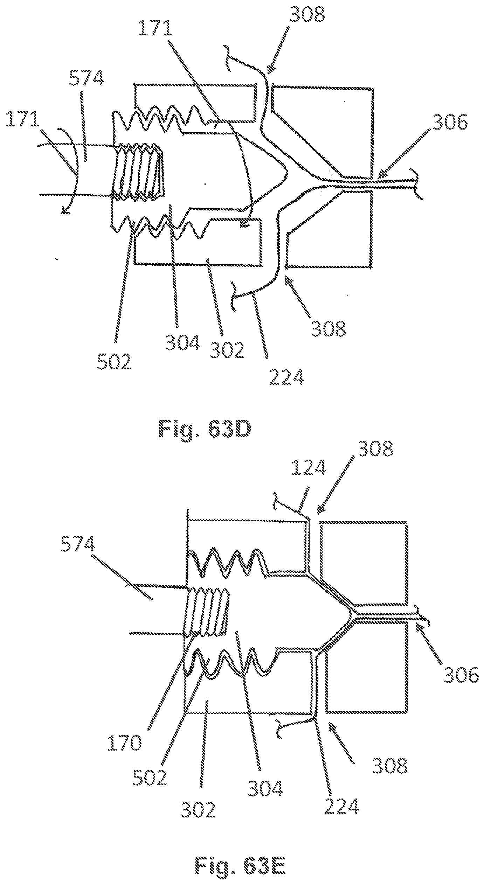

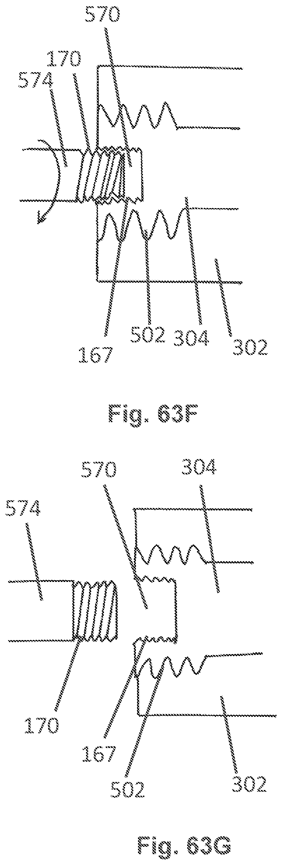

Figure 59 illustrates an operating end of the tool 550 with a line locking device 240 inserted into the recess 563 of the socket 562. In one example, the drive member 574 is moveable between an engaged or expanded condition (Figure 59) and a disengaged or collapsed condition (indicated by arrows in Figure 63). The drive member 574 can be configured to engage and disengage in a wide variety of different forms. In the example illustrated by Figure 59, the driver 574 is positioned in the insert 304 recess 570 before the driver 574 is moved from the disengaged condition to the engaged condition. In the disengaged condition, a retaining rod 572 is not disposed in the end of the drive member. Once in the recess 570, the retaining rod 572 is moved to the position illustrated by Figure 59 and causes the driver 574 to expand to the engaged condition in the recess 570 of the insert 304, and thereby couple the driver 574 to the insert 304 (until the rod 572 is removed). Figure 60 shows a perspective view of the line locking device 240 which shows the recess 570 located in the insert 304. Figure 61 shows another view of the driver 574, without the retaining rod 572.

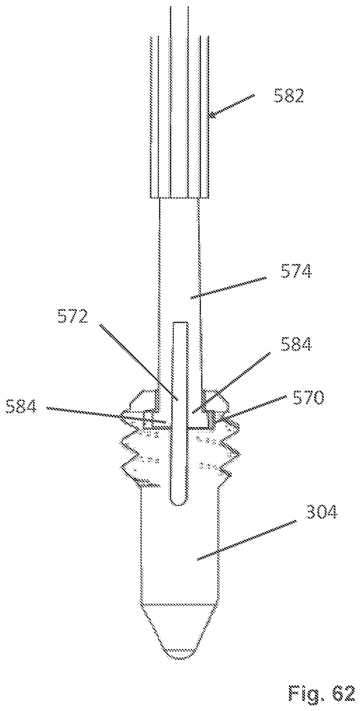

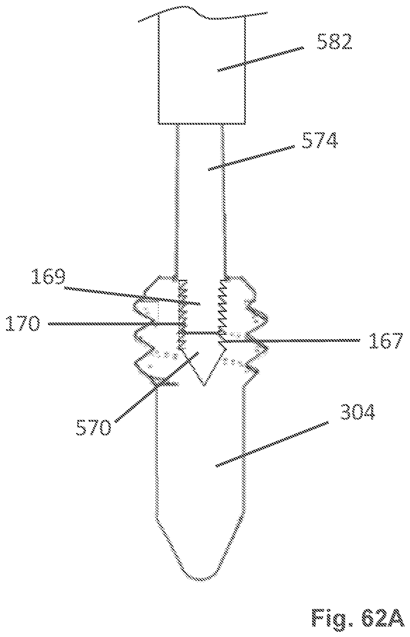

Figure 62 illustrates a view of the insert 304 which is positioned on the drive member 574 with the retaining rod 572 positioned to secure the insert 304 to the engagement device 574. A shaft 582 is attached to the driver 574, which allows for rotation of the driver 574 relative to the catheter 552 and socket 562. The illustrated driver 574 comprises fingers 584 that are forced outward by the retaining rod 572. This causes the fingers 584 to expand into the recess 570 of the insert 304 and secure the insert 304 to the engagement device 574. For clarity, this view omits the remaining components of the tool 550.

As illustrated in Figure 63, when the retaining rod 572 is withdrawn from the insert 304, the fingers 584 retract to the disengaged state. When the fingers retract to the disengaged state, the end of the driver 574 becomes smaller than the recess 570 in the insert 304, thereby releasing the insert 304. For example, the fingers 584 can be made from steel, a shape memory alloy or other resilient material spring toward the disengaged state when the retaining rod 572 is removed. Figure 64 illustrates the socket 562 in proximity to a line locking device 240 that has been released. Also illustrated is a portion of a catheter 552 attached to the socket 562.

Figures 53A, 54A, 55A, 56A, 57F-57J, 58A, 59A, 60A, 61A, 62A, 63A-63G, and 64A illustrate another example of a locking device 240 having a body 302 and a threaded insert 304 for positioning within the body. In this example, as in other examples described herein, the threaded insert can have exterior threads 502 on a proximal exterior portion of the threaded insert 304. The insert 304 can be substantially cylindrical and can have a tapered or conical distal end 408. The insert can be rotated into the body 302 by applying torque to rotate and screw the insert into the body. The exterior insert threads 502 screw into the mating threads 504 of the body 302, and the threads are aligned or mate in a first direction. This first direction can be that which requires the insert to be rotated clockwise to screw the insert into the body, i.e., the threads are right-handed. In another example, the threads can be left-handed.

Referring to Figure 62A, in an example, the insert can have an opening 570 with interior threads 167. A driver 574 (see Figure 59A) having external threads 170 can be rotated into the interior threads 167 of the opening 570 to secure the driver to the insert. This attachment of the driver to the insert can be done before the insert is rotated to attach the insert to the body. The threads of the driver that mate with the interior threads of the body can be aligned or mate in a second direction. In examples where the first direction is clockwise (right-handed), the threads of the second direction can require the driver to be rotated counterclockwise to attach the driver to the insert, i.e., left-handed threads (or vice versa). When the driver 574 is secured to the insert 304, the insert can be rotated into the body by rotation of the driver, described in detail below.

To deploy the implant, the driver 574 is secured to the insert 304. The driver is secured to the insert by rotating it in the second direction. Once the driver is secured to the insert, the driver is rotated in the first direction to rotate the insert into the body. This rotation in the first direction twists the insert into the body 302 of the device 240 so that the insert and body are secured together by being screwed together. Once the threads are secured between the insert and the body, the driver can continue to be rotated in the first direction. Then, the distal end of the driver will unscrew from the insert so that the driver can be removed. In particular, the rotation of the driver in the first direction when secured to the insert twists the insert into the body. Once the insert is fully twisted into the body, the driver which is still turning in the first direction, causes the torsional load to increase until it reaches a torsional preload value. Once the torsional preload value is reached, the insert will stop rotating with the driver. Continued application of the rotational force on the driver will cause the torque to increase past the threshold value, which will cause the distal end of the driver to begin to unscrew from the internal threads of the insert. The torque then drops when the driver begins to rotate with respect to the insert. Continued rotation of the driver in this direction causes the driver to detach from the insert, so that the delivery tools (driver, catheter) can be removed.

The line locking device 240 and the threaded insert 304 for positioning within the body 302 can take a wide variety of different forms. An example of a line locking device 240 is illustrated in Figures 53A and 54A. Figure 53A illustrates an exploded side view of the line locking device 240 which is comprised of a body 302 and a threaded insert 304. The body 302 has an input opening 306 and two outlets 308 (the second outlet is not visible in each of the figures). Figure 54A illustrates an orthogonal view of Figure 53A. The threaded insert 304 can have a threaded opening 570 at the top (proximal) end 168 of the insert 304. The threaded insert can have exterior threads 502 in a first orientation, for example, right-handed threads, which align with the threads 504 in the body. The interior threads 570 of this same insert can be left-handed threads. (The opposite can also be true; the exterior threads 502 can be left-handed with right-handed interior threads 570 in the opening 570.) The driver 574 can be a threaded rod with threads that fit into the opening 570 having left-handed threads.

Figures 55A and 56A illustrate a cross section view of the line locking device 240 of Figures 53A and 54A. As illustrated in Figure 55A, the threaded insert 304 is partially inserted into the body 302. The threaded distal end 169 of driver 574 is secured into the threaded opening 570 of the insert 304 (the threaded distal end 169 is shown smaller than the threaded opening 570 to illustrate bit features. However, in most examples these two features will mate and/or be substantially the same size). A first line 124 and a second line 224 are shown entering the body 302 via the input opening 306 where they pass between a conical-shaped clamping surface 406 of the body 302 and a conical surface 408 of the insert 304. After passing between the two clamping surfaces, the first line 124 exits the line locking device 240 through one outlet 308 and the second line 224 extends through the other outlet 308. The lines 124, 224 can take a wide variety of different forms. For example, the lines can be sutures, wires, cables, chords, bendable rods, or any combination thereof.

Figure 56A illustrates the threaded insert 304 inserted into the body 302 such that the first and second lines (124 and 224) are captured and/or clamped between the conical-shaped clamping surface 406 of the body 302 and the conical surface 408 of the insert 304. This insertion is performed by causing the threaded insert 304 to be rotated such that the threaded insert is drawn into the body 302 by the action of threads 502 formed on the insert and mating threads 504 formed in a body chamber. In Figure 56A, the driver 574 with a threaded distal end 169 remains inserted into the opening 570 of the insert 304.

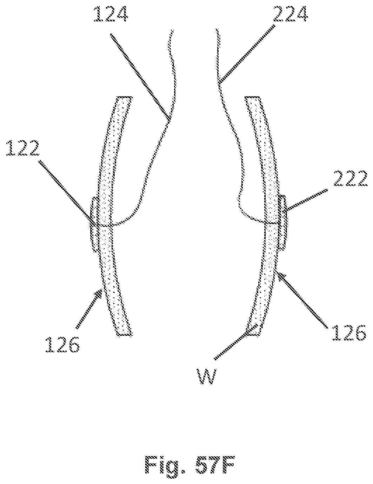

Figures 57F-57J illustrate the clamp in use. As shown, a first anchor 122 and second anchor 222 are positioned on an outside surface 126 of a heart wall W. The first anchor 122 is attached to the first line 124 and the second anchor 222 is attached to the second line 224. The first and second anchors 122, 222 and lines 124, 224 can be deployed in the manner described above.

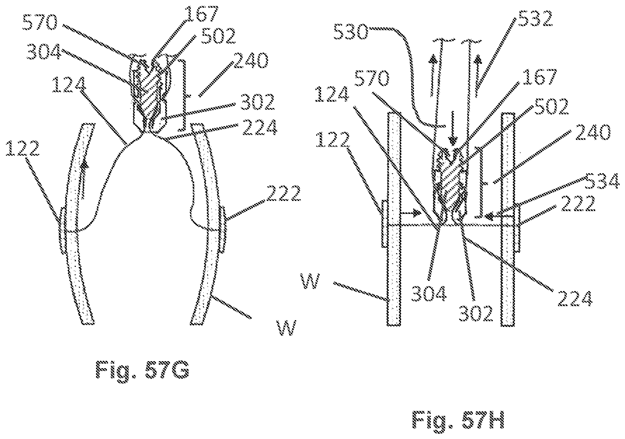

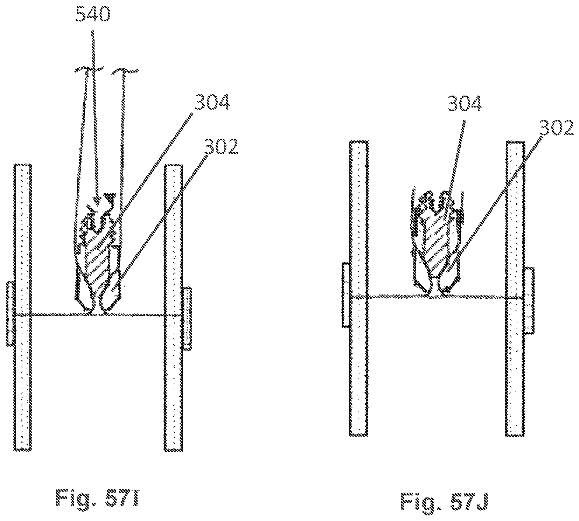

As illustrated in Figure 57G, the first and second lines 124 and 224 are positioned in a line locking device 240, such as the device illustrated by Figure 55A. In Figure 57H, the line locking device 240 is pushed along the lines 124, 224 as indicated by arrow 530 and is positioned and held proximately to the first and second anchors 122 and 222. The first and second lines 124 and 224 are pulled as indicated by arrows 532 through the clamp, causing the first and second anchors to be drawn inward as shown as indicated by arrows 534. This has the effect of drawing the heart walls W inward. Once the heart walls W are in the position desired, the threaded insert 304 is rotated as shown in Figure 57I at 540. This serves to secure the first and second lines 124 and 224 as illustrated in Figure 56A. This securing maintains the tension on the walls W and thereby keeps the heart walls in the remodeled shape. When the first and second lines 124 and 224 are secured they can be cut as illustrated in Figure 57J.

Still referring to Figures 57F-57J, in one example the line locking device 240 is configured to delivered through a catheter (i.e. transcatheter) to a ventricle LV, RV. Tension is applied to the lines 124, 224 while the line locking device is inside the ventricle by pulling on the lines from outside the patient's body (i.e. from the patient's groin, collar bone area, or chest). The line locking device 240 is locked while the line locking device is inside the ventricle by rotating (or otherwise actuating) a line locking tool or driver 550 from outside the patient's body (i.e. also from the patient's groin, collar bone area, or chest). As such, in one example the operation of rotating the threaded insert 304 includes a tool or driver 550 that can position the line locking device 240 and rotate the threaded insert 304 inside a heart.

The tool or driver 550 can take a wide variety of different forms. In the example illustrated by Figures 58A and 59A, the tool 550 includes a catheter 552, a socket 562, and a drive member 574. The socket 562 and drive member 574 are configured to retain the line locking device 240, while the catheter 552 moves the line locking device 240 along the lines 124, 224 through the patient's vasculature (typically through one or more guide catheters) to the patient's ventricle. The socket 562 includes a recess 563 for receiving the body 302 and is shaped to releasably engage and prevent or inhibit rotation of the body 302 when the threaded insert 304 is rotated. Referring again to Figure 55A, a recess 570 for engagement with the drive member 574 is located in the threaded insert 304, opposite the conical surface 408 of the insert 304. The drive member 574 with a threaded distal end 169 can mate to and disengage from the threaded insert 304 by mating threads 170 of the driver with the interior threads 167 of the recess 570 of the insert 304.

Figure 59A illustrates an operating end of the tool 550 with a line locking device 240 inserted into the recess 563 of the socket 562. In one example, the drive member 574 is moveable between an engaged condition (FIG. 59A) and a disengaged condition. The drive member 574 can be configured to engage and disengage in a wide variety of different forms. In the example illustrated by FIG. 59A, the driver 574 is threaded within the threaded opening 570 of the insert 304. Figure 60A shows a perspective view of the line locking device 240 which shows the threaded insert 304 located in the body 302 of the line locking device 240. Figure 61A shows a view of the threaded distal end 169 of the driver 574.

Figure 62A illustrates a view of the insert 304 which is threaded onto the drive member 574 A shaft 582 is attached to the driver 574, which allows for rotation of the driver 574 relative to the catheter 552 and socket 562. The illustrated driver 574 comprises a threaded distal end 169. The threads 170 of the distal end 169 mate with the threads 502 in the recess 540 of the insert 304 to secure the driver to the insert, so that continued rotation of the driver 574 can rotate the insert 304 into the body 302. For clarity, this view omits the remaining components of the tool 550.

As illustrated in Figure 63A, after the insert 304 bottoms out in the body the continued rotation of the driver 574 in the same direction of rotation that was used to secure the insert 304 into the body 302, causes the driver to unscrew from the interior threads 502 of the cavity 570 of insert 304. Figure 64A illustrates the socket 562 in proximity to a line locking device 240 that has been released. Also illustrated is a portion of a catheter 552 attached to the socket 562.

Referring now to Figures 63B and 63C, schematics of insert 304 and the driver 574 engaging with each other are illustrated. In Figure 63B, the insert 304 has an opening 570 with interior threads 167. The driver 574 has a distal end 169 with threads 170 that are oriented to mate with the interior threads 167 of the insert. The threads 170 at the distal end of the driver can be left-handed threads. In the same example, the interior threads 167 of the insert 304 are left-handed threads, and the exterior threads 502 of the insert 304 that mate with the body 302 are right-handed threads. In another example, all the threads are the opposite hand of those just described. In Figure 63C, the driver has been rotated in a first direction, as indicated by arrow 171 such that the threads are torqued to a preload threshold value, and the driver is engaged with the insert.

Referring now to Figures 63D and 63E, schematics of the insert being deployed into the body 302 of the device 240 are illustrated. In Figure 63D, the insert 304 is deployed into the body 302 by rotation of the insert 304 with the driver 574 to rotate the external threads 502. Torque is applied to the driver 574 at the catheter handle (not pictured), and the torsion translates through the system to the insert, as indicated by arrows 171. The torque increases throughout the system as the insert is further rotated into the body and the insert engages the body 302 and/or the lines 124. In Figure 63D, the first line 124 and second line 224 are threaded through the body 302 and can be pulled to remodel the heart walls. In Figure 63E, the insert 304 is fully deployed into the body 302, and the first line 124 and second line 224 are each secured between the conical end 408 of the insert 304 and the body 302. Continued rotation from this position causes the insert 304 to reach or exceed the torsional preload value necessary to secure the lines 124. The insert is about to stop rotating upon application of any more torque from the driver. Further, in this position, the driver is about to start rotating relative to the insert, once its torsional preload within the insert is surpassed.

Referring now to Figures 63F and 63G, schematics of the driver 574 disengaging from the insert 304 are illustrated. In Figure 63F, torque is applied to the driver in the same direction that it was applied in to secure the insert 304 to the body 302. The torque can be applied in the same direction as before because the external insert threads 502 and interior insert threads 167 are threaded in opposite directions. The driver begins to unscrew and therefore disengage from the insert in Figure 63F. In Figure 63G, the driver is fully unthreaded and separated from the insert and can be removed from the implanted device 240 by being pulled in a proximal direction through the catheter.

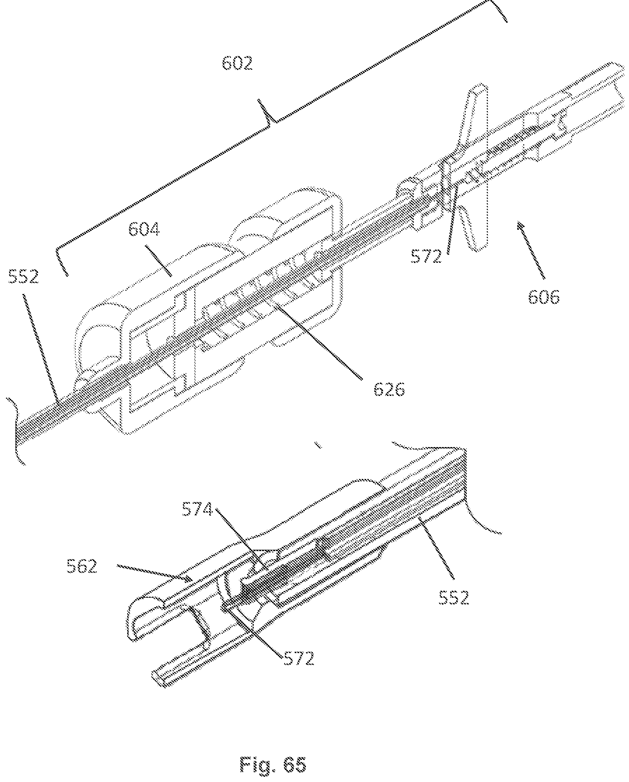

The tool 550 can be operated in a wide variety of different ways to lock the lines 124, 224 with the line lock device 240. Figure 65 illustrates one example of a handle 602 for operating the tool 550 described above. The handle 602 can operate the tool 550 from outside the patient's body, while the socket 562 and driver 574 are in the patient's ventricle. The socket 562 is shown slightly enlarged relative to the handle 602 for clarity. Additionally, for clarity, a portion of the catheter 552 between the handle 602 and the socket 562 is also not shown. One of ordinary skill in the art will understand that the shaft or catheter 552 can be of sufficient length to allow the socket 562 to be positioned inside a patient's heart while allowing the operating handle 602 to be outside the patient's body, with the catheter 552 extending through the patient's vasculature.

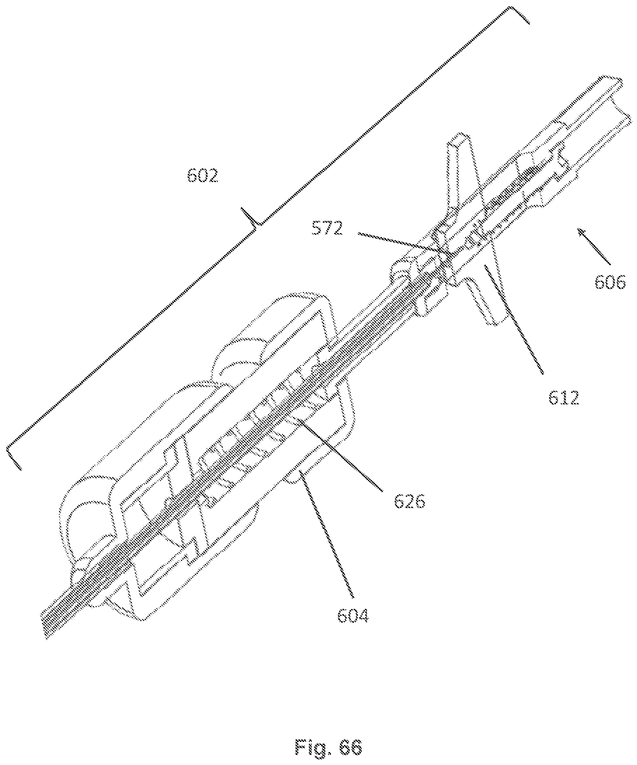

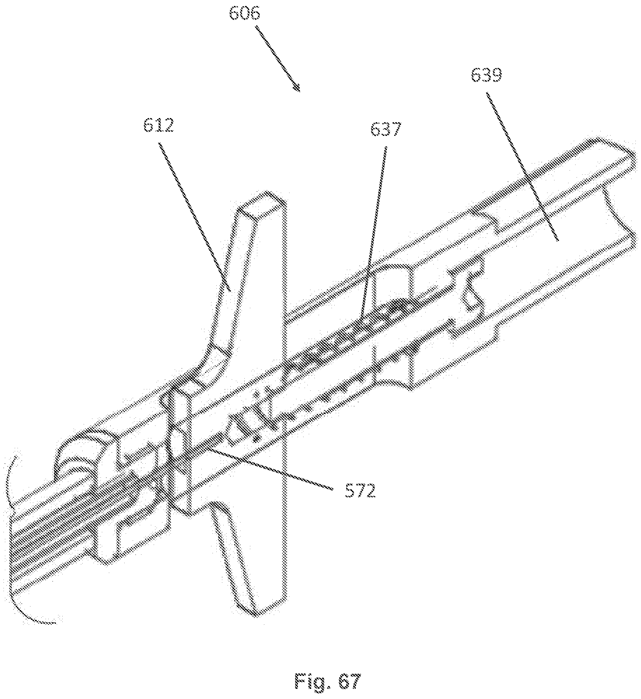

As illustrated, the operating handle 602 comprises a grip 604 and an engagement handle 606. Figure 66 shows a detailed view of the operating handle 602 including the grip 604, the engagement handle 606 and a release lever or control 612. Figure 67 provides an enlarged view of the engagement handle 606. The release lever or control 612 can take a wide variety of different forms. For example, the control 612 can be a lever, a button, a trigger, etc. In the illustrated example, the release control 612 has a wing shape for ease of operation and is connected to an end of the retaining rod 572.

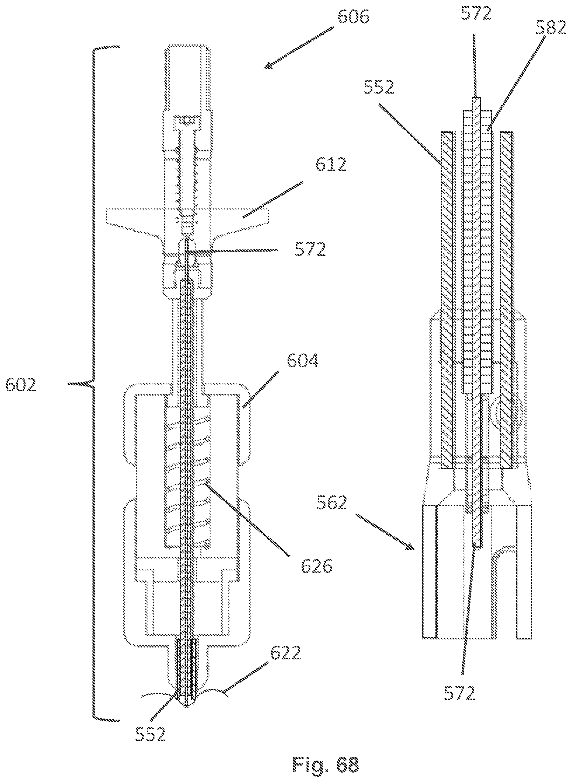

Figure 68 illustrates a cut-away view of the socket 562 and the operating handle 602. As shown the retaining rod 572 passes from the release lever 612 through the catheter 552 to the socket 562. The shaft can pass into the body of a patient around location 622 where it can be threaded through a vein or artery to position the line locking device 240. As is illustrated, an inner driver shaft 582 passes through the catheter 552 from the engagement handle 606 to an area near the socket 562. As will be illustrated, this shaft 582 functions to move the line locking device 240 further into the socket 562 and to rotate the drive member 574. This takes place when a user pushes the engagement handle 606 relative to the grip 604 and rotates the grip. A spring 626 positioned within the grip 604 resists this pushing motion and rotational movement and biases the engagement handle 606 and the connected drive member 574 toward a retracted position.

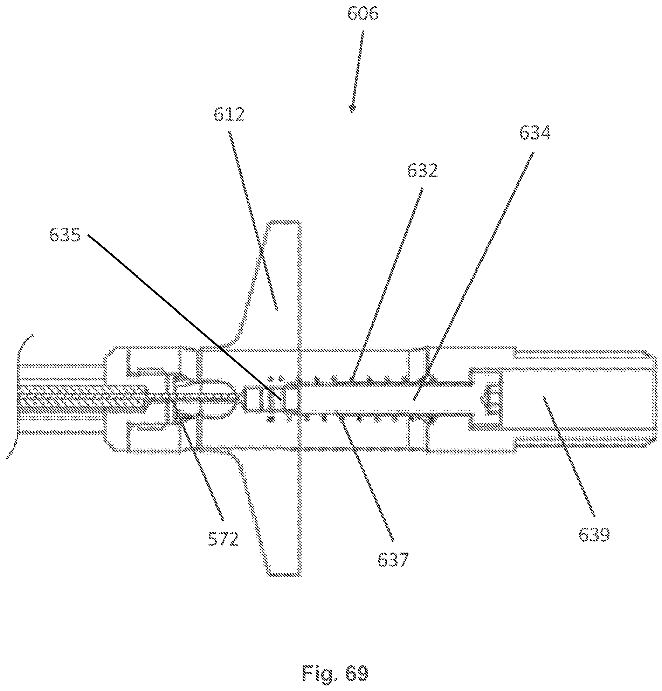

As illustrated in Figure 69, a second spring 632 positions the release control 612 such that the retaining rod extends from the engagement device 574 (not shown). This position secures the line locking device 240 to the engagement device 574 located at the end of the inner shaft 582 opposite the engagement handle 606. In an example, a positioning shaft 634 serves to keep the release lever 612 aligned as the release lever moves between the engaged and disengaged positions. The positioning shaft 634 is connected to the release control 612 at an end 635 and slides within bores 637, 639.

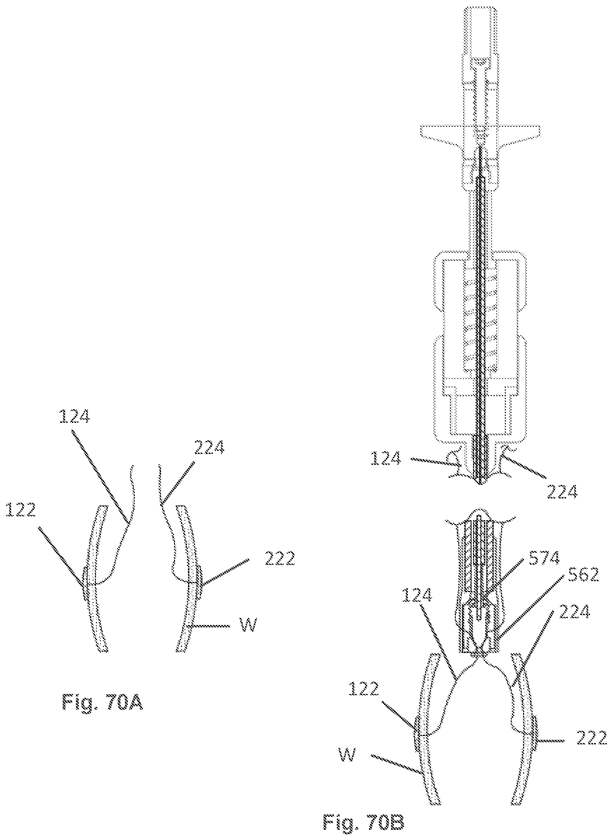

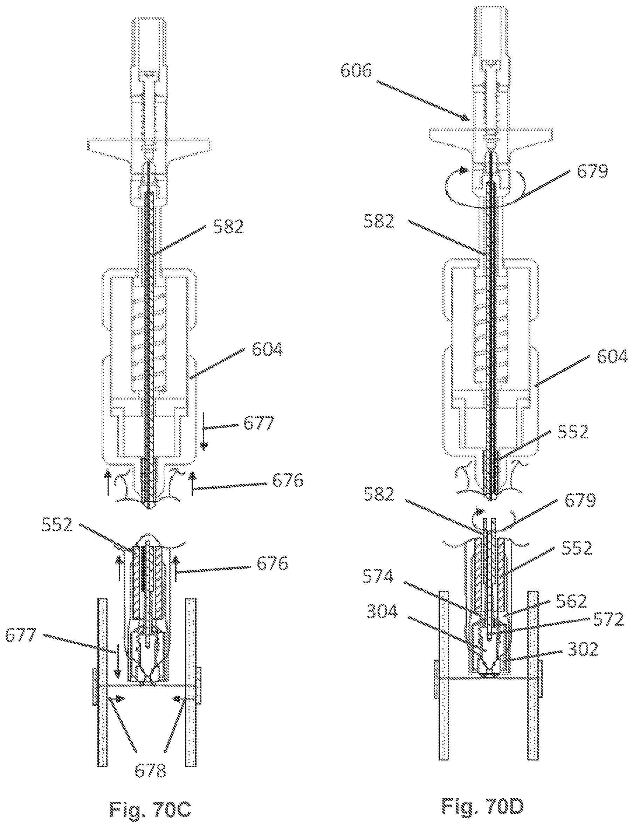

Referring to Figure 70A, in an example, during use, the first anchor 122 and second anchor 222 are positioned on an outer surface of the heart wall W with a first line 124 and a second line 224 attached and threaded thru the heart wall W. The lines (124 and 224) are threaded through the line locking device 240 as illustrated in Figure 55. This threading through the locking device 240 is done external to the patient's body. The line locking device 240 is secured inside the socket 562 to the driver 574 as shown in Figure 70B. The socket 562 is inserted into a patient and threaded through the vein or artery through a guide catheter as the lines (124 and 224) are held in place outside the patient. The line locking device 240 is positioned adjacent to the first and second anchor (122 and 222) using the socket 562. Referring to Figure 70C, when this positioning is done, the first and second lines (124 and 224) are pulled as indicated by arrows 676 while the socket is maintained in position or advanced as indicated by arrows 677. This pulling 676 of the lines 124, 224 and maintaining or advancing 677 of the line locking device 240 causes the first anchor 122 and second anchor 222 to be drawn inward 678 as first illustrated in Figure 70C,

After the first anchor 122 and second anchor 222 are drawn inward 678, the line locking device 240 is secured as illustrated and described in Figures 56 and 57D. This is performed by rotating as indicated by arrow 679 the engagement handle 606 relative to the grip 604. This rotation causes the shaft 582 to rotate 574 relative to the catheter 552, which in turn causes the driver 574 to rotate relative to the socket 562. The rotation of the driver 574 relative to the socket 562 causes the threaded insert 304 to rotate and axially advance in the body 302. As such, the engagement threaded insert 304 and the body 302 secure the first and second lines (124 and 224) as illustrated in Figure 56.

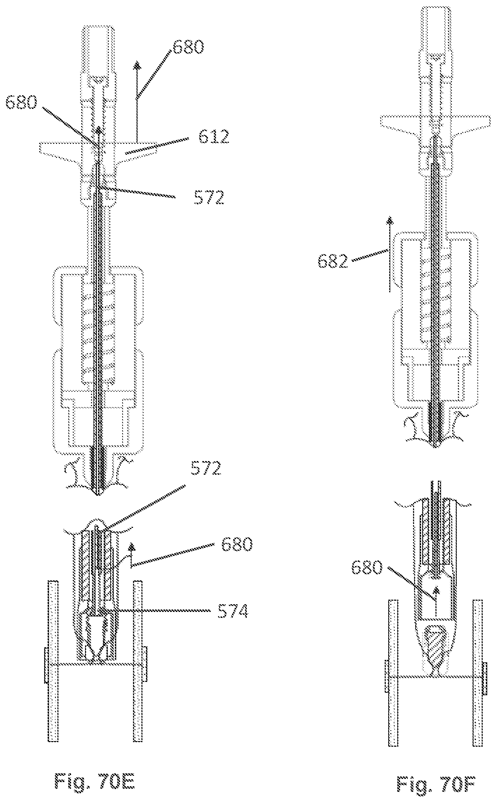

Referring to Figure 70E, after the first and second lines (124 and 224) are secured by the line locking device 240, the engagement device 574 is released from the threaded insert 304 portion of the line locking device 240 by retracting the retaining rod 572 by pulling the release control 612 rearward as illustrated by arrow 680. This rearward pulling pulls 680 the driver shaft out of the end of the driver 574. This allows the driver 574 to spring back to its retracted state, which is smaller than the recess 570 (See Figure 60), releasing the driver 574 from the insert 304.

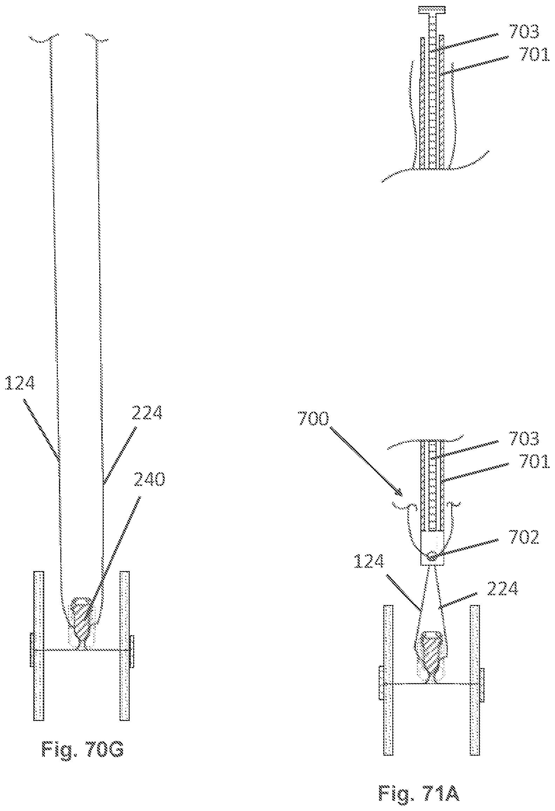

Referring to Figure 70F, after the driver 574 is released from the line locking device 240, the tool 550 is withdrawn from the patient as indicated by arrows 682. The line locking device 240 and first and second lines (124 and 224) remain in the patient as illustrated by Figure 70G.

The first and second lines (124 and 224) can be trimmed at the line locking device 240 using a trimming tool 700. The trimming tool can take a wide variety of different forms. An example of such a trimming tool 700 is illustrated in Figure 71A. The trimming tool 700 includes an outside component or sleeve 701 and an inside component or plunger 703 that are slidably or telescopically coupled together. As illustrated, the first and second lines 124 and 224 are threaded through an opening 702 in the trimming tool 700. For example, the lines 124, 224 can be threaded through the opening 702 outside the patients' body.

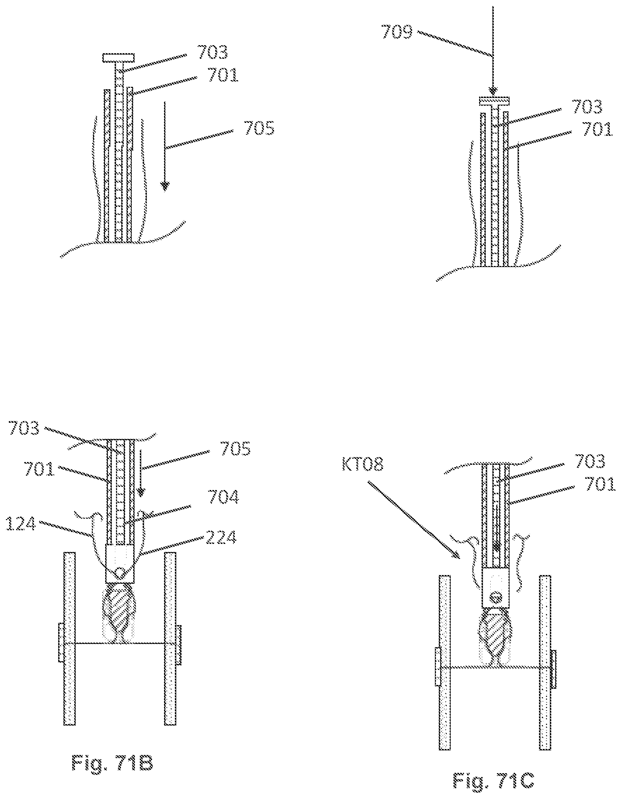

The trimming tool 700 is advanced 705 through a guide catheter to position a cutting end 704 of the tool at the line locking device 240 as illustrated in Figure 71B. In an example, the lines 124, 224 are held in place outside the patient's body as the cutting end 704 is advanced into place.

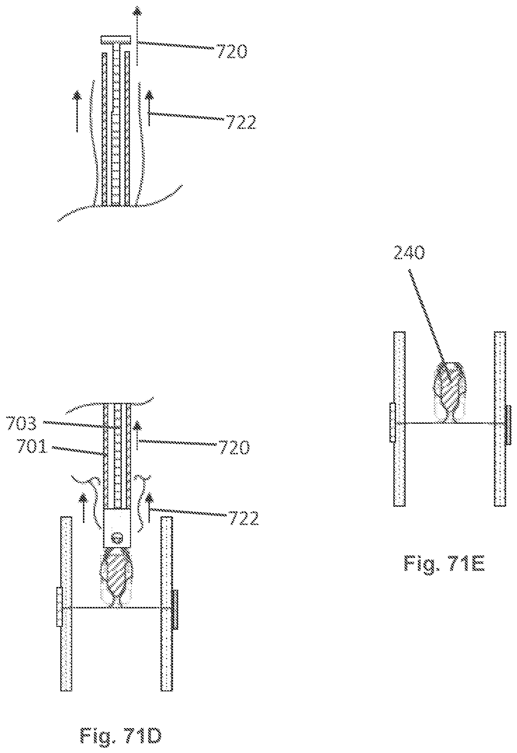

Referring to Figure 71C, in an example, a plunger or inner shaft 703 is depressed as indicated by arrow 709, causing the first and second lines (124 and 224) to be trimmed. Referring to Figure 71D, after the lines 124, 224 are cut, the trimming tool 700 and first and second lines 124 and 224 are withdrawn from the patient as indicated by arrows 720, 722 respectively. The result is the line locking device 240 remaining in place as shown in Figure 71E.

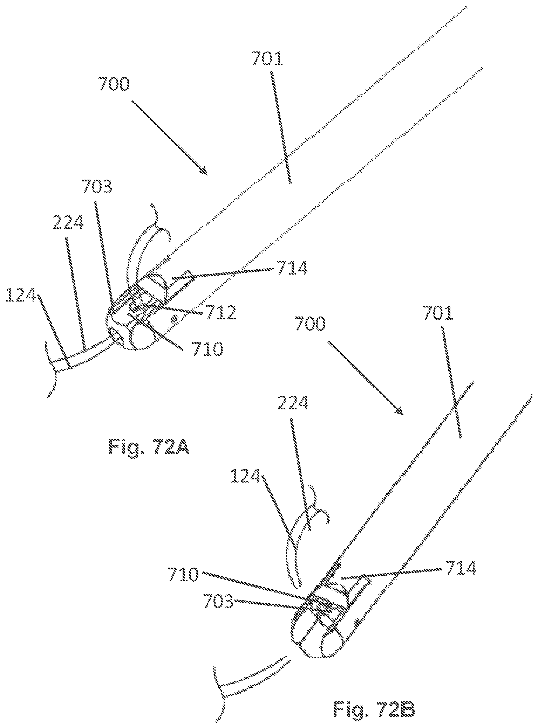

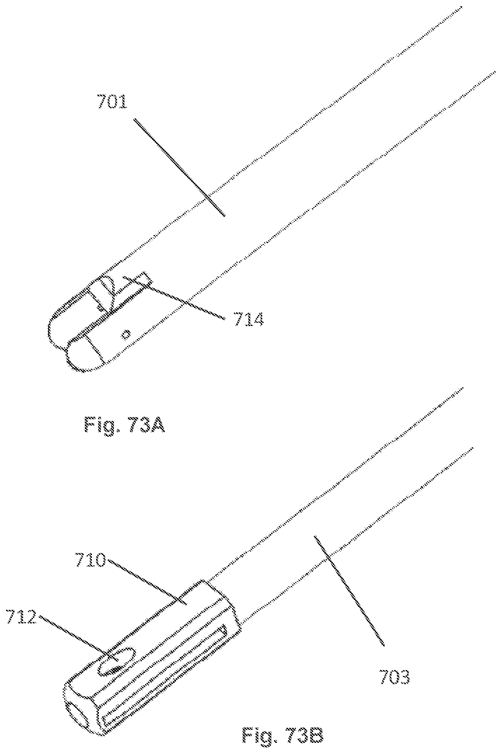

A more specific example of a trimming tool 700 is illustrated in Figures 72A and 72B. The trimming tool includes an outer sleeve 701 and an inner shaft 703. The outer shaft 701 is shown separately in Figure 73A and the inner shaft 703 of the trimming tool 700 is illustrated in Figure 73B. The inner shaft 703 includes a head 710 with a passage 712 that the lines 124, 224 can be routed through. For example, the lines 124, 224 can be routed through the passage 712 outside the patient's body. The inner shaft 703 is slidably disposed in the outer sleeve 701, such that the head 710 pulls the lines 124, 224 against a blade 714 of the outer sleeve to cut the lines 124, 224.

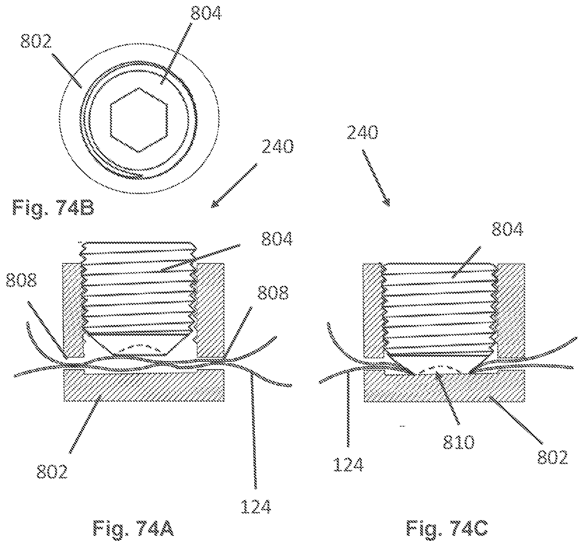

The line securing device 240 can take a wide variety of different forms. Figures 74A-93B illustrate a variety of non-limiting examples of line securing devices 240 that can be used in the heart wall remodeling techniques described herein. An example of a line locking device 240 is illustrated in Figures 74A and 74B. As illustrated, the line locking device 240 is comprised of a housing 802 and a threaded insert 804. Figure 74B shows a view of the line locking device 240 looking at the outward end of the threaded insert 804. As shown in Figure 74A, when the threaded insert 804 is partially inserted into the housing 802, one or more lines 124 can be threaded through a pair of openings 808. With the lines 124 threaded as shown in Figure 74A, the threaded insert 804 can be turned such that the treaded insert is drawn into the housing 802, causing the lines to be trapped between the threaded insert 804 and the housing 802 as shown in Figure 74C. As illustrated in Figure 74C, the threaded insert 804 can be formed with a concave surface 810 or a flat surface located at the inward end of the threaded insert 804. In certain example, this concave surface can result in a more secure clamping action because the configuration avoids the small area of contact that might occur if the insert 804 were provided with a convex shaped pointed or rounded end.

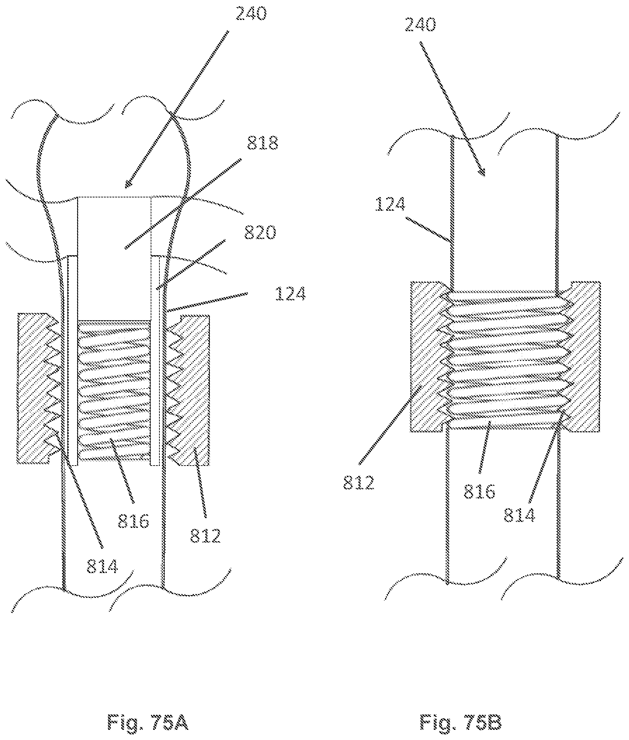

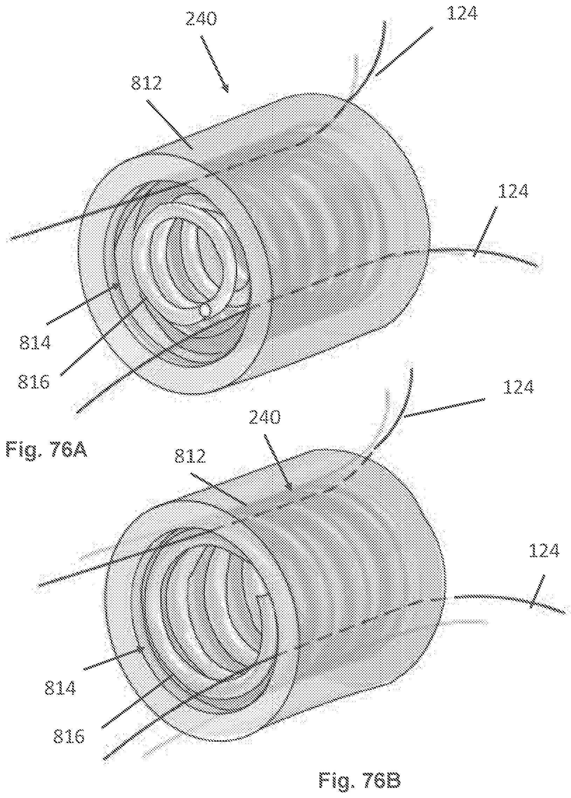

Figures 75A, 75B, 76A and 76B illustrate another example of a line locking device 240. In this example, an outer housing 812 has a threaded or serrated opening 814 passing longitudinally through the outer housing 812. Lines 124 are caused to pass through the threaded opening 814 and a spring 816 is disposed within the threaded opening 814. The spring 816 can be formed from a shape memory metal such as, without limitation, Nickel titanium (Nitinol) or another resilient material, such as steel, etc. The resilient characteristic of the spring 816 cause it to expand to engage the threads in the threaded opening 814, trapping the lines between the threads of the threaded opening 814 and the spring 816.

Figures 75A and 75B illustrate one example of a system for deploying the line locking device illustrated by Figures 76A and 76B. In the illustrated example, the spring 816 is compressed inside a catheter 820. The catheter 820 is positioned inside the outer housing 812. The lines 124 are threaded between the catheter 820 and the body 812. The spring 816 is released from the catheter 820 by retracting the catheter 820 while a pusher 818, such as a rod or a catheter, holds the axial position of the spring 816. Once the spring 816 is released, the spring 816 expands outward to the position illustrated by Figure 75B. In the position illustrated by Figure 75B, the lines are captured between the spring 816 and the threaded or serrated opening 814 of the housing 812.

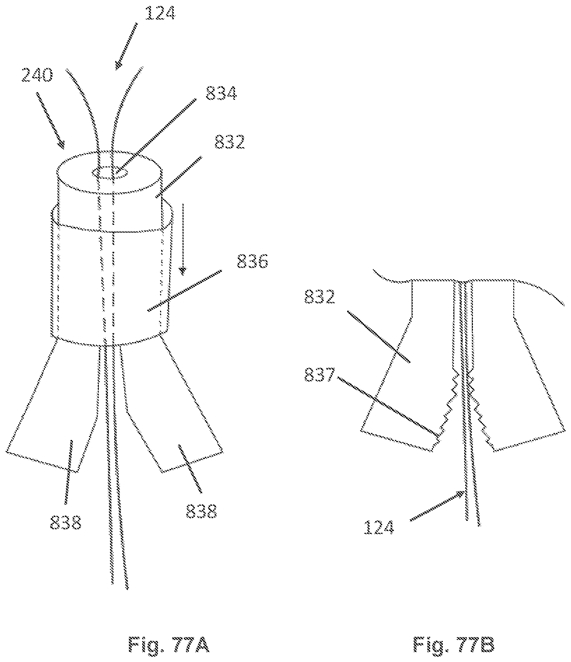

Figures 77A and 77B illustrate another example of a line locking device 240. In this example, lines are passed through a clamp member 832 that has an opening 834 passing longitudinally through the clamp member 832. When it is desired to clamp the lines 124, a sleeve 836 is moved along the clamp member 832 to cause jaws 838 formed in the clamp member 832 to close on and secure the lines 124. As illustrated in Figure 77B, teeth or serrations 837 can be formed in the clamp member 832 to more securely engage the lines 124. The sleeve 836 can be maintained in the closed position on the clamp member 832 in a wide variety of different ways. For example, the sleeve 836 can have an interference fit with the clamp member 832, the sleeve 836 and the clamp member 832 can threadedly engage one another, etc.

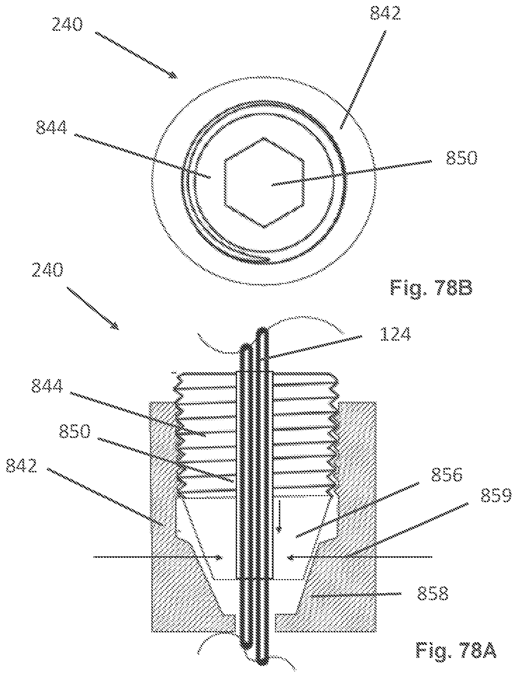

An example of a line locking device 240 is illustrated in Figures 78A and 78B. As shown in Figure 78A, the line locking device 240 is comprised of a housing 842 and a threaded insert 844. The threaded insert 844 is formed with a passage 850 that extends longitudinally through the insert. In use, lines 124 are passed through the opening 850 as illustrated. The threaded insert 844 is rotated such that the threads formed in the insert engage with threads formed in the housing 842. As the threaded insert 844 is drawn into the housing 842, a tapered end 856 of the threaded insert 844 engages a taper 858 formed in the housing. This engagement causes the tapered end of the insert to deform as indicated by arrows 859 and close a portion of the passage onto the lines 124, thus clamping the lines 124 in position relative to the line locking device 240. Figure 78B shows a top view of the line locking device 240 that illustrates the end of the threaded insert 844 opposite the tapered end 856. As illustrated, an example has a hex shaped opening 850 in the threaded insert 844 that facilitates the use of a tool (not illustrated) to rotate the threaded insert 844 relative to the housing 842, thus clamping the lines 124 as described herein.

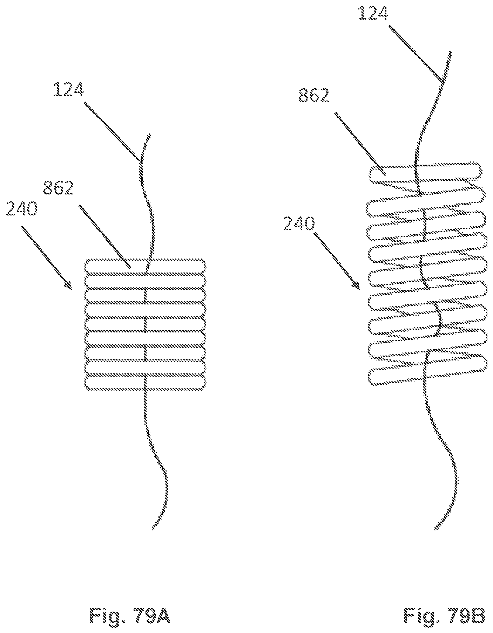

Another example of a line locking device 240 is illustrated in Figures 79A and 79B. In this example, lines 124 are woven through a spring 862 as illustrated in Figure 79b. The spring 862 can be formed from a resilient material, such as steel, a shape memory metal such as, without limitation, Nickel titanium (Nitinol), etc. As shown, the spring 862 will return to its tightly coiled state as illustrated in Figure 79A. This serves to trap the lines 124 between the coils of the spring 862, locking the lines 124 in place.

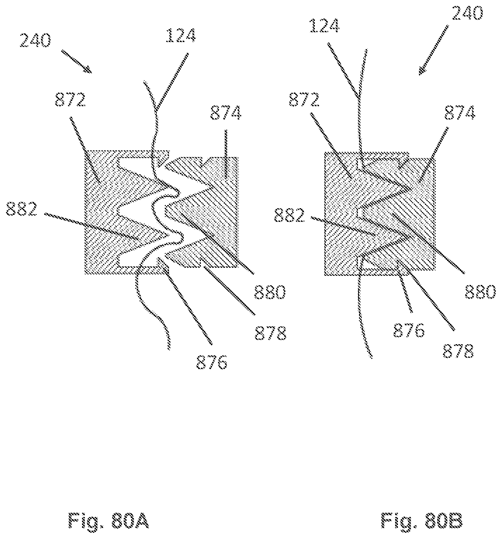

Another example of a line locking device 240 is shown in Figures 80A and 80B. The line locking device 240 is comprised of two parts, a receiver 872 and a locking insert 874. As shown, lines 124 are placed between the receiver 872 and locking insert 874 and the two are brought together as shown in Figure 80B. The receiver 872 and the locking insert can be coupled together in a wide variety of different ways to connect the lines and lock them in place. In the example illustrated by Figures 80A and 80B, the receiver has locking hooks 876 that engage locking grooves 878 in the locking insert 874. When the locking hooks 876 are engaged in the locking grooves 878, the teeth 880 of the locking insert 874, interweave with the teeth 882 of the receiver 872. These two sets of teeth 880 and 882 capture the line 124, locking it in place with respect to the line locking device 240.

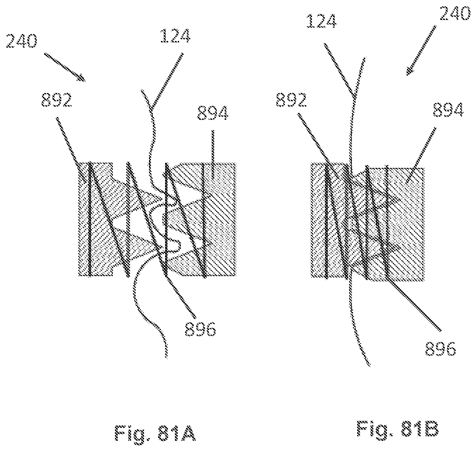

Another example of a line locking device 240 is shown in Figures 81A and 81B. As shown, the device has a receiver 892 and a locking insert 894. Lines 124 are placed between the receiver 892 and locking insert 894 and the two are brought together as shown in Figure 81B. A spring 896 draws the receiver 892 and locking insert 894 together and holds them in this state, locking the line 124 as shown in Figure 81B.

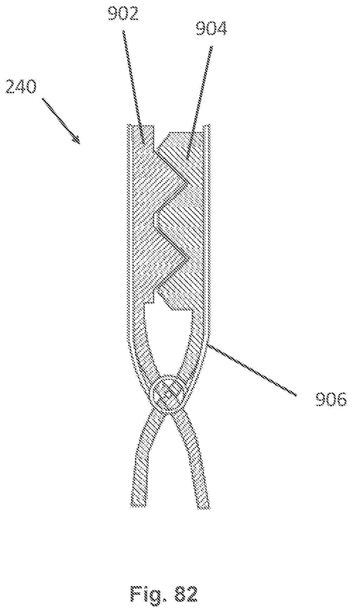

As shown in Figure 82, an example of a line locking device 240 comprises a pair of toothed jaws 902 and 904 that are held together by a spring 906. Closing force exerted by the spring 906 causes the toothed jaws 902 and 904 to lock a line (not shown) in place relative to the clamp 240.

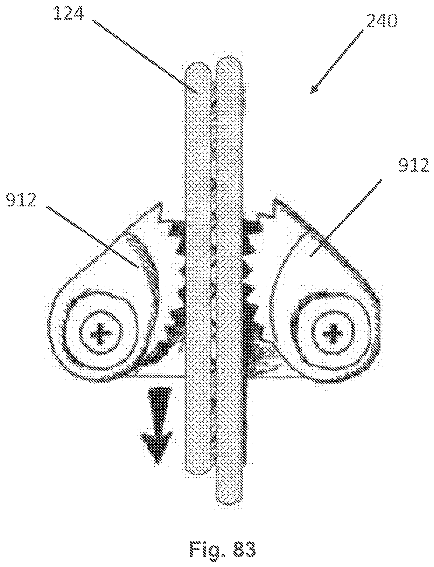

In the example of the line locking device 240 illustrated in Figure 83, a pair of toothed cams 912 are positioned such that movement of a pair of lines 124 results in the rotation of the cams 912. Depending on the direction the lines are pulled and the corresponding direction the cams 912 rotate, the locking device 240 either allows the lines to move through the locking device or prevents or inhibits the lines from moving through the locking device. For example, when the lines 124 are pulled relative to the locking device 240 in the direction indicated by the arrow, the cams 912 clamp the lines 124 such that the lines 124 are locked in place relative to the line locking device 240. The tension achieved by a pair of anchors 122 in the examples described above can cause the force indicated by the arrow, and thereby cause the locking device 240 to lock the position of the lines 124. In some examples, the cams can optionally be spring-loaded such that the cams 912 are biased against the lines 124.

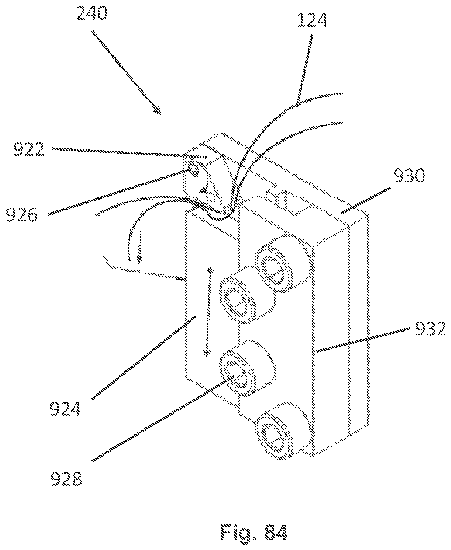

In another example, a line locking device 240 is formed from a movable plate and cleat that are used to secure two or more lines. As illustrated in Figure 84, a line 124 is threaded between a cleat 922 and an adjustable plate 924. The cleat 922 rotates about a pin 926 to secure the lines 124 between the cleat 922 and the plate 924. The plate 924 can be moved closer or farther from the cleat 922 depending upon the thickness and number of lines 124. In the illustrated example, the plate 924 can be secured in position using one or more fasteners 928 which secure the plate 924 between a backing plate 930 and a clamp plate 932.

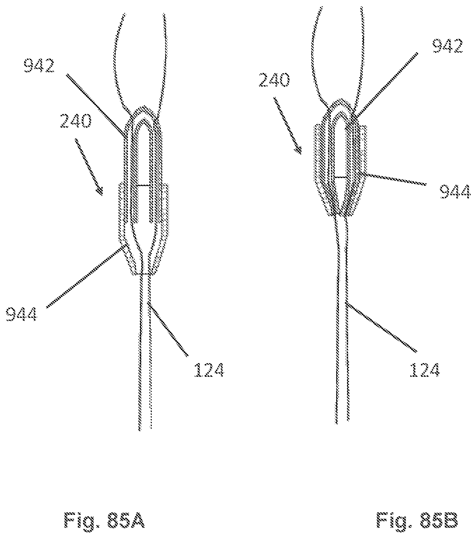

In another example, a line locking device 240, illustrated in Figures 85A and 85B, secures a line 124 that is passed through an inner component 942 of the line locking device 240. The inner component 942 is inserted into an outer component 944 which serves to compress the inner component 942 as illustrated in Figure 85B, such that the lines 124 are secured by the compression of the inner component 942.

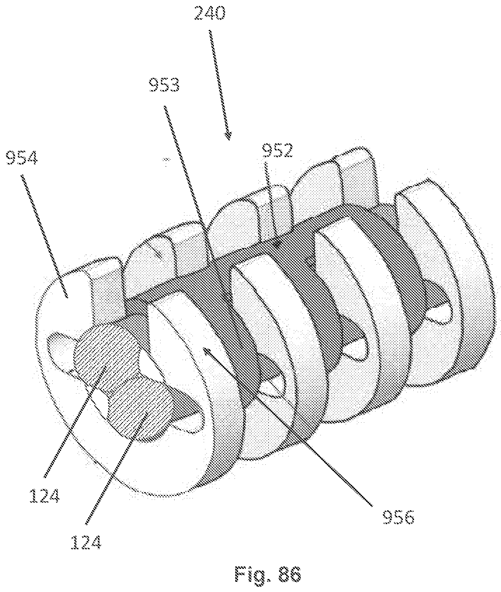

An example of a line locking device 240 is shown in Figure 86. Such a line locking device could be made from a resilient material, such as steel or a shape memory metal such as, without limitation, Nickel titanium (Nitinol), etc. As shown, a line 124 is passed between an inner lock component 952 and a memory metal outer lock component 954. The inner lock housing 952 has fingers 953 that fit within slots between fingers 956 of the outer lock housing 954 The two parts 952, 954 are brought together to snap onto the lines 124. The fingers 953, 956 mesh together to hold the lines 124 in place in the line locking device 240.

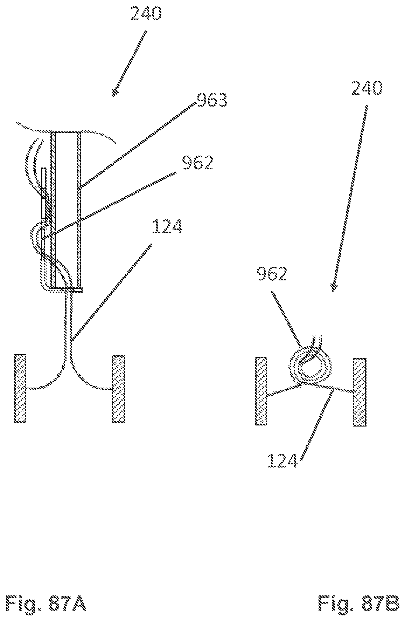

In another example of a line locking device 240 is illustrated in Figures 87A and 87B that comprises a material strap 962. In Figure 87A, the strap 962 is held in an elongated condition by a holder 963. A line 124 is weaved in and out of an elongated strap 962 and easily slides relative to the strap, when the strap is in the elongated position. The strap can be made of a shape memory material such as, without limitation, Nickel titanium (Nitinol) and shape set to the coiled shape (or other line constraining shape) illustrated by Figure 87B. When the locking strap 962 is released from the holder, it returns to its set position as a spiral shape. The spiral shape causes the line to be wrapped up with the strap locked in place as illustrated in Figure 87B.

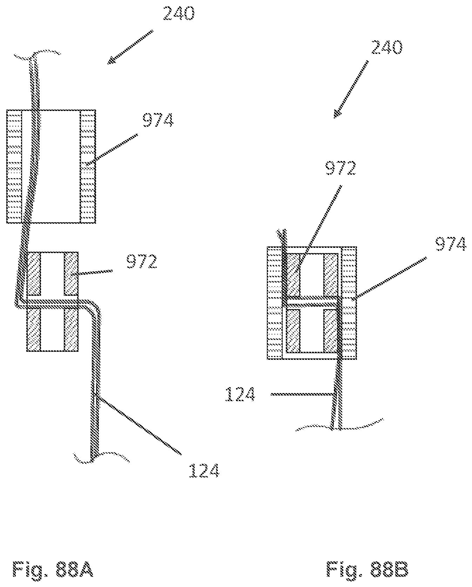

In another example of a line locking device 240, as illustrated in Figures 88A and 88B, a line 124 is passed through an inner clamp barrel 972. The inner clamp barrel 972 is placed inside an outer clamp barrel 974 with an inner diameter slightly larger than the outer diameter of the inner clamp barrel 972. The result is that the line 124 is wedged between the inner barrel 972 and outer barrel 974, locking the line 124 in place relative to the line locking device 240.

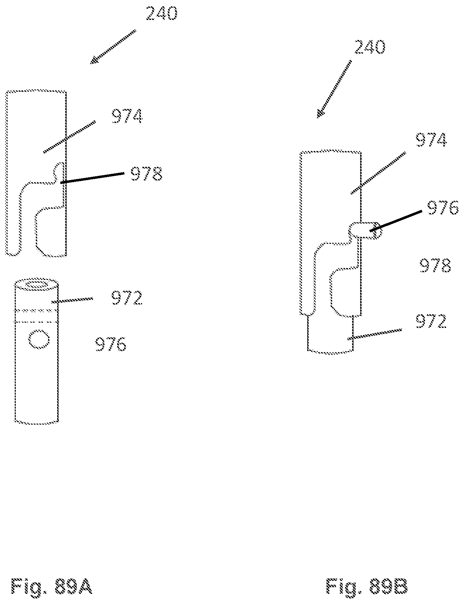

Figures 89A and 89B illustrate an example of a line locking device 240 that is similar to the device of Figures 88A and 88B. In the example illustrated by Figures 88A and 88B, the inner clamp barrel 972 includes a pin 976 and the outer clamp barrel 974 includes a slot 978. To lock the lines 124, the pin 976 is placed in the slot 978, inner clamp barrel 972 is advanced into the outer clamp barrel 974, and the inner clamp barrel 972 is rotated in the outer barrel 974 to move the pin circumferentially along the slot. As a result, the inner clamp barrel 972 has to be rotated relative to the outer clamp barrel 974 before the inner clamp barrel can be withdrawn from the outer clamp barrel. As such, the pin 976 and slot 978 lock the device 240 to prevent or inhibit unintentional release of the lines 124.

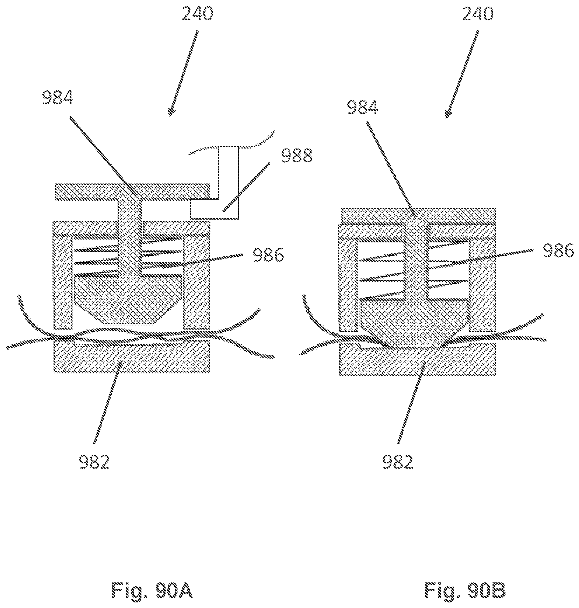

Figures 90A and 90B shown another example of a line locking device 240. As shown, a line 124 is passed through an opening in an outer housing 982. A plunger 984 and spring 986 are deposed inside the outer housing 982. As shown in Figure 90A, during installation a spacer 988 is positioned such that the plunger 984 is held in a retracted state. The spacer 988 can be a part of an installation tool (not shown) that guides the line locking device 240 into position. The retracted state allows the line 124 to move freely relative to the line locking device 240. When the spacer 988 is removed, the plunger 984 is pressed against the outer housing 982 by the spring 986, trapping the lines 124 in place.

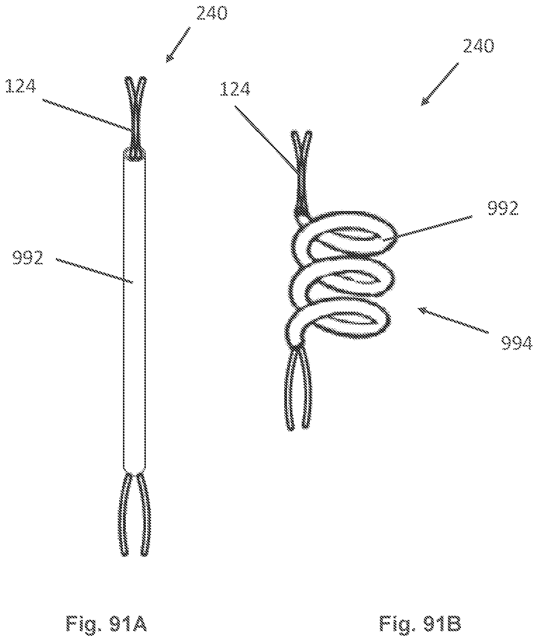

The properties of a shape memory metal such as, without limitation, Nickel titanium (Nitinol) can be used in an example of a line locking device. One such example of a line locking device 240 is illustrated in Figures 91A and 91B. As shown, lines 124 are threaded through a tube 992 formed from a shape memory metal. When it is desired to clamp the lines 124 in place, the memory metal of the tube 992 is allowed to return a shape set coiled state 994 as illustrated in Figure 91B. This forces the lines 124 into a circuitous path, holding them in place.

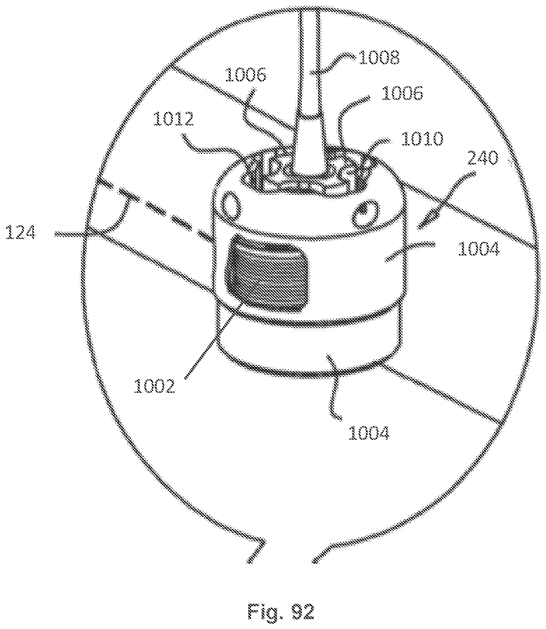

**In** another example, a line locking device 240 uses a spool to retract a line, locking it in place. Such a line locking device 240 is illustrated in Figure 92. As is illustrated, a spool 1002 is used to draw a line 124 into an outer housing 1004. The line locking device 240 comprises an engagement hub 1006 that is affixed to the spool 1002. The engagement hub 1006 can be turned by an installation tool 1008. The engagement hub 1006 is formed with teeth 1010 that engage pawls 1012 that allow the engagement hub 1006 to turn only in one direction such that the spool 1002 tightens the line 124 and holds it in the tightened state.

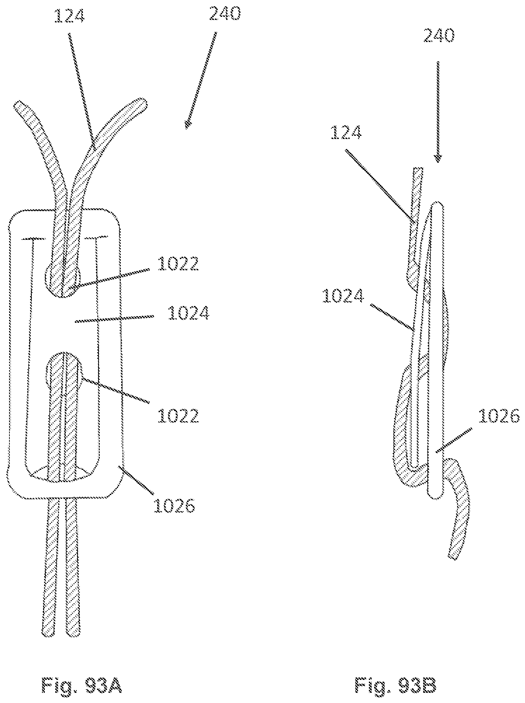

An example of a one-piece line locking device 240 is illustrated in Figures 93A and 93B. As shown, lines 124 are threaded through openings 1022 formed in the line locking device 240. A tab portion 1024 is formed in the line locking device 240. The tab portion 1024 captures the lines 124 between the tab portion 1024 and a base portion 1026. This capture, together with the circuitous path that the line 124 takes through the openings 1022, serves to lock the line 124 in place relative to the clamp 240. **In** certain examples, a line locking device 240 can be formed from a resilient material, such as a shape memory metal such as, without limitation, Nickel titanium (Nitinol), etc.

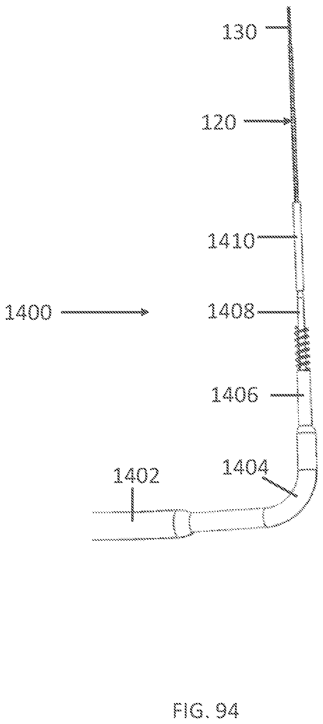

Referring to Figures 94-111, an example of a device 120 for remodeling the shape of a heart wall and a system 1400 and method for delivering the device 120 against an exterior surface of the heart H is illustrated. The system 1400 and the device 120 can be configured in a variety of ways.

**In** the illustrated example, the system 1400 includes a guide sheath 1402, a steerable catheter 1404, a delivery catheter 1406, a pusher 1408, a hemostatic plug 1410, a piercing device 130, and the device 120.

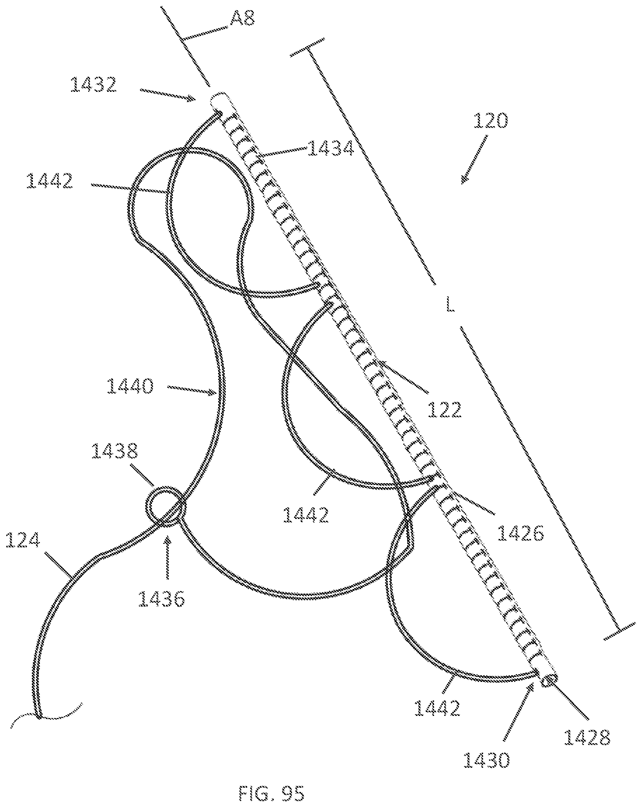

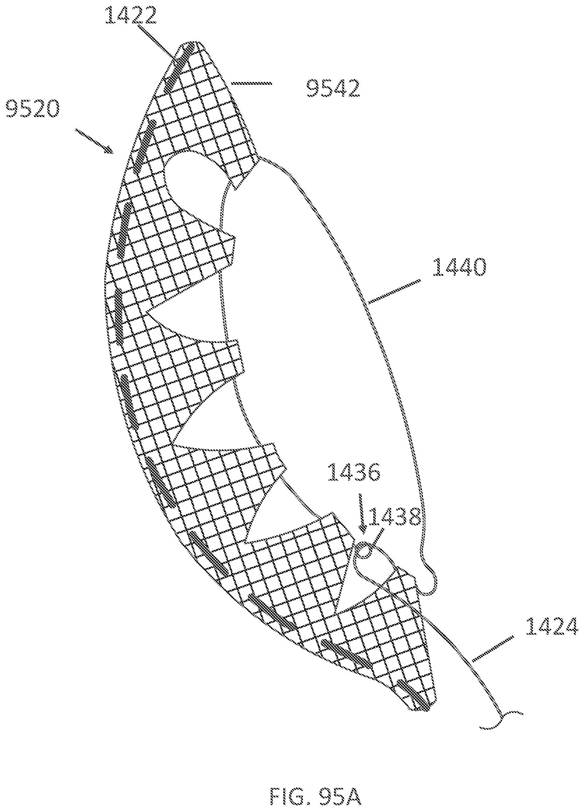

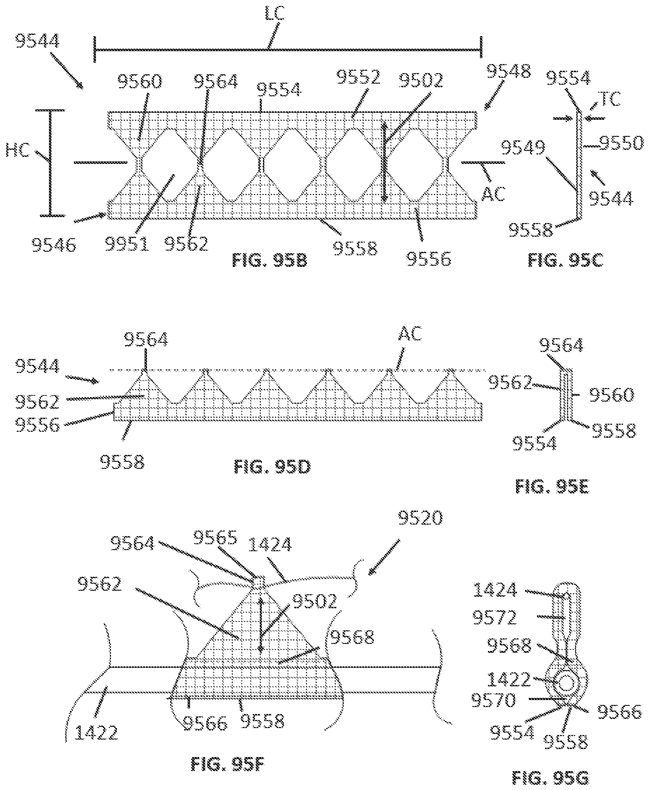

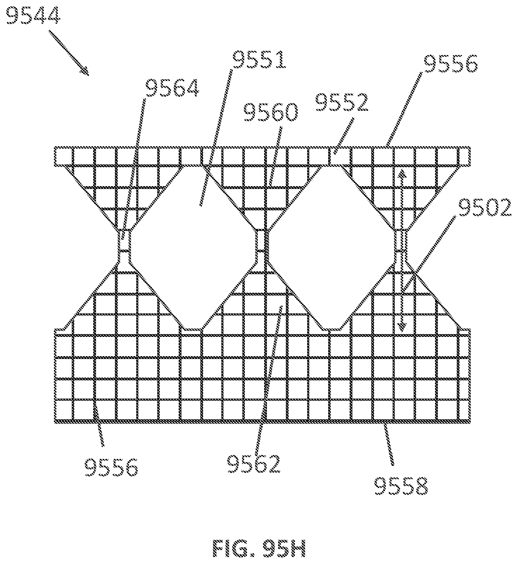

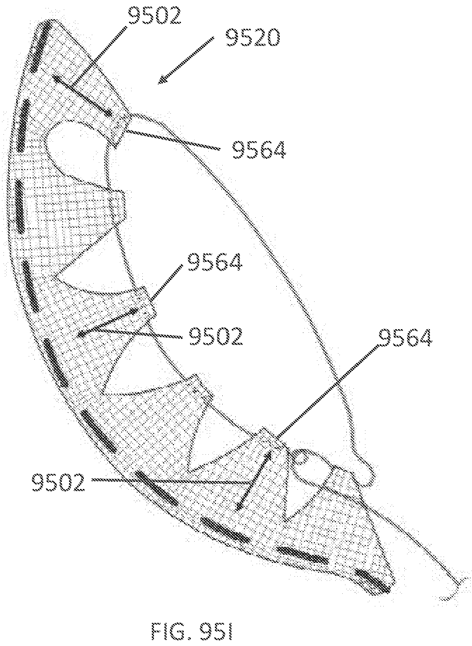

Referring to Figure 95, an example of the device 120 for remodeling the shape of a heart wall is illustrated. The device 120 includes an anchor 122 and a line 124. The anchor 122 can be configured in a variety of ways. Any configuration that can be positioned to engage an outward facing surface of a heart wall W to support pulling a portion of the heart wall W inward (i.e., toward an internal chamber of the heart) can be used. **In** the illustrated example, the anchor 122 is reconfigurable, such that the anchor can be delivered through a catheter or sheath in a delivered state (e.g., elongated as shown in Figure 95) that fits within a lumen of delivery catheter 1406, and can be reshaped to a deployed state once it has been delivered to the appropriate location.

**In** the illustrated example, the anchor 122 has a generally cylindrical elongated body 1426 forming a tube having a central passage 1428. **In** other examples, however, the body 1426 can be shaped other than cylindrical. For example, the elongated body 1426 can have an oval, rectangular, or other shaped cross section. The body 1426 has a length L and includes a first end portion 1430 and a second end portion 1432 opposite the first end portion.

In the illustrated example, the body 1426 includes one or more features to facilitate bending of the body 1426. The features can be configured in a variety of ways. In the illustrated example, the features include a series of traverse cuts 1434 along the body 1426. In one example, the series of cuts 1434 are a plurality of cuts where each of the cuts is generally perpendicular to a longitudinal axis A8 of the body 1426. In the illustrated example, the cuts in the series of cuts 1434 are evenly spaced along the body 1426 and extend from the first end portion 1430 to the second end portion 1432. For example, the series of cuts 1434 can extend over at least 80% of the length L of the body 1426. In other examples, the series of cuts 1434 are not be evenly spaced and can extend less than 80% of the length L of the body 1426.

Further, each of the cuts of the series of cuts 1434 extends partially into the body 1426. In one example, each of the cuts extend between 25%-75% through the body.

The anchor 122 can be made from any suitable material that can be reshaped from an elongated state to a curved, deployed state. In one example, the anchor 122 includes a shape-memory alloy-such as Nitinol-to provide shape-setting capability.

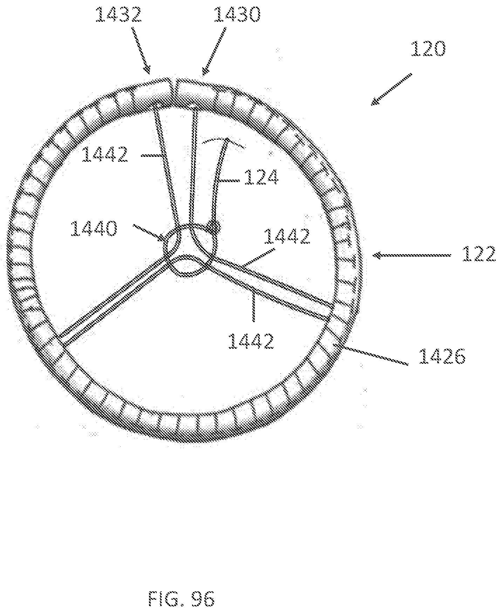

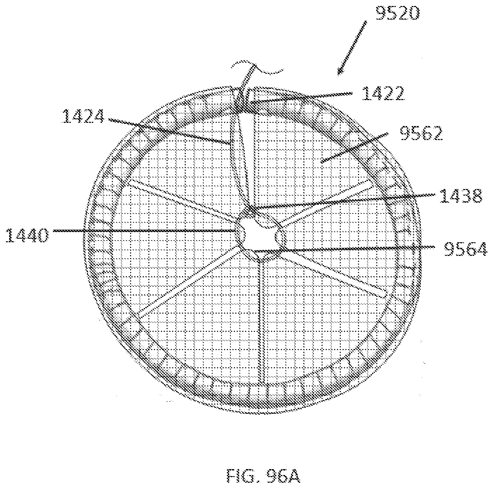

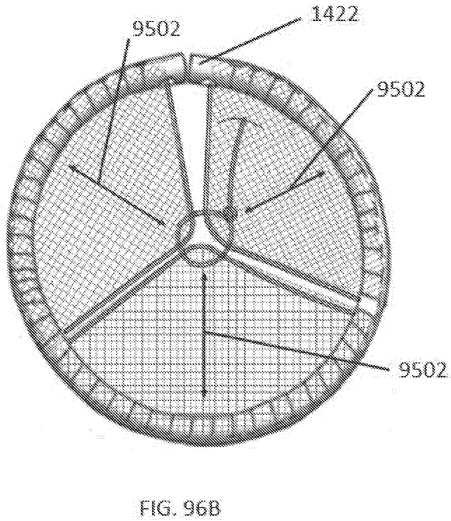

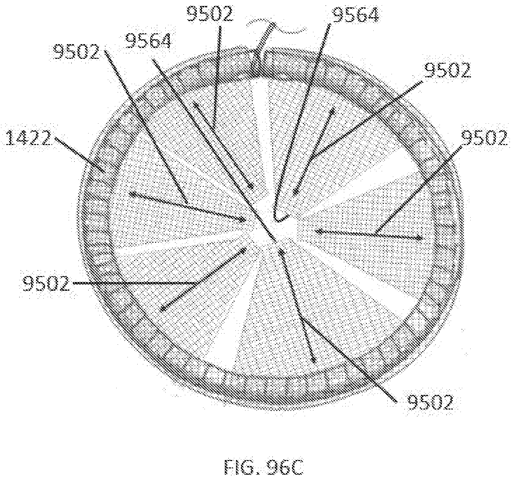

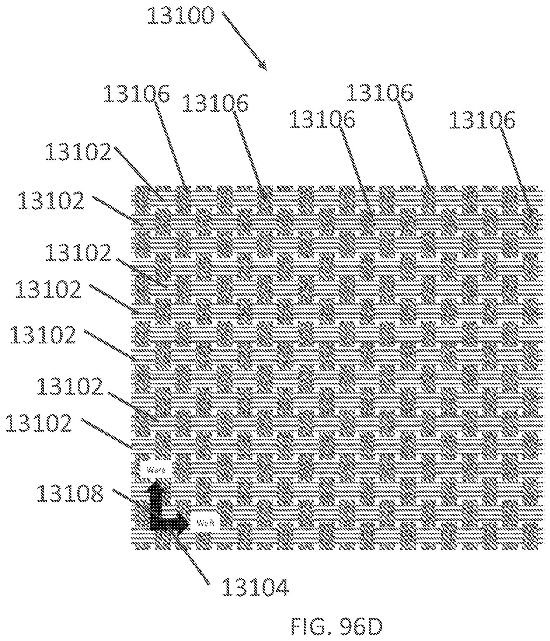

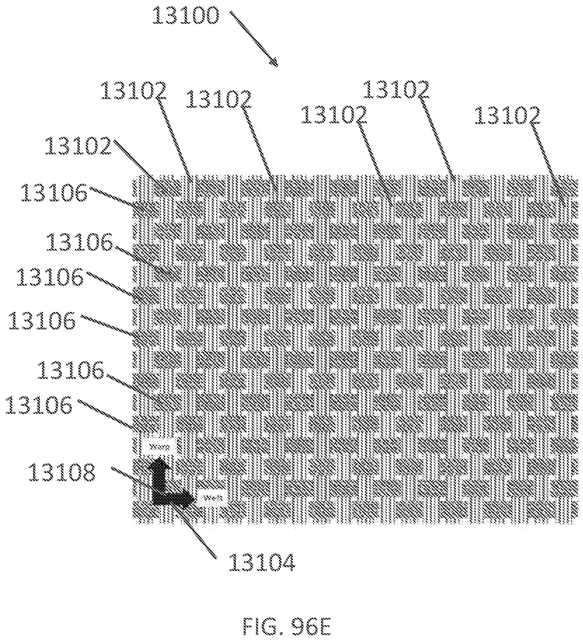

The line 124 is connected to the anchor 122 such that pulling the line 124 can pull the anchor 122. In some examples, pulling the line 124 can also reshape the anchor from an elongated state (Figure 95) to a curved, deployed state (Figure 96). In the illustrated example, the line 124 has a terminal end 1436 formed in a closed shape 1438, such as a circle 1438. The line 124 is arranged such that the line 124 passes through the closed-shaped terminal end 1436 to form a loop 1440 in the line 124 that can be withdrawn by pulling the line 124.

The line 124 can be connected to the anchor 122 in a variety of ways. In the illustrated example, the anchor includes a plurality of loops 1442 and the line 124 passes through each of the loops 1442 to connect the line 124 to the anchor 122. The number, size, location, and configuration of the loops 1442 can vary in different examples. In the illustrated example, the anchor includes three equal sized and evenly spaced loops 1442 along the length L of the anchor 122.

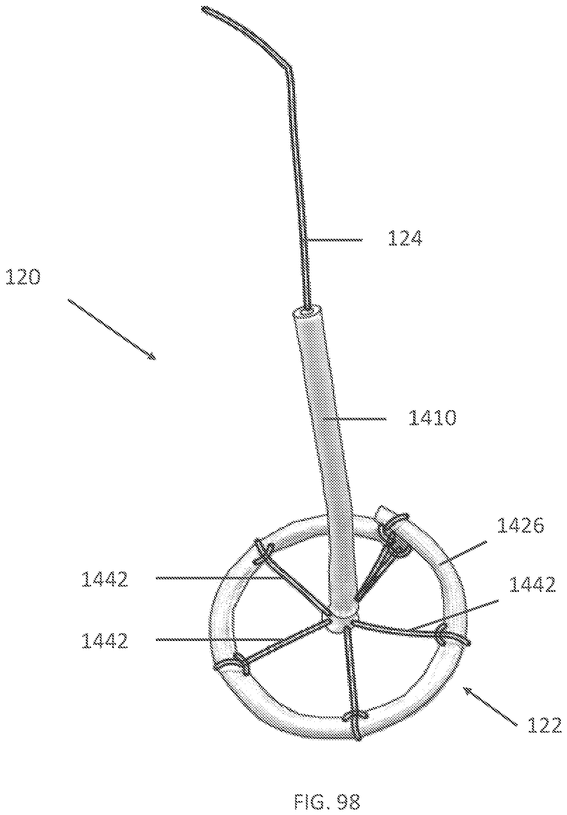

In the illustrated example, each of the loops 1442 is formed by a line fixedly attached, at its ends, to the body 1426 of the anchor 122. The line forming the loops 1442 can be fixedly attached in any suitable manner. In some examples, the body 1426 can include openings that accommodate insertion of the ends of the line forming the loops 1442 into the interior of body 1426 to attach the ends to the body 1426 122 (see, for example, Figure 96). In other examples, the lines that form the loops 1442 can be tied to the exterior of the body 1426 of the anchor 122 (see, for example, Figure 98) or otherwise attached to the exterior, such as for example, by an adhesive or other fastening device.

In other examples, however, the loops 1442 can be formed from a material other than a line. The loop 1440 in the line 124 passes through each of the loops 1442 to connect the line 124 to the anchor 122.

Referring to Figures 95A-95G, another example of a device 9520 for remodeling the shape of a heart wall is illustrated. The device 9520 is similar to the illustrated device 120 of Figure. 95 in that the device 9520 includes an anchor 1422 and a line 1424. The device 9520, however, instead of utilizing a plurality of loops 1442, formed by lines, through which the line 124 passes through, the device 9520 utilizes a cloth, fabric, or similar pliable material that is configured to facilitate reshaping the anchor in a deployed state that can be used to pull a portion of a heart wall inward to remodel the shape of the heart wall.

The anchor 1422 can be configured in a variety of ways. Any configuration that can be positioned to engage an outward facing surface of a heart wall W to support pulling a portion of the heart wall W inward (i.e., toward an internal chamber of the heart) can be used. In the illustrated example, the anchor 1422 is the same or substantially similar to the anchor 122 of Figure 95. Thus, the description of the anchor 122 applies equally to the anchor 1422

The line 1424 is connected to the anchor 1422 such that pulling the line 1424 pulls the anchor 1422. In some examples, pulling the line 1424 can also reshape the anchor from a substantially elongated state (Figure 95A) to a round, deployed state (Figure 96A). In the illustrated example, the line 1424 has a terminal end 1436 formed in a closed shape 1438, such as a circle. The line 1424 is arranged such that the line 1424 passes through the closed-shaped terminal end 1436 to form a loop 1440 in the line 1424 that can be withdrawn by pulling the line 1424.

The line 1424 can be connected to the anchor 1422 in a variety of ways. In the illustrated example, a connector 9542, made from a cloth, fabric, or similar pliable material, is used to connect the line 1424 to the anchor 1422. The connector 9542 can be configured in a variety of ways, such as for example, different shapes and different materials. In one example, the connector 9542 is made from a low-profile cloth. This cloth can take a wide variety of different forms. It can be woven, knitted, braided or non-woven. When woven, knitted or braided the cloth can optionally utilize high-strength yarns. The cloth can comprise any of the fabrics, yarns, and yarn components disclosed by US Patent No. 8,833,402, issued on September 16, 2014 to Rasmussen et al.. In one example, a permanent implant grade high strength yarn can be made from ultra-high molecular weight polyethylene (UHMwPE), polyethylene terephthalate (PET) and/or other materials and blends of materials. In some examples, the materials of the yarns are oriented to increase the strength of the yarn. In some examples, the connector 9542 is made from a thin material that when used with the disclosed anchor 1422 has a minimum pull strength of 60N or more in combination with the pulling line 1424. In one example, the cloths have a low-profile yarns and high orientation in molecular chains of the polymer to achieve high strength and low connector profile. The yarn used in the functional direction for realization of high pull-strength can possess a tenacity of at least 40 grams/denier. In one example, high-strength Dyneema^{®} yarn or other UHMwPE yarn with PET yarn can yield approximately 3 times the pull-out strength of the line 1424 through the connector compared to an otherwise identical connector made only from high density PET cloth.

In the following the non-SI unit inch corresponds to 2.54 cm. In one example, the connector cloth is a high density PET cloth and/or a hybrid cloth (described below) that has between 150 and 270 ends per inch, such as between 170 and 250 ends per inch, such as between 190 and 230 ends per inch, such as 210 ends per inch or about 210 ends per inch and between 110 and 230 picks per inch, such as between 130 and 210 picks per inch, such as between 150 and 190 picks per inch, such as about 170 picks per inch, such as 170 picks per inch. A wide variety of different yarns can be used for warp and weft. For example, a 40d/24f PET yarn can be used for warp and weft. In one example, a cloth thickness of a cloth made from a high strength yarn, such as a UHMwPE yarn, such as a Dyneema^{®} yarn with a conventional yarn, such as PET yarn is also lower compared to an otherwise identical cloth made only from conventional yarns, such as PET. The use of cloth as the connector 9542 provides an even force distribution against the tissue

Figures 96D and 96E illustrate two examples of woven cloth 13100 that can be used to make the connector 9542. In the example illustrated by Figure 96D, the woven cloth 13100 includes high strength yarn 13102 in the weft direction 13104 and a conventional yarn 13106 in the warp direction 13108. In the example illustrated by Figure 96E, the woven cloth 13100 includes a conventional yarn 13106 in the weft direction 13104 and a high strength yarn 13102 in the warp direction. The high strength yarn 13102 can take a wide variety of different forms. For example, the high strength yarn 13102 can be an UHMwPE yearn, such as a Dyneema^{®} yarn. The conventional yarn 13106 can take a variety of different forms. For example, the conventional yarn 13106 can be a conventional plastic, such as PET. The conventional yarn 13106 and the high strength yarn 13102 are woven to form a hybrid fabric. In one example, the high strength yarn size can be 25dtex/10filament and the conventional yarn size can be 44dtex/24filament, with the fabric being constructed using at least 180 picks per inch and 160 ends per inch. For example, a UHMWPE yarn size can be 25dtex/10filament and a PET yarn can be 44dtex/24filament, with the fabric being constructed using at least 180 picks per inch and 160 ends per inch. The high strength yarn and the conventional yarn can be multifilament or monofilament. For example, a UHMWPE yarn and a PET yarn can be multifilament or monofilament. UHMPWPE yarn can be of size 11dtex up to 55dtex while PET yarn can be 11dtex up to 44dtex. UHMPWPE yarn can be of size 11dtex up to 55dtex while PET yarn can be 11dtex up to 44dtex.

The connector 9542 can take a variety of different shapes. In some examples, the connector 9542 is configured such that pulling the line 1424 also reshapes the anchor from an elongated state (Figure 95A) to a round, deployed state (Figure 96A). In some other examples, the anchor 1422 is shape-set to the deployed state and the connector 9542 is not configured such that pulling the line reshapes the anchor. In the example illustrated by Figures 95B-95C, the connector 9542 includes a patterned cloth or other material 9544 that is laser-cut, or otherwise formed. In the illustrated example, the patterned material 9544 has a first end 9546, a second end 9548, a first face 9549, and a second face 9550 opposite the first face 9549.

The patterned material 9544 includes a series of cut-outs 9551 extending across the length LC of the patterned material 9544 along a central longitudinal axis AC. The cut-outs 9551 can be configured in a variety of ways, such as the shape, size, and a number of cut-outs. In the illustrated example, the cut-outs 9551 are generally diamond-shaped, with the cut-outs 9551 adjacent the first end 9546 and adjacent the second end 9548 being half-diamonds. In other examples, however, the cutouts 9551 can take other shapes, such as for example, circular, oval, triangular hexagonal, octagonal and/or rectangular, etc. The cut-outs 9551, in the illustrated example, are positioned and shaped such that the patterned material 9544 is symmetric relative to the longitudinal axis AC. Further, in the illustrated example, the patterned material 9544 includes five, full cut-outs, and two half, open cut-outs adjacent the first end 9546 and adjacent the second end 9548. In other examples, however, the patterned material 9544 can include more or less than five, full cut-outs.

In one example, the cut-out dimensions at the folded section (i.e. the section connecting the diamonds in Figure 95B) are selected to achieve a required pull-strength. Based upon the desired minimum pull strength, the number of cut-outs, the width of the section connecting the diamonds and cloth parameters such as tenacity, size and yarn density of high strength yarn in the cloth is optimized. In one example, a hybrid cloth of high strength yarn, such as UHMWPE yarn with conventional yarn, such as PET yarn utilizes melt-sealing of edges from the filaments of plastic conventional filaments, such as PET filaments when the connector 9542 component is cut into different shapes to avoid fraying of the yarns and/or premature failure of components i.e. low pull strength. In one example, the low melting temperature and melt flow characteristics of high strength yarns, such as UHMWPE yarns do not evenly melt at the edges when cutting the fabric into the shape of the connector 9542 using high temperature techniques, such laser cutting. The inclusion of the conventional yarns, such as PET yarn results in an edge having an even thickness, even though the edge is cut using a high temperature technique, such as laser cutting and the fabric includes the high strength yarns, such as UHMWPE yarns.

The cut-outs 9551 result in the patterned material 9544 having a first band 9552 forming an upper edge 9554 and a lower band 9556 forming a lower edge 9558. The cut-outs 9551 are defined by a series of first inward tapering sections 9560 and a series of opposing second inward tapering sections 9562 aligned with the series of first inward tapering sections 9560. Each of the first and second inward tapering sections 9560, 9562 are generally triangular, such as for example, forming an isosceles or equilateral triangle. However, the sections 9560 can have a wide variety of different shapes. Each of the first inward tapering sections 9560 is connected to a corresponding second inward tapering section 9562 by a bridge 9564.

Referring to Figures 95D-95E, to form the connector 9542, the patterned material 9544 is folded over upon itself, lengthwise along the central longitudinal axis AC, as best shown in Figure 95E. Thus, the bridges 9564 are folded in half such that the upper edge 9554 is positioned adjacent the lower edge 9558 and the first inward tapering sections 9560 are positioned adjacent the second inward tapering sections 9562. In this folded configuration, the material 9544 resembles a saw-tooth shape.

The connector can be connected to the anchor in a wide variety of different ways. Referring to Figures 95F-95G, to connect the connector to the anchor and to connect the line 1424 to the anchor 1422, the first band 9552 is connected to the second band 9556 at two locations. In the illustrated example, first, the first band 9552 is connected to the second band 9556 adjacent the upper edge 9554 and the lower edge 9558 as shown by line 9566. Second, the first band 9552 is connected to the second band 9556 adjacent the first inward tapering sections 9560 and the second inward tapering sections 9562 as shown by line 9568. The first band 9552 can be connected to the second band 9556 in any suitable manner, such as stitching, stapling, and/or adhesive or other suitable attachment. As a result of the first band 9552 being connected to the second band 9556 as disclosed, the material 9544 forms a first passage (or passages) 9570 through which the anchor 1422 extends and a second passage 9572 through which the line 1424 extends.

Figure 95H illustrates another patterned material 9544 where the first band 9552 is narrower than the second band 9556. To form the connector 9542, from the patterned material 9544 illustrated by Figure 95H, the material is folded over upon itself, lengthwise along centers of the bridges 9564. The upper edge 9554 is positioned significantly inward of the lower edge 9558 and the first inward tapering sections 9560 are positioned adjacent the second inward tapering sections 9562. The connector can be connected to the anchor in a wide variety of different ways.

In the Figure 95H example, the second band 9556 is folded over the anchor. The folded second band 9556 can be connected to itself (since it folds back onto itself) and/or to the first band 9552. The second band 9556 can be connected to the first band 9552 and/or itself in any suitable manner, such as stitching, stapling, and/or adhesive or other suitable attachment. As a result of the second band 9556 being folded, the material 9544 forms a first passage (or passages) through which the anchor extends and a second passage through which the line extends.

Referring to Figures 95B, 95F, 95H, 95I, 96B, and 96C in some examples the cloth 13100 can be oriented and cut into a pattern (Figure 95B) that maximizes the pull strength of the connector 9542. In one example, the cloth 13100 is oriented and cut such that the high strength fibers 13102 (See Figures 96D and 96E) extend in the direction indicated by arrow 9502 (See Figures 95I, 96B, and 96C). In the examples illustrated by Figures 95I, 96B, and 96C, the direction 9502 corresponds to a line that is perpendicular to an edge 9565 (or the axis AC) of the connection portions 9564. This direction can also be characterized as extending in the direction of a shortest line that extend from the anchor 1422 to the edge 9565 of the connection portions. In another example, the cloth 13100 is oriented and cut such that the high strength fibers 13102 extend in a direction that is perpendicular to the direction indicated by arrow 9502 (See Figures 95I, 96B, and 96C). In the examples illustrated by Figures 95I, 96B, and 96C, the direction that is perpendicular to the direction 9502 corresponds to a line that is parallel to an edge 9565 of the connection portions 9564 and/or to the edge 9558.

Referring to Figure 96, the anchor 122 is illustrated in a deployed state. The deployed state of the anchor 122 can be a variety of shapes. Any shape that can be used to pull a portion of a heart wall inward to remodel the shape of the heart wall can be used, such as for example, a curved shape. In the illustrated example, the anchor 122 is ring-shaped in the deployed state such that the first end portion 1430 is adjacent the second end portion 1432 and the body 1426 is curved in a circle. In some examples, the first end portion 1430 and the second end portion 1432 can abut. In other examples, the first end portion 1430 and the second end portion 1432 do not abut. For example, the first end portion 1430 and the second end portion 1432 can be spaced apart from one another or can overlap,

The anchor 122 can be reshaped from the elongated state to the deployed state in a variety of ways. For example, the anchor 122 can include a shape-memory alloy and be shape set to the shape of the deployed state. Alternatively, or in conjunction with the anchor being shape set, the line 124 can be used to reshape the anchor 122. In particular, since the line loop 1440 passes through each of the loops 1442, by pulling on the line 124, the line loop 1440 is withdrawn which pulls the first end portion 1430 and second end portion 1432 together while bending the body 1426 into an annulus.

Referring to Figure 96A, the anchor 1422 is illustrated in a deployed state. The deployed state of the anchor 1422 can be a variety of shapes. Any shape that can be used to pull a portion of a heart wall inward to remodel the shape of the heart wall can be used, such as for example, a curved shape. In the illustrated example, the anchor 1422 is ring-shaped in the deployed state.

The anchor 1422 can be reshaped from the elongated state to the deployed state in a variety of ways. For example, the anchor 1422 can include a shape-memory alloy and be shape set to the shape of the deployed state. Alternatively, or in conjunction with the anchor being shape set, the line 1424 can be used to reshape the anchor 1422. In particular, since the line loop 1440 passes through the second passage 9572, by pulling on the line 1424, the line loop 1440 decreases in size. The decreasing size of the line loop 1440 pulls the bridges 9564 toward one another, which pulls the anchor 1422 into an annular shape. The bridges 9564 on the connector body converge toward a center point of the annulus. Since the overlapping tapering sections 9560, 9562 are generally triangular, when the connector body into pulled into an annulus, the overlapping tapering sections 9560, 9562 are pulled together to substantially form a solid disc, which can be pulled against tissue, such as heart wall tissue.

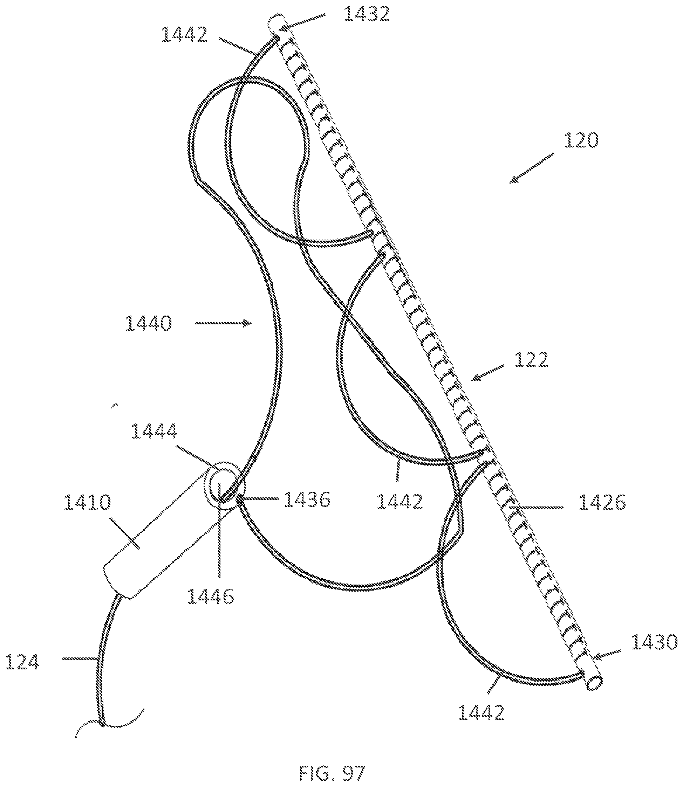

In Figure 97, the device 120 includes the hemostatic plug 1410. The hemostatic plug 1410 can be configured in a variety of ways. Any device that can stop bleeding from occurring from a passage formed by the piercing device 130 in a heart wall can be used. In the illustrated example, the hemostatic plug 1410 is formed as a cylindrical tube having a distal end 1444 and a central passage 1446 through which the line 124 extends. In other examples, however, the hemostatic plug 1410 can be configured other than cylindrical.

In the illustrated example, the terminal end 1436 of the line 124 is attached to the distal end 1444 of the hemostatic plug 1410. The line 124 forms the loop 1440 and is connected to the anchor 122 by passing through each of the loops 1442.

Referring to Figure 98, the anchor 122 is illustrated in a deployed state. In the illustrated example, the anchor 122 is ring-shaped in the deployed state such that the first end portion 1430 is adjacent the second end portion 1432, the body 1426 is curved in a circle, and the distal end 1444 of the hemostatic plug 1410 is generally adjacent the center of the circle.

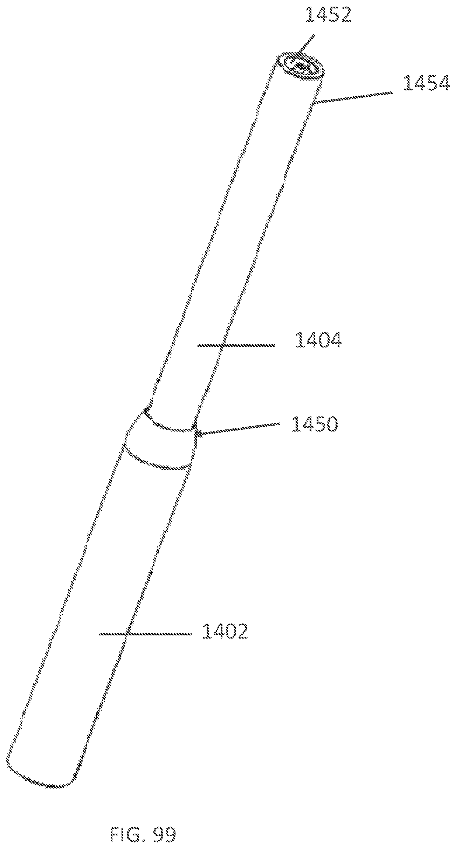

In Figure 99, an example of a portion of the guide sheath 1402 and a portion of the steerable catheter 1404 is illustrated. Both the guide sheath 1402 and the steerable catheter 1404 can be configured in a variety of ways. Any suitable known guide sheath 1402 and steerable catheter 1404 can be used. In the illustrated example, the guide sheath 1402 and the steerable catheter 1404 are concentric where the guide sheath 1402 includes an inner lumen (not shown) and the steerable catheter extends through the inner lumen and out of a distal end 1450 of the guide sheath 1402. Likewise, the steerable catheter 1404 includes an inner lumen 1452 that is open at a distal end 1454 of the steerable catheter 1404.

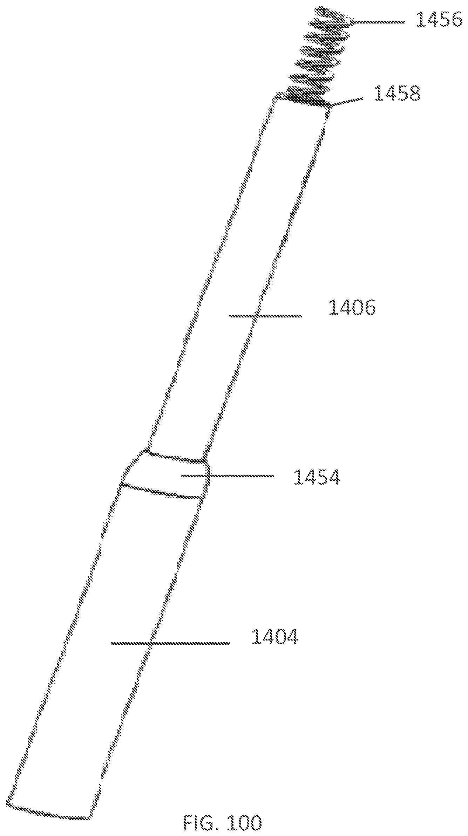

In Figure 100, a portion of the steerable catheter 1404 and a portion of the delivery catheter 1406 are illustrated. The delivery catheter 1406 includes an anchoring device 1456 attached to a distal end 1458 of the delivery catheter 1406. The anchoring device 1456 can be any suitable device capable of attaching to the heart wall W. In the illustrated example, the anchoring device 1456 is a wire formed in a helical shape configured to be screwed into the heart wall W to secure the delivery catheter 1406 to the heart wall W. The delivery catheter 1406 includes an inner lumen (not shown) open at the distal end 1458.

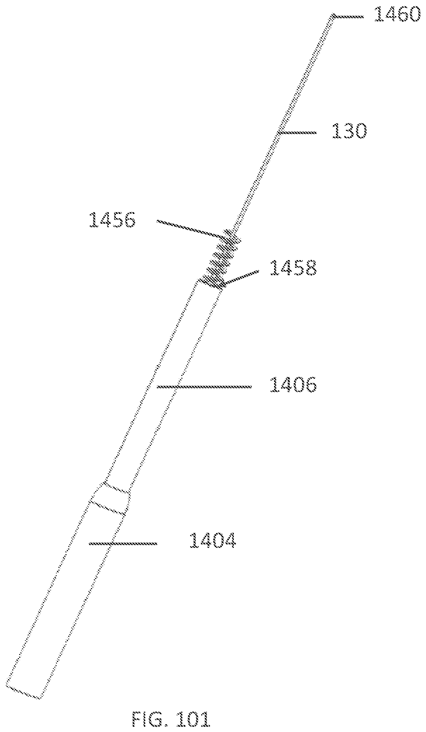

In Figure 101, a portion of the steerable catheter 1404, a portion of the delivery catheter 1406, and the piercing device 130 are illustrated. The piercing device 130 can be any suitable device for piercing or creating a passage into a human heart wall, such as for example, a needle, wire, or other similar device. In the illustrated example, the piercing device 130 is a needle or hollow wire having an inner passage (not shown) and an opening (not shown) proximate a distal end 1460 of the piercing device 130 the fluidly connects the inner passage (not shown) to the exterior of the piercing device 130.

In the illustrated example, the piercing device 130 is delivered through the inner lumen (not shown) of the delivery catheter 1406 and extends out of the distal end 1458 of the delivery catheter 1406.

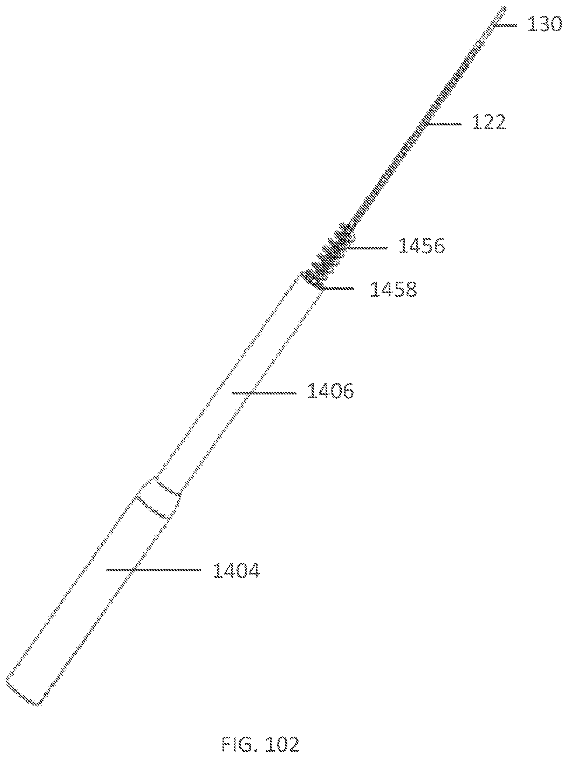

In Figure 102, a portion of the steerable catheter 1404, a portion of the delivery catheter 1406, the piercing device 130, and the anchor 122 are illustrated. The anchor 122 is shown in the elongated state and it extends from the distal end 1458 of the delivery catheter 1406 over top of the piercing device 130 such that the piercing device 130 is received in the central passage 1428 of the anchor 122 and the anchor 122 and the piercing device 130 are concentric.

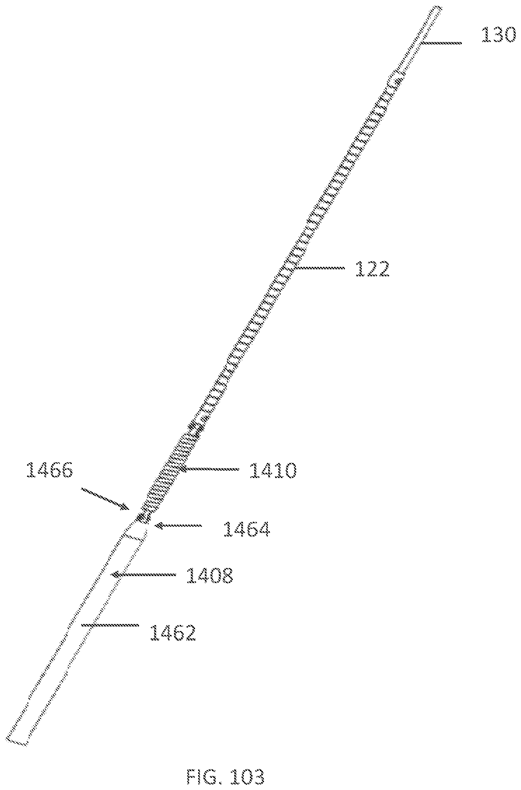

In Figure 103, examples of the piercing device 130, the anchor 122, the hemostatic plug 1410, and the pusher 1408 are illustrated. The pusher 1408 can be configured in a variety of ways. Any configuration capable of pushing the hemostatic plug 1410 and anchor 122 over the piercing device 130 and beyond the distal end 1460 of the piercing device 130 can be used. In the illustrated example, the pusher 1408 has an elongated, cylindrical body 1462 having a distal end 1464 configured to abut a proximal end 1466 of the hemostatic plug 1410. The body 1462 includes an inner passage (not shown) extending through the pusher body 1462. The piercing device 130 can be received in the passage (not shown) such that the pusher 1408 is slidable over the piercing device 130 and concentric with the piercing device 130.

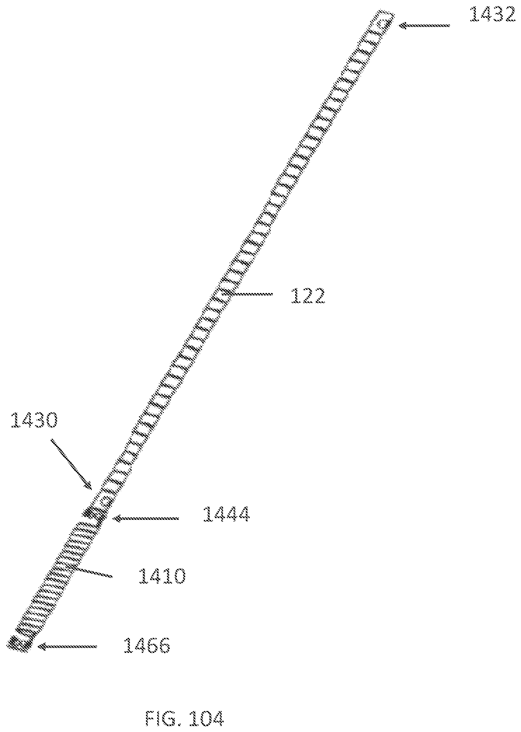

In Figure 104, the anchor 122 and the hemostatic plug 1410 are illustrated. The anchor 122 is shown in the elongated state. and the hemostatic plug 1410 is illustrated as a cylindrical tube. Both the anchor 122 and the hemostatic plug 1410 are configured to be concentric with and slidable over the piercing device 130. When being delivered over the piercing device 130, the anchor 122 and the hemostatic plug 1410 are longitudinally aligned, with the distal end 1444 of the hemostatic plug 1410 adjacent the first end 1430 of the anchor 122, as shown in Figure 104.

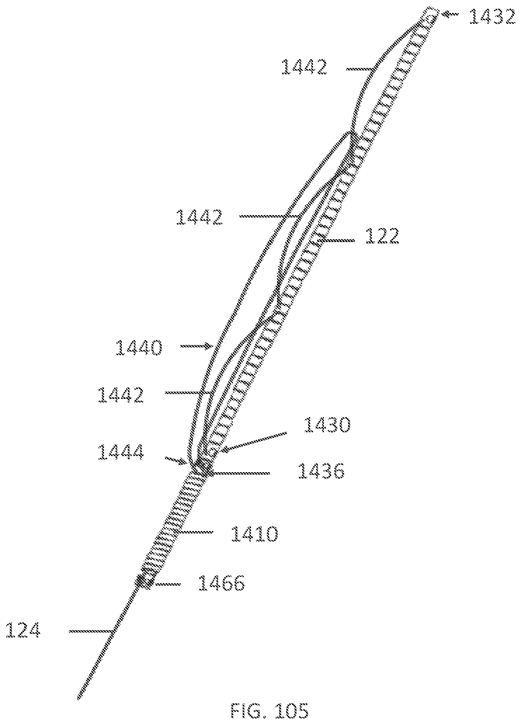

In Figure 105, the anchor 122 and the hemostatic plug 1410 are illustrated along with the line 124 and the loops 1442 on the anchor 122. The terminal end 1436 of the line 124 is attached to the distal end 1444 of the hemostatic plug 1410. The loop 1440 of the line 124 passes through each of the loops 1442 to connect the line 124 to the anchor 122. The line 124 then extends through the central passage 1446 (Figure 97) of the hemostatic plug from the distal end 1444 to the proximal end 1466.

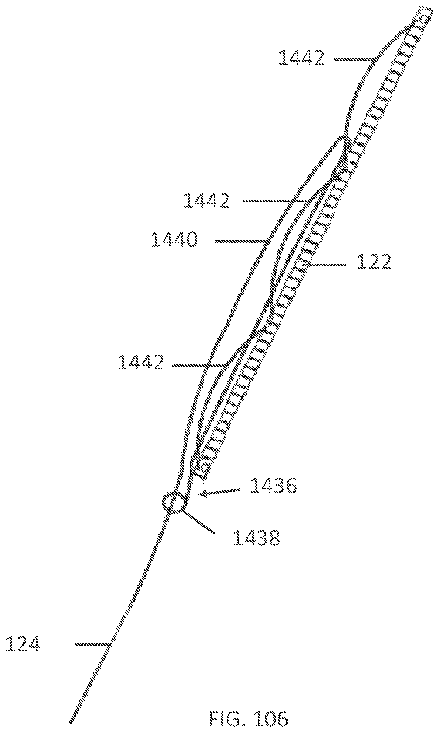

In Figure 106, the anchor 122 without a hemostatic plug 1410 is illustrated along with the line 124 and the loops 1442 on the anchor 122. The terminal end 1436 of the line is formed in a closed, circular shape 1438 and the line 124 is arranged such that the line 124 passes through the closed-shaped terminal end 1436. The loop 1440 of the line 124 passes through each of the loops 1442 to connect the line 124 to the anchor 122.

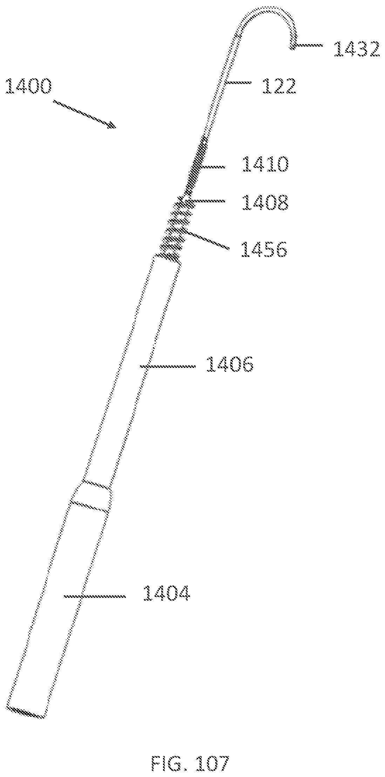

In Figure 107, the system 1400 is illustrated showing the anchor 122 partially deployed beyond the distal end 1460 of the piercing device 130 (Figure 101). The delivery catheter 1406 is illustrated partially extending from the steerable catheter 1404 and the pusher 1408 is illustrated partially extending from the delivery catheter 1406 through the anchoring device 1456. As the pusher 1408 extends from the delivery catheter 1406, the distal end 1464 of the pusher 1408 engages the proximal end 1466 of the hemostatic plug 1410 to push both the hemostatic plug 1410 and the anchor 122 over the piercing device 130. As the distal end 1432 of the anchor 122 moves beyond the distal end 1460 of the piercing device 130 (Figure 101), the anchor 122 can begin to reshape into its curved deployed state. For example, the anchor 122 can include a shape memory alloy that is shape set to the curved deployed state. Thus, the portion of the anchor 122 that is no longer received on the piercing device 130 can revert to the curved deployed state that that shape memory alloy was set to.

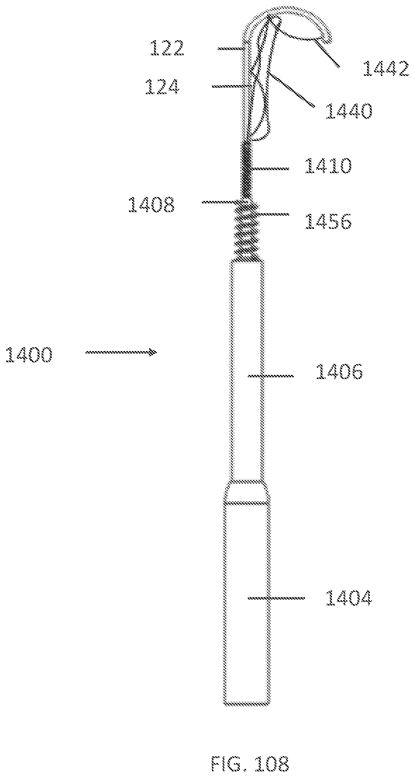

In Figure 108, the system 1400 is illustrated showing the anchor 122 partially deployed beyond the distal end 1460 of the piercing device 130 (Figure 101) along with the line 124 connected to the anchor 122. In particular, the line 124 connects to the hemostatic plug 1410, extends through the loops 1442, and then loops around and extend though the central passage 1446 (Figure 97) of the hemostatic plug 1410.

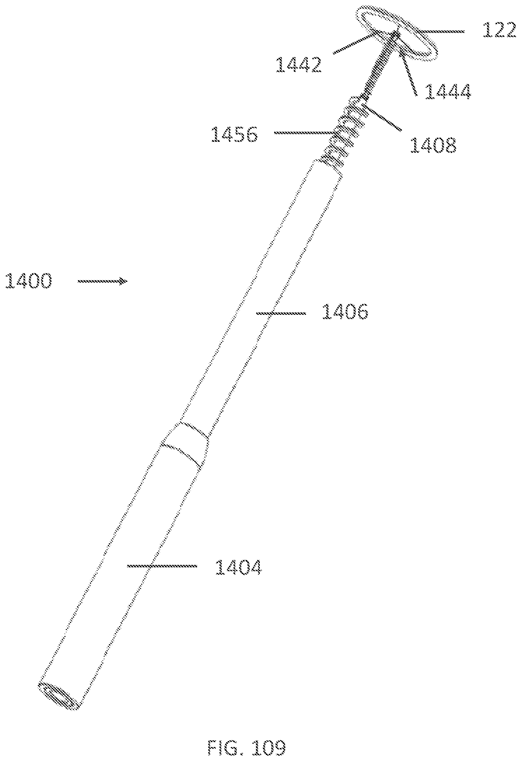

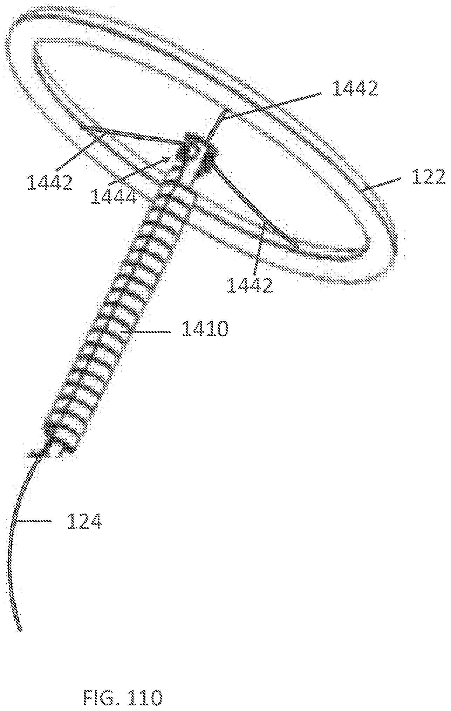

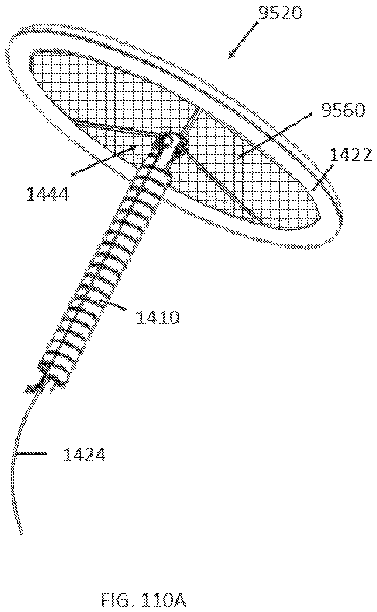

In Figures 109-110, the system 1400 is shown with the anchor 122 in the deployed state. In particular, the line 124 (Figure 110) is withdrawn by pulling the line 124 in a direction away from the anchor 122 and into the delivery catheter 1406. Since the loop 1440 (Figure 97) of the line 124 passes through the loops 1442 on the anchor 122, pulling the line 124 closes the loop 1440 and pulls the loops 1442 together to help facilitate, along with any shape setting properties of the anchor, reshaping the anchor 122 from the elongated state to the curved deployed state shown in Figures 109-110. At the same time, the distal end 1444 of the hemostatic plug 1410 is positioned adjacent the center of the curved anchor 122.

Similarly, in Figure 110A, the system 9520 is shown with the anchor 1422 in the deployed state. In particular, the line 1424 is withdrawn by pulling the line 1424 in a direction away from the anchor 1422 and into the delivery catheter 1406. Since the loop 1440 (Figure 96A) of the line 1424 passes through the second passage 9572 on the anchor 1422, pulling the line 1424 closes the loop 1440 and pulls the overlapping tapering sections 9560, 9562 together to help facilitate, along with any shape setting properties of the anchor, reshaping the anchor 1422 from the elongated or undeployed state to the curved deployed state. At the same time, the distal end 1444 of the hemostatic plug 1410 is positioned adjacent the center of the curved anchor 1422.

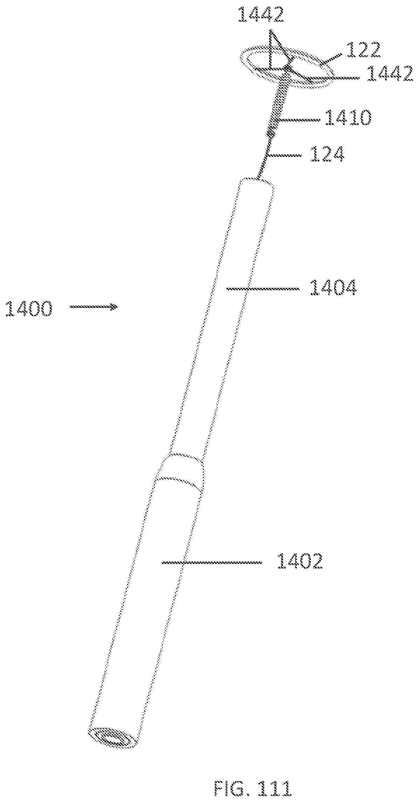

In Figure 111, the system 1400 is shown with the anchor 122 in the deployed state and the delivery catheter 1406 retracted back into the steerable catheter 1404. The line 124 (see Figure 110) is withdrawn by pulling the line 124 in a direction away from the anchor 122 and into the delivery catheter 1406. Since the loop 1440 (Figure 97) of the line 124 passes through the loops 1442 on the anchor 122, pulling the line 124 closes the loop 1440 and pulls the loops 1442 together to help facilitate, along with any shape setting properties of the anchor, reshaping the anchor 122 from the elongated state to the curved deployed state shown in Figures 109-110. At the same time, the distal end 1444 of the hemostatic plug 1410 is pulled up to the center of the curved anchor 122 as the loop 1440 is shortened by pulling the line 124.

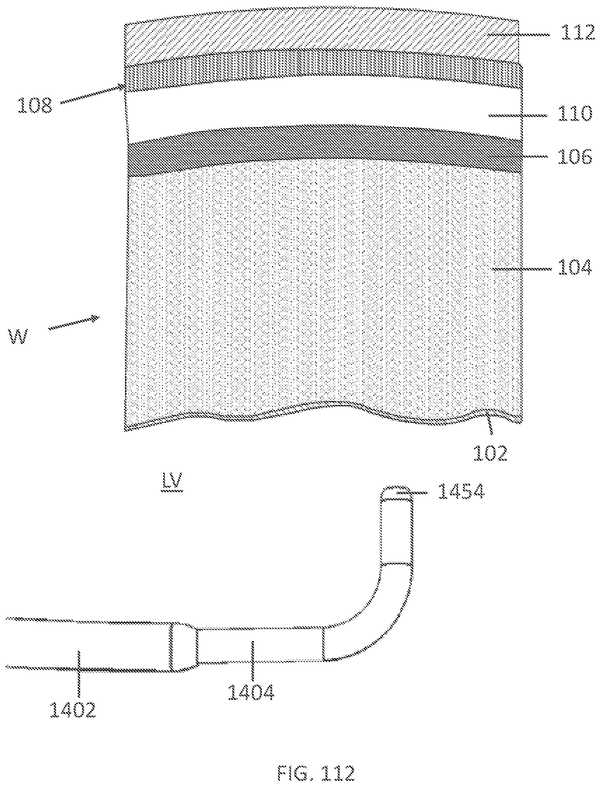

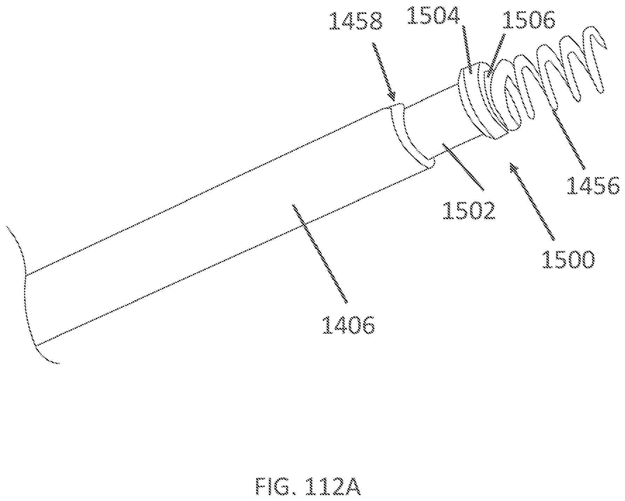

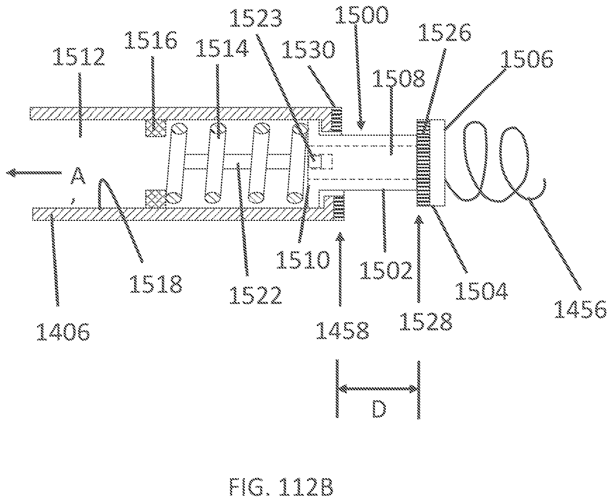

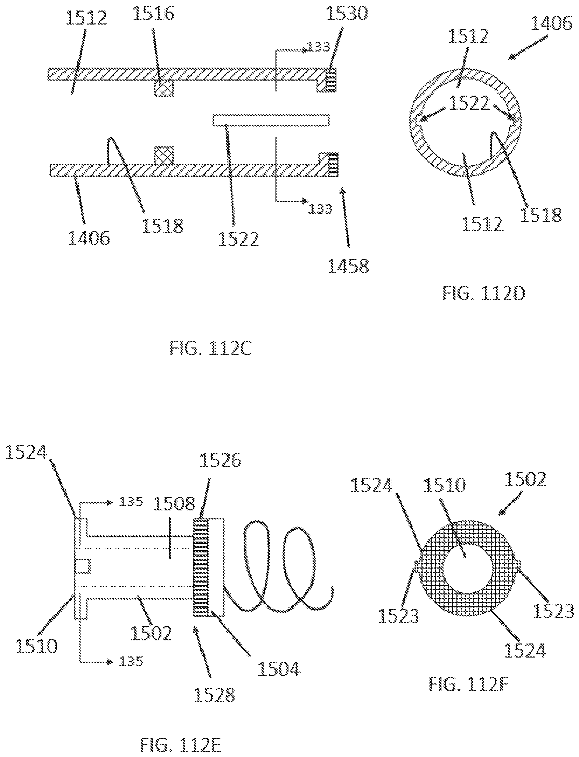

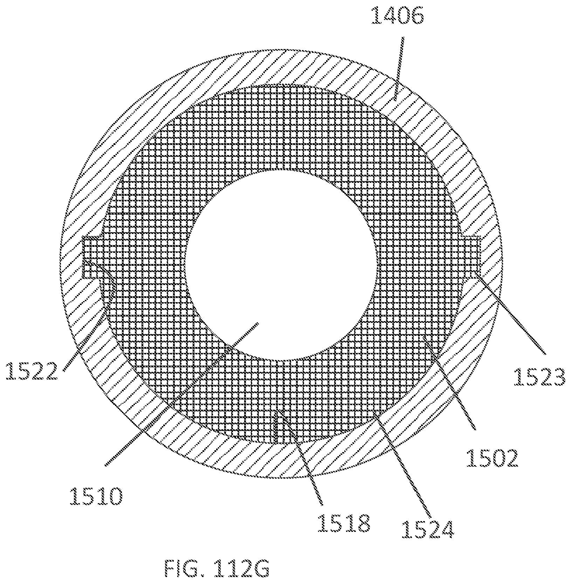

Referring to Figures 112-120, the deployment of the device 120 into the pericardial cavity 110 for remodeling the shape of a heart wall W and system and method for delivering the device 120 is illustrated. Referring to Figure 112, deployment of the device 120 includes delivering the guide sheath 1402 and the steerable catheter 1404 into an internal chamber (e.g., left ventricle LV) of the heart H. The steerable catheter 1404 is arranged such that the distal end 1454 of the steerable catheter 1404 is adjacent the heart wall W.

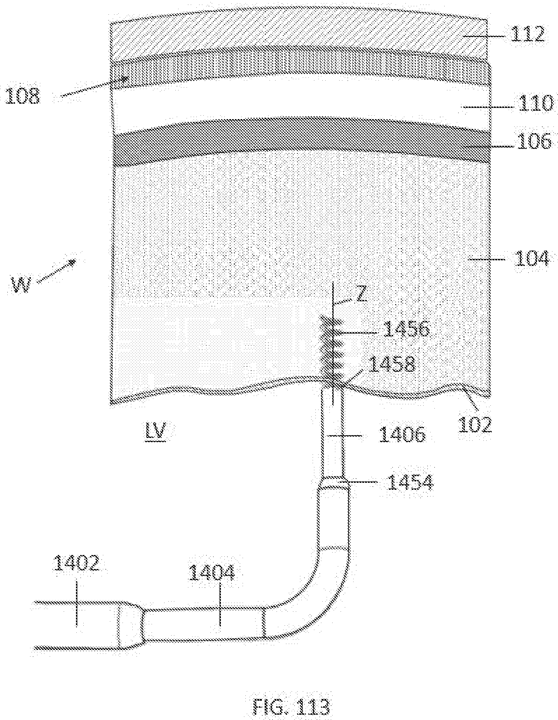

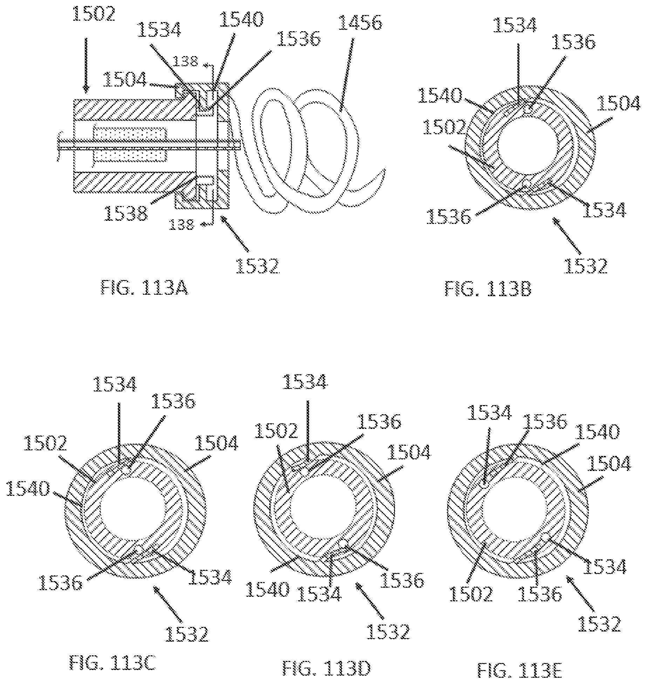

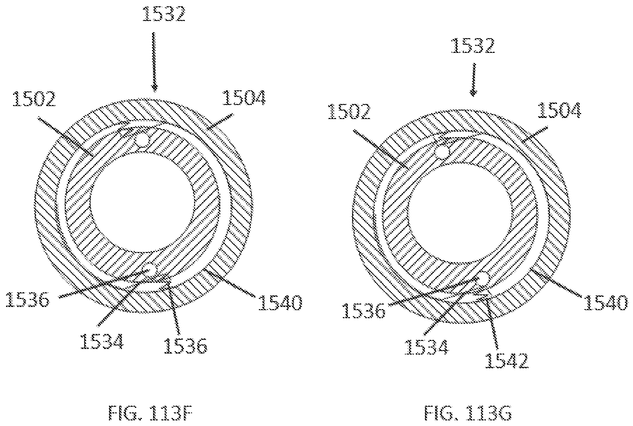

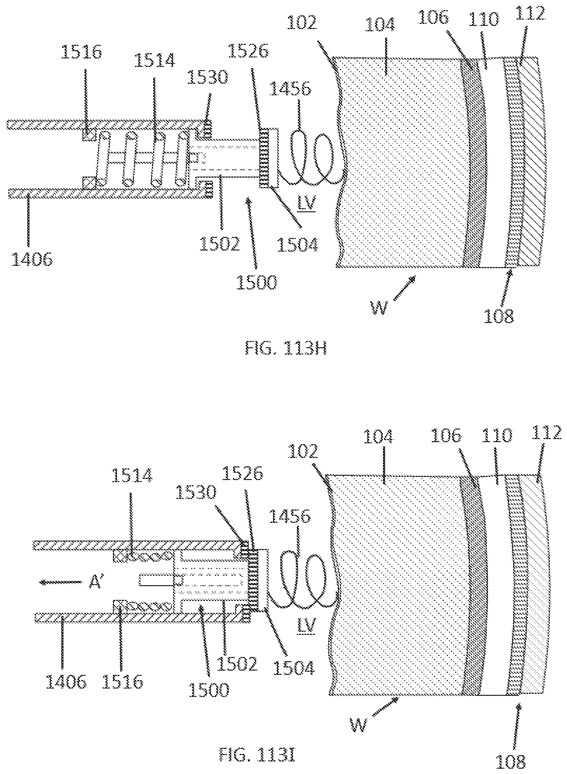

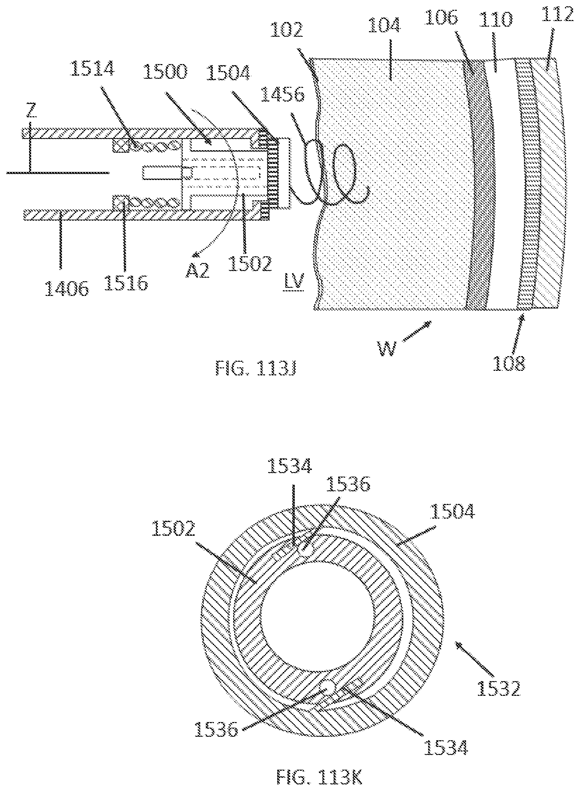

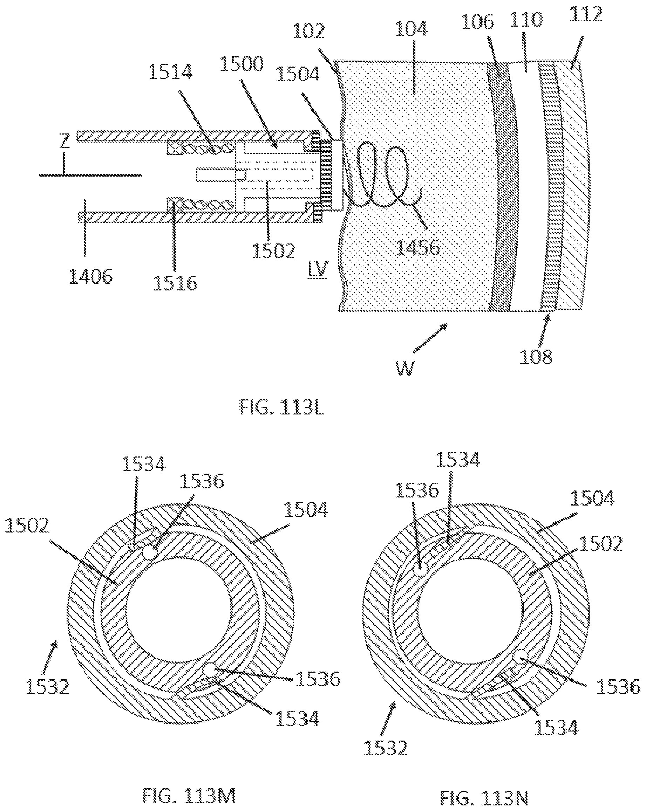

Referring to Figure 113, the delivery catheter 1406 is extended from the distal end 1454 of the steerable catheter 1404. The anchoring device 1456 of the delivery catheter 1406 is attached to the heart wall W, such as for example by rotating the delivery catheter 1406 about axis Z relative to the steerable catheter 1404 to screw the anchoring device 1456 into the heart wall (i.e. through the endocardium 102 and into the myocardium 104). In the illustrated example, the distal end 1458 of the delivery catheter 1406 abuts, or is adjacent, the endocardium 102.

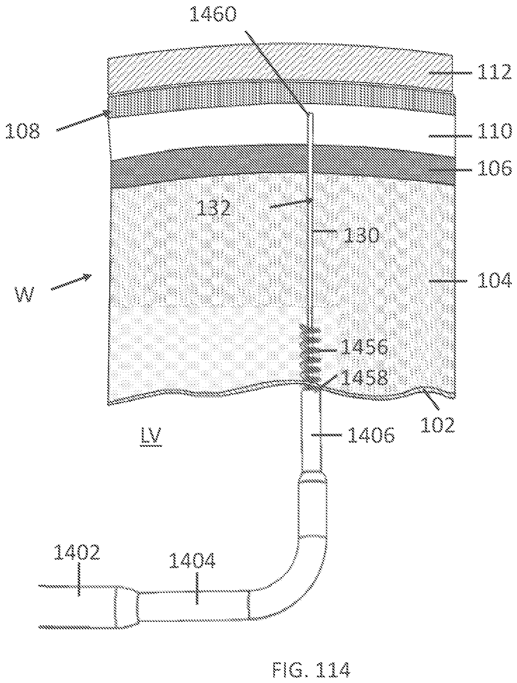

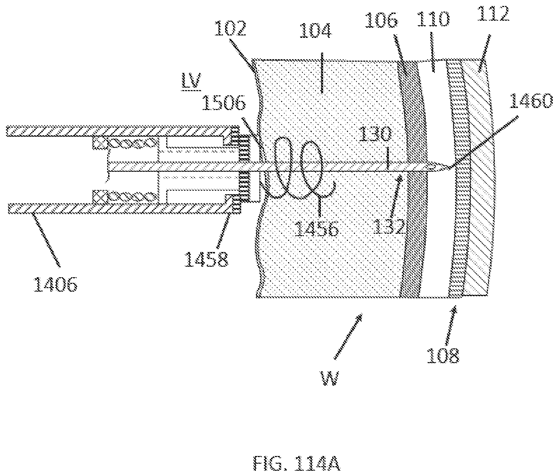

Referring to Figure 114, the piercing device 130 is delivered into an internal chamber (e.g., left ventricle LV) of the heart H via the delivery catheter 1406. The piercing device 130 can be extended from the distal end 1458 of the delivery catheter 1406 such that the distal end 1460 of the piercing device 130 is extended into the heart wall W. In the illustrated example, the distal end 1460 of the piercing device 130 is extended through the endocardium 102, the myocardium 104, the epicardium 106, and into the pericardial cavity 110 to form the passage 132. Since the delivery catheter 1406 is anchored to the heart wall W, the location for insertion of the piercing device 130 can be precisely controlled.

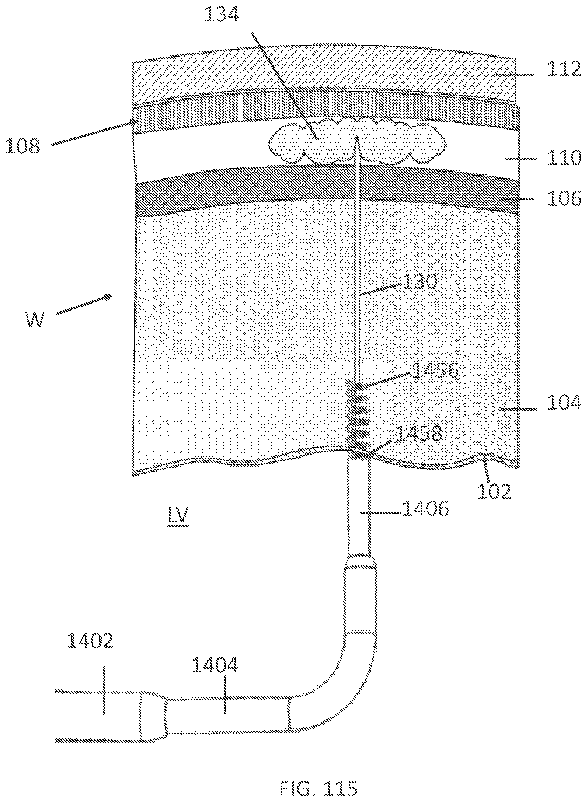

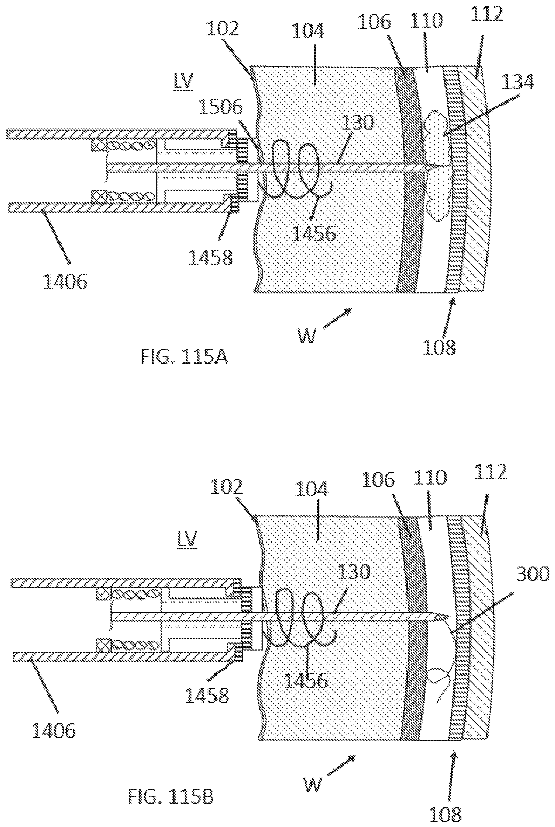

To verify that the piercing device 130 is properly positioned for deploying the anchor 122 into the pericardial cavity 110, a dye 134, or other detectable fluid, can be delivered through the piercing device 130 and injected into the pericardial cavity 110, as shown in Figure 115. The dye 134 can be detected by any suitable technique such as X-ray, to verify that the piercing device 130 is properly positioned.

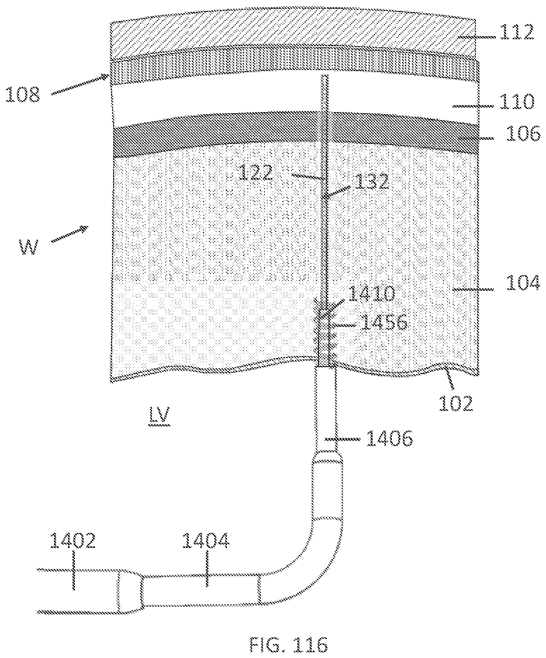

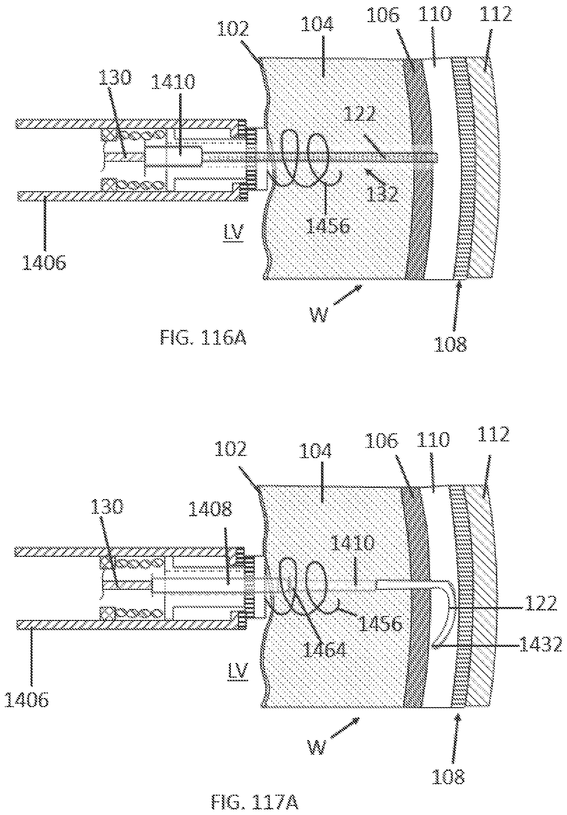

In Figure 116, the anchor 122 and the hemostatic plug 1410 are extended from the delivery catheter 1406 over the piercing device 130 (Figure 115) and through the passage 132. As shown in Figure 116, the anchor 122 remains in the elongated state while sliding along the piercing device 130 into the pericardial cavity 110.

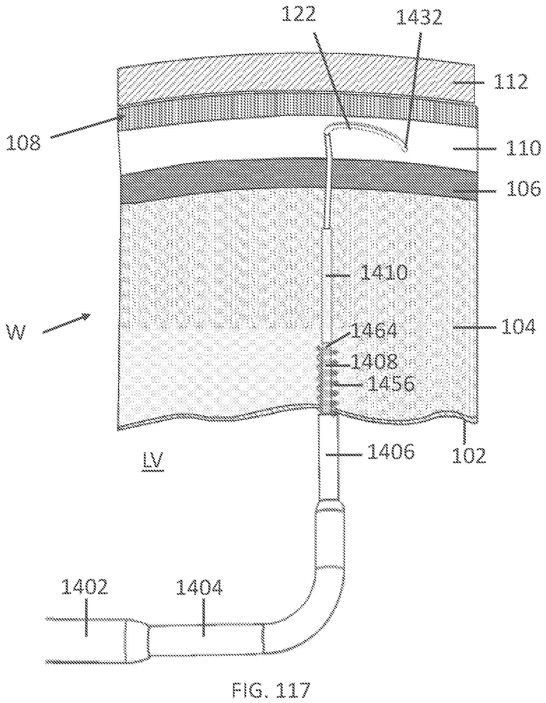

In Figure 117, the anchor 122 is partially deployed beyond the distal end 1460 (Figure 114) of the piercing device 130 (Figure 101). The delivery catheter 1406 is illustrated partially extending from the steerable catheter 1404 and the pusher 1408 is illustrated partially extending from the delivery catheter 1406 through the anchoring device 1456. As the pusher 1408 extends from the delivery catheter 1406, the distal end 1464 of the pusher 1408 engages the hemostatic plug 1410 to push both the hemostatic plug 1410 and the anchor 122 over the piercing device 130 (Figure 101). As the distal end 1432 of the anchor 122 moves beyond the distal end 1460 of the piercing device 130 (Figure 101), the anchor 122 can begin to reshape into its curved deployed state. For example, the anchor 122 can include a shape memory alloy that is shape set to the curved deployed state. Thus, the portion of the anchor 122 that is no longer received on the piercing device 130 can revert to the curved deployed state that that shape memory alloy was set to.

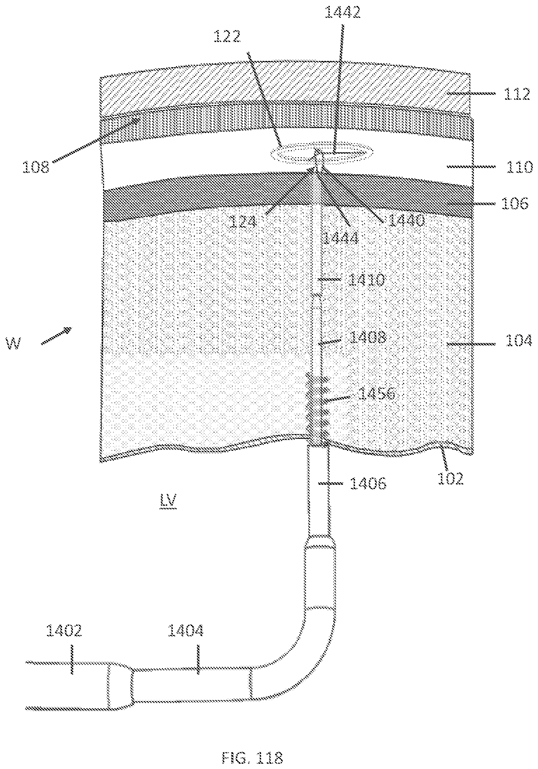

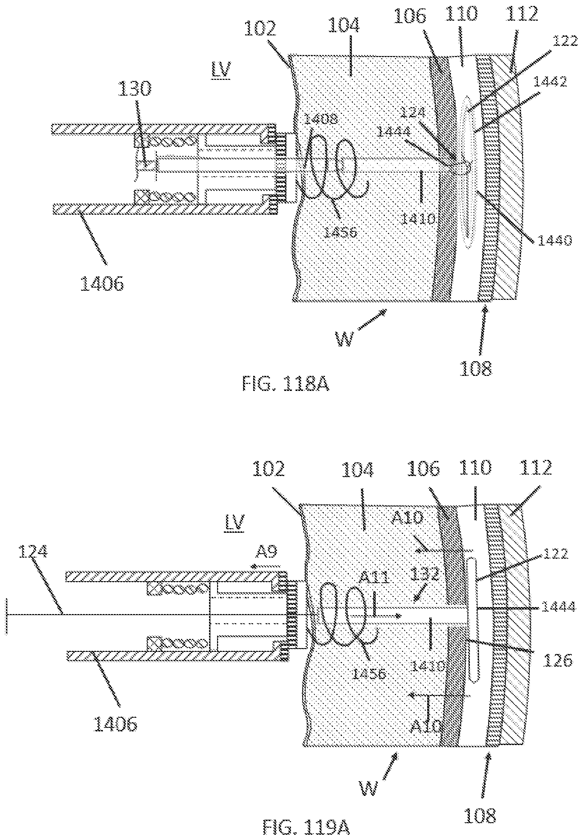

In Figure 118, the anchor 122 is in the deployed state and the hemostatic plug 1410 is positioned within the passage 132 to prevent or inhibit bleeding from the passage 132. In the illustrated example, the distal end 1444 of the hemostatic plug 1410 is at or near the inner wall of the pericardial cavity 110. To reshape the anchor 122, the line 124 is withdrawn by pulling the line 124 in a direction away from the anchor 122 and into the hemostatic plug 1410. Since the loop 1440 (Figure 97) of the line 124 passes through the loops 1442 on the anchor 122, pulling the line 124 closes the loop 1440, pulls the hemostatic plug 1410 and the anchor 122 together, and pulls the loops 1442 together to help facilitate, along with any shape setting properties of the anchor 122, reshaping the anchor 122 from the elongated state to the curved deployed state shown in Figures 118.

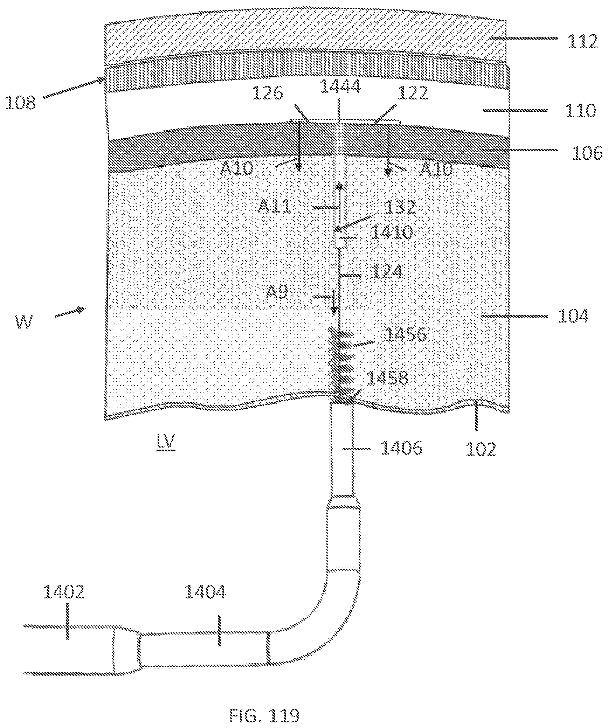

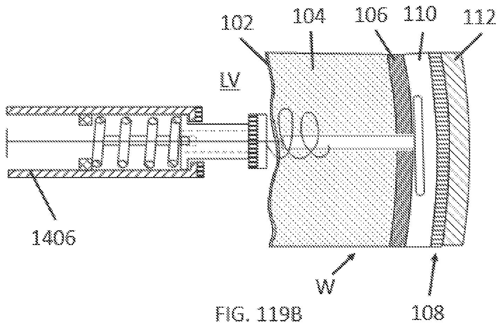

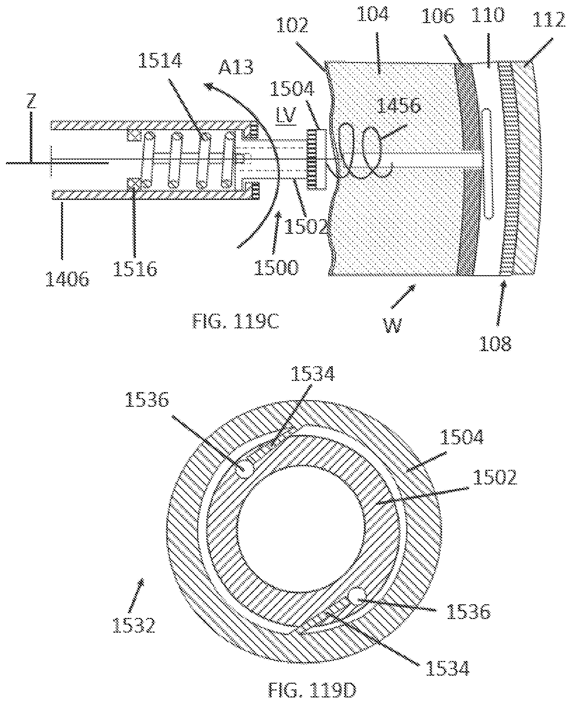

As shown in Figures 119-120, the pusher 1408 and piercing device can be removed by withdrawing them from the passage 132 into the delivery catheter 1406. The anchor 122, hemostatic plug 1410, and line 124 are left deployed in the heart wall W with the anchor 122 within the pericardial cavity 110 and the line 124 extending through the epicardium 106, the myocardium 104, and the endocardium 102.

To seat the anchor 122, the delivery catheter 1406 can remain attached to the heart wall W and the distal end 1458 can be pushed against the heart wall W. At the same time, the line 124 can be placed in tension by pulling the line 124 in an inward direction toward the heart chamber as shown by the arrow A9. As a result, the anchor 122 will be pulled against the outward facing surface 126 that partially defines the pericardial cavity 110, as shown by arrows A10. As the line 124 is being pulled in the direction of A9, the hemostatic plug 1410 is urged in the opposite direction, as shown by arrow A11, such that the distal end 1444 of the hemostatic plug 1410 is positioned at or adjacent the center of the anchor 122. The anchor 122 in its deployed state is too large to fit through the passage 132 formed by the piercing device 130. Thus, once the delivery catheter 1406 is removed, further tensioning of the line 124 can pull the heart wall W inward toward the heart chamber (e.g., the left ventricle LV).

Referring to Figures 112A-112G, 113A-113N, 114A, 115A, 115B, 116A, 117A, 118A, 119A-119D, 120A, system 1400 and method for delivering the device 120 against a surface of a heart can include piston 1500. Piston 1500 can be disposed between and coupled with delivery catheter 1406 and anchoring device 1456.

Referring to Figures 112A-112B, a portion of the delivery catheter 1406, piston 1500, and anchoring device 1456 are illustrated. Piston 1500 includes piston body 1502 coupled with piston head 1504. Anchoring device 1456 can be coupled with distal end 1506 of piston head 1504.

Referring to Figure 112B, delivery catheter 1406 can include an inner lumen 1512 that extends through the distal end 1458 of the delivery catheter 1406. Piston 1500 can comprise inner lumen 1508 extending through piston body 1502 and piston head 1504. Inner lumen 1508 is open at distal end 1506 of piston head 1504 and proximal end 1510 of piston body 1502.

Proximal end 1510 of piston body 1502 is positioned within the inner lumen 1512 of delivery catheter 1406 between a resistance member 1514 and distal end 1458 of delivery catheter 1406. Piston body 1502 can be configured to slide through distal end 1458 of the delivery catheter 1406 and at least partially into inner lumen 1512 of delivery catheter 1406.

Delivery catheter 1406 can include stopper 1516 coupled to inner wall 1518 of delivery catheter 1406. Stopper 1516 couples with and secures the proximal end of resistance member 1514 in place.

Piston 1500 can be configured to slide in distal end 1458 of the delivery catheter 1406. Resistance member 1514 resists the sliding motion of piston 1500 and biases the piston 1500 toward an extended position. Resistance member 1514 can comprise a variety of shapes and materials. Resistance member 1514 can be a spring, as shown in Figure 112B, a wire formed into a helical shape, or a spring-loaded or force-dampening material.

The piston 1500 and delivery catheter 1406 can have one or more recesses 1522 that slidably engage one or more protrusions 1523. The mating of the recesses 1522 with the protrusions 1523 couples the piston 1500 and delivery catheter 1406, such that the piston 1500 and delivery catheter 1406 can axially slide relative to one another but cannot rotate relative to one another. The resistance member 1514 biases the piston 1500 distally out of the delivery catheter 1406 that can include one or more guide slots 1522. That is, the piston 1500 engages with the delivery catheter 1406 to prevent or inhibit the rotation of the piston 1500 with respect to the delivery catheter 1406. This engagement can take place in a variety of ways other than the illustrated slot and protrusion arrangement.

Figure 112D illustrates a cross section of a portion of delivery catheter 1406 as shown in Figure 112C. In the example illustrated by Figures 112C and 112D, the recess 1522 is a slot or a plurality of slots in the inner wall 1518 of delivery catheter 1406. Figure 112F illustrates a cross section of proximal end 1510 of piston body 1502, as shown in Figure 112E. In the illustrated example, piston body 1502 includes the projection 1523 extending from outer wall 1524 of piston body 1502. Piston body 1502 can include a plurality of projections 1523. Projection 1523 can comprise a variety of sizes and shapes. Slot 1522 can comprise a shape complementary to projection 1523.

In the example illustrated by Figure 112G, an outer wall 1524 of the proximal end 1510 of piston body 1502 can engages with inner wall 1518 of delivery catheter 1406. The coupling of proximal end 1510 of piston body 1502 with the inner wall 1518 of delivery catheter 1406 allows the piston 1500 to slide into delivery catheter 1406.As delivery catheter 1406 is rotated, the engagement of projection 1523 with slot 1522 rotates piston 1500 with delivery catheter 1406.

With reference back to Figure 112B, piston 1500 is shown in a non-engaged position, characterized by proximal end 1510 of piston body 1502 in contact with the resistance member 1514, inner wall 1518, and distal end 1458 of delivery catheter 1406. A force in a direction A' can be exerted on anchoring device 1456, which in turn exerts a force on piston 1500 and moves piston 1500 proximally relative to the delivery catheter 1406, in the direction A'. The force on anchoring device 1456 can cause piston head 1504 to contact distal end 1458 of delivery catheter 1406. This position shown in Figure 113I and can be referred to as the "activated or engaged position." Force can be applied by pushing anchor 1456 of delivery catheter 1406 against heart wall W or any other material in contact with anchoring device 1456.

The relative position of piston 1500 and delivery catheter 1406 can be detected. A minimum or an optimal force at which anchoring device 1456 is pressed against the material before rotation can be detected based on the relative position of piston 1500 and delivery catheter 1406. Engagement of the piston head 1504 with the distal end 1458 of delivery catheter 1406 (the compressed or activated position) can indicate to a user that anchoring device 1456 is in suitable engagement for use. For example, the engaged or activated position can indicate that anchoring device 1456 is pressed against heart wall W at a suitable pressure for the temporary implanting of the anchoring device 1456 to the heart.

A user can determine if additional force should be applied to anchoring device 1456 by observing the relative position of the piston head 1504 with respect to the distal end 1458 of delivery catheter 1406. With reference to Figures 112B and 112E, to detect the relative position of piston head 1504 with respect to the distal end 1458 of delivery catheter 1406, piston head 1504 and/or catheter 1406 can include a marker 1526, 1530, respectively. The marker 1526 can be located on proximal end 1528 of piston head 1504 as illustrated or any other portion of the piston head. With reference to Figures 112B and 112C, the delivery catheter 1406 marker 1530 can be located on distal end 1458 of delivery catheter 1406.

Referring to Figure 112B, in the non-activated or extended position, marker 1526 is distance D from marker 1530. Piston 1500 can travel in the A' direction upon exertion of a force, for example as anchoring device 1456 is pressed against a material, such as a heart wall, such as the endocardium and/or papillary muscle in a ventricle. As piston 1500 travels in the direction A', the distance between marker 1526 and the distal end 1458 of delivery catheter 1406 decreases. The distance between marker 1526 and the marker 1530 also decreases. Marker 1526 can abut marker 1530 when the piston 1500 reaches the compressed or activated position. A user can be able to detect the relative position of piston 1500 and delivery catheter 1406 by tracking the position of the marker 1526 and marker 1530 using a visual scope, an electrical sensor that detects contact between markers 1526, 1530, or other suitable means.

The contact, positioning of, and/or pressure applied to anchoring device 1456 and piston 1500 can be determined by a variety of other means. For example, marker 1530 and marker 1526 can be otherwise positioned or replaced. Delivery catheter 1406 or piston 1500 can comprise, for example a force measuring device or pressure sensing device and means for communicating the pressure or force to the user, such as a visual display, indicator light, audible indicator, etc...

Anchoring device 1456 can be rotated into the receiving material, for example heart wall W. As anchoring device 1456 is further rotated into the receiving material, the amount of torque required can increase, until a "torque threshold" is reached. The torque threshold for optimal engagement of the anchoring device 1456 into heart wall W can be pre-determined based on many factors, including the composition of the receiving material, the size shape of the anchoring device, and the distance that the anchoring device must travel into the receiving material. In one example, the torque threshold is based on a force increase that occurs when the anchor 1456 bottoms out on the tissue. For example, during initial engagement of the anchor 1456 with the tissue, the torque will increase gradually as more of the anchor is engaged in the tissue. But, when the anchor 1456 bottoms out, the torque required to continue rotating the catheter 1406 spikes. In one example, the "threshold torque" is set between the between the gradually increasing torque and the torque spike that results from bottoming out of the anchor.

With reference to Figure 113A-113B, system 1400 can include an over-torque prevention device 1532 designed to prevent or inhibit excessive torque of the anchoring device 1456 into the heart wall W. Over-torque can occur when the torque applied to the anchoring device 1456 is greater than the pre-determined torque threshold. Over-torque prevention device 1532 can comprise a clutch mechanism configured to de-couple the delivery catheter 1406 from the piston 1500 and/or anchoring device 1456 when the torque applied to the anchoring device 1456 is greater than the pre-determined torque threshold. The clutch mechanism automatically allows for the rotation of delivery catheter 1406 and piston 1500 separate from the rotation of anchoring device 1456 when the threshold torque is reached, and thus prevents or inhibits over rotation of anchoring device 1456 into the receiving material. In another example, the clutch mechanism can be between the delivery catheter 1406 and the piston 1500, instead of between the piston 1500 and the anchor 1456.

The over-torque prevention devise can take a wide variety of different forms. For example, any clutch mechanism can be used. For example, in the example illustrated by Figures 113A-113E, the piston head includes a cap that is rotatably connected to the piston body. The over-torque prevention device 1532 can include one or more arms 1534 extending from inner wall 1540 of the rotatable cap piston head 1504. Arm 1534 can be configured to contact pin 1536 extending distally from distal end 1538 of piston body 1502. Distal end 1538 of piston body 1502 can include one or a plurality of pins 1536.

In the illustrated example, the cap piston head 1504 includes two arms 1534 positioned about 180 degrees apart, and distal end 1538 of piston body 1502 includes two pins 1536 positioned 180 degrees apart. However, any number of arms and pins can be used. The torque threshold value can be altered by various means, including by increasing or decreasing the length of arms 1534 relative to the size of the pins 1536.

Figures 113B-113C correspond to situations where the torque applied to the delivery catheter 1406 and anchor 1456 is less than a pre-determined torque threshold. With reference to Figure 113C, when delivery catheter 1406 is rotated, pins 1536 contact and push arms 1534. Due to this contact, piston head 1504 and anchoring device 1456 rotate at the same rate as delivery catheter 1406 and piston body 1502. Arms 1534 making contact with pins 1536 can result in a resistance experienced by a user applying the torque. The resistance can signify that the torque threshold has not been reached or exceeded by the applied force.

Figures 113D and 113E illustrates situations where the torque applied to the delivery catheter 1406 reaches and exceeds a pre-determined torque threshold. At the position illustrated by Figure 113D, arms 1534 can flex against and begin to slide past pins 1536. Arms 1534 travel past pins 1536, and delivery catheter 1406 and piston body 1502 decouple from piston head 1504 and anchoring device 1456. Delivery catheter 1406 and piston body 1502 rotate with respect to piston head 1504 and anchoring device 1456. The arms 1534 slipping past the pins 1536 prevents or inhibits excessive torque being applied to the anchoring device 1456, and thereby can prevent or inhibit the anchoring device 1456 being overexerted into the receiving material. Arms 1534 traveling past pins 1536 can cause a sudden decrease in resistance experienced by the user applying the torque. The decrease in resistance can signify that the torque threshold has been reached and that the rotation of delivery catheter 1406 should be slowed, stopped, or reversed.

Arms 1534 and pins 1536 can have numerous shapes, sizes and other configuration variations to optimize setting and controlling the torque threshold. For example, with reference to Figures 113F-113G, over-torque prevention device 1532 can comprise arms 1534 made of a thin sheet metal material. Arms 1534 can extend along inner wall 1540 of the cap of the piston head 1504. Arms 1534 can couple with flexible spring members 1542, forming a ramp-like surface. The size of the arms and spring members 1542 can be altered to increase or decrease the desirable pre-determined threshold torque value.

Figure 113F corresponds to situations where the torque applied to the delivery catheter 1406 is less than a pre-determined torque threshold. When delivery catheter 1406 is rotated, pins 1536 contact arms 1534. Due to this contact, piston head 1504 rotates at the same rate as delivery catheter 1406 and piston body 1502.

Figure 113G corresponds to when the torque applied to the delivery catheter 1406 reaches a pre-determined or exceeds the torque threshold. Arms 1534 are can flexed by pins 1536, and piston body 1502 begins to rotate faster relative to piston head 1504. The pins 1536 slip past the arms 1534 to decouple the anchor 1456 from the catheter 1406.

Referring to Figures 113H-113N, 114A, 115A, 115B, 116A, 117A, 118A, 119A-119D, 120A, 121A, and 122A, the deployment of the device 120 for remodeling the shape of a heart wall W and system and method for delivering the device 120 is illustrated. The method illustrated by Figures 113H-113N, 114A, 115A, 115B, 116A, 117A, 118A , 119A-119D, 120A, 121A, and 122A,can be adapted to any of the examples disclosed herein. For example, the deployment described by Figures 113H-113N, 114A, 115A, 115B, 116A, 117A, 118A, 119A-119D, 120A, 121A, and 122A, can be adapted to any of the anchors, hemostasis tubes, delivery devices, and methods described herein.

Referring to Figure 113H, deployment of the device 120 includes delivering delivery catheter 1406 into an internal chamber (e.g., left ventricle LV) of the heart H and adjacent the heart wall W. Delivery catheter 1406 is extended from the distal end 1454 of the steerable catheter (not shown). The desired position on endocardium 102 is selected and anchoring device 1456 is positioned to abut or be adjacent to the endocardium 102. Delivery catheter 1406 is shown in a non-compressed position.

Referring to Figures 113H and 113I, anchoring device 1456 is pressed against endocardium 102 with a force causing piston 1500 to move proximally into delivery catheter 1406. The force at which anchoring device 1456 is pressed against endocardium 102 can be selected to optimize delivery of anchoring device 1456 into endocardium 102 and myocardium 104. Referring to Figure 113I, the movement of piston 1500 in the A' direction relative to delivery catheter 1406 causes the distance between marker 1526 and marker 1530 to decrease.

With reference to Figure 113I, anchoring device 1456 is positioned at a suitable pressure against heart wall W and piston 1500 is shown in the activated position. Marker 1526 abuts marker 1530 and distal end 1458 of delivery catheter 1406. A user using a scope can be observe the activated position by determining that marker 1526 abuts marker 1530. This can signify to the user that the anchoring device 1456 is ready to be attached to heart wall W.

With reference to Figure 113J-113N, anchoring device 1456 is attached to the heart wall W. In the illustrated example, the anchoring device 1456 can be attached to the heart wall W by rotating the delivery catheter 1406 about illustrated axis Z in the direction of arrow A2 (Figure 113J). As a result, the anchoring device 1456 is screwed into the heart wall W through the endocardium 102 and into the myocardium 104.

With reference to Figures 113A and 113J-113K, anchoring device 1456 is partially rotated into the heart wall W. The torque applied to the delivery catheter 1406 is less than a pre-determined torque threshold. With reference to Figure 113K, when delivery catheter 1406 or piston body 1502 is rotated, arms 1534 contact pins 1536. Due to this contact, piston head 1504 and anchoring device 1456 rotate at the same rate as delivery catheter 1406 and piston body 1502. The resulting gradually increasing torque can signify to a user to continue rotating anchoring device 1456 into heart wall W.

With reference to Figures 113A and 113L-113N, anchoring device 1456 is adequately attached to heart wall W. The torque applied to the delivery catheter 1406 meets and surpasses the pre-determined torque threshold. With reference to Figure 113M, the torque applied to the delivery catheter 1406 reaches a pre-determined torque threshold. Arms 1534 can flex as the pins 1536 travel over the arms.

With reference to Figure 113N, the torque applied to the delivery catheter 1406 surpasses the pre-determined torque threshold. Pins 1536 travel past the arms 1534, causing delivery catheter 1406 and piston body 1502 to rotate with respect to cap of the piston head 1504. The pins 1536 moving past the arms 1534 prevents or inhibits excessive torque being applied to the anchoring device 1456, and thereby can prevent anchoring device 1456 being over exerted into the heart wall W. Pins 1536 slipping past the arms 1534 can cause a sudden decrease in resistance experienced by the user applying the torque. The decrease in resistance can signify that the threshold torque value has been surpassed and that the rotation of delivery catheter 1406 should be slowed, stopped, or reversed.

Referring to Figures 114A, the piercing device 130 is delivered into an internal chamber (e.g., left ventricle LV) of the heart H via delivery catheter 1406 and piston 1500. The piercing device 130 can be extended from the distal end 1458 of the delivery catheter 1406 and through distal end 1506 of piston head 1504, such that the distal end 1460 of the piercing device 130 is extended into the heart wall W. In the illustrated example, the distal end 1460 of the piercing device 130 is extended through the endocardium 102, the myocardium 104, the epicardium 106, and into the pericardial cavity 110 to form the passage 132. Since the delivery catheter 1406 is anchored to the heart wall W, the location for insertion of the piercing device 130 can be precisely controlled.

To verify that the piercing device 130 is properly positioned for deploying the anchor 122 into the pericardial cavity 110, a dye 134, or other detectable fluid, can be delivered through the piercing device 130 and injected into the pericardial cavity 110, as shown in Figure 115A. The dye 134 can be detected by any suitable technique such as X-ray, to verify that the piercing device 130 is properly positioned. Referring to Figure 115B, in another example a wire 300 or other radio-opaque marker can be delivered through the piercing device 130 and into the pericardial cavity 110 to verify that the piercing device 130 is properly positioned, instead of the dye.

In Figure 116A, the anchor 122 and the hemostatic plug 1410 are extended from the delivery catheter 1406 over the piercing device 130 (Figure 114A) and through the passage 132. As shown in Figure 116A, the anchor 122 remains in the elongated state while sliding along the piercing device 130 into the pericardial cavity 110.

In Figure 117A, the anchor 122 is partially deployed beyond the distal end 1460 (Figure 114A) of the piercing device 130. Pusher 1408 is illustrated partially extending from the delivery catheter 1406 and piston 1500 through the anchoring device 1456. As the pusher 1408 extends from the piston 1500, the distal end 1464 of the pusher 1408 engages the hemostatic plug 1410 to push both the hemostatic plug 1410 and the anchor 122 over the piercing device 130. As the distal end 1432 of the anchor 122 moves beyond the distal end 1460 of the piercing device 130, the anchor 122 can begin to reshape into its curved deployed state. For example, the anchor 122 can include a shape memory alloy that is shape set to the curved deployed state. Thus, the portion of the anchor 122 that is no longer received on the piercing device 130 can revert to the curved deployed state that shape memory alloy was set to.

In Figure 118A, the anchor 122 is in the deployed state and the hemostatic plug 1410 is positioned within the passage 132 to prevent or inhibit bleeding from the passage 132. In the illustrated example, the distal end 1444 of the hemostatic plug 1410 is at or near the inner wall of the pericardial cavity 110. To reshape the anchor 122, the line 124 is withdrawn by pulling the line 124 in a direction away from the anchor 122 and into the hemostatic plug 1410. Since the loop 1440 of the line 124 passes through the loops 1442 on the anchor 122, pulling the line 124 closes the loop 1440, pulls the hemostatic plug 1410 and the anchor 122 together, and pulls the loops 1442 together to help facilitate, along with any shape setting properties of the anchor 122, reshaping the anchor 122 from the elongated state to the curved deployed state shown in Figure 119A.

As shown in Figures 119A-119B, the pusher 1408 and piercing device 130 can be removed by withdrawing them from the passage 132 into the delivery catheter 1406. The anchor 122, hemostatic plug 1410, and line 124 are left deployed in the heart wall W with the anchor 122 within the pericardial cavity 110 and the line 124 extending through the epicardium 106, the myocardium 104, and the endocardium 102.

Referring to Figure 119A, to seat the anchor 122, line 124 can be placed in tension by pulling the line 124 in an inward direction toward the heart chamber as shown by the arrow A9. As a result, the anchor 122 will be pulled against the outward facing surface 126 that partially defines the pericardial cavity 110, as shown by arrows A10. As the line 124 is being pulled in the direction of A9, the hemostatic plug 1410 is urged in the opposite direction, as shown by arrow A11, such that the distal end 1444 of the hemostatic plug 1410 is positioned at or adjacent the center of the anchor 122. The anchor 122 in its deployed state is too large to fit through the passage 132 formed by the piercing device 130.

With reference to Figure 119C, anchoring device 1456 can be detached from the heart wall W. In the illustrated example, the anchoring device 1456 can be detached from the heart wall W by rotating the delivery catheter 1406 about illustrated axis Z in the direction of arrow A13, the direction opposite of direction A2 (Figure 113J). As a result, the anchoring device 1456 can screw out from heart wall W. Piston 1500 can be in the activated or de-activated position when detaching anchoring device 1456 from heart wall W.

With reference to Figures 113A and 119D, anchoring device 1456 is partially rotated out from the heart wall W. When delivery catheter 1406 is rotated about illustrated axis Z in the direction of arrow A13 (Figure 119C), ends of arms 1534 contact pins 1536. Due to this contact, piston head 1504 and anchoring device 1456 rotate at the same rate as delivery catheter 1406 and piston body 1502. When removing anchoring device 1456, pins 1536 will maintain constant contact with arms 1534 at all torques. Arms 1534 will not flex to allow pins 1536 to pass arms 1534 as anchoring device is rotated out from heart wall W, even if the torque applied in the A13 direction reaches or surpasses the torque threshold predetermined in the A2 direction. Torque is therefore not limited in the A13, removal direction as it is in the A2, anchoring direction. This is due to the hard stop between the end of the arms and the pins in the removal direction, instead of the gradual ramp engagement in the am boring direction.

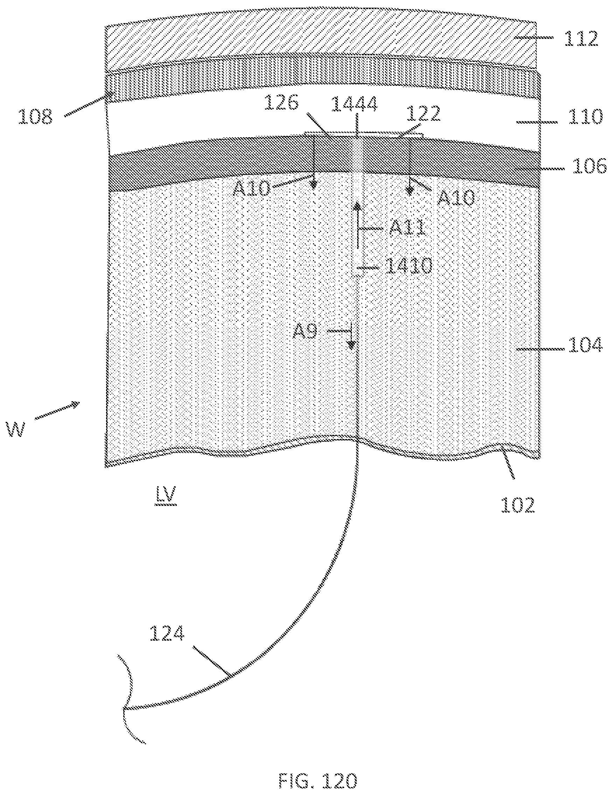

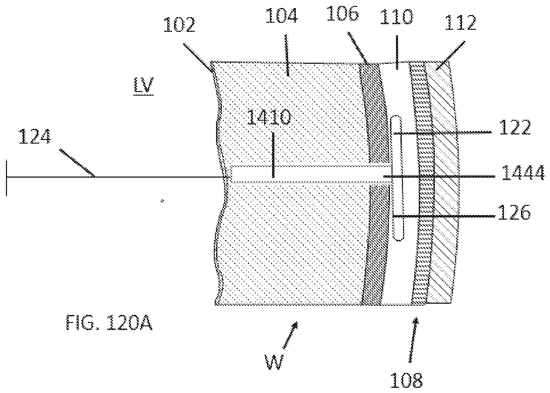

With reference to Figure 120A, once the delivery catheter 1406 is removed, further tensioning of the line 124 can pull the heart wall W inward toward the heart chamber (e.g., the left ventricle LV).

The piston and/or clutch examples of Figures 112A-112G, 113A-113N, 114A, 115A, 115B, 116A, 117A, 118A, 119A-119D, 120A can be used in any of the examples of the present application. For example, Figures 26A, 27A, and 28A illustrate use of the piston and/or clutch in the example of Figures 26-28 that is described above. In Figures 26A and 27A, device 120 for remodeling the shape of a heart wall W can be delivered through the delivery catheter 136. In particular, the delivery catheter 136 is extended through piston 1500 and through the passage 132 such that the distal end 138 of the delivery catheter 136 is positioned within the pericardial cavity 110. As shown in Figure 27A, with the distal end 138 of the delivery catheter 136 is positioned within the pericardial cavity 110, the device 120 for remodeling the shape of a heart wall W can be delivered through the delivery catheter 136. In particular, the anchor 122 can be extended out of the distal end of the 138 of the delivery catheter 136 and into the pericardial cavity 110 while the attached line 124 extends through the delivery catheter 136 in the passage 132.

With reference to Figures 28A, the delivery catheter 136 and the piercing device 130 can be removed by withdrawing them from the passage 132 and from the heart chamber. The device 120 is left deployed through heart wall W with the anchor 122 engaging an outward facing surface 126 of the heart wall W and the line 124 extending through the epicardium 106, the myocardium 104, and the endocardium 102 and into the heart chamber (e.g., the left ventricle LV). Line 124 can be placed in tension by pulling the line 124 in an inward direction toward the heart chamber as shown by the arrow A12.

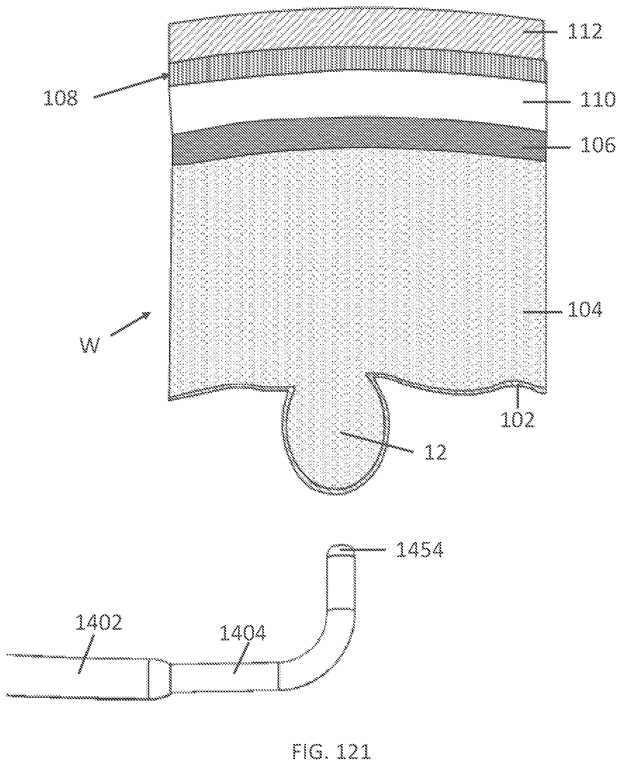

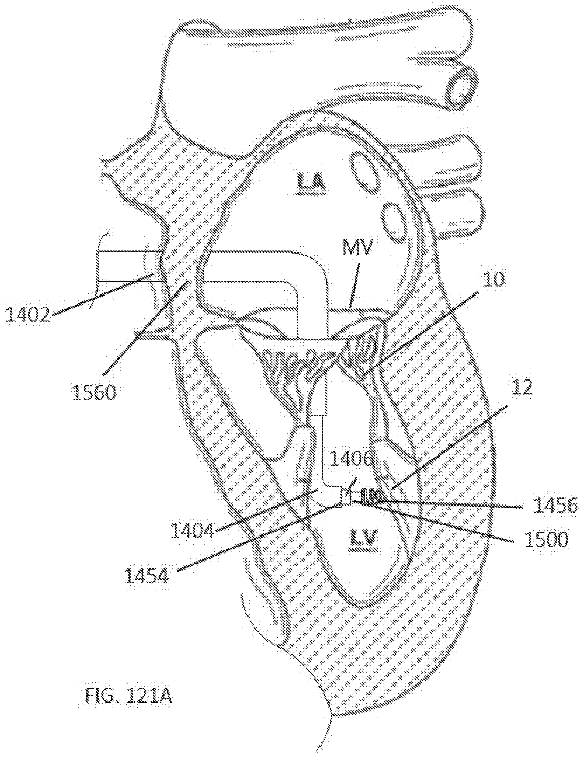

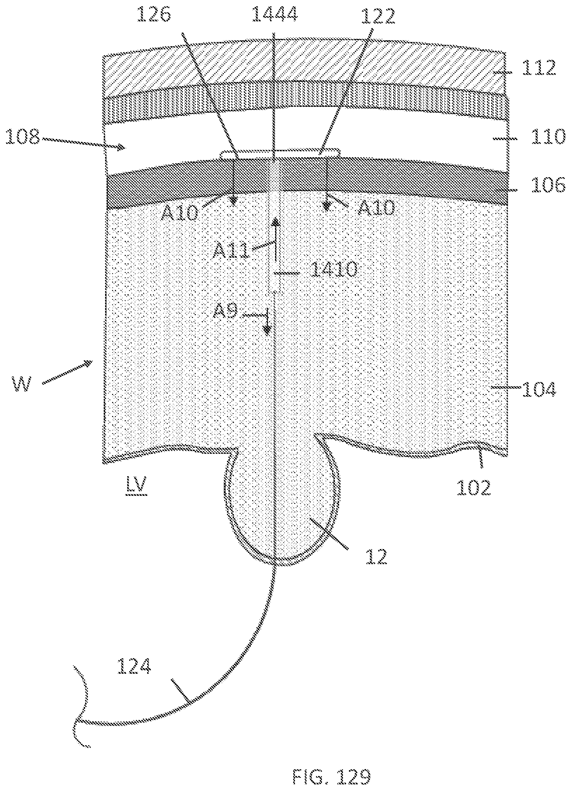

Referring to Figures 121-129, the deployment of the device 120 into the pericardial cavity 110, and through one of the papillary muscles 12, for remodeling the shape of a heart wall W and system and method for delivering the device 120 is illustrated. Referring to Figure 121, deployment of the device 120 includes delivering the guide sheath 1402 and the steerable catheter 1404 into an internal chamber (e.g., left ventricle LV) of the heart H. The steerable catheter 1404 is arranged such that the distal end 1454 of the steerable catheter 1404 is adjacent one of the papillary muscles 12 of the heart H.

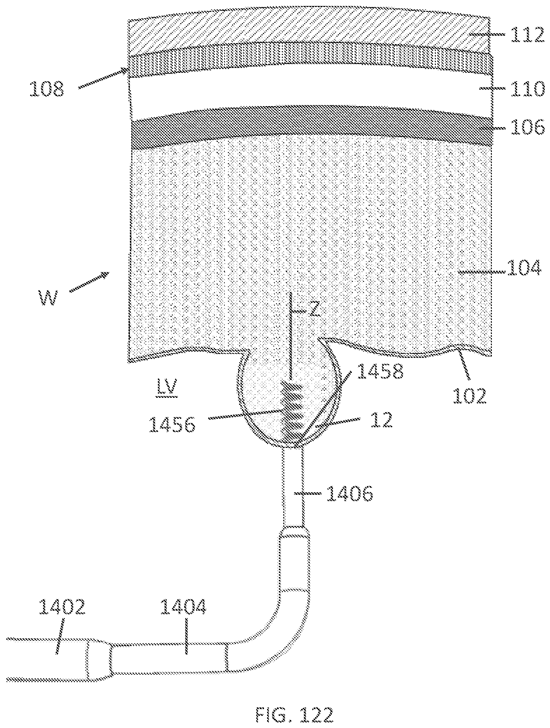

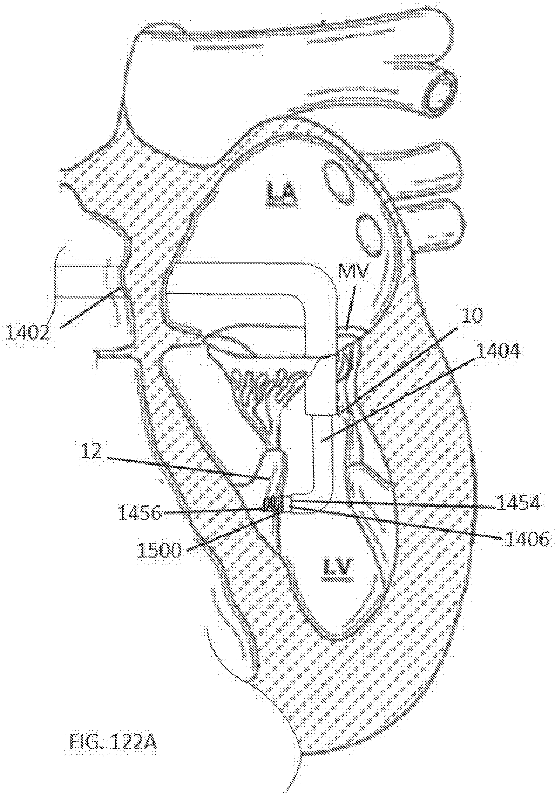

Referring to Figure 122, the delivery catheter 1406 is extended from the distal end 1454 of the steerable catheter 1404. The anchoring device 1456 of the delivery catheter 1406 is attached to the papillary muscle 12, such as for example by rotating the delivery catheter 1406 about axis Z relative to the steerable catheter 1404 to screw the anchoring device 1456 into papillary muscle 12. In the illustrated example, the distal end 1458 of the delivery catheter 1406 abuts, or is adjacent, the papillary muscle 12.

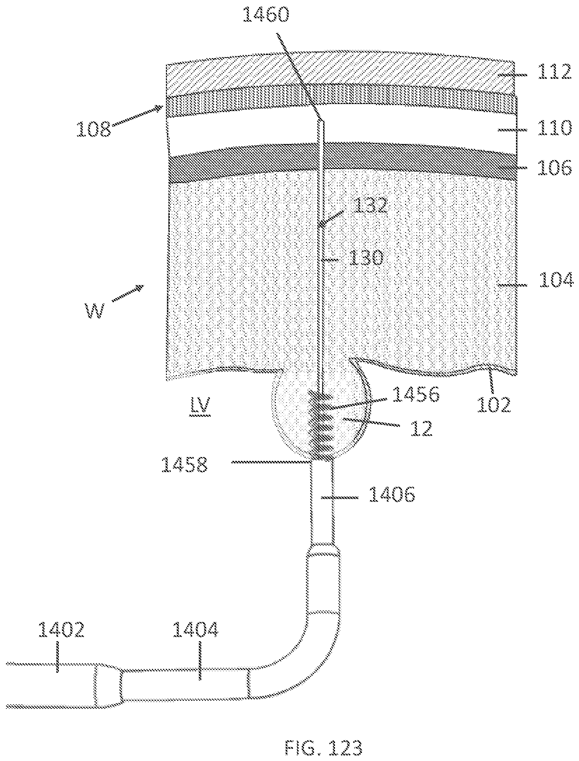

Referring to Figure 123. the piercing device 130 is delivered into an internal chamber (e.g., left ventricle LV) of the heart H via the delivery catheter 1406. The piercing device 130 can be extended from the distal end 1458 of the delivery catheter 1406 such that the distal end 1460 of the piercing device 130 is extended through the papillary muscle 12 and the heart wall W. In the illustrated example, the distal end 1460 of the piercing device 130 is extended through the papillary muscle 12, the endocardium 102, the myocardium 104, the epicardium 106, and into the pericardial cavity 110 to form the passage 132. Since the delivery catheter 1406 is anchored to the papillary muscle 12, the location for insertion of the piercing device 130 can be precisely controlled.

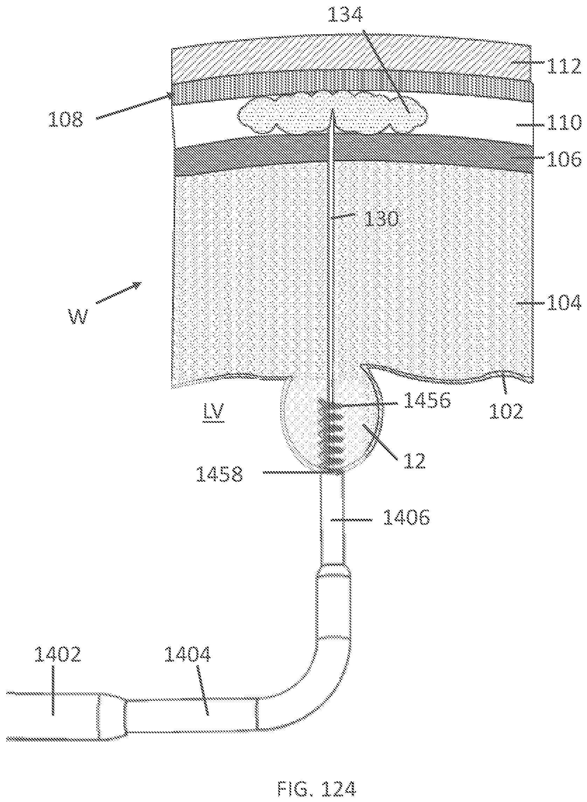

To verify that the piercing device 130 is properly positioned for deploying the anchor 122 into the pericardial cavity 110, a dye 134, or other detectable fluid, can be delivered through the piercing device 130 and injected into the pericardial cavity 110, as shown in Figure 124. The dye 134 can be detected by any suitable technique such as X-ray, to verify that the piercing device 130 is properly positioned.

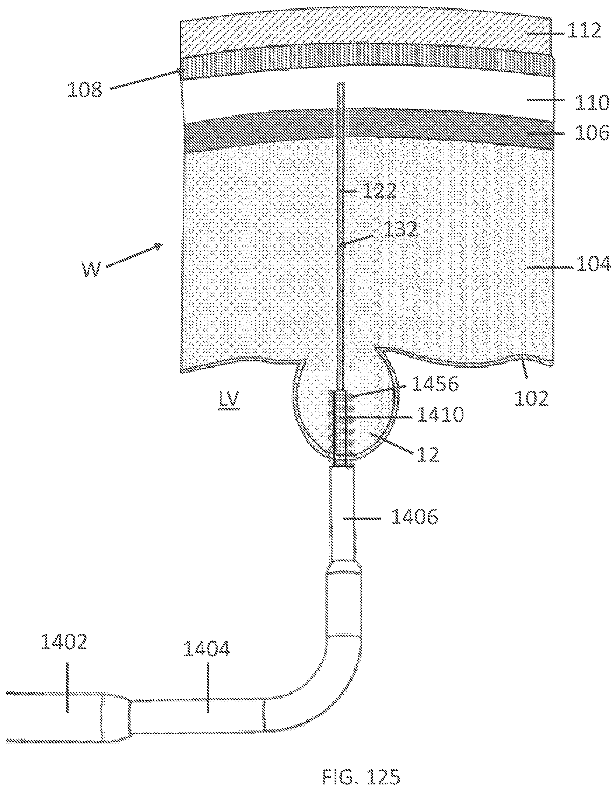

In Figure 125, the anchor 122 and the hemostatic plug 1410 are extended from the delivery catheter 1406 over the piercing device 130 (Figure 115) and through the passage 132. As shown in Figure 116, the anchor 122 remains in the elongated state while sliding along the piercing device 130 into the pericardial cavity 110.

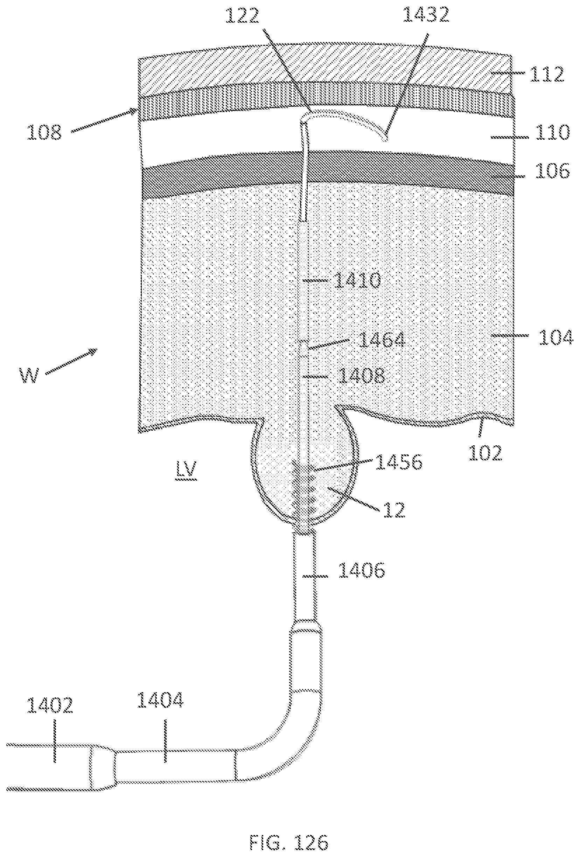

In Figure 126, the anchor 122 is partially deployed beyond the distal end 1460 of the piercing device 130 (Figure 123). The delivery catheter 1406 is illustrated partially extending from the steerable catheter 1404 and the pusher 1408 is illustrated partially extending from the delivery catheter 1406 through the anchoring device 1456. As the pusher 1408 extends from the delivery catheter 1406, the distal end 1464 of the pusher 1408 engages the hemostatic plug 1410 to push both the hemostatic plug 1410 and the anchor 122 over the piercing device 130 (Figure 123). As the distal end 1432 of the anchor 122 moves beyond the distal end 1460 of the piercing device 130 (Figure 123), the anchor 122 can begin to reshape into its curved deployed state. For example, the anchor 122 can include a shape memory alloy that is shape set to the curved deployed state. Thus, the portion of the anchor 122 that is no longer received on the piercing device 130 can revert to the curved deployed state that that shape memory alloy was set to.

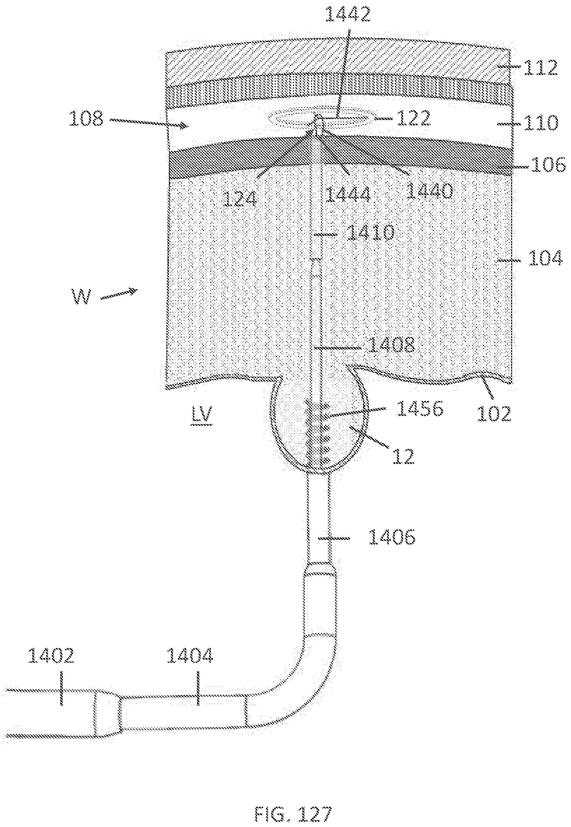

In Figure 127, the anchor 122 is in the deployed state and the hemostatic plug 1410 is positioned within the passage 132 to prevent or inhibit bleeding from the passage 132. In the illustrated example, the distal end 1444 of the hemostatic plug 1410 is at or near the inner wall of the pericardial cavity 110. To reshape the anchor 122, the line 124 is withdrawn by pulling the line 124 in a direction away from the anchor 122 and into the hemostatic plug 1410. Since the loop 1440 (Figure 97) of the line 124 passes through the loops 1442 on the anchor 122, pulling the line 124 closes the loop 1440, pulls the hemostatic plug toward the anchor, and pulls the loops 1442 together to help facilitate, along with any shape setting properties of the anchor 122, reshaping the anchor 122 from the elongated state to the curved deployed state shown in Figures 127.

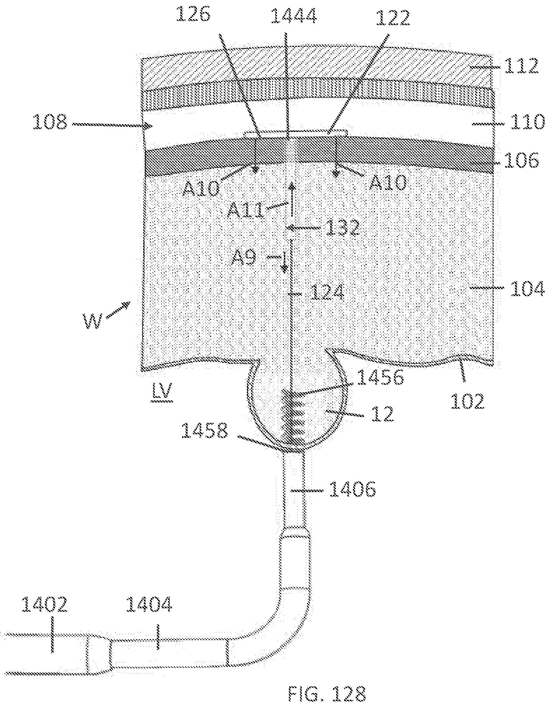

As shown in Figures 128-129, the pusher 1408 and piercing device can be removed by withdrawing them from the passage 132 into the delivery catheter 1406. The anchor 122, hemostatic plug 1410, and line 124 are left deployed in the heart wall W with the anchor 122 within the pericardial cavity 110 and the line 124 extending through the epicardium 106, the myocardium 104, the endocardium 102, and the papillary muscle 12.

To seat the anchor 122, the delivery catheter 1406 can remain attached to the heart wall W and the distal end 1458 can be pushed against the heart wall W. At the same time, the line 124 can be placed in tension by pulling the line 124 in an inward direction toward the heart chamber as shown by the arrow A9. As a result, the anchor 122 will be pulled against the outward facing surface 126, which in the illustrated example is the inner pericardium layer (i.e., the visceral layer of the serous pericardium) that partially defines the pericardial cavity 110, as shown by arrows A10. As the line 124 is being pulled in the direction of A9, the hemostatic plug 1410 is urged in the opposite direction, as shown by arrow A11, such that the distal end 1444 of the hemostatic plug 1410 is positioned at or adjacent the center of the anchor 122. The anchor 122 in its deployed state is too large to fit through the passage 132 formed by the piercing device 130. Thus, once the delivery catheter 1406 is removed, further tensioning of the line 124 can pull the heart wall W inward toward the heart chamber (e.g., the left ventricle LV).

The piston and/or clutch examples of Figures 112A-112G, 113A-113N, 114A, 115A, 115B, 116A, 117A, 118A, 119A-119D, 120A can be used to deploy one or more devices 120 into one or more papillary muscles 12, for remodeling the shape of a heart wall W. For example, with reference to Figures 121A and 122A, guide sheath 1402 can puncture and extend through atrial-septal wall and can optionally be guided through the mitral valve MV. Steerable catheter 1404 can extend from guide sheath 1402. The steerable catheter 1404 is arranged such that the distal end 1454 of the steerable catheter 1404 can be steered to be adjacent one of the papillary muscles 12 of the heart H. Delivery catheter 1406, piston 1500, and anchoring device extend from the distal end 1454 of the steerable catheter 1404. Device 120 can be delivered through the papillary muscle 12 in accordance with Figures 112A-112G, 113A-113N, 114A, 115A, 115B, 116A, 117A, 118A, 119A-119D, 120A.

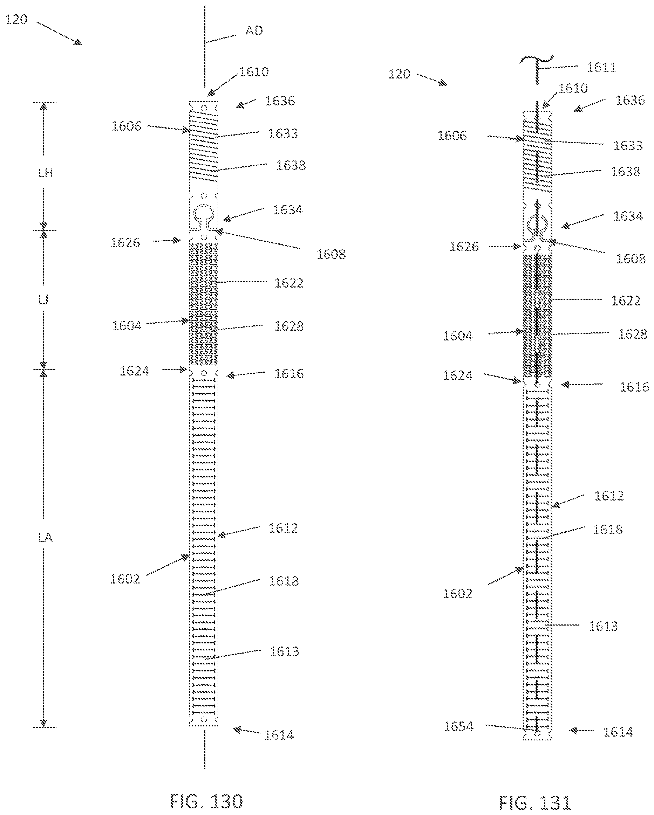

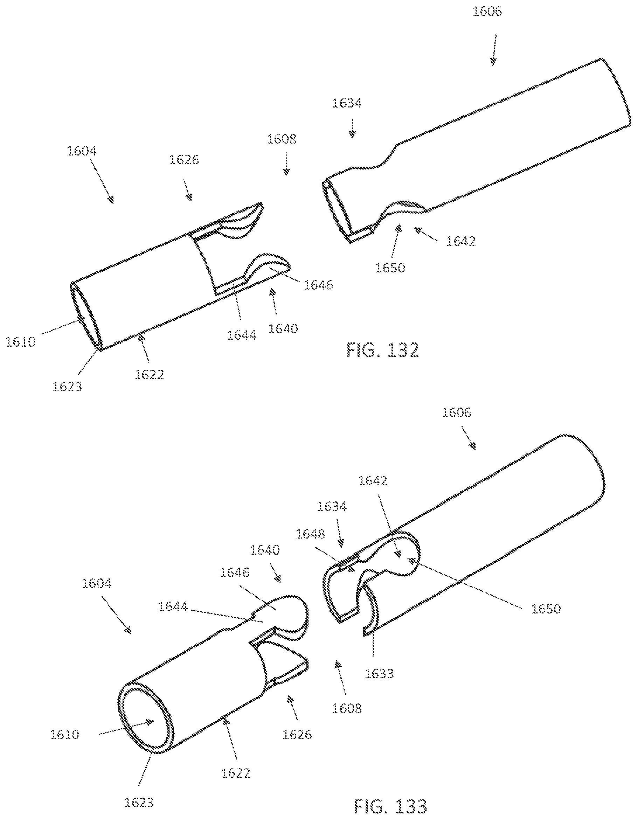

Referring to Figure 130, an example of the device 120 for remodeling the shape of a heart wall is illustrated. The device 120 includes a longitudinal axis AD, an anchoring portion 1602, a joint portion 1604, and a hemostasis element 1606 detachably connected to the joint portion 1604 by and an attachment portion 1608. In the illustrated example, the anchoring portion 1602, the joint portion 1604, the hemostasis element 1606, and the attachment portion 1608 are formed from a single tube having a central passage 1610 (Figures 132-133). For example, a single tube can be laser cut to form the device 120. In other examples, however, the device 120 can be formed from separate components. The inner passage 1610 is configured to receive an elongating device 1611 (depicted in dashed lines in Figure 131) therethrough. The elongating device 1611 can be any suitable device that when received in the inner passage 1610 coaxially aligns the anchoring portion 1602, the joint portion 1604, and the hemostasis element 1606. When the elongating device 1611 is received within the central passage 1610, the device 120 is held in an elongated delivered state by the elongating device 1611. In some examples, the elongating device 1611 is configured as a piercing device capable of creating a passage into a human heart wall, such as for example, a needle, wire, or other similar device.

The anchoring portion 1602 can be configured in a variety of ways. Any configuration that can be positioned to engage an outward facing surface of a heart wall W to support pulling a portion of the heart wall W inward (i.e., toward an internal chamber of the heart) can be used. In the illustrated example, the anchoring portion 1602 is reconfigurable such that the anchoring portion 1602 can be delivered through a catheter or sheath in a delivered state (e.g., elongated as shown in Figure 130) that fits within a lumen of a delivery catheter (e.g., delivery catheter 1406 of Figure 107), and can be reshaped to a deployed state once it has been delivered to the appropriate location.

The anchoring portion 1602 has a generally cylindrical, elongated anchor body 1612 having a cylindrical sidewall 1613 forming a tube. In other examples, however, the anchor body 1612 can be shaped other than cylindrical. For example, the elongated anchor body 1612 can have an oval, rectangular, or other shaped cross section. The anchor body 1612 has a length LA and includes a distal first end portion 1614 and a proximal second end portion 1616 opposite the first end portion 1614.

In the illustrated example, the anchor body 1612 includes one or more features to facilitate bending of the anchor body 1612. The features can be configured in a variety of ways. In the illustrated example, the features include a series of traverse cuts 1618 along the anchor body 1612. In one example, the series of cuts 1618 are a plurality of cuts where each of the cuts is generally perpendicular to the longitudinal axis AD. In the illustrated example, the cuts in the series of cuts 1618 are evenly spaced along the anchor body 1612 and extend from the first end portion 1614 to the second end portion 1616. For example, the series of cuts 1618 can extend over at least 80% of the length LA of the anchor body 1612. In other examples, the series of cuts 1618 are not be evenly spaced and can extend less than 80% of the length LA of the anchor body 1612. Further, each of the cuts of the series of cuts 1618 extends partially into the anchor body 1612. In one example, each of the cuts extend between 25%-75% through the anchor body 1612.

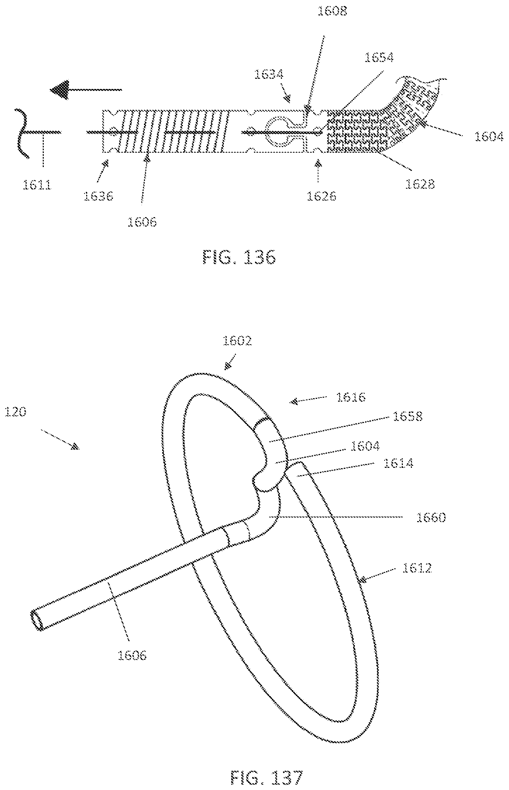

In the illustrated example, the anchoring portion 1602 includes a shape-memory alloy-such as Nitinol-to provide shape-setting capability such that anchoring portion 1602 is shape set to a curved, deployed state (as shown in Figure 137).

The joint portion 1604 can be configured in a variety of ways. In the illustrated example, the joint portion 1604 has a generally cylindrical, elongated joint body 1622 having a cylindrical sidewall 1623 forming a tube. In other examples, however, the joint body 1622 can be shaped other than cylindrical. For example, the elongated joint body 1622 can have an oval, rectangular, or other shaped cross section. The joint body 1622 has a length LJ and includes a distal first end portion 1624 and a proximal second end portion 1626 opposite the first end portion 1624. In the illustrated example, the joint portion 1604 is formed integrally with the anchoring portion 1602 (i.e. formed from the same tube). In other examples, however, the joint portion 1604 are not be integrally formed with the anchoring portion 1602 but formed as a separate piece fixedly attached to the anchoring portion 1602 in any suitable manner.

In the illustrated example, the joint body 1622 includes one or more features to facilitate bending of the joint body 1622. The features can be configured in a variety of ways. In the illustrated example, the features include a series of cuts 1628 along the joint body 1622 that form a pattern that allows for bending of the joint body 1622 in multiple directions with a large degree of rotation of the joint body. For example, the joint body can be configured to allow between 180 degrees and 720 degrees of rotation of the joint body 1622, such as rotation between 270 degrees and 480 degrees of rotation of the joint body 1622, such as rotation between 315 degrees and 415 degrees of rotation of the joint body 1622, such as about 360 degrees of rotation of the joint body 1622. Any suitable pattern of cuts that allows for bending of the joint body 1622 in multiple directions between 180 degrees and 720 degrees of the joint body 1622 can be used. In one example, the series of cuts 1628 are an interlocking pattern of projections and grooves that extend from the majority of the length LJ of the joint body 1622. For example, the series of cuts 1628 can extend over at least 80% of the length LJ of the joint body 1622. In other examples, the series of cuts 1628 can extend less than 80% of the length LJ of the joint body 1622. Further, each of the cuts of the series of cuts 1618 extends partially into the joint body 1622. In one example, each of the cuts extend between 25%-75% through the joint body 1622.

In the illustrated example, the joint portion 1604 includes a shape-memory alloy-such as Nitinol-to provide shape-setting capability such that joint portion 1604 is shape set to a curved, deployed state (as shown in Figure 137).

The hemostasis element 1606 can be configured in a variety of ways. Any device that can stop bleeding from occurring from a passage formed by the elongating device 1611, or another piercing device, in a heart wall W can be used. In the illustrated example, the hemostasis element 1606 includes a cylindrical sidewall 1633 forming as a tube having a length LH, a distal first end 1634, and a proximal second end 1636 opposite the distal first end 1634. In other examples, however, the hemostatic element 1606 can be configured other than cylindrical.

In the illustrated example, the hemostasis element 1606 includes one or more features to facilitate bending of the hemostasis element 1606. The features can be configured in a variety of ways. In the illustrated example, the features include a series of traverse cuts 1638 along the hemostasis element 1606. In one example, the series of cuts 1638 are a plurality of cuts where each of the cuts is slanted relative to a longitudinal axis AD of the device 120. In the illustrated example, the cuts in the series of cuts 1638 are evenly spaced along the hemostasis element 1606 and extend between the first end portion 1634 to the second end portion 1636. For example, the series of cuts 1638 can extend over at least 50%, or at least 60%, or at least 70%, of the length LH of the hemostasis element 1606. In other examples, the series of cuts 1638 are not evenly spaced and can extend less than 50% of the length LH of the hemostasis element 1606. Further, each of the cuts of the series of cuts 1638 extends partially into the hemostasis element 1606. In one example, each of the cuts extend between 25%-75% through the hemostasis element 1606.

Referring to Figures 132-133, the distal first end 1634 of the hemostasis element 1606 and the proximal second end portion 1626 of the joint body 1622 cooperatively form the attachment portion 1608. The attachment portion 1608 can be configured in a variety of ways. Any structure that can connect the hemostasis element 1606 to the joint body 1622 during delivery of the device 120 and allow the hemostasis element 1606 to detach from the joint body 1622 when desired can be used. In the illustrated example, the attachment portion 1608 includes a pair of projections 1640 on the joint body 1622 and a pair of recesses 1642 on the hemostasis element 1606 configured to receive the pair of projections 1640. In other examples, the attachment portion 1608 can include more or less than two projections 1640 and recesses 1642. Further, in other examples, the projections can be located on the hemostasis element 1606 and the recesses 1642 can be located on the joint body 1622.

In the illustrated example, the pair of projections 1640 extend proximally from the cylindrical sidewall 1623 at the proximal second end portion 1626 of the joint body 1622. The projections 1640 are positioned on opposite sides of the cylindrical sidewall 1623. In other examples, the projections 1640 can be positioned at any suitable location on the joint body 1622. Each projection 1640 includes a stem portion 1644 adjacent the proximal second end portion 1626 and a head portion 1646 adjacent the stem portion 1644. The head portion 1646 is wider or enlarged as compared to the stem portion 1644.

The pair of recesses 1642 are formed in the cylindrical sidewall 1633 of the hemostasis element 1606 and extend proximally from the distal first end 1634. Each of the recesses 1642 is positioned to align axially with a corresponding one of the projections 1640. Thus, in the illustrated example, the pair of recesses 1642 are located on opposite sides of the cylindrical sidewall 1633. Each of the recesses 1642 includes a channel portion 1648 adjacent the distal first end 1634 and a head receiving portion 1650 adjacent the channel portion 1648. The channel portion 1648 is sized to receive the stem portion 1644 of the projection 1640 and the head receiving portion 1650 is wider than the channel portion 1648 and sized to the receive the head portion 1646. The head portion 1646 is wider than the channel portion 1648 such that when the head portion 1646 is received within the head receiving portion 1650 in a coupling position, the channel portion 1648 blocks movement of the head portion 1646 distally such that the hemostasis element 1606 and the joint body 1622 are coupled together.

The pair of projections 1640 includes a shape-memory alloy-such as Nitinol-to provide shape-setting capability. In the illustrated example, the pair of projections are shape set to a release position where the projections 1640 bend radially inward relative to the cylindrical sidewall 1623. In the release position, the projections 1640 bend inward sufficiently to be clear of the recesses 1642 and allow the hemostasis element 1606 to decouple from the joint portion 1604.

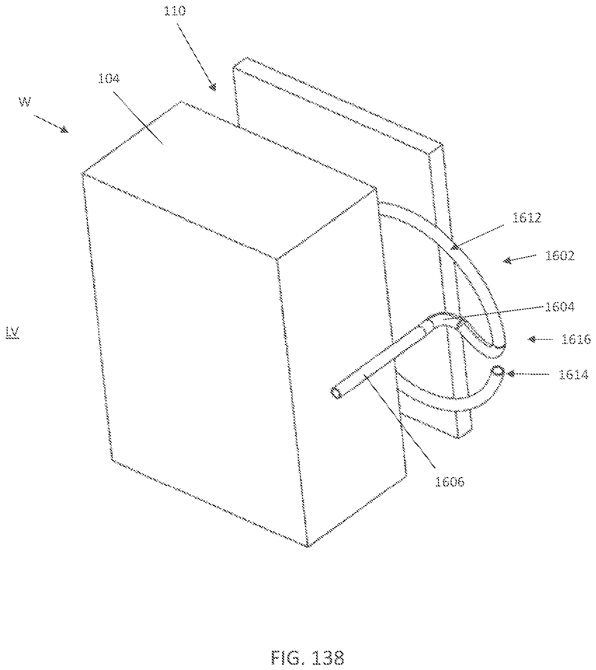

The device 120 can be deployed by any suitable delivery system, such as for example, any delivery system disclosed herein (e.g., the system 1400). The device 120 is deployed in the elongated delivered state over the elongating device 1611, as shown in Figure 131. In one example, the device 120 can be deployed in the pericardial cavity 110, as shown in Figure 138. The elongating device 1611 can form a passage (not shown) from an internal chamber (e.g., left ventricle LV) of the heart H, through the myocardium 104, and into the pericardial cavity 110.

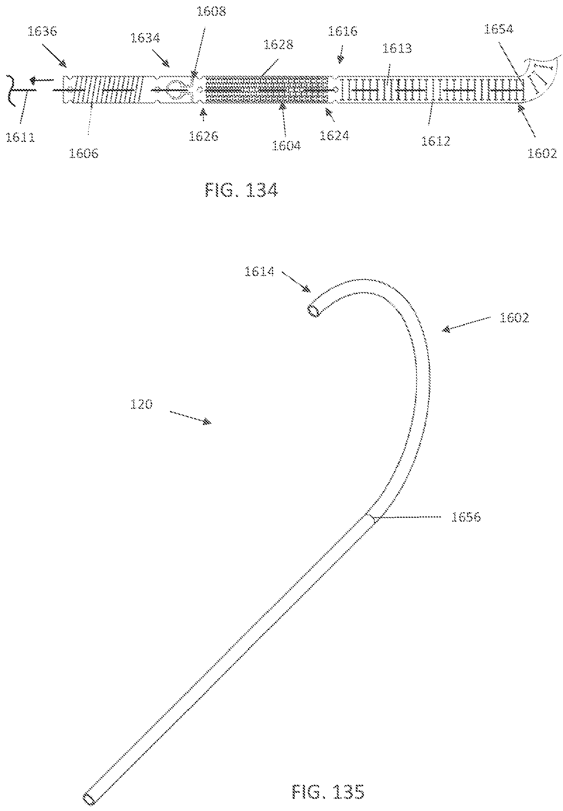

With the elongating device 1611 in position within the passage (not shown) and a tip 1654 of the elongating device 1611 within the pericardial cavity 110, the device 120 can be extended through the passage (not shown) over the elongating device 1611 and into the pericardial cavity 110. As shown by Figures 134-135, as the tip 1654 moves proximally relative to the device 120, shown by line 1656 in Figure 135, whether by distal movement of the device 120, proximal movement of the elongating device 1611, or a combination thereof, the portion of the device 120 distal to the tip 1654 will revert to the deployed state (i.e. the shape set shape). In the illustrated example, the section of the anchoring portion 1602 distal to the tip 1654 curves in a circular or semicircular shape.

Referring to Figures 136-138, when all of the anchoring portion 1602 of the elongating device 1611 extends beyond the tip 1654, the anchoring portion 1602 reverts to the ring-shaped, deployed state such that the distal first end portion 1614 is adjacent the proximal second end portion 1616 and the anchor body 1612 is curved in a circular or oval shape.

When the device 120 and/or are moved such that the joint portion 1604 extends beyond, the tip 1654, the section of the joint portion 1604 that no longer houses the elongating device 1611 reverts to the deployed state. In the deployed state, the shape of the joint portion 1604 can be set to curve in multiple directions to allow the operator to position the deployed anchoring portion 1602 within the pericardial cavity as desired. For example, in one example, a distal portion 1658 of the joint portion 1604 can curve in a first direction while a proximal portion 1660 can curve in a second direction that is different than the first direction. Thus, the amount that the elongating device 1611 extends through the joint portion 1604 can be used to control the curve of the joint portion 1604 and the position of the anchoring portion 1602.

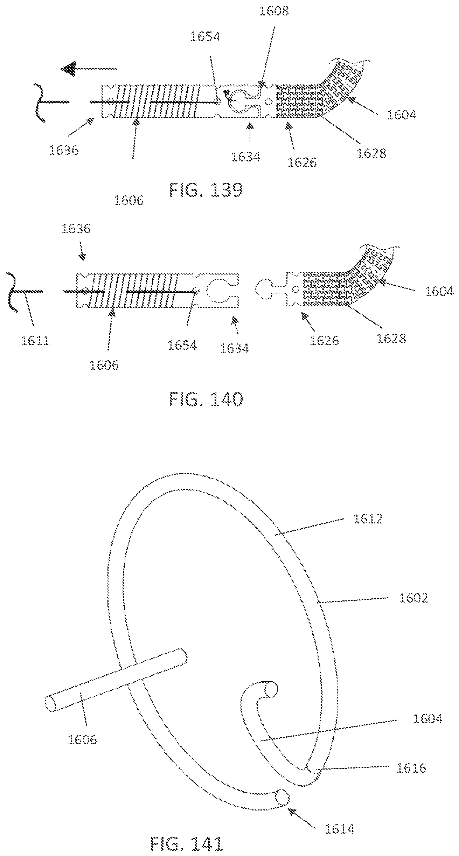

Referring to Figures 139-142, once the anchoring portion 1602 is correctly positioned within the pericardial cavity 110, the elongating device 1611 and/or the device 120 can be moved such that the attachment portion 1608 extends distally beyond the tip 1654 of the elongated device 1611. With the elongating device 1611 removed from the attachment portion 1608, the projections 1640 can revert to the release position where the projections 1640 bend radially inward relative to the cylindrical sidewall 1623 sufficiently to be clear of the recesses 1642 and allow the hemostasis element 1606 to decouple from the joint portion 1604.

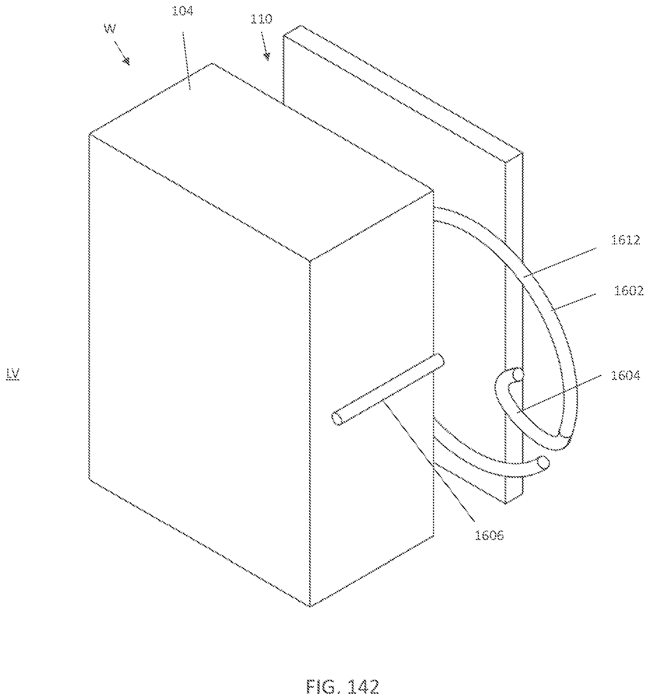

After decoupling the joint portion 1604 from the hemostasis element 1606, the anchoring portion 1602 and joint portion 1604 remain in the pericardial cavity 110 while the hemostasis element 1606 is positioned within the passage (not shown) in the myocardium 104, as shown in Figure 142. The elongating device 1611 can be withdrawn from the hemostasis element 1606 such that the hemostasis element 1606 remains positioned in the passage (not shown) after deployment of the device 120.

While not shown in Figures 130-142, one or more lines (e.g., line 124 of Figure 95) are connected to the device 120. The one or more lines can be connected to the device 120 in a variety of ways, such as any manner described herein (e.g., by the plurality of loops 1442 as shown in Figure 95). To seat the anchoring portion 1602, the one or more lines can be placed in tension by pulling the one or more lines in an inward direction toward the heart chamber (e.g., the left ventricle LV). Further tensioning of the line 124 can pull the heart wall W inward to a remodeled position.

Prior to decoupling the joint portion 1604 from the hemostasis element 1606, the device 120 can be withdrawn from the pericardial cavity 110 to reposition the device 120 or abort the deployment. In particular, the device 120 can be pulled back onto the elongating device 1611 to revert back to the elongated delivered state. The device 120 can then be pulled back through the passage (not shown) in the myocardium 104 to abort the deployment or redeployed within the pericardial cavity 110. Various devices and methods herein involve one or more anchors used to reshape human tissue, especially heart tissue. Such example devices and methods are described in detail in application WO 2020/219281. The present application illustrates example tissue remodeling systems using one or more anchors. It is appreciated that while the figures of the present application may depict a certain configuration of anchor (e.g. one or more helical anchors), the use of many different types of anchors is contemplated (including those disclosed by application WO 2020/219281), including the use of multiple types of anchors in a system.

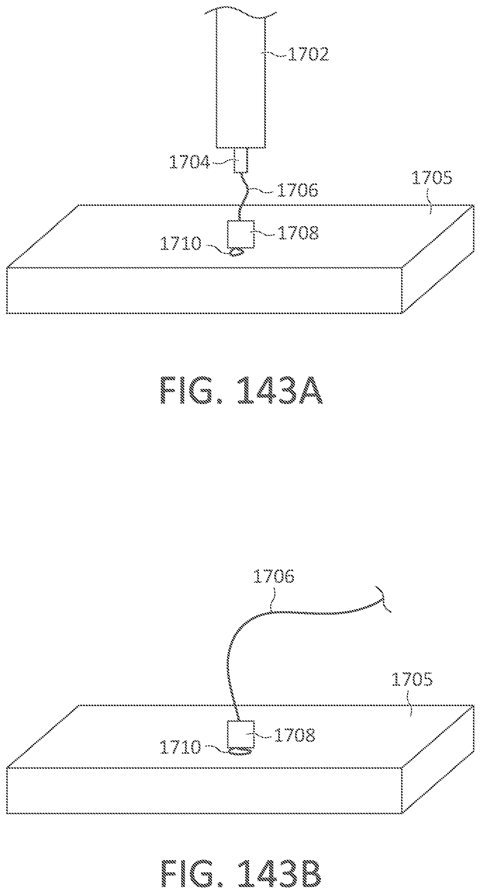

Figure 143A shows a fully deployed anchor 1710 into a target tissue area 1705. In certain examples the anchor 1710 has a stich depth SD between 0.02 mm and 2.0 mm. The anchor 1710 can be deployed using a deployment system 1702 comprising a deployment device 1704 which facilitates engagement of the anchor 1710 into the target issue area 1705. In some examples, a tether 1706 can be connected to the anchor 1710. In some examples, the tether 1706 is connected to the anchor 1710 via a surgical knot. In some examples, the tether 1706 is disposed within the anchor 1710. In other examples, the tether 1706 is attached to a coupler 1708. It is appreciated that tension between the anchor 1710 and the tether 1706 can be used to reshape tissue at the target tissue area 1705. In certain examples, the coupler 1708 is configured to create a variable tension between the tether 1706 and the anchor 1710, for example, by using a spring connected to the tether 1706. Figure 143B shows the anchor 1710 fully deployed with deployment system 1702 and the deployment device 1704 removed and the tether 1706 exposed.

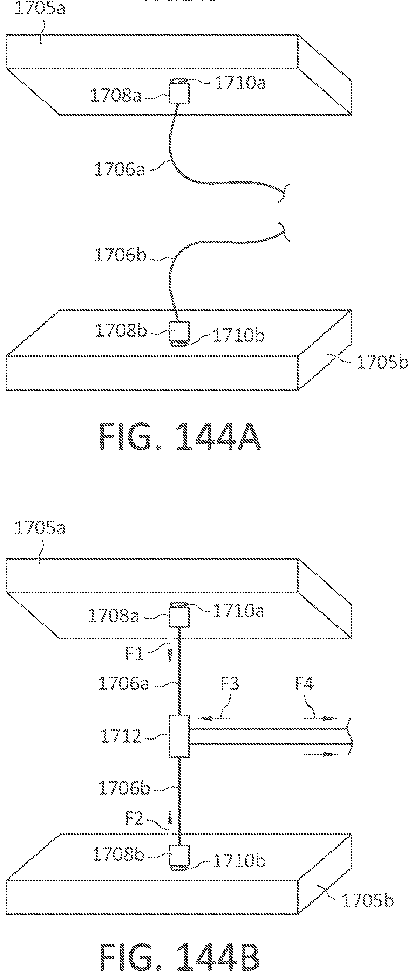

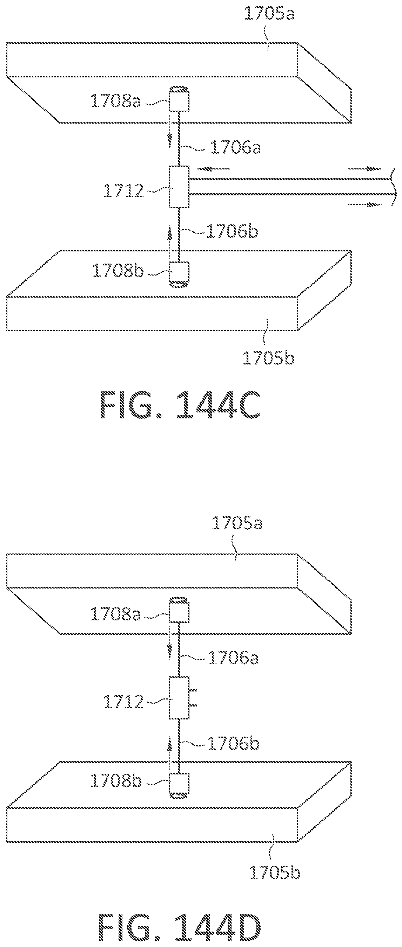

Figures 144A-D illustrate an example of tissue remodeling using a pair of anchors 1710. Figure 144A shows a first anchor 1710a deployed in a first target tissue area 1705a. Connected to the first anchor 1710a is a first coupler 1708a and a first tether 1706a. Proximate to the deployment location of the first anchor 1710a is a second target tissue area 1705b, where a second anchor 1710b, a second coupler 1708b, and a second tether 1706b are deployed. Figure 144B shows a tether lock 1712 being advanced along the first tether 1706a and the second tether 1706b as indicated by arrow F3. As the first tether 1706a and the second tether 1706b are pulled in the direction of arrow F4, the first anchor 1710a and the second anchor 1710b are pulled toward each other, which manipulates the engaged tissue at first and second target tissue areas 1705a, 1705b. Figure 144C shows the tether lock 1712 being further advanced along the first and second tethers 1706a, 1706b. Once the desired tissue manipulation has been achieved, the tether lock 1712 can be locked, which holds the first and second target tissue areas 1705a, 1705b in a remodeled position. Once locked in remodeled position, ends of first and second tethers 1706a, 1706b can be cut off as shown in Figure 144D. The tether lock 1712 can take a variety of forms. For example, any of the connectors described in detail in International Patent Publication WO 2020/219281 can be used as the tether lock 1712. In some examples, the tether lock 1712 is configured to create a variable tension at the first tether 1706a and/or the second tether 1706b. Various examples relating to the variable tension mechanisms used with the tether lock 1712, tether(s), and/or anchor(s) are described in detail herein.

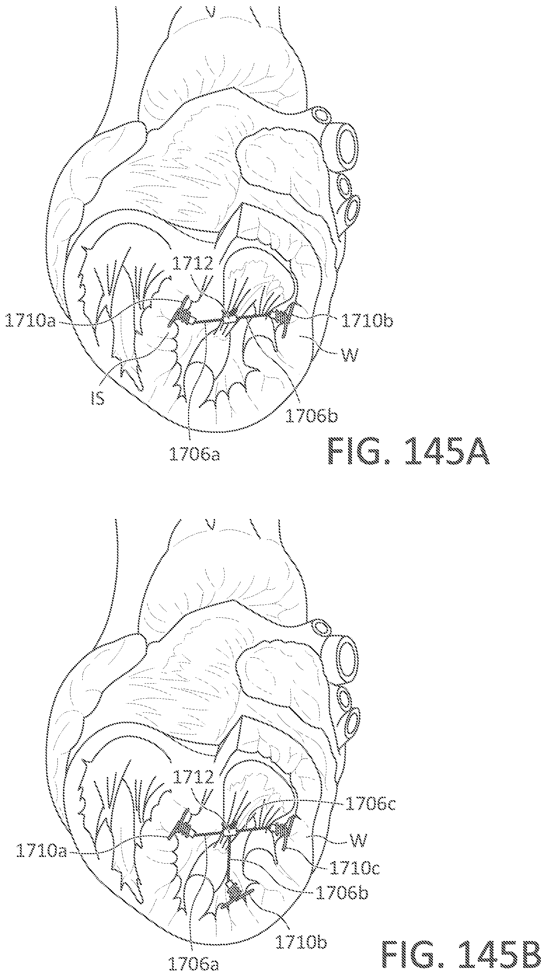

In Figure 145A, the second anchor 1710b is attached to the heart wall W and the first anchor 1710a is implanted on the interventricular septum IS. The first and second tethers 1706a, 1706b can be pulled inward to pull the heart wall W inward to remodel the shape of the heart wall W and the intraventricular septum IS. To hold the heart wall W and the intraventricular septum IS in the remodeled position, the first and second tethers 1706a, 1706b, while in tension, can be connected within the left ventricle LV, such as for example, by the tether lock 1712 or other suitable means for connecting the tethers. In some examples, first and second anchors 1710a, 1710b can be associated with one or more strain reliefs. Strain reliefs can be disposed within the anchor(s) and can limit and/or damp the load on first and second anchors 1710a, 1710b. For example, the strain reliefs can stretch and contract as the heart walls move toward and away from one another as the heart beats. As such, the strain reliefs stretch and contract to control the load applied to the heart walls by the tethered anchors.

The first and second anchors 1710a, 1710b can be used in a wide variety of different ways to remodel the heart and/or approximate the papillary muscles. A tether lock 1712 and two or more anchors can be deployed or more than one tether lock 1712 with two or more anchors per tether lock can be deployed to remodel the heart of a single patient. For example, any of the configurations disclosed herein can be used in combination on the heart of a single patient. In certain other examples, anchors (e.g., anchor 1710) can be associated with a coupler which can provide a variable force on an attached tether for remodeling heart tissue.

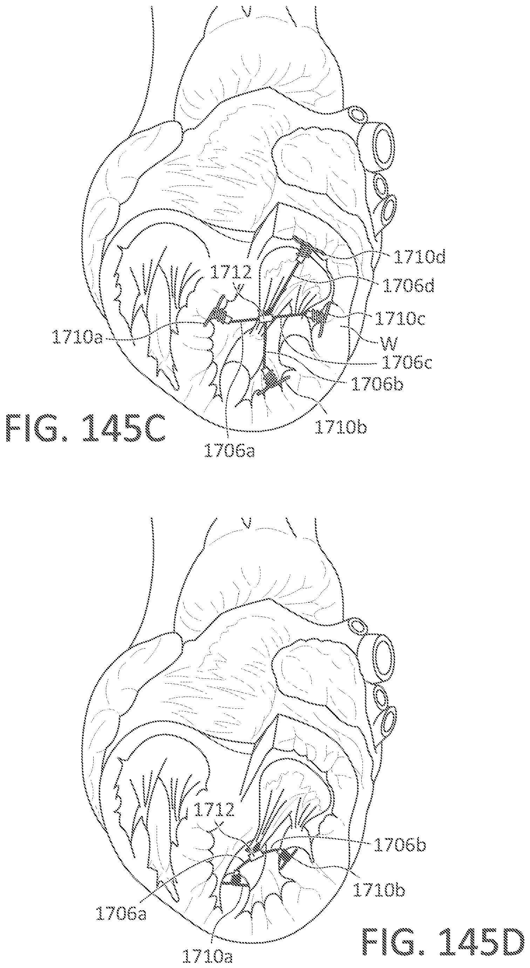

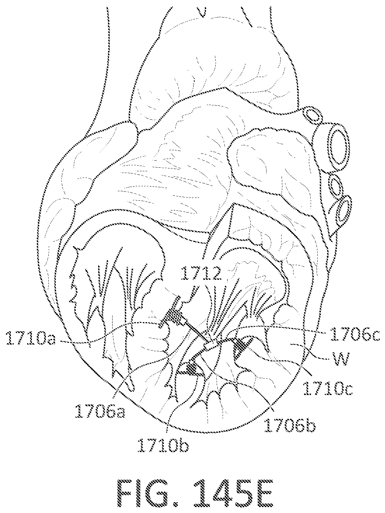

Figure 145B shows remodeling of the heart wall W and intraventricular septum IS using a first anchor 1710a deployed in intraventricular septum IS and second and third anchors 1710b, 1710c deployed in heart wall W. Figure 145C shows remodeling of the heart wall W and intraventricular septum IS using a first anchor 1710a deployed in intraventricular septum IS and second, third, and fourth anchors 1710b, 1710c, 1710d deployed in heart wall W. First, second, third, and fourth tethers 1706a-1706d connect the respective anchors 1710a-1710d. Figure 145D shows remodeling of papillary muscles using anchors 1710a and 1710b. Figure 145E shows remodeling of papillary muscles and intraventricular septum IS using a first anchor 1710a deployed in intraventricular septum IS and second and third anchors 1710b, 1710c deployed within the papillary muscles.

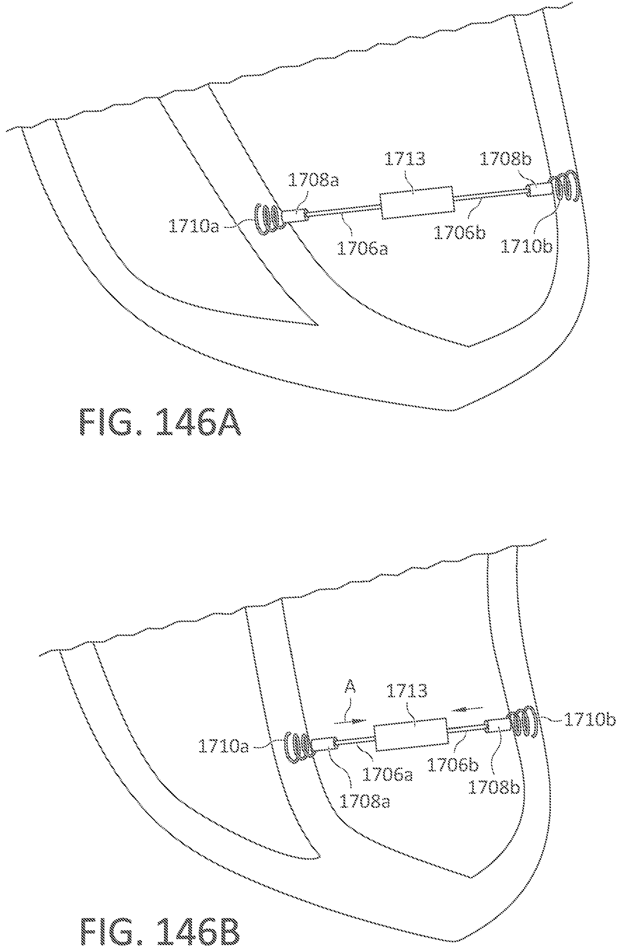

Figures 146A-146B illustrate an example of tissue remodeling using first and second anchors 1710a, 1710b, first and second tethers 1706a, 1706b, first and second couplers 1708a, 1708b, and an example variable tether tensioner 1713 applying tension between the first and second anchors 1710a, 1710b. The variable tether tensioner 1713 can take a wide variety of different forms. For example, the variable tether tensioner 1713 can be part of the first anchor 1710a, part of the second anchor 1710b, part of the first tether 1706a, part of the second tether 1706b, and/or part of a tether lock (e.g., tether lock 1712 of Figure 144b). Further, the variable tether tensioner 1713 can be one or more separate components that are attached to the first anchor 1710a, the second anchor 1710b, the first tether 1706a, the second tether 1706b, and/or the tether lock 1712. Figure 146A shows the first and second tethers 1706a, 1706b connected by the variable tether tensioner 1713. In an example, the variable tether tensioner 1713 is a tether lock configured to provide variable tension to first and the second tethers 1706a, 1706b.

Figure 146B shows the variable tether tensioner 1713 increasing tension between the first and the second tethers 1706a, 1706b. The increasing tension pulls the first and second anchors 1710a, 1710b (and heart tissue to which the anchors are attached) toward each other as indicated by the arrows A. The variable tether tensioner 1713 can be configured to increase tension in a wide variety of different ways. For example, the variable tether tensioner 1713 can be configured to increase tension using a mechanical system, such as a resilient component, such as a spring. In certain examples, the variable tether tensioner 1713 is configured to increase tension using a bio-absorbable material to manipulate a resilient component, such as a spring, over time. As the bio-absorbable material degrades and is absorbed, either by natural breakdown of the bio-absorbable material and/or through enhanced breakdown caused by one or more tools, the spring returns to its natural shape. (e.g., expands, contracts, winds, or unwinds, depending on the type of spring).

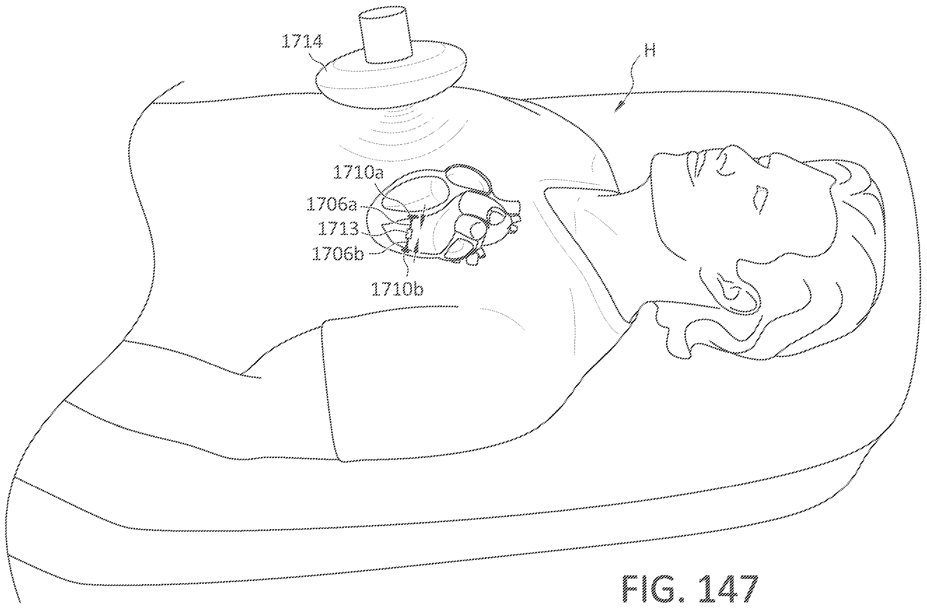

Figure 147 illustrates an example variable tether tensioner 1713 being modified by an optional adjustment device 1714 outside a human body H. The optional adjustment device 1714 can take a wide variety of different forms. Activation of the adjustment device 1714 can manipulate the variable tether tensioner 1713 to remodel tissue by cinching or pulling on one or more tethers 1706a, 1706b attached to two or more anchors 1710a, 1710b embedded in tissue. The optional adjustment device 1714 can apply heat, electricity, a magnetic field, vibratory energy or force, etc. to the variable tether tensioner 1713 through the human body H such that the tension applied by the variable tether tensioner 1713 (e.g., the tension between tethers 1706a, 1706b, attached to two or more anchors 1710a, 1710b) increases. The adjustment device 1714 can also (or instead) be configured to modify variable tension anchors, couplers, etc., as disclosed herein, in substantially the same ways as described herein.

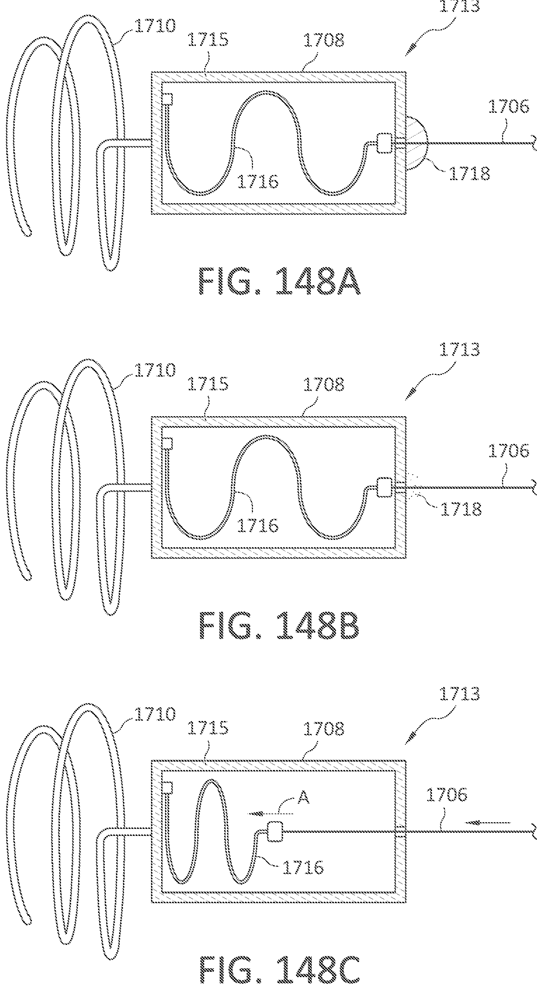

In some examples, a similar variable tether tensioner 1713 can be used in connection with a coupler that couples an anchor 1710 to a tether 1706. For example, Figures 148A-148C illustrate an example coupler 1708 with an example variable tether tensioner 1713. The coupler 1708 is associated with an anchor 1710 which can be implanted in tissue as described in detail herein. The coupler 1708 includes a body 1715 and a spring 1716 positioned within the body 1715 and attached to a tether 1706. In Figure 148A, the spring 1716 is held in an extended position by a stopper 1718. It is appreciated that different springs (e.g., different size, shape, material, spring constant) can be used with the coupler 1708. In some examples, a stopper 1718 can be made of a bio-absorbable material that degrades over time. In certain configurations, the degradation of the bio-absorbable stopper 1718 can be activated, sped-up, or slowed down by various methods, such as for example, by an adjustment device 1714, as described herein, and/or via an injection of additional material, compounds, solutions, etc. that materially alter the rate of breakdown of the stopper 1718. In some examples, the bio-absorbable material degrades at a predetermined rate. In Figure 148B the stopper 1718 is shown in a degraded state. As the stopper 1718 degrades, the spring 1716 recoils toward the anchor 1710 creating a force toward anchor 1710, as shown by arrow A in Figure 148C. The force can be utilized to remodel the tissue area in which anchor 1710 is engaged. It is appreciated that different springs (e.g., different size, shape, stiffness, material, spring constant) will apply different force according to Hooke's Law.

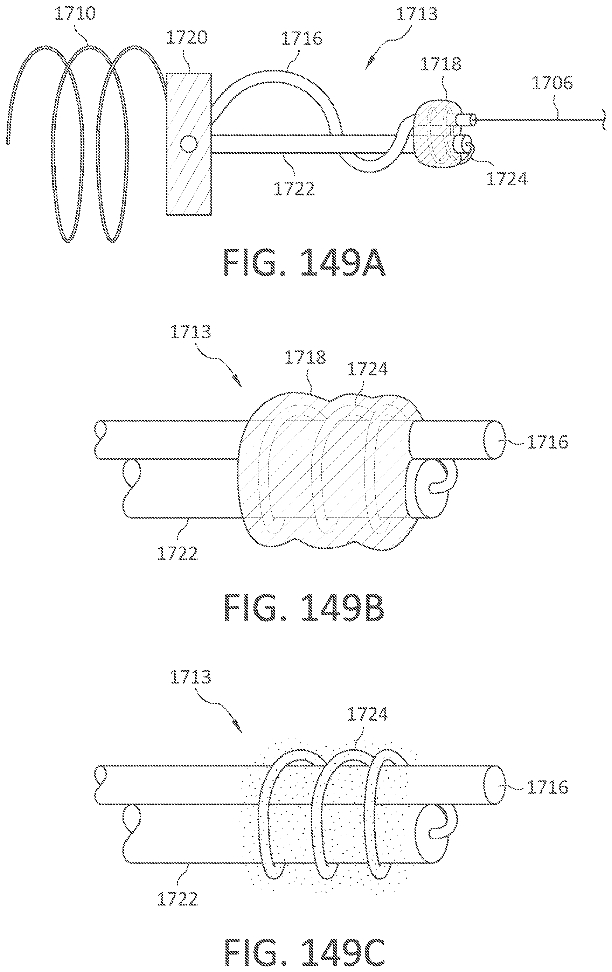

Another example variable tether tensioner 1713 is shown in Figure 149A. The mechanism comprises at least a base 1720, a pin 1722 extending from the base 1720, and the spring 1716. The spring 1716 can be held in an extended position by the stopper 1718. As described herein, it is appreciated that the stopper 1718 can be made from a bio-absorbable material. In some examples, the pin 1722 has a suture 1724, or other suitable line, extending from one end of the pin 1722. The suture 1724 can be wound around the spring 1716 and the pin 1722 and covered by the stopper 1718. A close-up view of the suture 1724 wrapped around the spring 1716 and the pin 1722 is shown in Figure 149B. In Figure 149C, the stopper 1718 has partially or fully degraded exposing the suture 1724 wrapped around the pin 1722 and the spring 1716. In certain examples, the suture 1724 can be connected to the spring 1716 and/or pin 1722 such that after partial or full degradation of the stopper 1718, the spring 1716 compresses or retracts. In some examples, the suture 1724 limits (either temporarily or permanently) the distance that the spring 1716 compresses or retracts after the stopper 1718 degrades.

In some examples, the suture 1724 can be made of a bio-absorbable material as well as the stopper 1718 or the suture 1724 can be made of a bio-absorbable material and the stopper can be omitted. In some examples where both a bio-absorbable stopper and a bioabsorbable suture are included, the bio-absorbable material of the stopper 1718 can degrade at a faster rate than the bio-absorbable material of the suture 1724. In such an example, the spring 1716 would then be capable of three different positions (i.e., with suture and stopper, stopper degraded, and suture degraded) allowing for controlled variable tension on the tether 1706 over time.

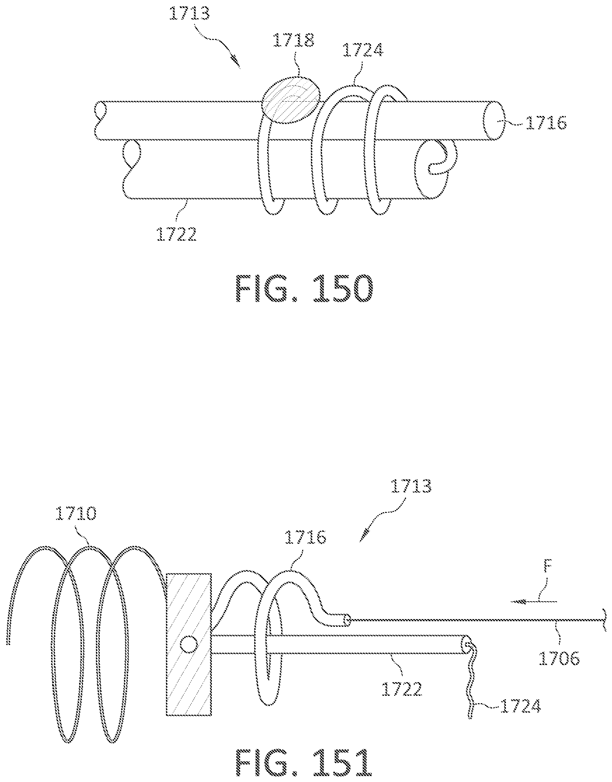

The rate of degradation of the bio-absorbable material of the stopper 1718 can be controlled, for example, by varying the size (e.g., the amount of material) and/or position of the stopper 1718. In general, the more bio-absorbable material of the stopper 1718, the greater amount of time it will take for the stopper 1718 to degrade. It is appreciated that the amount of bio-absorbable material used in the stopper 1718 can also be limited to prevent or minimize fragmental embolization. In Figure 150, an alternative positioning of the stopper 1718 is shown wherein the stopper 1718 is positioned on only a portion of the suture 1724 and the spring 1716. The configuration of Figure 150 can be used to apply the tension more quicky than the configuration illustrated by Figures 149A and 149B.

In certain examples, as the bio-absorbable material of the stopper 1718 degrades, the suture 1724 is exposed and is free to unwrap from the spring 1716 and the pin 1722, allowing the spring 1716 to create a force toward the anchor 1710 as the spring 1716 returns to an unbiased position. As shown in Figure 151, the spring 1716 has returned to its unbiased position pulling the tether 1706 toward the anchor 1710 in the direction of force F. This force can be applied at multiple anchor sites to remodel tissue in a desired manner.

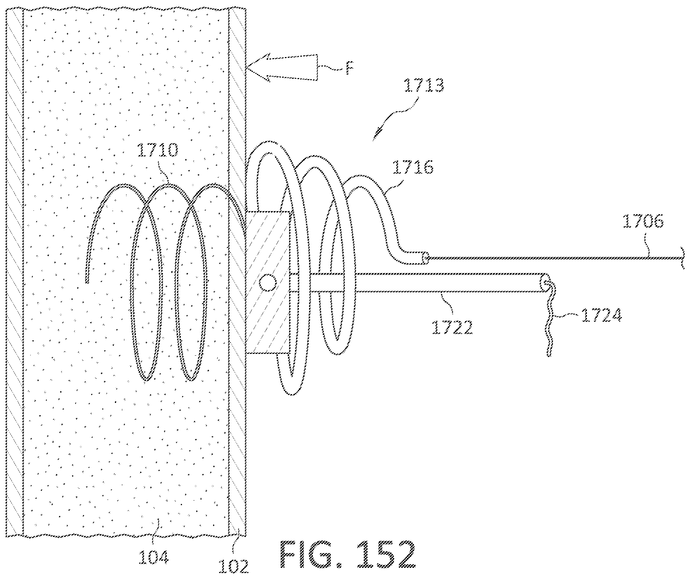

Figure 152 illustrates an anchor system that is similar to the anchor system of Figure 149A deployed in a target tissue area 1705. In this particular example, the anchor 1710 is penetrating the endocardium 102 and is embedded in the tissue at the myocardium 104. It is appreciated that, in various other examples, the anchor 1710 can be similarly embedded in other tissue areas. As shown in Figure 152, the stopper 1718 has degraded and the spring 1716 is applying a force F towards the target tissue area 1705. It is appreciated that the force F also pulls on the tether 1706 in the same direction. In the example illustrated by Figure 152, the retracted or compressed spring 1716 is configured to press against heart tissue, such as against the endocardium 102. This force of the spring against the heart tissue can prevent or inhibit the anchor 1710 from backing or rotating out of the tissue.

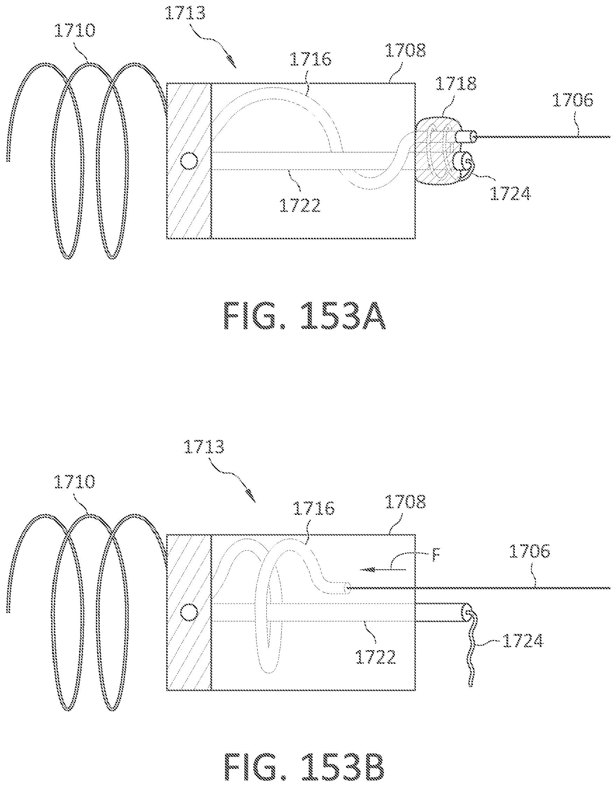

In certain examples, the variable tension mechanisms described herein can be optionally disposed in a housing, for example, within the coupler 1708. In Figure 153A, the coupler 1708 is attached to the anchor 1710 and contains the base/pin/spring assembly described above. The spring 1716 is held in an expanded position by the stopper 1718. In certain examples, the spring 1716 protrudes from the coupler 1708. In Figure 153B, the coupler 1708 is shown with the spring 1716 retracted into the coupler 1708 after the bio-absorbable material of the stopper 1718 has degraded, creating a force F in the direction of the anchor 1710.

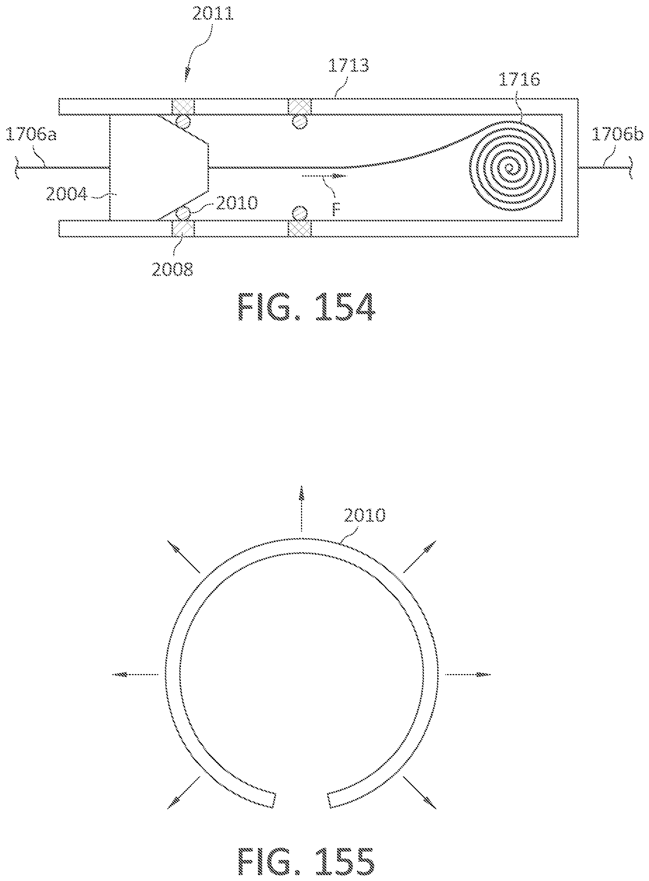

Figure 154 illustrates another example variable tether tensioner 1713. Like the other tensioners described herein, the variable tether tensioner 1713 can be a separate device (as illustrated by Figure 154) or can be part of an anchor, a tether or line, and/or a line lock. In the illustrated example, the variable tether tensioner 1713 is connected to a first tether 1706a and a second tether 1706b. It is appreciated that each tether 1706a, 1706b can be attached to an embedded anchor system as described herein. In this example, the first tether 1706a is attached to a plug 2004. The plug 2004 is disposed within the variable tether tensioner 1713 and attached to a spring 1716. The spring 1716 pulls on the plug 2004 in an axial direction. A retaining device, such as a c-clip 2010, is positioned within the variable tether tensioner 1713 to hold the plug 2004 in place by blocking axial movement of the plug 2004, which prevents or inhibits the spring 1716 from pulling the plug 2004 in the direction of the spring bias. The c-clip 2010 is disposed along the inside walls of the variable tether tensioner 1713 and applies an outward radial force. In certain examples, the c-clip 2010 is positioned at or within a groove or cavity 2011 of the inside walls of the variable tether tensioner 1713. The groove or cavity 2011 can be partially, or fully, filled with a compound 2008 that blocks the c-clip from expanding into the groove or cavity 2011 such that axial movement of the plug 2004 is no longer blocked by the c-clip 2010. The compound 2008 can be a low-melting compound, a bio-absorbable compound, or other suitable compound that degrades, melts, or otherwise allows the c-clip to expand into the groove or cavity 2011.

In certain examples, the compound 2008 melts when exposed to inductive heat. In certain examples, the c-clip 2010 heats up when exposed to inductive heat and melts the compound 2008. When the compound 2008 melts, or otherwise degrades, the c-clip 2010 expands into the groove or cavity 2011 of the variable tether tensioner 1713. As the c-clip 2010 expands into the groove or cavity 2011, axial movement of the plug 2004 is no longer blocked by the c-clip 2010 and the plug 2004 can be pulled by the spring 1716 in the direction of force F. The force F creates tension on the first tether 1706a as the plug 2004 moves inward toward the spring 1716. In some examples, a plurality of c-clips 2010 can be used in the same manner to create variable degrees of tension on the first tether 1706a attached to a plug 2004. In some examples, different compounds 2008 can be used at different positions within the variable tether tensioner 1713, allowing for different melting points or degradation rates, and therefore controlled expansion of the c-clips 2010 into the grooves or cavities 2011. A close up of an example c-clip 2010 is shown in Figure 155. It is appreciated that the above-described example could work in a substantially similar way using only a compound 2008 to hold the plug 2004 into place. In alternative examples, the c-clip 2010 can be replaced with a similar spring or other suitable device to apply pressure on the plug 2004 and be capable of expansion into the groove or cavity 2011 upon degradation of the compound 2008.

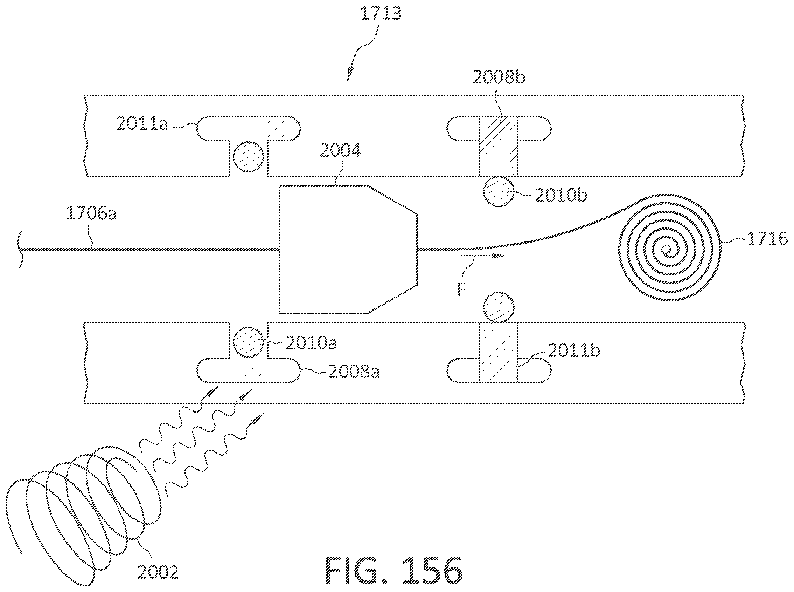

In Figure 156, an inductive heating element 2002 is used to apply inductive heat to a first c-clip 2010a. When heated, the first c-clip 2010a melts a first compound 2008a located in a first groove or cavity 2011a in the inside wall of the variable tether tensioner 1713. As a result, the first c-clip 2010a expands into the first groove or cavity 2011a as the first compound 2008a melts, freeing the plug 2004 to move under the bias of the spring 1716 in the direction of force F. As shown in Figure 156, after melting of the first compound 2008a, the plug 2004 will moves axially until blocked by a second c-clip 2010b. The inductive heating element 2002 can be used to heat the second c-clip 2010b. When heated, the second c-clip 2010b melts a second compound 2008b located in a second groove or cavity 2011b in the inside wall of the variable tether tensioner 1713. As a result, the second c-clip 2010b expands into the second groove or cavity 2011b as the second compound 2008b melts, freeing the plug 2004 to move under the bias of the spring 1716 in the direction of force F. It is appreciated that in certain examples, each groove or cavity 2011a, 2011b can be shaped in a way that accommodates the melted compound 2008 and allows for the c-clip to expand into the groove or cavity 2011a, 2011b allowing the plug 2004 to move freely in the axial direction toward the spring 1716.

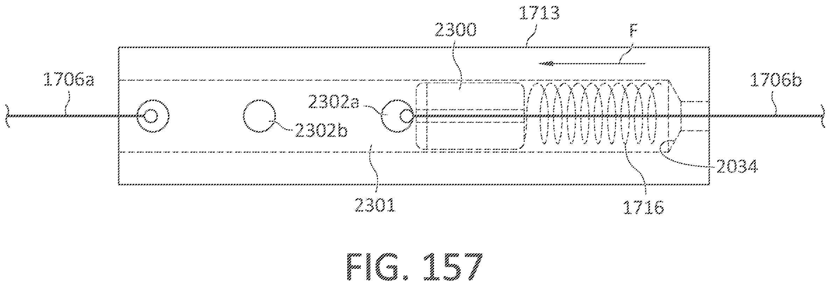

Another variable tension tensioner 1713 is illustrated in Figure 157. Figure 157 shows the variable tether tensioner 1713 operably connected to a first tether 1706a and a second tether 1706b. As shown, the second tether 1706b is connected to a piston 2300 movably disposed within a passage 2301 inside of the variable tether tensioner 1713. Within the passage 2301 of the variable tether tensioner 1713, a spring 1716 is compressed between the piston 2300 and a radial shoulder 2034. The piston 2300 is blocked from moving axially away from the spring 1716 by a first stopping element 2302a that holds the piston 2300 in place. The first stopping element 2302a can be made of a bio-absorbable material, a compound (e.g., a low melting compound), a polymer (e.g., a low melting Polycaprolactone - PCL). The first stopping element 2302a can be degraded and/or melted (e.g., via induction heating). Once the first stopping element 2302a has substantially degraded/melted, the force from the spring 1716 pushes the piston 2300 axially along the passage 2301 in the direction of force F. Axial movement of the piston 2300 in the direction of force F increases tension in the second tether 1706b.

A second stopping element 2302b is positioned within the passage 2301 to prevent or inhibit additional movement of the piston 2300. The second stopping element 2302b can be similarly degraded and/or melted allowing the force from the spring 1716 to push the piston 2300 further axially along the passage 2301 in the direction of force F. With each progressive move of the piston 2300 beyond a stopping element (e.g., the first and second stopping elements 2302a, 2302b), the tension in the second tether 1706b increases. It is appreciated that there can be any number of stopping elements allowing for incremental tension increases within the variable tether tensioner 1713.

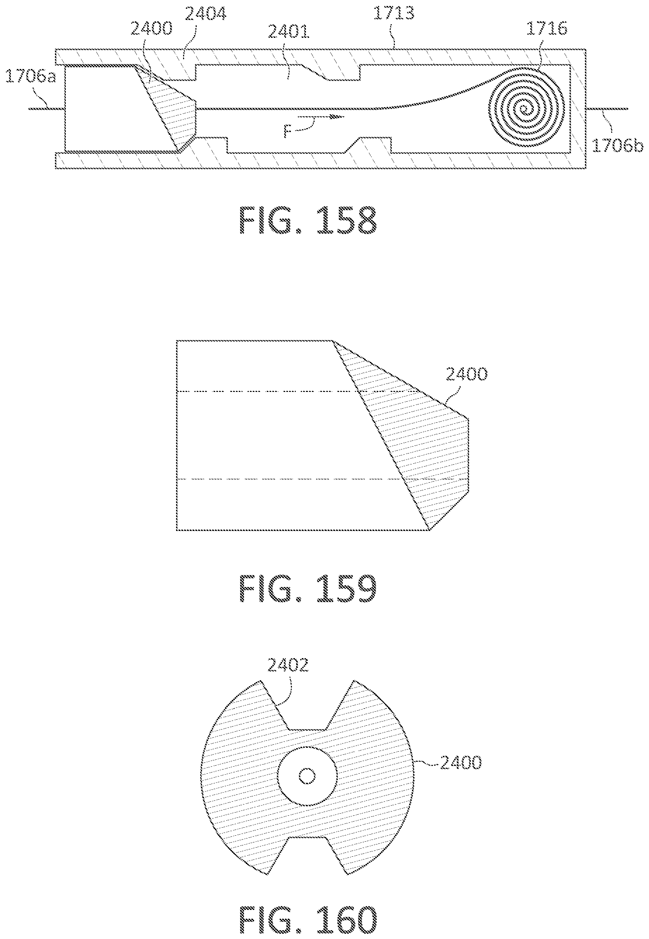

Figure 158 illustrates another example variable tether tensioner 1713. In the illustrated example, the variable tether tensioner 1713 is attached to a first tether 1706a via a plug nut 2400 disposed within a passage 2401 of the variable tether tensioner 1713 and is attached to a second tether 1706b opposite the first tether 1706a. A spring 1716 is positioned within the passage 2401 and is attached to the plug nut 2400 to pull the plug nut 2400 in the direction of force F creating tension at the first tether 1706a. The plug nut 2400 is held in place (i.e., blocked from being pulled toward the spring 1716) within the passage 2401 by one or more tapered stops 2404 engaging the plug nut 2400. In the illustrated example, the tapered stops 2404 are formed within the passage 2401 of the variable tether tensioner 1713. In some examples, the plug nut 2400 and the tapered stops 2404 have tapered surfaces that are mating and similar to those of a screw thread. The plug nut 2400 can advance past the tapered stops 2404 as vibratory energy (e.g., via an external mechanical vibration or ultrasonic vibrator) sweeps through a frequency range to hit natural angular frequency to cause the plug nut 2400 to slip past the tapers toward spring 1716. Figure 159 shows a close up of an example of geometry of plug nut 2400. Figure 160 shows an end view of the plug nut 2400 and an example of angular tapers 2402 of the plug nut 2400.

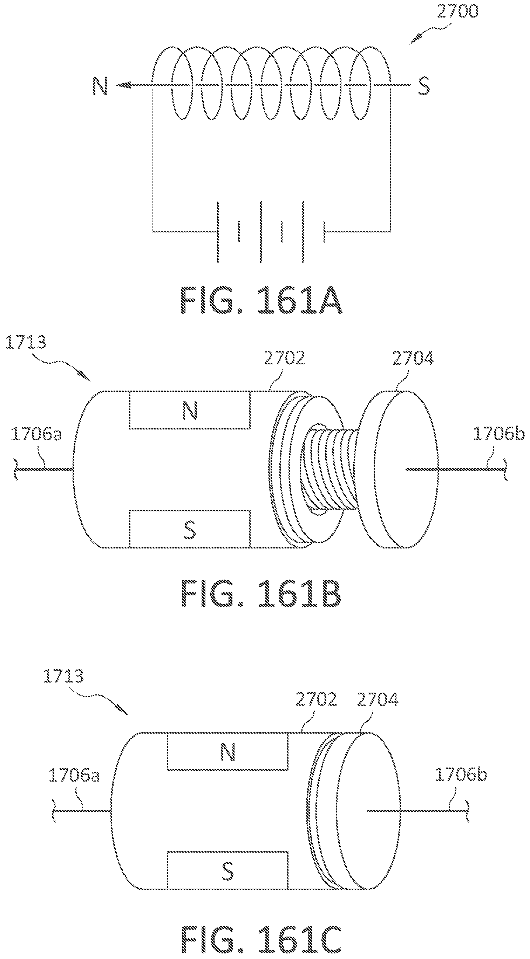

Figures 161A-162C illustrate yet another variable tension mechanism for use with a variable tether tensioner 1713. Figure 161A illustrates how an example electromagnetic field can be created by a coil winding 2700. Energizing the coil winding 2700 creates an electromagnetic field with a north and south pole as shown in Figure 161A. A stator can be used to carry the magnetic field and a movable rotor can be used which will align itself with the magnetic field. The magnetic field can be altered by sequentially energizing or "stepping" the stator coils which generates rotary motion of the rotor. This scientific principle is commonly referred to as "stepper motor" theory. In an example shown in Figure 161B, the variable tether tensioner 1713 has a magnetic base 2702 with a north and a south polarity. The magnetic base 2702 is attached to a first tether 1706a. The variable tether tensioner 1713 also has a cap 2704 which is threaded into the magnetic base 2702 and is attached to a second tether 1706b. By using an external electromagnetic field generator placed outside the body, the magnetic base 2702 can be caused to rotate via the changing magnetic field. Rotation of the magnetic base 2702 results in the cap 2704 threading into the magnetic base 2702 and increasing tension at the second tether 1706b. In some examples, the same principles can apply to a magnetic wheel which turns or screws into a stationary tube. It is appreciated that the above-described process can be both used to screw the cap into the base (increasing tension) as well as reverse the cap out of the base (decreasing tension). This grants greater control over the amount of tension applied by the example variable tether tensioner 1713.

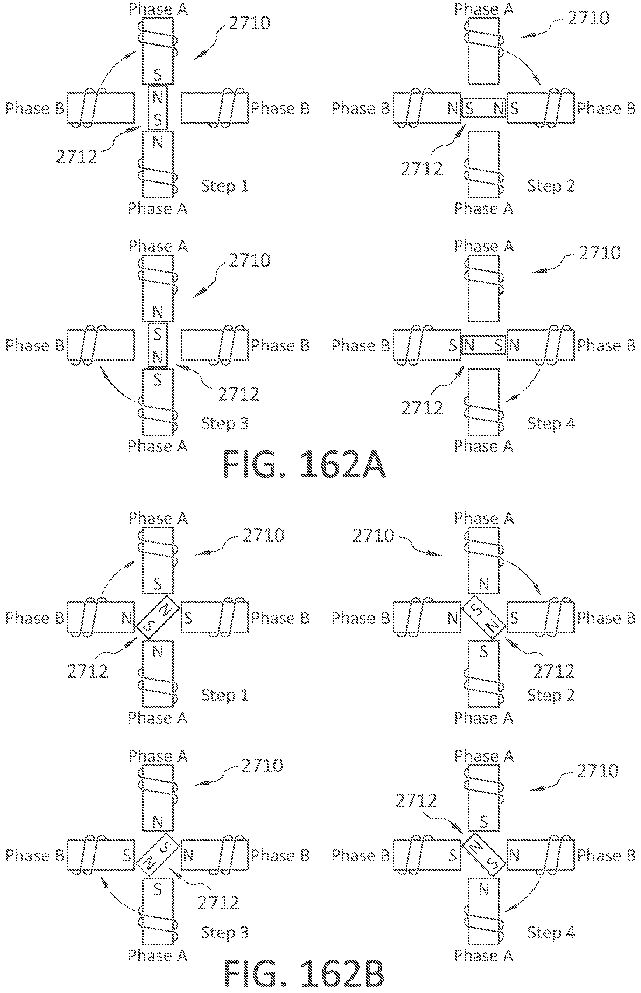

Figures 162A-C further describe the "stepper motor" theory for creating rotational energy using an electromagnetic field. Figure 162A illustrates a typical step sequence for a two-phase motor. In Step 1, phase A of a two-phase stator 2710 is energized. In step 1, a rotor 2712 is magnetically locked in the position shown, since unlike poles attract. When phase A is turned off and phase B is turned on, the rotor 2712 rotates 90° clockwise. In Step 3, phase B is turned off and phase A is turned on but with the polarity reversed from Step 1. This causes another 90° rotation of the rotor 2712. In Step 4, phase A is turned off and phase B is turned on, with polarity reversed from Step 2. Repeating this sequence causes the rotor 2712 to rotate clockwise in 90° steps.

The stepping sequence illustrated in Figure 162A is called "one phase on" stepping. A more common method of stepping is "two phase on" where both phases of the motor are always energized, as illustrated in Figure 162B. However, only the polarity of one phase is switched at a time. With two-phase on stepping, the rotor 2712 aligns itself between the "average" north and "average" south magnetic poles. Since both phases are always on, this method gives 41.4% more torque than "one phase on" stepping, but with twice the power input.

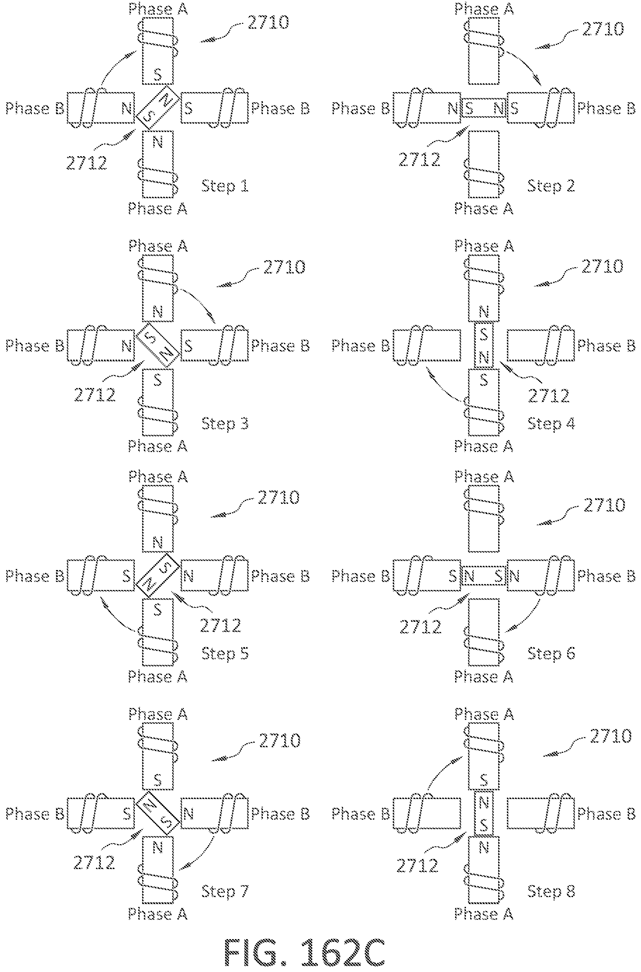

The motor can also be "half stepped" by inserting an off state between transitioning phases. This cuts a stepper's full step angle in half. For example, a 90° stepping motor would move 45° on each half step, as illustrated in Figure 162C. However, half stepping typically results in a 15% - 30% loss of torque depending on step rate when compared to the two-phase on stepping sequence. Since one of the windings is not energized during each alternating half step there is less electromagnetic force exerted on the rotor 2712 resulting in a net loss of torque.

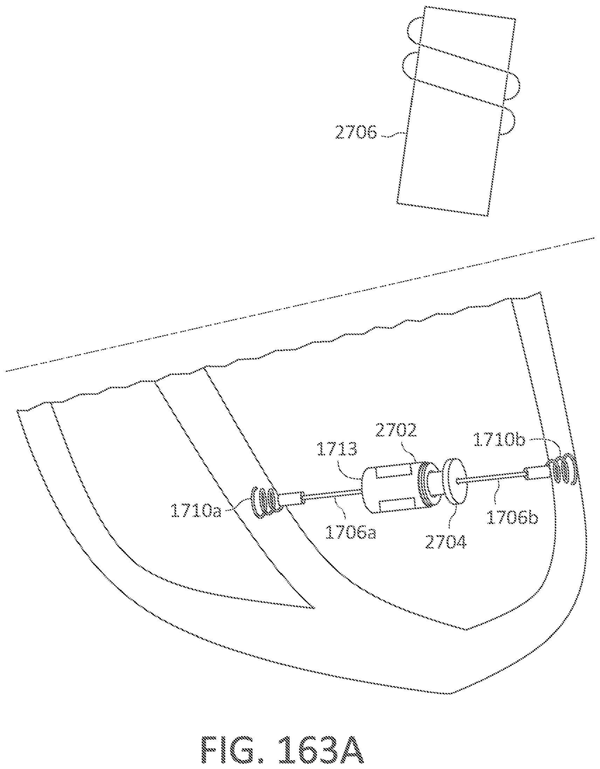

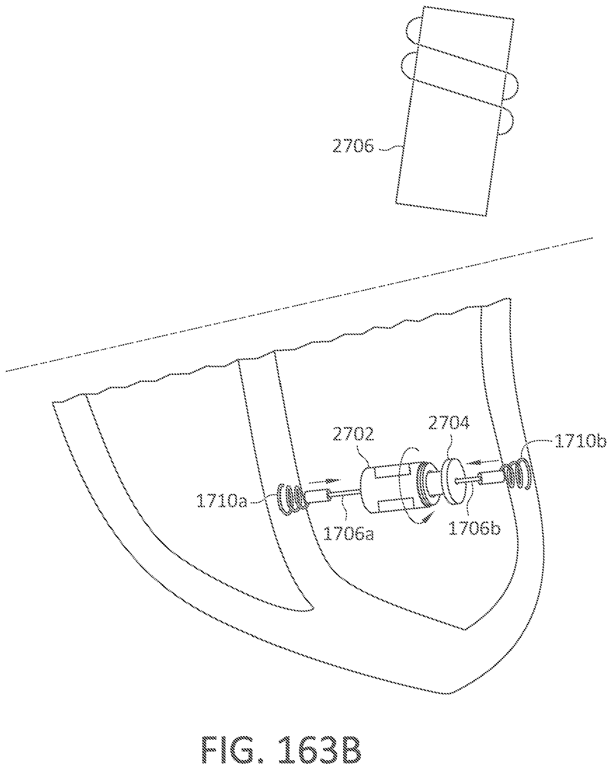

Figure 163A illustrates an example magnetic variable tether tensioner 1713 with a magnetic base 2702 attached to a first tether 1706a which is attached to a first anchor 1710a and a cap 2704 which is attached to a second tether 1706b attached to a second anchor 1710b. An electromagnet 2706 can be used to create a rotational energy at the variable tether tensioner 1713 and cause the magnetic base 2702 and the cap 2704 rotate (i.e., turn or screw) relative to each other. The relative rotation of the magnetic base 2702 and the cap 2704 increases tension in the first and second tethers 1706a, 1706b and thereby remodeling tissue at the embedded locations of the first and second anchors 1710a, 1710b. This tissue remodeling movement is illustrated in Figure 163B.

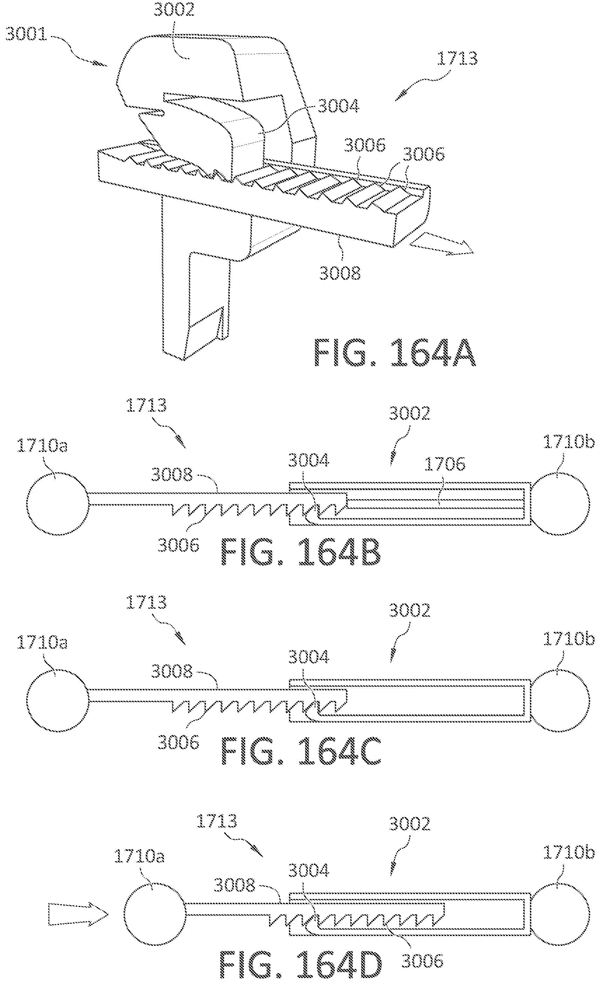

Figures 164A-164D illustrate example variable tether tensioner 1713 utilizing a ratcheting system 3001. Figure 164A illustrates an example ratcheting system 3001 that can be used to variably increase tension between two embedded anchors. In the illustrated example, the ratcheting system 3001 includes a ratcheting member 3008 having a plurality of teeth or serration 3006. The ratcheting member 3008 extends through a ratchet head 3002. In some examples, the ratchet head 3002 has an attached sheath for receiving the ratcheting member 3008. The ratchet head 3002 has a tongue 3004 which can engage the teeth or serrations 3006 of the ratcheting member 3008. Any number of teeth or serrations 3006 can be engaged with the tongue 3004 at a time. After the teeth or serrations 3006 have disengaged with the tongue 3004 (e.g., after the ratcheting member 3008 has been further advanced through the rachet head 3002), the teeth or serrations 3006 can enter an optional sheath.

In some examples, the ratcheting member 3008 can be attached to a first anchor 1710a as shown in FIG. 164B. The sheath can be attached to a second anchor 1710b. In some examples, the ratcheting member 3008 is attached to the second anchor 1710b via a tether 1706, as shown in Figure 164B. As the ratcheting member 3008 is pushed through the ratchet head 3002, the tongue 3004 engages the teeth or serrations 3006 and prevents or inhibits backward movement of the ratcheting member 3008. The ratcheting member 3008 extends through the ratchet head 3002 and the teeth or serrations 3006 engage with the tongue 3004 (not shown). As the ratcheting member 3008 is advanced, the force applied to the first and second anchors 1710a, 1710b can be used to remodel tissue. As shown in Figure 164D, the ratcheting member 3008 has been advanced through the ratchet head 3002 pulling or cinching the first and second anchors 1710a, 1710b closer together. In some example, a spring can be used within the sheath portion of ratchet head 3002 to push back on the advancing ratcheting member 3008.

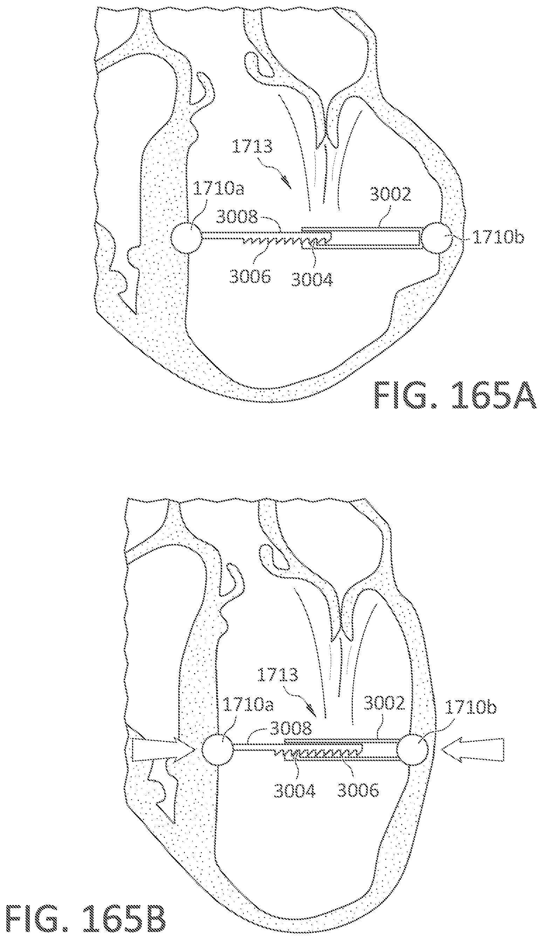

In some examples, as the heart muscle expands and contracts over time, the ratcheting member 3008 can be incrementally advanced through the ratchet head 3002 thereby applying a proportionally incremental increase in tension between the first and second anchors 1710a,1710b. This process is illustrated in Figures 165A and 165B. As the heart beats, and the muscles expand and contract, over time the ratcheting effect will be more pronounced as each progressive tooth or serration moves past the ratcheting head 3002.

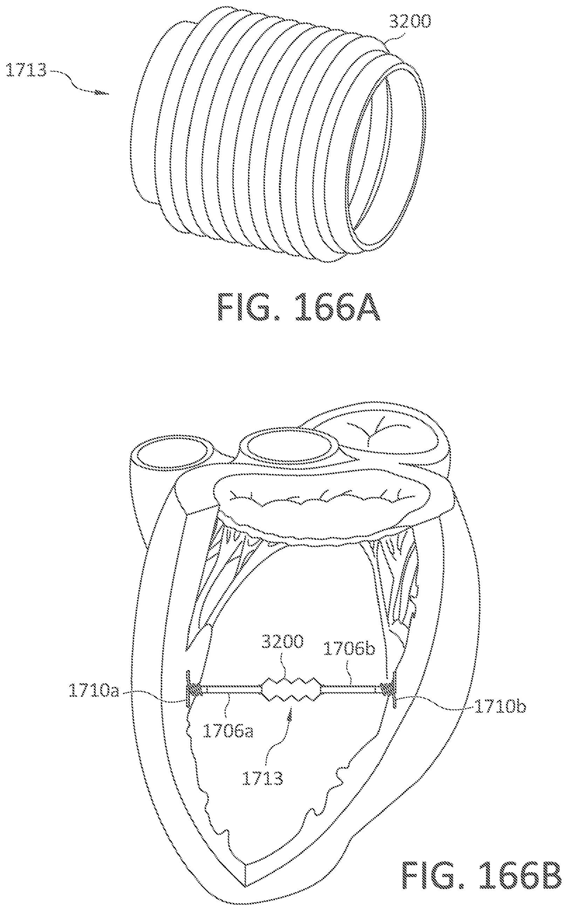

Figures 166A-166B illustrate another example variable tether tensioner 1713. In the example illustrated by Figure 166A, the variable tether tensioner 1713 includes a bellows 3200. The bellows 3200 can have one or more chambers which can be filled with a fluid causing the bellows 3200 to expand or contract. In some examples, the bellows 3200 contains one or more perforations that allow fluid to pass through the bellows 3200. In some examples, the bellows 3200 can be filled with a certain amount of fluid and the perforations act as a one-way valve, letting fluid escape over time. In this way, the bellows 3200 to be implanted in a certain position with a desired tension between one or more tethers (and corresponding anchors) and over time, as the bellows 3200 releases the internal fluid through the one-way perforations, the bellows 3200 contracts, thereby increasing tension between the attached tethers.

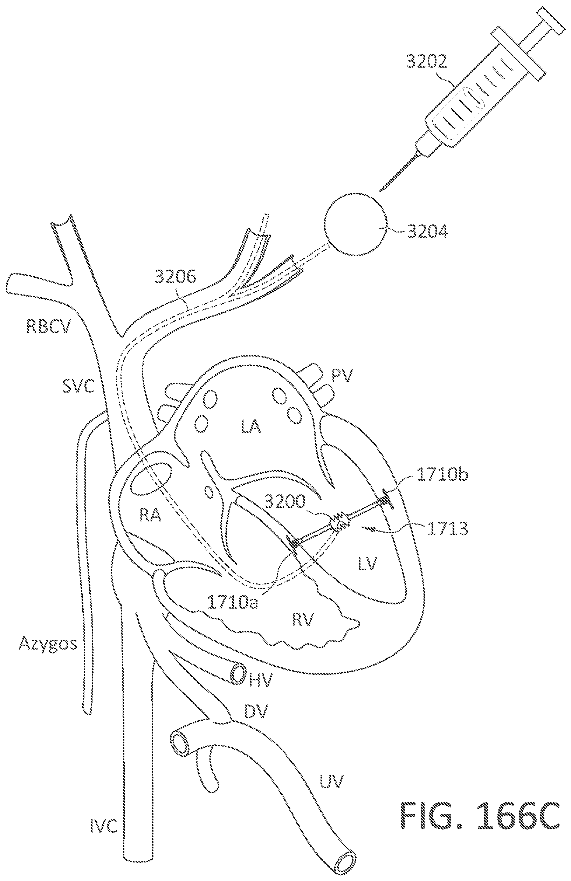

Figure 166B shows an example bellows 3200 attached to a first anchor 1710a by a first tether 1706a and to a second anchor 1710b by a second tether 1706b. In some examples, the bellows 3200 can have additional fluid added or fluid removed via a catheter or syringe. In some examples, the fluid is a calcium and/or saline solution. Figure 166C illustrates the process of adding or subtracting fluid from the bellows 3200. In an example, a syringe 3202 can add or subtract fluid through a port 3204 which is operably connected to the bellows 3200. As described above, as fluid is removed from the bellows, the bellows 3200 contracts causing an increase in tension between the first and second embedded anchors 1710a, 1710b. Similarly, if fluid is added, the bellows 3200 expands relieving force applied. The fluid can be added and removed in a wide variety of different ways. In the example illustrated in Figure 166C, the fluid is delivered through a conduit 3206 that extends into the right atrium RA from the superior vena cava SVC, into the right ventricle RV from the right atrium RA, through the ventricular septum, and into the left ventricle LV. However, any path the provides access to the bellows 3200 can be used.

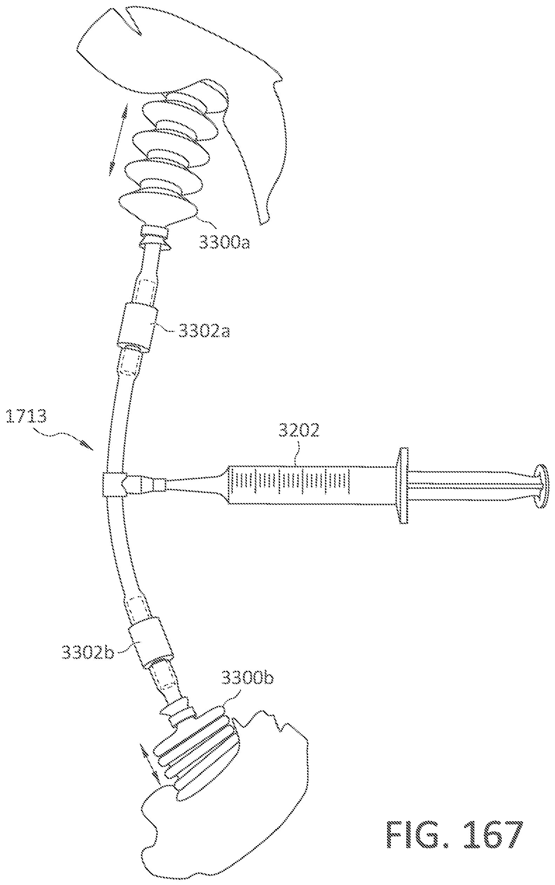

Figure 167 illustrates an example of a bellows-type variable tether tensioner 1713. In the illustrated example, a syringe 3202 can add or remove fluid from a first bellows 3300a and/or a second bellows 3300b. However, fluid flow is restricted by a first one-way valve 3302a associated with the first bellows 3300a and a second one-way valve 3302b associated with the second bellows 3300b. Depending on the orientation of the first and second one-way valves 3302a, 3302b, removing liquid via the syringe 3202 could contract the first bellows 3300a and expand the second bellows 3300b, or vice versa. It is appreciated that the first and second bellows 3300a, 3300b can be anchor locks attached to one or more tethers (e.g., bellows 3200) or, in the alternative, as couplers attached directly to an anchor implanted in tissue.

While various inventive aspects, concepts and features of the disclosures may be described and illustrated herein as embodied in combination in the examples, these various aspects, concepts, and features may be used in many alternative examples, either individually or in various combinations and sub-combinations thereof.

Additionally, even though some features, concepts, or aspects of the disclosures may be described herein as being a preferred arrangement or method, such description is not intended to suggest that such feature is required or necessary unless expressly so stated. Still further, example or representative values and ranges may be included to assist in understanding the present application, however, such values and ranges are not to be construed in a limiting sense and are intended to be critical values or ranges only if so expressly stated.

Moreover, while various aspects, features and concepts may be expressly identified herein as being inventive or forming part of a disclosure, such identification is not intended to be exclusive, but rather there may be inventive aspects, concepts, and features that are fully described herein without being expressly identified as such or as part of a specific disclosure, the disclosures instead being set forth in the appended claims. Descriptions of example methods or processes are not limited to inclusion of all steps as being required in all cases, nor is the order that the steps are presented to be construed as required or necessary unless expressly so stated. The words used in the claims have their full ordinary meanings.

## Claims

1. A device (120) for remodeling a shape of one or more walls of a human heart, the human heart having an internal chamber, the device comprising:
an anchoring portion (1602) having an elongated anchor body (1612) with a distal anchor end portion (1614) and a proximal anchor end portion (1616) opposite the distal anchor end portion;
a joint portion (1604) comprising a joint body (1622), wherein the joint body is configured to allow bending of the joint body between 180 degrees and 720 degrees of rotation of the joint body;
wherein the joint portion has a distal joint end portion (1624) fixedly attached to the proximal anchor end portion and a proximal joint end portion (1626) opposite the distal joint end portion;
a hemostasis element (1606) having a distal hemostasis end portion (1634) detachably connected to the proximal joint end portion;
wherein the device is configured to receive an elongating device (1611) through an inner passage (1610) in the device;
wherein when the elongating device is received in the inner passage through the anchoring portion, the anchoring portion is held in an elongated delivered state; and
wherein when the elongating device is removed from the inner passage of the anchoring portion, the anchoring portion forms a curved deployed state.

2. The device of claim 1, wherein the anchoring portion:
a) is annular in the curved deployed state; and/or
b) includes a shape memory alloy and the anchoring portion is shape set to the curved deployed state.

3. The device of claim 1 or claim 2, wherein the elongating device is configured to pierce a heart wall to form a passage therein,
optionally wherein the elongating device is a wire.

4. The device of any preceding claim, wherein the elongated anchor body includes a series of cuts traverse to a longitudinal axis along the elongated anchor body.

5. The device of any preceding claim, the joint portion is integrally formed with the anchoring portion.

6. The device of any preceding claim, wherein the distal hemostasis end portion is detachably connected to the proximal joint end portion by an attachment portion (1608) movable between a coupling position and a release position.

7. The device of claim 6, wherein the attachment portion includes a plurality of projections and a plurality of recesses, wherein each of the plurality of recesses is configured to received one of the plurality of projections.

8. The device of claim 7, wherein the attachment portion include a pair of projections extending proximally from a cylindrical sidewall of the joint portion and a pair of recesses extending proximally in a cylindrical sidewall of the hemostasis element.

9. The device of claim 7, wherein each of the plurality of projections includes a shape memory alloy and each of the plurality of projections is shape set to the release position.

10. The device of claim 9, wherein each of the plurality of projections extends proximally from a cylindrical sidewall of the joint portion and wherein, in the release position, each of the plurality of projections bend radially inward,
optionally wherein each of the plurality of projections is held in the coupling position by the elongating device when the elongating device is positioned in the inner passage and extending through the attachment portion.

11. A method of remodeling a shape of a heart wall that at least partially defines a heart chamber, the method comprising:
extending an elongating member through the heart wall from the heart chamber to a pericardial cavity;
extending a device through a passage in the heart wall and over the elongating member, wherein the device includes an anchoring portion having an elongated anchor body, and a joint portion (1604) comprising a joint body (1622), wherein the joint body is configured to allow bending of the joint body between 180 degrees and 720 degrees of rotation of the joint body, wherein the joint portion fixedly attached to a proximal end of the anchoring portion, and a hemostasis element detachably connected to a proximal end of the joint portion;
shaping the elongated anchor body into an annular deployed state within the pericardial cavity;
bending the joint portion to reposition the anchor body in the pericardial cavity; and
disconnecting the joint portion from the hemostasis element such that the joint portion and the anchoring portion are in the pericardial cavity and the hemostasis element is positioned in the passage;
wherein the method is performed on a cadaver, cadaver heart or a simulator.

12. The method of claim 11, wherein extending the device over the elongating member comprises the elongating member extending through an inner passage of the device that extends through the elongated anchor body, the joint portion, and the hemostasis element.

13. The method of claim 12, wherein:
a) shaping the elongated anchor body into the annular deployed state comprises removing the elongating member from the inner passage through the elongated anchor body; and/or
b) bending the joint portion to reposition the anchor body comprises removing the elongating member from the inner passage through the joint portion.

14. The method of claim 12 or 13, wherein disconnecting the joint portion from the hemostasis element comprises:
a) removing the elongating member from the inner passage through an attachment portion cooperatively formed by the joint portion and the hemostasis element; or
b) bending one or more projections radially inward.

15. The method of any of claims 11 to 14, wherein the joint portion is integrally formed with the anchoring portion.

## Patentansprüche

1. Vorrichtung (120) zur Remodellierung einer Form einer oder mehrerer Wände eines menschlichen Herzens, wobei das menschliche Herz eine innere Kammer aufweist, wobei die Vorrichtung Folgendes umfasst:
einen Verankerungsabschnitt (1602), der einen länglichen Ankerkörper (1612) mit einem distalen Ankerendabschnitt (1614) und einem proximalen Ankerendabschnitt (1616) gegenüber dem distalen Ankerendabschnitt aufweist;
einen Verbindungsabschnitt (1604), der einen Verbindungskörper (1622) umfasst, wobei der Verbindungskörper dazu ausgelegt ist, ein Biegen des Verbindungskörpers zwischen 180 Grad und 720 Grad der Drehung des Verbindungskörpers zu ermöglichen;
wobei der Verbindungsabschnitt einen distalen Verbindungsendabschnitt (1624), der fest an dem proximalen Ankerendabschnitt angebracht ist, und einen proximalen Verbindungsendabschnitt (1626) gegenüber dem distalen Verbindungsendabschnitt aufweist;
ein Hämostaseelement (1606) mit einem distalen Hämostaseendabschnitt (1634), der lösbar mit dem proximalen Verbindungsendabschnitt verbunden ist;
wobei die Vorrichtung dazu ausgelegt ist, eine Verlängerungsvorrichtung (1611) durch einen inneren Durchgang (1610) in der Vorrichtung aufzunehmen;
wobei, wenn die Verlängerungsvorrichtung in dem inneren Durchgang durch den Verankerungsabschnitt aufgenommen wird, der Verankerungsabschnitt in einem länglichen zugeführten Zustand gehalten wird; und
wobei, wenn die Verlängerungsvorrichtung aus dem inneren Durchgang des Verankerungsabschnitts entfernt wird, der Verankerungsabschnitt einen gekrümmten entfalteten Zustand bildet.

2. Vorrichtung nach Anspruch 1, wobei der Verankerungsabschnitt:
a) im gekrümmten entfalteten Zustand ringförmig ist; und/oder
b) eine Formgedächtnislegierung beinhaltet und der Verankerungsteil in den gekrümmten entfalteten Zustand formgesetzt ist.

3. Vorrichtung nach Anspruch 1 oder Anspruch 2, wobei die Verlängerungsvorrichtung dazu ausgelegt ist, eine Herzwand zu durchstechen, um einen Durchgang darin zu bilden,
wobei optional die Verlängerungsvorrichtung ein Draht ist.

4. Vorrichtung nach einem der vorhergehenden Ansprüche, wobei der längliche Ankerkörper eine Reihe von Schnitten beinhaltet, die quer zu einer Längsachse entlang des länglichen Ankerkörpers verlaufen.

5. Vorrichtung nach einem der vorhergehenden Ansprüche, wobei der Verbindungsabschnitt integral mit dem Verankerungsabschnitt ausgebildet ist.

6. Vorrichtung nach einem der vorhergehenden Ansprüche, wobei der distale Hämostaseendabschnitt durch einen Befestigungsabschnitt (1608), der zwischen einer Kupplungsposition und einer Freigabeposition bewegbar ist, lösbar mit dem proximalen Verbindungsendabschnitt verbunden ist.

7. Vorrichtung nach Anspruch 6, wobei der Befestigungsabschnitt mehrere Vorsprünge und mehrere Aussparungen beinhaltet, wobei jede der mehreren Aussparungen dazu ausgelegt ist, einen der mehreren Vorsprünge aufzunehmen.

8. Vorrichtung nach Anspruch 7, wobei der Befestigungsabschnitt ein Paar von Vorsprüngen, die sich proximal von einer zylindrischen Seitenwand des Verbindungsabschnitts erstrecken, und ein Paar von Aussparungen, die sich proximal in einer zylindrischen Seitenwand des Hämostaseelements erstrecken, aufweist.

9. Vorrichtung nach Anspruch 7, wobei jeder der mehreren Vorsprünge eine Formgedächtnislegierung beinhaltet und jeder der mehreren Vorsprünge auf die Freigabeposition formgesetzt ist.

10. Vorrichtung nach Anspruch 9, wobei sich jeder der mehreren Vorsprünge proximal von einer zylindrischen Seitenwand des Verbindungsabschnitts erstreckt und wobei sich in der Freigabeposition jeder der mehreren Vorsprünge radial nach innen biegt,
wobei optional jeder der mehreren Vorsprünge durch die Verlängerungsvorrichtung in der Kupplungsposition gehalten wird, wenn die Verlängerungsvorrichtung in dem inneren Durchgang positioniert ist und sich durch den Befestigungsabschnitt erstreckt.

11. Verfahren zur Remodellierung einer Form einer Herzwand, die zumindest teilweise eine Herzkammer definiert, wobei das Verfahren Folgendes umfasst:
Ausdehnen eines Verlängerungselements durch die Herzwand von der Herzkammer zu einer Perikardhöhle;
Ausdehnen einer Vorrichtung durch einen Durchgang in der Herzwand und über das Verlängerungselement, wobei die Vorrichtung einen Verankerungsabschnitt mit einem länglichen Ankerkörper und einen Verbindungsabschnitt (1604), der einen Verbindungskörper (1622) umfasst, wobei der Verbindungskörper dazu ausgelegt ist, ein Biegen des Verbindungskörpers zwischen 180 Grad und 720 Grad der Drehung des Verbindungskörpers zu ermöglichen, wobei der Verbindungsabschnitt fest an einem proximalen Ende des Verankerungsabschnitts angebracht ist, und ein Hämostaseelement, das lösbar mit einem proximalen Ende des Verbindungsabschnitts verbunden ist, aufweist;
Formen des länglichen Ankerkörpers in einen ringförmigen entfalteten Zustand innerhalb der Perikardhöhle;
Biegen des Verbindungsabschnitts, um den Ankerkörper in der Perikardhöhle neu zu positionieren; und
Trennen des Verbindungsabschnitts von dem Hämostaseelement, so dass sich der Verbindungsabschnitt und der Verankerungsabschnitt in der Perikardhöhle befinden und das Hämostaseelement in dem Durchgang positioniert ist;
wobei das Verfahren an einem Kadaver, einem Kadaverherz oder einem Simulator durchgeführt wird.

12. Verfahren nach Anspruch 11, wobei das Ausdehnen der Vorrichtung über das Verlängerungselement umfasst, dass sich das Verlängerungselement durch einen inneren Durchgang der Vorrichtung ausdehnt, der sich durch den länglichen Ankerkörper, den Verbindungsabschnitt und das Hämostaseelement erstreckt.

13. Verfahren nach Anspruch 12, wobei:
a) das Formen des länglichen Ankerkörpers in den ringförmigen entfalteten Zustand das Entfernen des Verlängerungselements aus dem inneren Durchgang durch den länglichen Ankerkörper umfasst; und/oder
b) das Biegen des Verbindungsabschnitts zum Neupositionieren des Ankerkörpers das Entfernen des Verlängerungselements aus dem inneren Durchgang durch den Verbindungsabschnitt umfasst.

14. Verfahren nach Anspruch 12 oder 13, wobei das Trennen des Verbindungsabschnitts von dem Hämostaseelement Folgendes umfasst:
a) Entfernen des Verlängerungselements aus dem inneren Durchgang durch einen Befestigungsabschnitt, der zusammenwirkend durch den Verbindungsabschnitt und das Hämostaseelement gebildet wird; oder
b) Biegen eines oder mehrerer Vorsprünge radial nach innen.

15. Verfahren nach einem der Ansprüche 11 bis 14, wobei der Verbindungsabschnitt integral mit dem Verankerungsabschnitt ausgebildet ist.

## Revendications

1. Dispositif de remodelage (120) d'une forme d'une ou plusieurs parois d'un cœur humain, le cœur humain présentant une chambre interne, le dispositif comprenant :
une partie d'ancrage (1602) ayant un corps d'ancrage allongé (1612) avec une partie d'extrémité d'ancrage distale (1614) et une partie d'extrémité d'ancrage proximale (1616) en regard de la partie d'extrémité d'ancrage distale ;
une partie articulation (1604) comprenant un corps d'articulation (1622), le corps d'articulation étant configuré pour permettre une flexion du corps d'articulation entre 180 degrés et 720 degrés de rotation du corps d'articulation ;
dans lequel la partie articulation présente une partie d'extrémité d'articulation distale (1624) fixée à demeure à la partie d'extrémité d'ancrage proximale et une partie d'extrémité d'articulation proximale (1626) en regard de la partie d'extrémité d'articulation distale ;
un élément d'hémostase (1606) présentant une partie d'extrémité d'hémostase distale (1634) reliée de manière amovible à la partie d'extrémité d'articulation proximale ;
dans lequel le dispositif est configuré pour recevoir un dispositif d'allongement (1611) à travers un passage interne (1610) dans le dispositif ;
dans lequel lorsque le dispositif d'allongement est reçu dans le passage interne à travers la partie d'ancrage, la partie d'ancrage est maintenue dans un état posé allongé ; et
dans lequel lorsque le dispositif d'allongement est retiré du passage interne de la partie d'ancrage, la partie d'ancrage forme un état déployé incurvé.

2. Dispositif selon la revendication 1, dans lequel la partie d'ancrage :
a) est annulaire à l'état déployé incurvé ; et/ou
b) comporte un alliage à mémoire de forme et la partie d'ancrage a une forme définie sur l'état déployé incurvé.

3. Dispositif selon la revendication 1 ou la revendication 2, dans lequel le dispositif d'allongement est configuré pour percer une paroi cardiaque pour former un passage dans celle-ci,
éventuellement dans lequel le dispositif d'allongement est un fil.

4. Dispositif selon une quelconque revendication précédente, dans lequel le corps d'ancrage allongé comporte une série de découpes transversales à un axe longitudinal le long du corps d'ancrage allongé.

5. Dispositif selon une quelconque revendication précédente, la partie d'articulation étant formée d'un seul tenant avec la partie d'ancrage.

6. Dispositif selon une quelconque revendication précédente, dans lequel la partie d'extrémité d'hémostase distale est reliée de manière détachable à la partie d'extrémité d'articulation proximale par une partie de fixation (1608) mobile entre une position d'accouplement et une position de libération.

7. Dispositif selon la revendication 6, dans lequel la partie de fixation comporte une pluralité de saillies et une pluralité d'évidements, dans lequel chaque évidement de la pluralité d'évidements est configuré pour recevoir une saillie de la pluralité de saillies.

8. Dispositif selon la revendication 7, dans lequel la partie de fixation comporte une paire de saillies s'étendant de manière proximale à partir d'une paroi latérale cylindrique de la partie d'articulation et une paire d'évidements s'étendant de manière proximale dans une paroi latérale cylindrique de l'élément d'hémostase.

9. Dispositif selon la revendication 7, dans lequel chaque saillie de la pluralité de saillies comporte un alliage à mémoire de forme et chaque saillie de la pluralité de saillies est mise en forme vers la position de libération.

10. Dispositif selon la revendication 9, dans lequel chaque saillie de la pluralité de saillies s'étend de manière proximale à partir d'une paroi latérale cylindrique de la partie d'articulation et dans lequel, dans la position de libération, chaque saillie de la pluralité de saillies fléchit radialement vers l'intérieur,
éventuellement dans lequel chaque saillie de la pluralité de saillies est maintenue dans la position d'accouplement par le dispositif d'allongement lorsque le dispositif d'allongement est positionné dans le passage interne et s'étend à travers la partie de fixation.

11. Procédé de remodelage d'une forme d'une paroi cardiaque qui définit au moins partiellement une chambre cardiaque, le procédé comprenant :
l'extension d'un élément d'allongement à travers la paroi cardiaque de la chambre cardiaque à une cavité péricardique ;
l'extension d'un dispositif à travers un passage dans la paroi cardiaque et sur l'élément d'allongement, dans lequel le dispositif comporte une partie d'ancrage ayant un corps d'ancrage allongé, et une partie d'articulation (1604) comprenant un corps d'articulation (1622), le corps d'articulation étant configuré pour permettre une flexion du corps d'articulation entre 180 degrés et 720 degrés de rotation du corps d'articulation, la partie d'articulation étant fixée à demeure à une extrémité proximale de la partie d'ancrage, et un élément d'hémostase étant relié de manière amovible à une extrémité proximale de la partie d'articulation ;
la mise en forme du corps d'ancrage allongé dans un état déployé annulaire au sein de la cavité péricardique ;
la flexion de la partie d'articulation pour repositionner le corps d'ancrage dans la cavité péricardique ; et
la désolidarisation de la partie d'articulation à partir de l'élément d'hémostase de telle sorte que la partie d'articulation et la partie d'ancrage soient dans la cavité péricardique et que l'élément d'hémostase soit positionné dans le passage ;
dans lequel le procédé est réalisé sur un cadavre, un cœur de cadavre ou un simulateur.

12. Procédé selon la revendication 11, dans lequel l'extension du dispositif sur l'élément d'allongement comprend l'élément d'allongement s'étendant à travers un passage interne du dispositif qui s'étend à travers le corps d'ancrage allongé, la partie d'articulation et l'élément d'hémostase.

13. Procédé selon la revendication 12, dans lequel :
a) la mise en forme du corps d'ancrage allongé dans l'état déployé annulaire consiste à retirer l'élément d'allongement du passage interne à travers le corps d'ancrage allongé ; et/ou
b) la flexion de la partie d'articulation pour repositionner le corps d'ancrage consiste à retirer l'élément d'allongement du passage interne à travers la partie d'articulation.

14. Procédé selon la revendication 12 ou 13, dans lequel la désolidarisation de la partie d'articulation à partir de l'élément d'hémostase comprend :
a) le retrait de l'élément d'allongement à partir du passage interne à travers une partie de fixation formée de manière coopérante par la partie d'articulation et l'élément d'hémostase ; ou
b) la flexion d'une ou plusieurs saillies radialement vers l'intérieur.

15. Procédé selon l'une quelconque des revendications 11 à 14, dans lequel la partie d'articulation est formée d'un seul tenant avec la partie d'ancrage.
